(19) Europäisches Patentamt
European Patent Office
Office européen des brevets

(11) Veröffentlichungsnummer: **0 299 379 B1**

(12) **EUROPÄISCHE PATENTSCHRIFT**

(45) Veröffentlichungstag der Patentschrift: **21.04.93**

(51) Int. Cl.$^5$: **C07D 213/40**, C07D 217/14, A61K 31/44, A61K 31/47

(21) Anmeldenummer: **88110934.2**

(22) Anmeldetag: **08.07.88**

Die Akte enthält technische Angaben, die nach dem Eingang der Anmeldung eingereicht wurden und die nicht in dieser Patentschrift enthalten sind.

# (54) Pentadienamide PAF-Antagonisten.

(30) Priorität: **10.07.87 US 72389**

(43) Veröffentlichungstag der Anmeldung: **18.01.89 Patentblatt 89/03**

(45) Bekanntmachung des Hinweises auf die Patenterteilung: **21.04.93 Patentblatt 93/16**

(84) Benannte Vertragsstaaten: **AT BE CH DE ES FR GB GR IT LI LU NL SE**

(56) Entgegenhaltungen:
**EP-A- 0 157 420**
**EP-A- 0 210 782**

**CHEMICAL ABSTRACTS, Band 105, Nr. 5, 4. August 1986, Seite 740, Zusammenfassung-Nr. 42810f, Columbus, Ohio, US**

**CHEMICAL ABSTRACTS, Band 104, Nr. 7, 17. Februar 1986, Seite 500, Zusammenfassung-Nr. 50683m, Columbus, Ohio, US**

**JOURNAL OF THE AMERICAN CHEMICAL SOCIETY, Band 103, 1981, Seiten 6261-6262; E. CIGANEK: "2,3,4,4a,5,6,7,7a-octahydro-1H-benzofuro(3,-2-e)isoquinoline: a new morphine fragment"**

(73) Patentinhaber: **F. HOFFMANN-LA ROCHE AG**
**Postfach 3255**
**CH-4002 Basel(CH)**

(72) Erfinder: **Guthrie, Robert William**
**102 Alberta Drive**
**Saddle Brook, N.J. 07665(US)**
Erfinder: **Kierstead, Richard Wightman**
**30 Willowbrook**
**North Caldwell, N.J. 07006(US)**
Erfinder: **Tilley, Jefferson Wright**
**19 Evergreen Drive**
**North Caldwell, N.J. 07007(US)**

(74) Vertreter: **Lederer, Franz, Dr. et al**
**Lederer, Keller & Riederer Patentanwälte**
**Lucile-Grahn-Strasse 22**
**W-8000 München 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die Erfindung betrifft Verbindungen der allgemeinen Formel

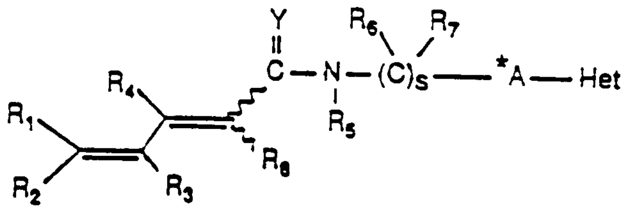

I

worin Y O oder S, *A Paraphenylen oder die Gruppe *-$(CH_2)_n$-$(X)_m$-$(CH_2)_r$-,X O, S oder -CH=CH-, n und r unabhängig voneinander eine ganze Zahl von 0 bis 3, s die Zahl 0 oder 1, m die Zahl 0 oder 1, wobei n+s mindestens 2 ist, wenn m die Zahl 1 ist, $R_1$ und $R_2$ unabhängig voneinander Wasserstoff, niederes Alkyl, $C_{3-6}$-Cycloalkyl, niederes Alkenyl, Het oder Aryl, $R_3$, $R_4$ und $R_8$ unabhängig voneinander Wasserstoff, niederes Alkyl oder Aryl, $R_5$ und $R_7$ unabhängig voneinander Wasserstoff oder niederes Alkyl, $R_6$ Wasserstoff, niederes Alkyl, $C_{3-6}$-Cycloalkyl, Het-niederes Alkyl oder Aryl, Het eine monocyclische, 6-gliedrige heteroaromatische oder eine bicyclische heteroaromatische Gruppe aus der Gruppe von Pyridinyl, Chinolinyl, Isochinolinyl, Indolyl, Benzimidazolyl und Pyrimidinyl und welche gegebenenfalls durch niederes Alkyl, Halogen oder Phenyl substituiert ist, bedeuten und das Sternchen die Verknüpfungsstelle bezeichnet und die mit nieder bezeichenten Reste bis zu 7 Kohlenstoffatome besitzen, und sofern $R_6$ und $R_7$ verschieden sind, Enantiomere und racemische Mischungen davon, sofern $R_1$ und $R_2$ verschieden sind, geometrische Isomeren davon und pharmazeutisch annehmbare Säureadditionssalze davon.

Im Vergleich zu den obigen Verbindungen, in denen Het eine monocyclische 6-gliedrige heteroaromatische Gruppe sein kann, sind die in C.A. 105 : 42810 f beschriebenen Verbindungen Imidazolderivate.

Die Verbindungen der Formel I sind wirksam als PAF (platelet activating factor)-Antagonisten und sind demnach nützlich bei Krankheiten, welche durch überschüssiges PAF charakterisiert sind, oder zur Verhütung oder Behandlung von Kreislauferkrankungen, Lungenkrankheiten, immunologischen Störungen, Entzündungen, dermatologischen Krankheiten, Schocks oder Transplantatabstossungen.

Gegenstand der vorliegenden Erfindung sind die Verbindungen der obigen Formel I als solche und als therapeutisch aktive Substanzen, ein Verfahren zu deren Herstellung, diese Verbindungen enthaltende Arzneimittel und ihre Herstellung, die Verwendung dieser Verbindungen bei der Verhütung oder Behandlung von Krankheiten und ihre Verwendung bei der Herstellung von Arzneimitteln.

Der Ausdruck "nieders Alkyl" bezeichnet eine geradkettige oder verzweigte gesättigte Kohlenwasserstoffgruppe mit 1 bis 7 Kohlenstoffatomen, z.B. Methyl, Ethyl, Propyl, Isopropyl, Butyl, t-Butyl, Neopentyl, Pentyl, Hexyl, Heptyl und dergleichen. Der Ausdruck "niederes Cycloalkyl" bezeichnet eine cyclische Alkylgruppe mit 3 - 6 Kohlenstoffatomen, z.B. Cyclopropyl, Cyclobutyl, Cyclopentyl, Cyclohexyl und dergleichen. Der Audruck "niederes Alkoxy" bezeichnet eine Alkylethergruppe, in welcher die Alkylgruppe wie oben beschrieben ist, z.B. Methoxy, Ethoxy, Propoxy, Pentoxy und dergleichen. Der Ausdruck "niederes Alkenyl" bezeichnet eine geradkettige oder verzweigte, ungesättigte Kohlen-wasserstoffgruppe mit 3 bis 7 Kohlenstoffatomen, z.B. Propenyl, Butenyl und dergleichen.

Der Ausdruck "Halogen" bezeichnet die Halogene Brom, Chlor, Fluor und Jod. Der Ausdruck "Aryl" bezeichnet vorzugsweise eine Naphtyl- oder Phenylgruppe, welche unsubstiuiert oder durch Halogen, Trifluormethyl, niederes Alkyl, Phenyl, niederes Alkoxy oder Nitro mono-, di- oder trisubstituiert ist.

Die Verbindungen der Formel I können in der (E)- bzw. trans- oder in der (Z)- bzw. cis-isomeren Form oder als Mischungen davon vorliegen. Insbesondere können die verschiedenen geometrischen Isomeren durch die nachfolgenden Formeln charakterisiert werden:

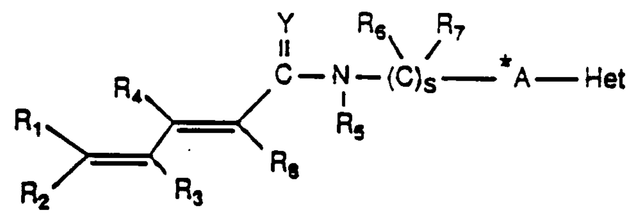

Ib

(E)- oder trans-Isomer und

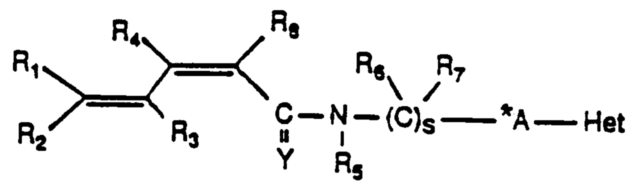

Ic

(Z)- oder cis-Isomer.

Darüberhinaus können die (E)- und (Z)-Isomeren, sofern $R_6$ und $R_7$ verschieden sind, auch als Enantiomere vorliegen, welche durch die folgenden Formeln charakterisiert werden können:

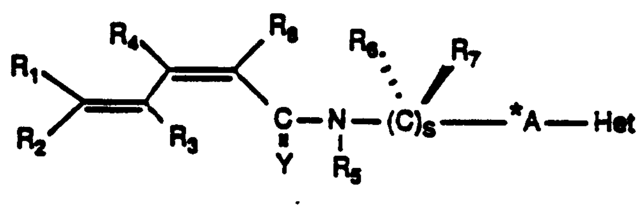

Id

[R-(Z)]-Isomer mit (R)-Konfiguration;

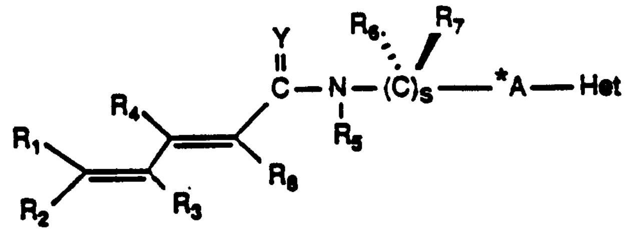

Ie

[R-(E)]-Isomer mit (R)-Konfiguration;

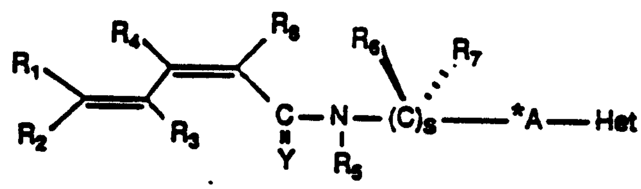

If

[S-(Z)]-Isomer mit (S)-Konfiguration und

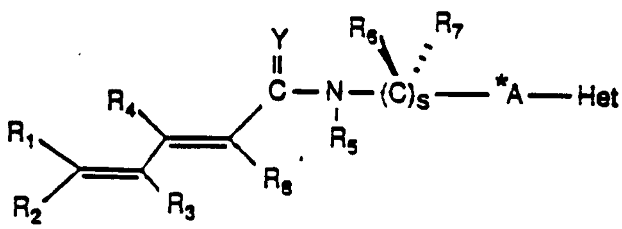

Ig

S-(E) -Isomer mit (S)-Konfiguration.

Die vorliegende Erfindung umfasst sämtliche möglichen Isomeren und Mischungen davon.

Eine bevorzugte Gruppe von Verbindungen der Formel I sind diejenigen, worin $R_1$ und $R_2$ unabhängig voneinander niederes Alkyl oder Aryl, $R_3$, $R_4$ und $R_8$ unabhängig voneinander Wasserstoff oder niederes Alkyl, $R_5$ und $R_7$ Wasserstoff, $R_6$ Wasserstoff, niederes Alkyl oder $C_{3-6}$ Cycloalkyl, *A *-$(CH_2)_n$-$(X)_m$-$(CH_2)_r$-, n+r eine ganze Zahl von 2 bis 6, die Zahl 0, Het eine monocyclische 6-gliedrige heteroaromatische Gruppe, welche ein oder zwei Heteroatome ausgewählt aus Stickstoff, Sauerstoff und Schwefel enthält, Y Sauerstoff oder Schwefel und s die Zahl 1 bedeuten.

Eine besonders bevorzugte Gruppe von Verbindungen der Formel I sind diejenigen, worin $R_1$ niederes Alkyl oder Aryl, $R_2$ Aryl, $R_3$, $R_4$ und $R_8$ unabhängig voneinander Wasserstoff oder niederes Alkyl, *A *-$(CH_2)_n$-$(X)_m$-$(CH_2)_r$-, n+r die Zahl 3, m die Zahl 0, Het gegebenenfalls durch niederes Alkyl substituiertes Pyridinyl oder Pyrimidinyl, $R_5$ und $R_7$ je Wasserstoff, $R_6$ Wasserstoff, niederes Alkyl oder Cyclopropyl, Y Sauerstoff und s die Zahl 1 bedeuten.

Eine ganz besonders bevorzugte Gruppe von Verbindungen der Formel I sind diejenigen, worin $R_1$ Butyl, Pentyl oder Hexyl oder Phenyl mit bis zu 3 Substituenten ausgewählt aus Halogen und niederem Alkoxy, $R_2$ Phenyl mit bis zu 3 Substituenten ausgewählt aus Halogen und niederem Alkoxy, $R_3$, $R_4$, $R_5$, $R_7$ und $R_8$ je Wasserstoff und Het 3-Pyridinyl oder 2-Methyl-3-pyridinyl bedeuten.

Bevorzugte erfindungsgemässe Verbindungen sind:

[R-(E,E)]-5-(4-Methoxyphenyl)-N-[1-methyl-4-(3-pyridinyl)butyl]-2,4-decadienamid;
[R,S-(E,E)]-5-(4-Methoxyphenyl)-N-[1-methyl-4-(2-methyl-3-pyridinyl)butyl]-2,4-decadienamid;
(E)-5,5-bis(3-Methoxyphenyl)-N-[4-(3-pyridinyl)butyl]-2,4-pentadienamid;
(E)-5,5-bis(3-Fluorophenyl)-N-[4-(3-pyridinyl)butyl]-2,4-pentadienamid;
[R-(E,E)]-5-(4-Methoxyphenyl)-N-[1-methyl-4-(3-pyridinyl)butyl]-2,4-nonadienamid; und
[R-(E)]-N-[1-Ethyl-4-(3-pyridinyl)butyl]-5,5-bis(4-methoxyphenyl)-2,4-pentadienamid.

Beispiele von Verbindungen der Formel I gemäss vorliegender Erfindung sind:

(E)-5,5-bis(Methoxyphenyl)-N-[4-(2,6-dimethyl-3-pyridinyl)butyl]-2,4-pentadienamid;
[R-(E,E)]-N-[4-(2,6-Dimethyl-3-pyridinyl)butyl]-5-(4-methoxyphenyl)-2,4-decadienamid;
[R-(E,E)]-5-(4-Methoxyphenyl)-N-[1-methyl-4-(4-methyl-3-pyridinyl)butyl]-2,4-decadienamid;
[R-(E)]-5,5-bis(4-Methoxyphenyl)-N-[1-methyl-4-(4-methyl-3-pyridinyl)butyl]-2,4-pentadienamid;
[R-(E,E)]-5-(4-Methoxyphenyl)-N-[1-methyl-4-(4-methyl-3-pyridinyl)butyl]-2,4-nonadienamid;
[R-(E,E)]-5-(4-Methoxyphenyl)-N-[1-methyl-4-[6-(1-methylethyl)-3-pyridinyl]butyl]-2,4-undecadienamid;
[R-(E,E)]-5-(4-Methoxyphenyl)-N-[1-methyl-4-[6-(1-methylethyl)-3-pyridinyl]butyl]-2,4-decadienamid;
[R-(E)]-5,5-bis(4-Methoxyphenyl)-N-[1-methyl-4-[6-(1-methylethyl)-3-pyridinyl]butyl]-2,4-pentadienamid;

[R-(E,E)]-5-(4-Methoxyphenyl)-N-[1-methyl-4-(5-methyl-3-pyridinyl)butyl]-5-(3-pyridinyl)-2,4-pentadienamid;
[R-(E,E)]-5-(4-Methoxyphenyl)-N-[1-methyl-4-(5-methyl-3-pyridinyl)butyl]-2,4-decadienamid;
[R-(E,E)]-5-(4-Methoxyphenyl)-N-[1-methyl-4-(5-methyl-3-pyridinyl)butyl]-2,4-nonadienamid;
(E,E)-5-(4-Methoxyphenyl)-N-[4-(3-pyridinyl)phenyl]-2,4-undecadienamid;
(E,E)-5-(4-Methoxyphenyl)-N-[4-(3-pyridinyl)phenyl]-2,4-nonadienamid;
(E,E)-5-(4-Methoxyphenyl)-N-[4-(3-pyridinyl)phenyl]-2,4-decadienamid;
(E)-5,5-bis(3,4-Dimethoxyphenyl)-2,4-dimethyl-N-[4-(3-pyridinyl)butyl]-2,4-pentadienamid;
[R-(E,E)]-5-(4-Methoxyphenyl)-3-methyl-N-[1-methyl-4-(3-pyridinyl)butyl]-2,4-decadienamid;
[R-(E,E)]-5-(4-Methoxyphenyl)-4-methyl-N-[1-methyl-4-(3-pyridinyl)butyl]-2,4-decadienamid;
(E)-5,5-bis(3,4-Dimethoxyphenyl)-3-methyl-N-[4-(3-pyridinyl)butyl]-2,4-pentadienamid;
[R-(E,E)]-5-(4-Methoxyphenyl)-2,4-dimethyl-N-[1-methyl-4-(3-pyridinyl)butyl]-2,4-undecadienamid;
[R-(E,E)]-5-(4-Methoxyphenyl)-4-methyl-N-[1-methyl-4-(3-pyridinyl)butyl]-2,4-nonadienamid;
(E)-5,5-bis(3,4-Dimethoxyphenyl)-4-methyl-N-[4-(3-pyridinyl)butyl]-2,4-pentadienamid;
[R-(E,E)]-5-(4-Methoxyphenyl)-2,4-dimethyl-N-[1-methyl-4-(3-pyridinyl)butyl]-2,4-decadienamid;
[R-(E,E)]-5-(3-Methoxyphenyl)-4-methyl-N-[1-methyl-4-(3-pyridinyl)butyl]-2,4-undecadienamid;
[R-(E,E)]-5-(4-Methoxyphenyl)-4-phenyl-N-[1-methyl-4-(3-pyridinyl)butyl]-2,4-decadienamid;
[R-(E,E)]-N-[1-Methyl-4-(3-pyridinyl)butyl]-5,5-bis(2,3,4-trimethoxyphenyl)-2,4-pentadienamid;
[R-(E,E)]-N-[1-Methyl-4-(3-pyridinyl)butyl]-5-(3-thienyl)-5-(2,3,4-trimethoxyphenyl)-2,4-pentadienamid;
[R-(E,E)]-N-[1-Methyl-4-(3-pyridinyl)butyl]-5-(2,3,4-trimethoxyphenyl)-2,4-nonadienamid;
[R-(E,E)]-N-[1-Methyl-4-(3-pyridinyl)butyl]-5-(2,3,4-trimethoxyphenyl)-2,4-decadienamid;
[R-(E)]-N-[1-Methyl-4-(3-pyridinyl)butyl]-5,5-bis(3,4,5-trimethoxyphenyl)-2,4-pentadienamid;
[R-(E,E)]-N-[1-Methyl-4-(3-pyridinyl)butyl]-5-(3-pyridinyl)-5-(3,4,5-trimethoxyphenyl)-2,4-pentadienamid;
[R-(E,E)]-N-[1-Methyl-4-(3-pyridinyl)butyl]-5-(3,4,5-trimethoxyphenyl)-2,4-decadienamid;
[R-(E)]-5,5-bis(3,5-Dimethoxyphenyl)-N-[1-methyl-4-(3-pyridinyl)butyl]-2,4-pentadienamid;
R-(E,E)]-5-(3,5-Dimethoxyphenyl)-N-[1-methyl-4-(3-pyridinyl)butyl]-2,4-nonadienamid;
[R-(E,E)]-5-(3,5-Dimethoxyphenyl)-N-[1-methyl-4-(3-pyridinyl)butyl]-2,4-decadienamid;
[R-(E)]-5,5-bis(2,3-Dimethoxyphenyl)-N-[1-methyl-4-(3-pyridinyl)butyl]-2,4-pentadienamid;
[R-(E,E)]-5-(2,3-Dimethoxyphenyl)-N-[1-methyl-4-(3-pyridinyl)butyl]-2,4-undecadienamid;
[R-(E)]-5,5-bis(2,6-Dimethoxyphenyl)-N-[1-methyl-4-(3-pyridinyl)butyl]-2,4-pentadienamid;
[R-(E,E)]-5-(2,6-Dimethoxyphenyl)-N-[1-methyl-4-(3-pyridinyl)butyl]-2,4-nonadienamid;
[R-(E,E)]-5-(2,6-Dimethoxyphenyl)-N-[1-methyl-4-(3-pyridinyl)butyl]-2,4-decadienamid;
(R-(E,E)]-5-(2,6-Dimethoxyphenyl)-N-[1-methyl-4-(3-pyridinyl)butyl]-2,4-undecadienamid;
[R-(E)]-5,5-bis(2,4-Dimethoxyphenyl)-N-[1-methyl-4-(3-pyridinyl)butyl]-2,4-pentadienamid;
[R-(E,E)]-5-(2,4-Dimethoxyphenyl)-N-[1-methyl-4-(3-pyridinyl)butyl]-2,4-nonadienamid;
[R-(E,E)]-5-(2,4-Dimethoxyphenyl)-N-[1-methyl-4-(3-pyridinyl)butyl]-2,4-decadienamid;
[R-(E,E)]-5-(4-Methoxynaphthalen-2-yl)-N-[1-methyl-4-(3-pyridinyl)butyl]-2,4-nonadienamid;
[R-(E,E)]-5-(4-Methoxynaphthalen-2-yl)-5-(4-methoxyphenyl)-N-[1-methyl-4-(3-pyridinyl)butyl]-2,4-pentadienamid;
[R-(E,E)]-5-(6-Methoxynaphthalen-2-yl)-N-[1-methyl-4-(3-pyridinyl)butyl]-5-(3-pyridinyl)-2,4-pentadienamid;
[R-(E,E)]-5-(6-Methoxynaphthalen-2-yl)-N-[1-methyl-4-(3-pyridinyl)butyl]-2,4-nonadienamid;
[R-(E,E)]-5-(6-Methoxynaphthalen-2-yl)-N-[1-methyl-4-(3-pyridinyl)butyl]-2,4-decadienamid;
[R-(E,E)]-5-(7-Methoxynaphthalen-2-yl)-N-[1-methyl-4-(3-pyridinyl)butyl]-5-phenyl-2,4-pentadienamid;
[R-(E,E)]-5-(7-Methoxynaphthalen-1-yl)-N-[1-methyl-4-(3-pyridinyl)butyl]-2,4-nonadienamid;
[R-(E,E)]-5-(7-Methoxynaphthalen-2-yl)-N-[1-methyl-4-(3-pyridinyl)butyl]-2,4-decadienamid;
[R-(E,E)]-5-(7-Methoxynaphthalen-1-yl)-N-[1-methyl-4-(3-pyridinyl)butyl]-2,4-undecadienamid;
[R-(E,E)]-5-(4,7-Dimethoxynaphthalen-2-yl)-N-[1-methyl-4-(3-pyridinyl)butyl]-2,4-decadienamid;
[R-(E,E)]-5-(7-Methoxynaphthalen-2-yl)-N-[1-methyl-4-(3-pyridinyl)butyl]-2,4-undecadienamid;
[R-(E,E)]-5-(7-Methoxynaphthalen-1-yl)-N-[1-methyl-4-(3-pyridinyl)butyl]-2,4-decadienamid;
[R-(E,E)]-5-(4,7-Dimethoxynaphthalen-2-yl)-N-[1-methyl-4-(3-pyridinyl)butyl]-2,4-nonadienamid;
[R-(E,E)]-5-(4-Methoxyphenyl)-N-[1-methyl-4-(5-pyrimidinyl)butyl]-5-phenyl-2,4-pentadienamid;
[R-(E)]-5,5-bis(3-Methoxyphenyl)-N-[1-methyl-4-(5-pyrimidinyl)butyl]-2,4-pentadienamid;
[R-(E,E)]-5-(4-Methoxyphenyl)-N-[1-methyl-4-(5-pyrimidinyl)butyl]-2,4-decadienamid;
[R-(E,E)]-5-(4-Methoxyphenyl)-N-[1-methyl-4-(3-pyridazinyl)butyl]-2,4-nonadienamid;
[R-(E,E)]-5-(4-Methoxyphenyl)-N-[1-methyl-4-(3-pyridazinyl)butyl]-2,4-decadienamid;
[R-(E,E)]-5-(4-Methoxyphenyl)-N-[1-methyl-4-(3-pyridazinyl)butyl]-2-(thienyl)-2,4-pentadienamid;
[R-(E,E)]-5-(4-Methoxyphenyl)-N-[1-methyl-4-(2H-chromen-3-yl)butyl]-2,4-undecadienamid;
[R-(E,E)]-5-(3-Chlorophenyl)-N-[1-methyl-4-(3-pyridinyl)butyl]-5-phenyl-2,4-pentadienamid;
[R-(E,E)]-5-(3-Chlorophenyl)-N-[1-methyl-4-(3-pyridinyl)butyl]-2,4-decadienamid;

[R-(E,E)]-5-(3-Chlorophenyl)-N-[1-methyl-4-(3-pyridinyl)butyl]-5-phenyl-2,4-pentadienamid;
[R-(E,E)]-5-(4-Chlorophenyl)-N-[1-methyl-4-(3-pyridinyl)butyl]-2,4-nonadienamid;
[R-(E)]-5,5-bis[4-(Trifluoromethyl)phenyl]-N-[1-methyl-4-(3-pyridinyl)butyl]-2,4-pentadienamid;
[R-(E,E)]-5-[4-(Trifluoromethyl)phenyl]-N-[1-methyl-4-(3-pyridinyl)butyl]-2,4-decadienamid;
[R-(E,E)]-5-(4-Nitrophenyl)-N-[1-methyl-4-(3-pyridinyl)butyl]-2,4-undecadienamid;
[R-(E,E)]-5-(4-Nitrophenyl)-N-[1-methyl-4-(3-pyridinyl)butyl]-2,4-nonadienamid;
[R-(E,E)]-5-[4-(1-Methylethyl)phenyl]-N-[1-methyl-4-(3-pyridinyl)butyl]-2,4-decadienamid;
[R-(E,E)]-5-[4-(1-Methylethyl)phenyl]-N-[1-methyl-4-(3-pyridinyl)butyl]-2,4-nonadienamid;
[R-(E)]-5,5-bis[4-(Methylthio)phenyl]-N-[1-methyl-4-(3-pyridinyl)butyl]-2,4-pentadienamid;
[R-(E,E)]-5-[4-(Methylthio)phenyl]-N-[1-methyl-4-(3-pyridinyl)butyl]-2,4-nonadienamid;
[R-(E,E)]-5-[4-(Methylsulfinyl)phenyl]-N-[1-methyl-4-(3-pyridinyl)butyl]-5-phenyl-2,4-pentadienamid;
[R-(E,E)]-5-[4-(Methylsulfinyl)phenyl]-N-[1-methyl-4-(3-pyridinyl)butyl]-2,4-undecadienamid;
[R-(E)]-5,5-bis[4-(Dimethylamino)phenyl]-N-[1-methyl-4-(3-pyridinyl)butyl]-2,4-pentadienamid;
[R-(E,E)]-5-[4-(Dimethylamino)phenyl]-N-[1-methyl-4-(3-pyridinyl)butyl]-2,4-decadienamid;
[R-(E)-5,5-bis(3,4-Methylenedioxyphenyl)-N-[1-methyl-4-(3-pyridinyl)butyl]-2,4-pentadienamid;
[R-(E,E)-5-(3,4-Methylenedioxyphenyl)-N-[1-methyl-4-(3-pyridinyl)butyl]-2,4-nonadienamid;
[R-(E,E)]-N-[1-Ethyl-4-(3-pyridinyl)butyl]-5-(4-methoxyphenyl)-2,4-nonadienamid;
[R-(E,E)]-N-[1-Ethyl-4-(3-pyridinyl)butyl]-5-(4-methoxyphenyl)-2,4-decadienamid;
[R-(E,E)]-N-[1-Ethyl-4-(3-pyridinyl)butyl]-5-(4-methoxyphenyl)-2,4-undecadienamid;
[S-(E,E)]-N-[1-Ethyl-4-(3-pyridinyl)butyl]-5-(4-methoxyphenyl)-2,4-decadienamid;
[R-(E,E)]-5-(4-Methoxyphenyl)-N-[1-propyl-4-(3-pyridinyl)butyl]-2,4-nonadienamid;
[R-(E,E)]-5-(4-Methoxyphenyl)-N-[1-propyl-4-(3-pyridinyl)butyl]-2,4-decadienamid;
[R-(E,E)]-5-(4-Methoxyphenyl)-N-[1-propyl-4-(3-pyridinyl)butyl]-5-phenyl-2,4-pentadienamid;
[R-(E,E)]-N-[1-Cyclopropyl-4-(3-pyridinyl)butyl]-5-(4-methoxyphenyl)-2,4-decadienamid;
[R-(E,E)]-N-[1-Cyclopropyl-4-(3-pyridinyl)butyl]-5-(4-methoxyphenyl)-2,4- nonadienamid;
[S-(E,E)]-N-[1-Cyclopropyl-4-(3-pyridinyl)butyl]-5-(4-methoxyphenyl)-2,4-decadienamid;
[R,S-(E,E)]-5-(4-Methoxyphenyl)-N-[1-(1-methylethyl)-4-(3-pyridinyl)butyl]-2,4-undecadienamid;
[R,S-(E,E)]-5-(4-Methoxyphenyl)-N-[1-(1-methylethyl)-4-(3-pyridinyl)butyl]-2,4-nonadienamid;
[R-(E,E)]-5-(4-Methoxyphenyl)-N-[1-methyl-2-(3-pyridinyl)ethyl]-2,4-nonadienamid;
[R-(E,E)]-5-(4-Methoxyphenyl)-N-[1-methyl-2-(3-pyridinyl)ethyl]-2,4-decadienamid;
R-(E,E)]-5-(4-Methoxyphenyl)-N-[1-methyl-3-(3-pyridinyl)propyl]-2,4-undecadienamid;
[R-(E)]-5,5-bis(4-Methoxyphenyl)-N-[1-methyl-3-(3-pyridinyl)propyl]-2,4-pentadienamid;
[R-(E,E)]-5-(4-Methoxyphenyl)-N-[1-methyl-5-(3-pyridinyl)pentyl]-2,4-decadienamid;
[R-(E,E)]-5-(4-Methoxyphenyl)-N-[1-methyl-5-(3-pyridinyl)pentyl]-2,4-undecadienamid;
[R-(E,E)]-5-(4-Methoxyphenyl)-N-[1-methyl-6-(3-pyridinyl)hexyl]-2,4-undecadienamid;
[R-(E,E)]-5-(4-Methoxyphenyl)-N-[1-methyl-7-(3-pyridinyl)heptyl]-2,4-decadienamid;
[R-(E,E)]-5-(4-Methoxyphenyl)-N-[1-methyl-6-(3-pyridinyl)hexyl]-2,4-nonadienamid;
[R-(E,E)]-5-(4-Methoxyphenyl)-N-[1-methyl-7-(3-pyridinyl)heptyl]-2,4-undecadienamid;
(E,E)-5-(4-Methoxyphenyl)-N-[3-(3-pyridinyloxy)propyl]-2,4-decadienamid;
[R-(E,E)]-5-(4-Methoxyphenyl)-N-[1-methyl-8-(3-pyridinyl)octyl]-2,4-decadienamid;
[R-(E,E)]-5-(4-Methoxyphenyl)-N-[1-methyl-8-(3-pyridinyl)octyl]-2,4-nonadienamid;
[R-(E,E)]-N-[1-Methyl-4-(3-pyridinyl)butyl]-5-(3-pyridinyl)-2,4-undecadienamid;
[R-(2E,4Z)]-N-[1-Methyl-4-(3-pyridinyl)butyl]-5-(3-pyridinyl)-2,4-decadienamid;
[R-(E,E)]-N-[1-Methyl-4-(3-pyridinyl)butyl]-5-(3-pyridinyl)-2,4-undecadienamid;
[R-(2E,4Z)]-N-[1-Methyl-4-(3-pyridinyl)butyl]-5-(3-pyridinyl)-2,4-decadienamid;
[R-(E,E)]-N-[1-Methyl-4-(3-pyridinyl)butyl]-5-(4-pyridinyl)-2,4-nonadienamid;
[R-(2E,4Z)]-N-[1-Methyl-4-(3-pyridinyl)butyl]-5-(4-pyridinyl)-2,4-nonadienamid;
(E,E)-5-(4-Methoxyphenyl)-N-[3-(3-pyridinyloxy)propyl]-2,4-decadienamid;
(E,E)-5-(4-Methoxyphenyl)-N-[3-(3-pyridinyloxy)propyl]-2,4-nonadienamid;
(E,E)-5-(4-Methoxyphenyl)-N-[3-(3-pyridinyloxy)propyl]-2,4-undecadienamid;
(E,E)-5-(4-Methoxyphenyl)-N-[3-(3-pyridinylamino)propyl]-2,4-undecadienamid;
(E,E)-5-(4-Methoxyphenyl)-N-[3-(3-pyridinylamino)propyl]-2,4-nonadienamid;
(E,E)-5-(4-Methoxyphenyl)-N-[3-(3-pyridinylamino)propyl]-2,4-decadienamid;
(E,E)-5-(4-Methoxyphenyl)-N-[3-[(3-pyridinyl)thio]propyl]-2,4-nonadienamid;
(E,E)-5-(4-Methoxyphenyl)-N-[3-[(3-pyridinyl)thio]propyl]-2,4-decadienamid;
(E,E)-5-(4-Methoxyphenyl)-N-[3-[(3-pyridinyl)thio]propyl]-2,4-undecadienamid;
(E,E)-5-(4-Methoxyphenyl)-N-[2-[3-pyridinylmethoxy]ethyl]-2,4-undecadienamid;
(E,E)-5-(4-Methoxyphenyl)-N-[2-[3-pyridinylmethoxy]ethyl]-2,4-nonaadienamid;

(E,E)-5-(4-Methoxyphenyl)-N-[2-[3-pyridinylmethoxy]ethyl]-2,4-decadienamid;
(E,E)-5-(4-Methoxyphenyl)-N-[2-[(3-pyridinylmethyl)thio]-ethyl]-2,4-undecadienamid;
(E,E)-5-(4-Methoxyphenyl)-N-[2-[(3-pyridinylmethyl)thio]ethyl]-2,4-nonadienamid;
(E,E)-5-(4-Methoxyphenyl)-N-[2-[(3-pyridinylmethyl)thio]ethyl]-2,4-decadienamid;
(E,E)-5-(4-Methoxyphenyl)-N-[2-[(3-pyridinylmethyl)amino]ethyl]-2,4-decadienamid;
(E,E)-5-(4-Methoxyphenyl)-5-(3-pyridinyl)-N-[2-[(3-pyridinylmethyl)amino]ethyl]-2,4-pentadienamid;
(E,E)-5-(4-Methoxyphenyl)-N-[2-[(3-pyridinylmethyl)amino]ethyl]-2,4-undecadienamid;
(E,E)-5-(4-Methoxyphenyl)-N-[2-[(3-pyridinylmethyl)amino]ethyl]-2,4-nonadienamid;
(E,E)-5-(4-Methoxyphenyl)-N-[2-[methyl-(3-pyridinylmethyl)amino]ethyl]-2,4-undecadienamid;
(E,E)-5-(4-Methoxyphenyl)-N-[2-[methyl-(3-pyridinylmethyl)amino]ethyl]-2,4-decadienamid;
(E,E)-5-(4-Methoxyphenyl)-N-[2-[methyl-(3-pyridinylmethyl)amino]ethyl]-2,4-nonadienamid;
[R-(E,E)]-5-(4-Methoxyphenyl)-9-methyl-N-[1-methyl-4-(3-pyridinyl)butyl]-2,4,8-decatrienamid;
[R-(E,E)]-8-Methoxy-5-(4-methoxyphenyl)-N-[1-methyl-4-[(3-pyridinyl)butyl]-2,4-octadienamid;
[R-(E,E)]-7-Ethoxy-5-(4-methoxyphenyl)-N-[1-methyl-4-[(3-pyridinyl)butyl]-2,4-heptadienamid; und ähnliche.

Die Verbindungen der Formel I und ihre pharmazeutisch annehmbaren Säureadditionssalze können erfindungsgemäss dadurch hergestellt werden, dass man

a) ein reaktives Derivat einer Carbonsäure der allgemeinen Formel

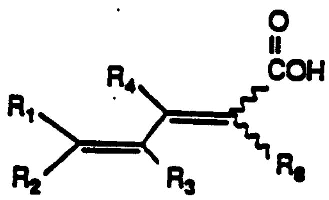

II

worin $R_1$, $R_2$, $R_3$, $R_4$ und $R_8$ obige Bedeutung besitzen,
mit einer Verbindung der allgemeinen Formel

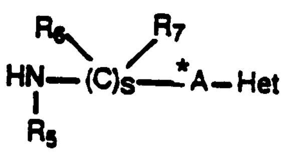

V

worin $R_5$, $R_6$, $R_7$, *A, Het und s obige Bedeutung besitzen, umsetzt, oder

b) eine Carbonsäure der obigen allgemeinen Formel II in Gegenwart eines Tri(niederen Alkyl)- oder Triarylphosphins mit einem Azid der allgemeinen Formel

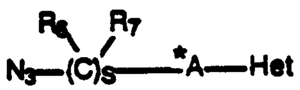

VIII

worin $R_6$, $R_7$, *A, Het und s obige Bedeutung besitzen, umsetzt, oder

c) eine Verbindung der allgemeinen Formel I, worin Y O bedeutet, mit Phosphorpentasulfid zu einer entsprechenden Verbindung der allgemeinen Formel I umsetzt, worin Y S bedeutet, und

d) erwünschtenfalls eine erhaltene Verbindung der allgemeinen Formel I in ein pharmazeutisch annehmbares Säureadditionssalz überführt.

Das erfindungsgemässe Verfahren wird im Zusammenhang mit bevorzugten Ausführungsformen nachfolgend noch etwas näher erläutert.

## Reaktionsschema I

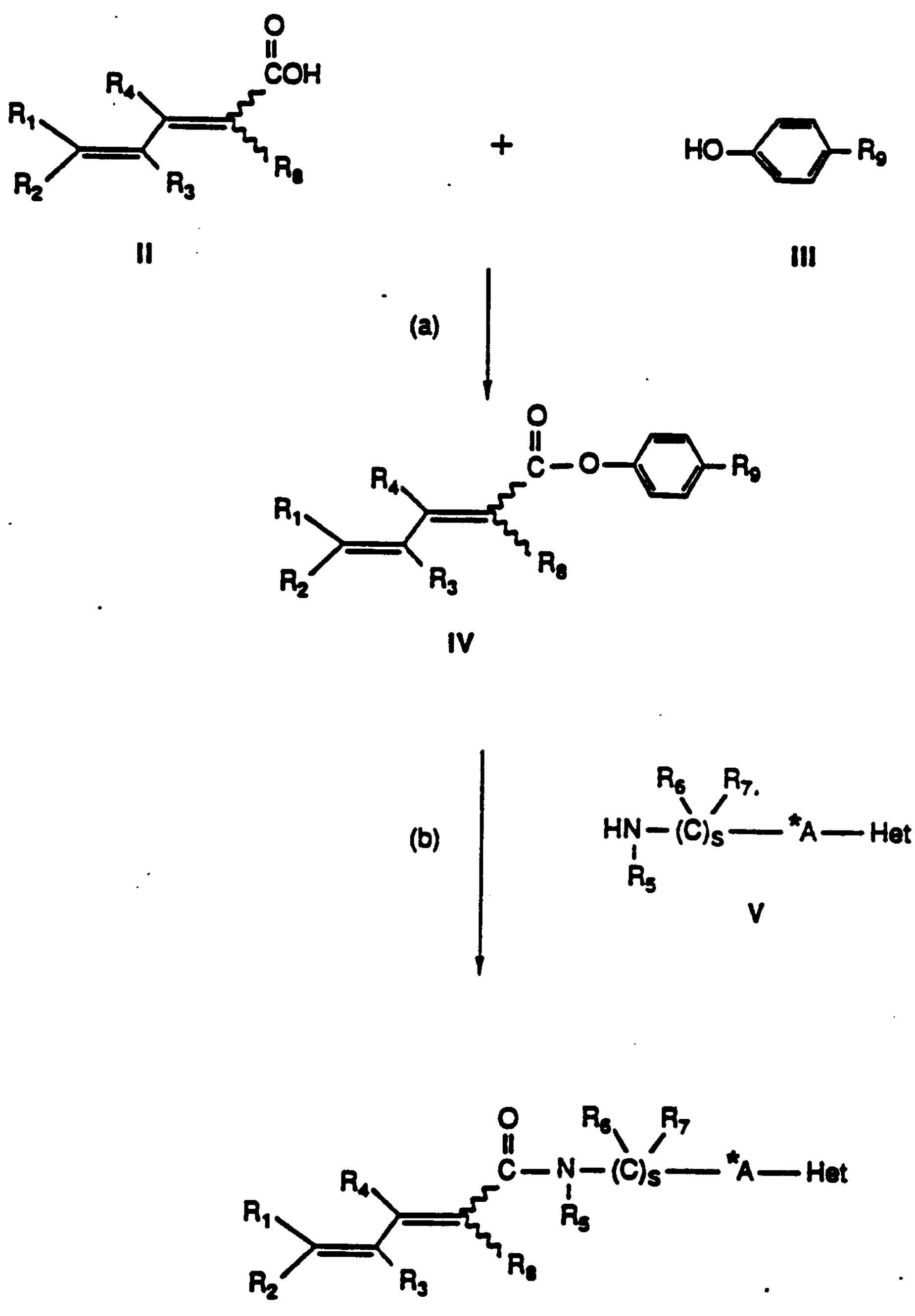


II

III

IV

V

Ih

worin $R_1$, $R_2$, $R_3$, $R_4$, $R_5$, $R_6$, $R_7$, $R_8$, *A, Het und s obige Bedeutung besitzen, und $R_9$ Wasserstoff, Halogen, niederes Alkoxy, niederes Alkyl, niederes Trihaloalkyl oder Nitro bedeutet.

Im Reaktionsschema I, Stufe (a), wird eine Pentadiencarbonsäure der Formel II, welche die (E)- und (Z)-Isomeren und Mischungen davon einschliesst, mit einem Phenol der Formel III in Gegenwart eines Kondensationsmittels, vorzugsweise Dicyclohexylcarbodiimid, in einem inerten Lösungsmittel, wie Methylenchlorid, Diethylether oder Dimethylformamid, bei einer Temperatur von -80°C bis Raumtemperatur umgesetzt. Die erhaltene Verbindung der Formel IV kann unter Verwendung von herkömmlichen Methoden, wie Kristallisation, Chromatographie oder dergleichen, isoliert werden.

In Stufe (b), wird ein aktivierter Ester der Formel IV mit einem Amin der Formel V, welche die (R)- und (S)-Enantiomeren und racemische Mischungen davon einschliesst, in einem inerten Lösungsmittel, vorzugsweise Tetrahydrofuran oder Diethylether, bei einer Temperatur von -80°C bis 100°C umgesetzt. Die Reaktion wird vorzugsweise mit der Bedeutung Nitro für $R_9$ durchgeführt. Die erhaltene Verbindung der Formel Ih kann unter Verwendung von herkömmlichen Methoden, z.B Kristallisation, Chromatographie oder dergleichen, isoliert werden.

## Reaktionsschema II

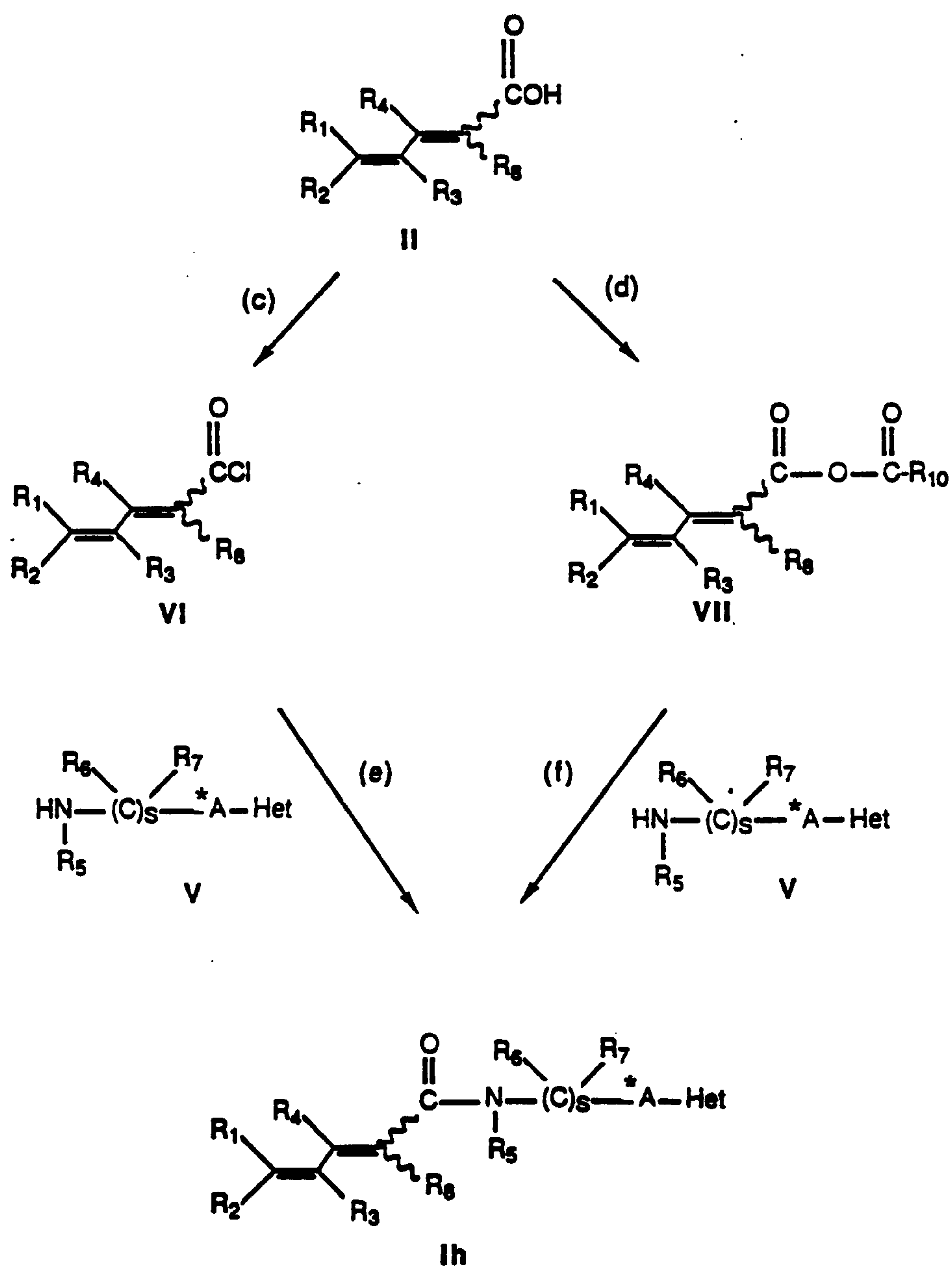

II, VI, VII, V, V, Ih

worin $R_1$, $R_2$, $R_3$, $R_4$, $R_5$, $R_6$, $R_7$, $R_8$, *A, Het und s obige Bedeutung besitzen, und $R_{10}$ niederes Alkyl bedeutet.

Im Reaktionsschema II, Stufe (C), wird eine Pentadiencarbonsäure der Formel II in Form des (E)- oder (Z)-Isomeren oder einer Mischung davon mit einem ein Acylhalogenid bildendes Mittel, vorzugsweise Oxalylchlordi oder Thionylchlorid, in einem inerten Lösungsmittel, vorzugsweise Methylenchlorid oder Toluol, bei einer Temperatur von -80 °C bis Raumtemperatur umgesetzt. Die erhaltene Verbindung der Formel VI kann durch Eindampfen der Reaktionsmischung isoliert und wie in Stufe (e) mit einem Amin der Formel V, in Form des (R)- oder (S)-Enantiomeren oder in Form einer racemischen Mischung davon in Gegenwart eines tertiären Amins, vorzugsweise Triethylamin, in einem inerten Lösungsmittel, wie Methylenchlorid oder Toluol, bei einer Temperatur von -80 °C bis Raumtemperatur umgesetzt werden. Die erhaltene

Verbindung der Formel Ih kann mittels herkömmlicher Methoden, wie Kristallisation oder Chromatographie oder dergleichen, isoliert werden.

In Stufe (d) wird eine Pentadiencarbonsäure der Formel II, in Form des (E)- oder (Z)-Isomeren oder in Form einer Mischung davon, mit einem niederen Alkylchloroformat in Gegenwart eines tertiären Amins, vorzugsweise Triethylamin, in einem inerten Lösungsmittel, vorzugsweise Diethylether oder Tetrahydrofuran, bei einer Temperatur von -20°C bis 10°C umgesetzt. Das erhaltene gemischte Anhydrid der Formel VII wird dann in situ wie in Stufe (f) mit einem Amin der Formel V, in Form des (R)-oder (S)-Enantiomeren oder in Form einer racemischen Mischung davon, bei einer Temperatur von -20°C bis Raumtemperatur umgesetzt.

## Reaktionsschema III

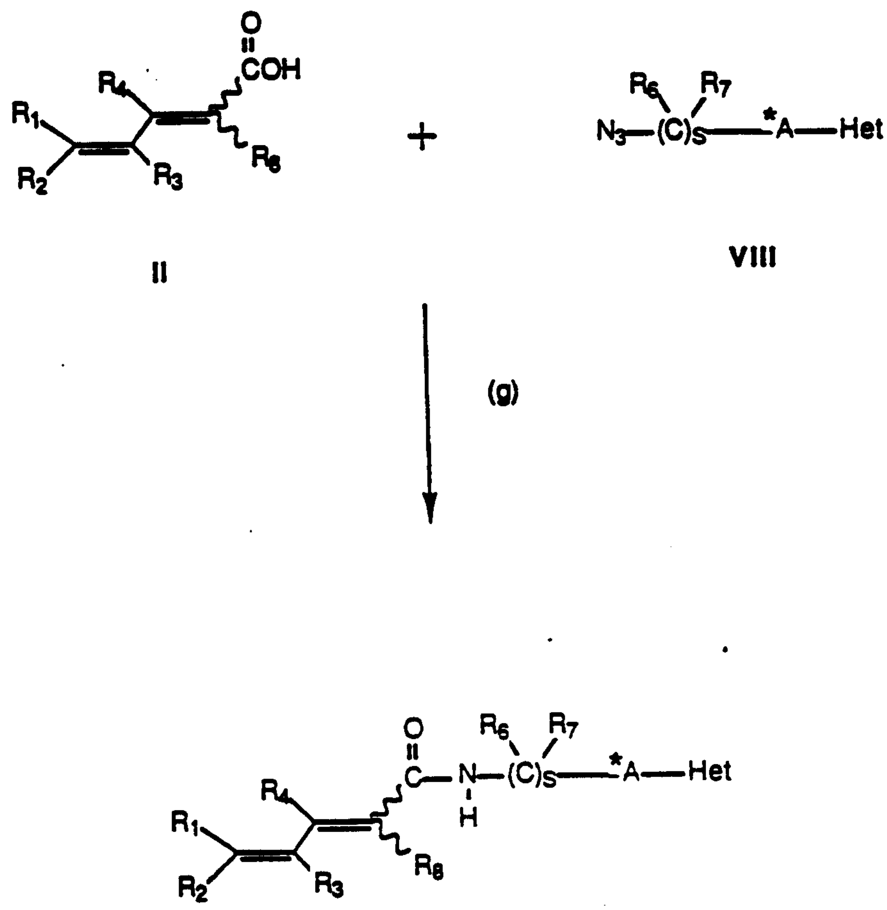


II

VIII

II

worin $R_1$, $R_2$, $R_3$, $R_4$, $R_6$, $R_7$, $R_8$, *A, Het und s obige Bedeutung besitzen.

Im Reaktionsschema III, Stufe (g), wird eine Pentadiencarbonsäure der Formel II mit einem Azid der Formel VIII in Gegenwart eines Tri(niederen Alkylen)- oder Triarylphosphins in einem inerten Lösungsmittel, vorzugsweise Toluol, bei einer Temperatur von Raumtemperatur bis 100°C umgesetzt.

## Reaktionsschema IV

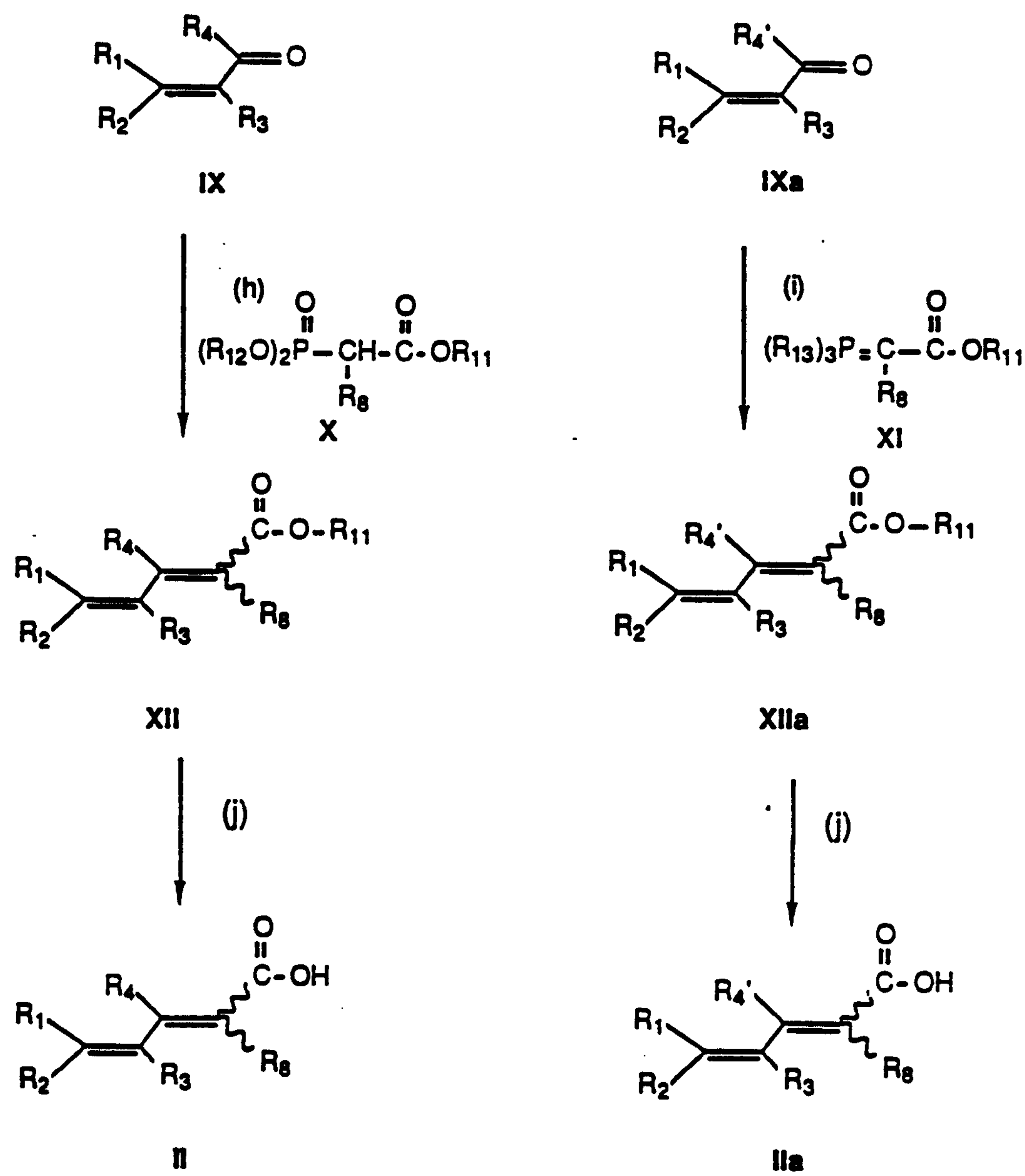


worin $R_1$, $R_2$, $R_3$, $R_4$ und $R_8$ obige Bedeutung besitzen, und $R_4'$ Wasserstoff, $R_{11}$, $R_{12}$ und $R_{13}$ unabhängig voneinander niederes Alkyl oder Aryl bedeuten.

Im Reaktionsschema IV, Stufe (h), wird ein Phosphonoacetattriester der Formel X mit einer starken Base, vorzugsweise Natrimhydrid oder Natriumamid, in einem inerten Lösungsmittel, vorzugsweise Dimethylsulfoxid, Dimethylformamid oder Tetrahydrofuran, bei einer Temperatur von 0 °C bis 80 °C in das entsprechende Carbanion übergeführt, welches dann in situ mit einer Carbonylverbindung der Formel IX umgesetzt wird. Die erhaltene Verbindung der Formel XII kann eine Mischung der Isomeren sein, welche gegebenenfalls unter Verwendung von herkömmlichen Methoden, wie Kristallisation, Chromatographie oder dergleichen, aufgetrennt werden kann.

In Stufe (i), wird ein Carboxaldehyd der Formel IXa mit einem (Carbalkoxyalkyliden)triarylphosphoran der Formel XI in einem geeigneten Lösungsmittel bei einer Temperatur von -20 °C bis -50 °C umgesetzt. Bei Verwendung eines aprotischen Lösungsmittels, vorzugsweise Methylenchlorid, Tetrachlorkohlenstoff oder Dimethylformamid, erhält man ein Isomerengemisch im Verhältnis von >10:1 zu Gunsten des (E)-Isomeren bezüglich der neu gebildeten Doppelbindung. Falls man ein protisches Lösungsmittel verwendet,

vorzugsweise Methanol oder Ethanol, erhält man einen höheren Anteil an (Z)-Isomer (E:Z ist ungefähr 3:2). Die erhaltene Verbindung der Formel XIIa kann gegebenenfalls unter Verwendung von herkömmlichen Methoden, wie Kristallisation, Chromatographie oder dergleichen, in die (E)- und (Z)-Isomeren aufgetrennt werden.

In Stufe (j) wird ein Pentadiencarbonsäureester der Formel XII oder XIIa mit überschüssigem Alkalimetalhydroxid in einem Lösungsmittelgemisch (vorzugsweise Methanol/wasser oder Ethanol/wasser), bei einer Temperatur von Raumtemperatur bis 85 ° C umgesetzt.

**Reaktionsschema V**

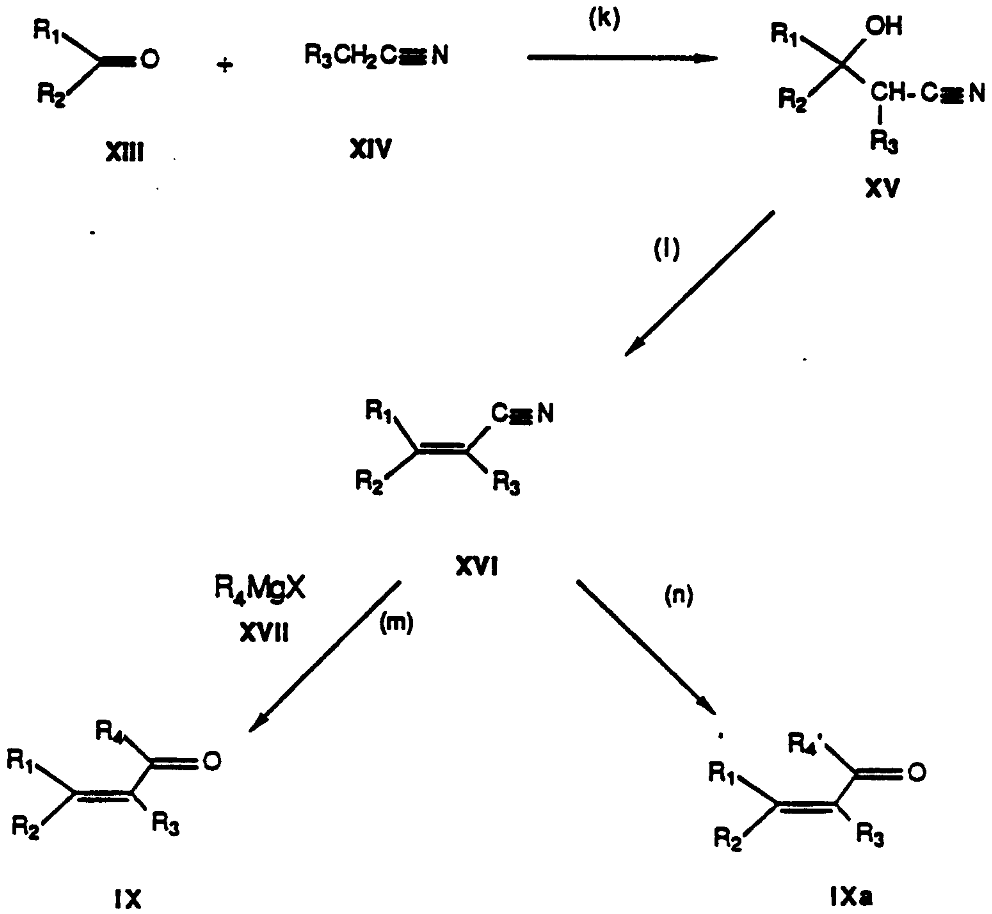


worin $R_1$, $R_2$, $R_3$, $R_4$ und $R'_4$ obige Bedeutung besitzen.

In Reaktionsschema V, Stufe (k), wird ein Acetonitril der Formel XIV durch Behandeln mit einer starken Base, vorzugsweise Natriumhydrid, Natriumamid oder Lithiumdiisopropylamid (LDA), in einem inerten Lösungsmittel, vorzugsweise Ether oder Tetrahydrofuran, bei einer Temperatur von -80 ° C bis Raumtemperatur in das entsprechende Carbanion übergeführt, welches dann in situ mit einer Carbonylverbindung der Formel XIII umgesetzt wird.

In Stufe (1) wird ein Carbinol der Formel XV mit einer Säure, vorzugsweise Trifluoressigsäure, Methansulfonsäure oder Schwefelsäure, gegebenenfalls in Gegenwart eines zusätzlichen Lösungsmittels, vorzugsweise Methylenchlorid oder Chloroform, bei einer Temperatur von -10 ° C bis 45 ° C umgesetzt. Man kam jedoch auch das Carbinol der Formel XV in Gegenwart eines tertiären Amins, vorzugsweise Triethylamin, in einem inerten Lösungsmittel, vorzugsweise Methylenchlorid, bei einer Temperatur von -80 ° C bis Raumtemperatur mit Methansulfonylchlorid behandeln. Sofern $R_1$ und $R_2$ verschieden sind, kann es sich bei der erhaltenen Verbindung der Formel XVI um ein Gemisch der (E)- und (Z)-Isomeren handeln, welches gegebenenfalls mittels herkömmlicher Methoden, wie Destillation, Kristallisation, Chromatographie und dergleichen, aufgetrennt werden kann.

In Stufe (m) wird eine Verbindung der Formel XVI mit einem niederen Alkyl- oder Arylmagnesiumhalogenid der Formel XVII in einem inerten Lösungsmittel, vorzugsweise Diethylether oder Tetrahydrofuran, bei

einer Temperatur von -80 ° C bis zur Rückflusstemperatur umgesetzt. Nach erfolgter saurer Hydrolyse des erhaltenen Imins, unter Verwendung einer wässrigen Lösung einer Säure, vorzugsweise Oxalsäure oder Schwefelsäure, wird eine erhaltene Verbindung der Formel IX mittels herkömmlicher Methoden, wie Destillation, Kristallisation, Chromatographie und dergleichen, isoliert.

In Stufe (n) wird eine Verbindung der Formel XVI mit einem Reduktionsmittel vorzugsweise Diisobutylaluminiumhydrid, in einem inerten Lösungsmittel, vorzugsweise Toluol, bei einer Temperatur von -80 ° C bis Raumtemperatur umgesetzt. Nach saurer Hydrolyse des erhaltenen Imins unter Verwendung einer wässrigen Lösung einer Säure, vorzugsweise Oxalsäure oder Schwefelsäure, wird die erhaltene Verbindung der Formel IXa mittels herkömmlicher Methoden, wie Destillation, Chromatographie, Kristallisation und dergleichen, isoliert.

**Reaktionsschema VI**

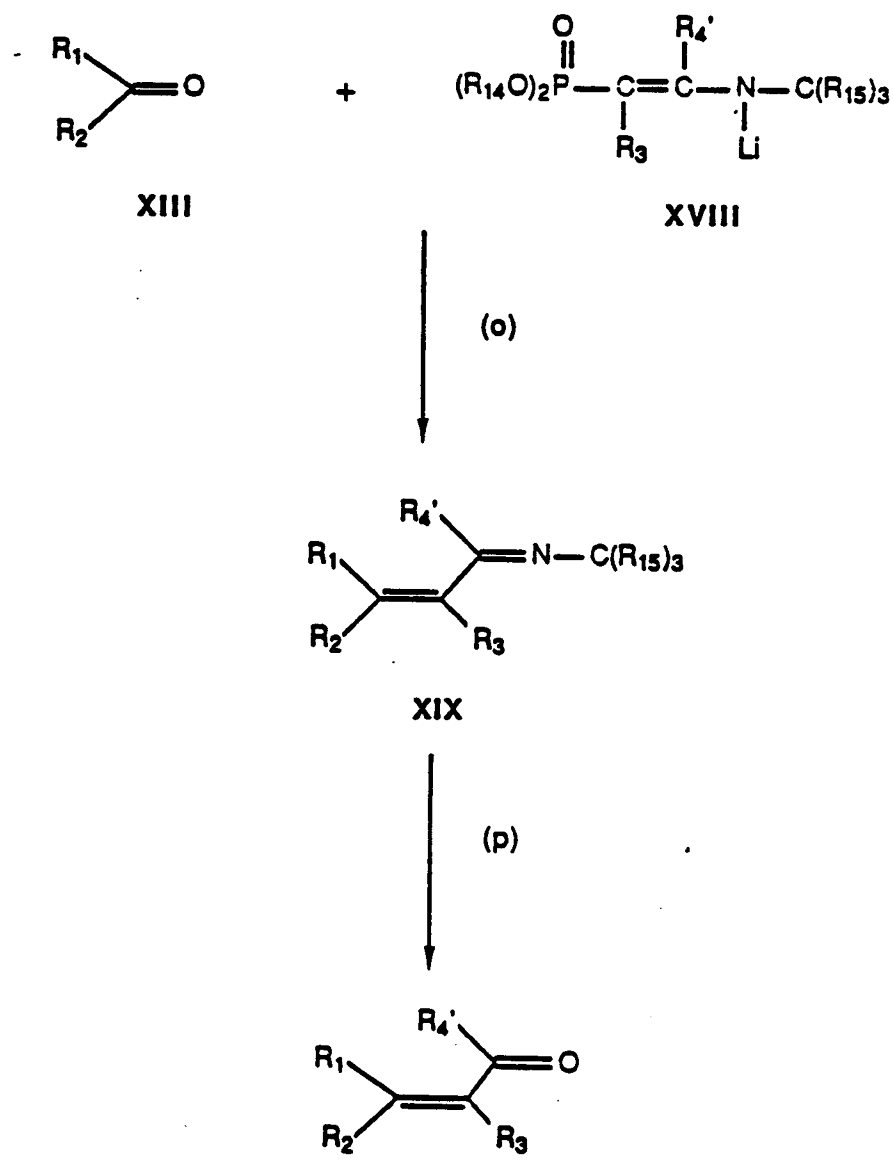


XIII + XVIII

XIX

IXa

worin $R_1$, $R_2$, $R_3$ und $R_4'$ obige Bedeutung besitzen, und $R_{14}$ niederes Alkyl oder Aryl und $R_{15}$ niederes Alkyl bedeuten.

In Reaktionsschema VI, Stufe (o), wird ein Lithioenaminophosphonate der Formel XVIII, das man in situ durch Umsetzen eines Carboxaldehyd-N-t-butylimins mit einem Dialkylchlorophosphonate in Gegenwart von überschüssigem Lithiumdiisopropylamid (LDA) hergestellt hat, mit einer Carbonylverbindung der Formel XIII in einem inerten Lösungsmittel, vorzugsweise Tetrahydrofuran oder Diethylether, bei einer Temperatur von -80 °C bis 50 °C umgesetzt. In Stufe (p) wird das erhaltene Imin der Formel XIX meist ohne vorgängige Isolierung unter Verwendung einer wässrigen Lösung einer Säure, vorzugsweise Oxalsäure, Salzsäure oder Schwefelsäure, hydrolisiert. Sofern $R_1$ von $R_2$ verschieden ist, kann es sich bei der erhaltenen Verbindung der Formel IXa um eine Mischung der (E)- und (Z)-Isomeren handeln, das gegebenenfalls mittels herkömmlicher Methoden wie Chromatographie, Kristallisation und dergleichen, aufgetrennt werden kann.

## Reaktionsschema VII

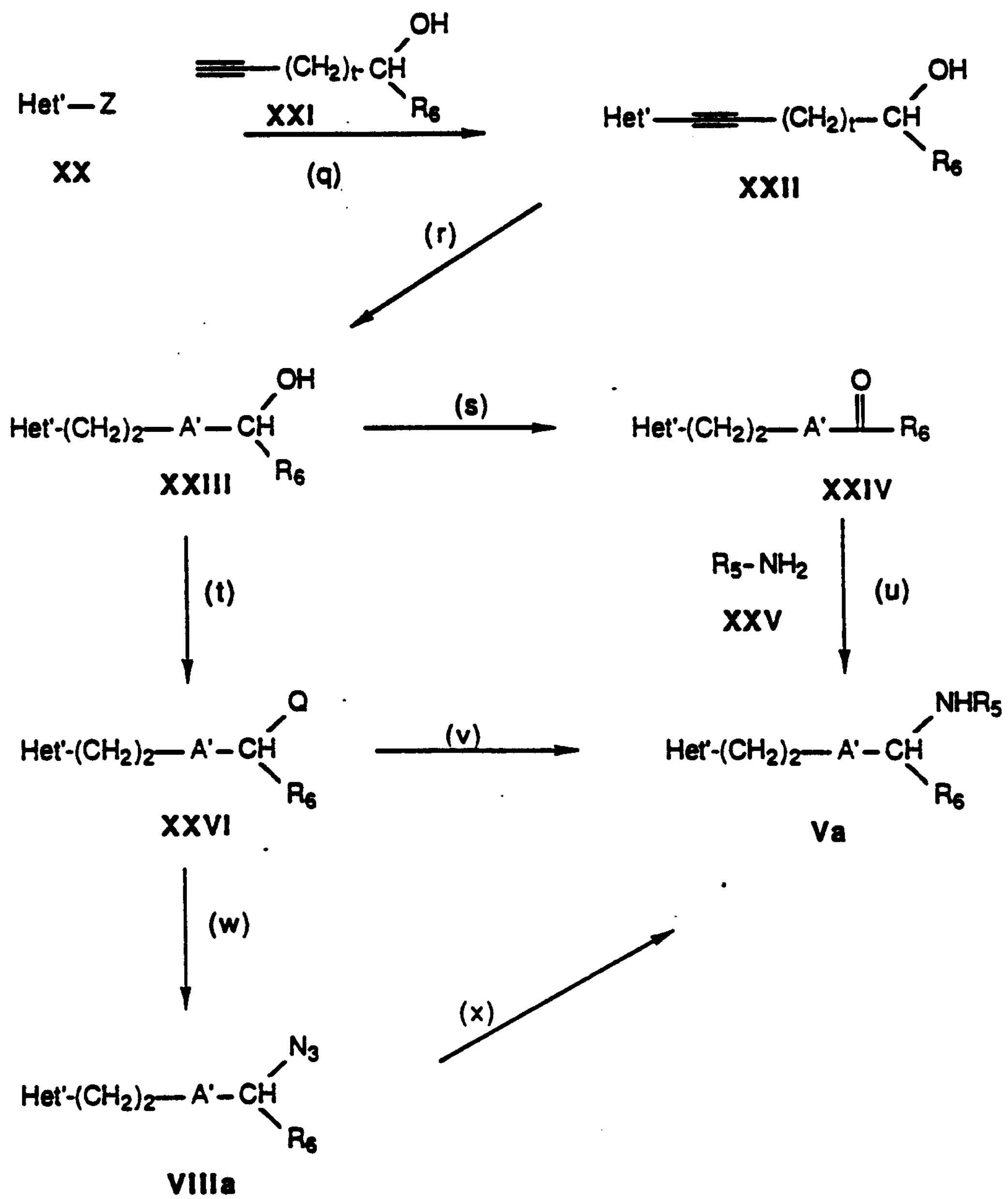

Het'-Z ist eine monocyclische 5- oder 6-gliedrige heteroaromatische oder eine bicyclische heteroaromatische Verbindung, welche ein oder zwei Heteroatome ausgewählt aus Stickstoff, Sauerstoff und Schwefel enthält, und welche durch Halogen, niederes Alkyl oder Aryl substituiert sein kann, wobei Z Jod, Brom oder eine niedere Perfluoralkylsulfonatgruppe ist, und wobei sich diese Gruppe an einer Position des heteroaromatischen Ringes befindet, welche in Uebergangsmetall-katalysierten Aryl/Alkinyl-Kupplungsreaktionen aktiviert ist. Die Verbindung der Formel XXI wird an die Stelle der Abgangsgruppe Z an den Ring gebunden. Beispiele von Het' sind: 3-Pyridinyl, 5-Pyrimidinyl, 2-Thienyl, 3-Thienyl, 2-Pyridinyl, 6-Methyl-2-pyridinyl, 2-Methyl-3-pyridinyl, 3-Chinolinyl, 4-Isochinolinyl und dergleichen. t ist eine ganze Zahl von 0 bis 4, A' ist Alkylen mit 1 bis 4 Kohlenstoffatomen, Q ist Brom, Chlor oder niederes Alkyl- oder Arylsulfonyloxy und $R_5$ und $R_6$ sind wie oben beschrieben.

In Reaktionsschema VII, Stufe (q), wird eine Verbindung der Formel XX mit einem Acetylen der Formel XXI in Gegenwart eines Ueberschusses an einem Protonenakzeptor, z.B. Triethylamin, und eines geeigneten Palladiumkatalysators, z.B. bis(Triphenylphosphin)palladium-dichlorid, gegebenenfalls in Gegenwart eines inerten Lösungsmittels, z.B. Methylenchlorid oder Dimethylformamid, bei einer Temperatur von Raumtemperatur bis 100 ° C, umgesetzt, wobei die Reaktionsbedingungen von der Wahl von Z, des Lösungsmittels und des heteroaromatischen Ringes abhängen. Die erhaltene Verbindung der Formel XXII kann mittels herkömmlicher Methoden, wie Destillation, Chromatographie und dergleichen, isoliert werden oder direkt in die nächste Stufe der Synthese eingesetzt werden.

In Stufe (r) wird ein Acetylen der Formel XXII in einem inerten Lösungsmittel aufgelöst, z.B. in einem niederen Alkanol, und dann über einen geeigneten Katalysator, z.B. Palladium/Kohle, Platinoxid oder dergleichen, bei einem Wasserstoffdruck von eins bis fünf Atmosphären, vorzugsweise bei Raumtemperatur bis zur Beendigung der Reduktion hydriert. Erhaltene Verbindungen der Formel XXIII, worin $R_6$ von Wasserstoff verschieden ist, können unter Verwendung von Standardmethoden in die Enantiomeren aufgespalten werden, z.B. durch Bildung von Estern mit chiralen Carbonsäuren, chromatographischer Auftrennung der Ester und Hydrolyse der erhaltenen Ester.

In Stufe (t) wird ein Alkohol der Formel XXIII mit einem niederen Alkyl- oder Arylsulfonylhalogenid, z.B. Methansulfonylchlorid oder p-Toluolsufonylchlorid, in Gegenwart eines Protonenakzeptors, z.B Pyridin oder Triethylamin, zu einer Verbindung der Formel XXVI, worin Q niederes Alkyl- oder Arylsulfonyloxy bedeutet, mit der gleichen absoluten Konfiguration wie der Ausgangsalkohol der Formel XXIII umgesetzt. Als Alternative kann man auch eine Verbindung der Formel XXIII mit einem Mittel umsetzen, das für die Ueberführung von Alkoholen in Halogenide geeignet ist, z.B. Thionylchlorid, wobei man in Gegenwart eines Protonenakzeptors, z.B. Pyridin, arbeitet und zwar so lange bis die Ueberführung in eine Verbindung der Formel XXVI, worin Q Chlor oder Brom bedeutet, vollständig ist. Die erhaltene Verbindung der Formel XXVI wird im allgemeinen nicht isoliert, sondern direkt in die nächste Stufe eingesetzt.

In Stufe (w) wird eine Verbindung der Formel XXVI mit einem Alkalimetallazid, z.B. Natriumazid, in Gegenwart eines polaren, inerten Lösungsmittels, z. B Dimethylformamid, N-Methylpyrrolidinon, Dimethylsulfoxid oder dergleichen, bei einer Temperatur von etwa Raumtemperatur bis 100 ° C umgesetzt, und zwar bis die Bildung des Azides vollständig ist. Die erhaltene Verbindung der Formel VIIIa kann mittels herkömmlicher Methoden z.B. Chromatographie oder dergleichen, isoliert werden. Diese Umwandlung erfolgt im allgemeinen unter Inversion in Bezug auf das Kohlenstoffatom der Verbindung der Formel XXVI, das den Substituenten Q trägt.

In Stufe (v) wird eine Verbindung der Formel XXVI mit einem Aequivalent eines Aminanions zu einem Zwischenprodukt umgesetzt, das zu einem Amin der Formel Va weiterverarbeitet werden kann. Man kann beispielsweise eine Verbindung der Formel XXVI mit einem Alkalimetalphthalimid, z.B. Kaliumphthalimid, in einem polaren aprotischen Lösungsmittel z.B. Dimethylformamid, Dimethylsulfoxid, N-Methylpyrrolidinon oder dergleichen, bei einer Temperatur von etwa 60 ° C bis 120 ° C zu einem Zwischenprodukt der Formel XXVI worin Q Phthalimido bedeutet, umsetzen, welche dann mittels herkömmlicher Methoden in eine Verbindung der Formel Va übergeführt werden kann, z.B. durch Behandeln mit Hydrazin in einem niederen Alkohol oder mit Methylamin in einem polaren aprotischen Lösungsmittel, wie Dimethylformamid. Als Alternative kann man eine Verbindung der Formel XXVI mit einem niederen Perfluoralkylsulfonamid (von einem primären Amin abgeleitet), z.B. einem N-(niederen Alkyl)trifluormethansulfonamid, in einem polaren, aprotischen Lösungsmittel, z. B. Aceton, Dimethylformamid, Dimethylsulfoxid oder dergleichen, in Gegenwart einer Base, z. B. einem Alkalimetallhydroxid, oder je nach dem einem Alkalimetallhydrid, z.B. Natriumhydrid, bei einer Temperatur von Raumtemperatur bis 100 ° C umsetzen. Eine erhaltene Verbindung der Formel Va kann mittels herkömmlicher Methoden, z. B. Destillation, Kristallisation der entsprechenden Säureadditionssalze, Chromatographie oder dergleichen, isoliert werden. Falls eine chirale Verbindung der Formel XXVI eingesetzt wird, verläuft diese Reaktion im allgemeinen unter Inversion der Konfiguration am Kohlenstoffatom der Verbindung der Formel XXVI, an das der Substituent Q gebunden ist.

In Stufe (s) wird ein Alkohol der Formel XXIII zu einer Carbonylverbindung der Formel XXIV oxidiert. Geeignete Reagenzien für diese Umwandlung sind beispielsweise Oxidationsmittel auf der Basis von Chrom, z.B. Pyridiniumchlorochromat. Eine bevorzugte Methode wird in K. Omura und D. Swern, Tetrahedron 34, 1651 (1978) beschrieben. Bei dieser Methode wird ein leichter Ueberschuss eines Carbonsäurehalogenides z.B. Oxalylchlorid, in einem inerten halogenierten Kohelnwasserstoff, z. B. Methylenchlorid, auf eine Temperatur von -50 ° C bis -80 ° C gekühlt, worauf man überschüssiges Dimethylsulfoxid dazugibt, während 0,25 bis 0,5 Stunden rührt, ein Aequivalent eines Alkohols der Formel XXIII dazugibt, weitere 0,25 bis 0,5 Stunden rührt, unter Beibehaltung einer Reaktionstemperatur von -50 ° C bis -80 ° C mit überschüssigem Triethylamin versetzt und das Reaktionsgemisch während 0,5 bis 1 Stunde erwärmen lässt, bevor man es mit Wasser und überschüssiger anorganischer Base behandelt. Man erhält eine Carbonylverbindung der Formel XXIV.

In Stufe (u) wird eine Carbonylverbindung der Fomel XXIV mit einem Amin der Formel XXV zu einer Schiffschen Base umgesetzt. Diese wird dann in Gegenwart eines geeigneten Reduktionsmittels zu einem Amin der Formel Va reduziert. Dieses Verfahren kann entweder als Einstufen- oder als Zweistufenverfahren durchgeführt werden. Man kann beispielsweise eine Verbindung der Formel XXIV mit einem grossen Ueberschuss an einem Amin der Formel XXV und einem Aequivalent einer schwachen organischen Säure, z.B. Essigsäure, in Gegenwart eines Reduktionsmittels, wie Natriumcyanoborhydrid in einem geeigneten Lösungsmittel, vorzugsweise einem niederen Alkohol, z.B. Methanol, bei Raumtemperatur behandeln, bis das Ausgangsmaterial aufgebraucht ist. Als Alternative kann man ein Amin der Formel XXV und eine Carbonylverbindung der Formel XXIV zusammen in einem aromatischen Lösungsmittel erhitzen, wobei man eine mit einem Wasserabscheider versehene Apparatur verwendet und bis zur Beendigung der Wasserbildung arbeitet. Die erhaltene Schiffbase kann über einen geeigneten Katalysator beispielsweise Raneynickel, bei einem Wasserstoffdruck von 1 bis 5 Atmosphären hydriert werden. Man erhält eine Verbindung der Formel Va. Sofern $R_6$ von Wasserstoff verschieden und $R_5$ ein chiraler Substituent ist, kann ein Diastereomer des Amins der Formel Va angereichert werden. Beispielsweise kann man, wenn $R_5$ eine chirale Benzylgruppe, z.B (R)-$\alpha$-Methylbenzyl ist, und $R_6$ niederes Alkyl, z.B Methyl bedeutet, die Verbindung der Formel Va diastereomer anreichern und die chirale Benzylgruppe dann abspalten, beispielsweise durch Hydrierung über Palladium/Kohle, wobei man ein mit einem Enantiomer angereichertes Amin der Formel Va erhält, worin $R_5$ Wasserstoff und $R_6$ niederes Alkyl bedeuten. Die Verbindungen der Formel Va können mittels herkömmlicher Methoden, beispielsweise Extraktion gefolgt von Destillation, Kristillisation ihrer Säureadditionssalze, Chromatographie oder dergleichen, isoliert werden.

In Reaktionsschema VII, Stufe (x), wird ein Azid der Formel VIIIa in einem Lösungsmittel, vorzugsweise einem niederen Alkohol, aufgelöst und bei einem Wasserstoffdruck von 1 bis 5 Atmosphären über einen Edelmetallkatalysator, z.B. Palladium/Kohle oder Platinoxid, hydriert. Die erhaltene Verbindung der Formel Va kann mittels herkömmlicher Methoden, beispielsweise Destillation, Kristallisation ihrer Säureadditionssalze, Chromatographie oder dergleichen, isoliert werden. Diese Reaktion verläuft ohne Aenderung der Konfiguration am Kohlenstoffatom, der Verbindung der Formel VIIIa, an das die Azido-Gruppe gebunden ist.

## Reaktionsschema VIII

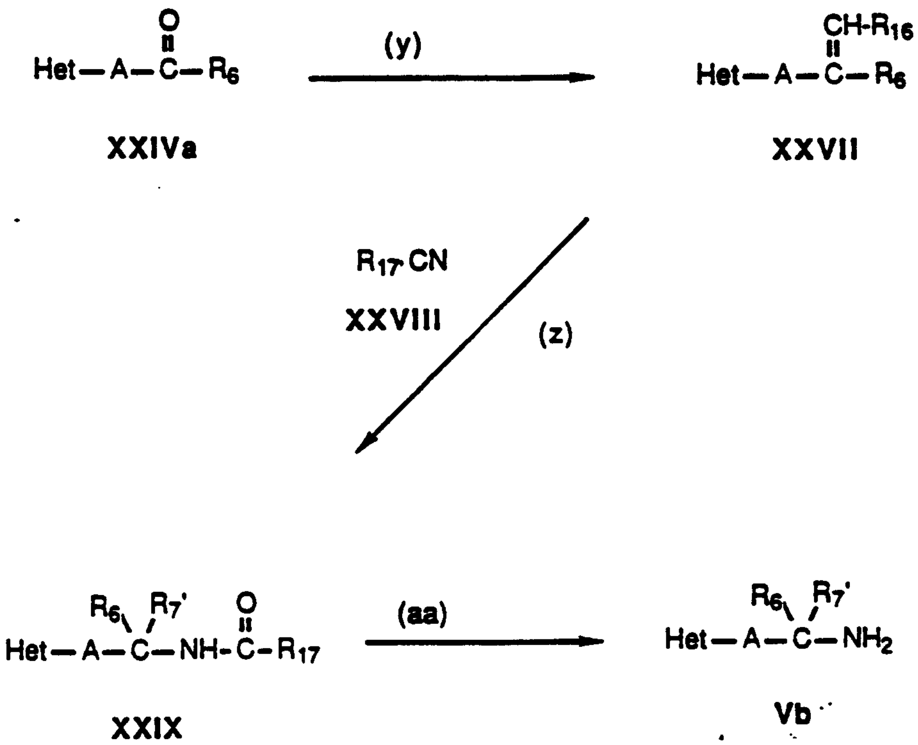


worin Het, A und $R_6$ obige Bedeutung besitzen, und $R'_7$ niederes Alkyl, $R_{16}$ Wasserstoff oder niederes Alkyl und $R_{17}$ niederes Alkyl oder Aryl bedeuten.

In Reaktionsschema VIII, Stufe (y), wird eine Carbonylverbindung der Formel XXIVa in einem inerten Lösungsmittel, vorzugsweise Tetrahydrofuran, Dimethylsulfoxid oder Diethylether, bei einer Temperatur von -80 ° C bis Raumtemperatur mit einem niederen Alkylidentriarylphosphoran behandelt. Die erhaltene Verbindung der Formel XXVII kann mittels herkömmlicher Methoden, z.B. Destillation, Chromatographie oder dergleichen, isoliert werden.

In Stufe (z) wird ein Nitril der Formel XXVIII in Gegenwart einer starken Mineralsäure, vorzugsweise Schwefelsäure, und einer kleinen Menge an Wasser mit einer Verbindung der Formel XXVII umgesetzt. Die erhaltene Verbindung der Formel XXIX kann mittels herkömmlicher Methoden, beispielsweise Destillation, Kristallisation, Chromatographie oder dergleichen, isoliert werden.

In Stufe (aa) wird eine Verbindung der Formel XXIX zu einem Amin der Formel Vb hydrolisiert. Vorzugsweise bedeutet dabei $R_{17}$ 2-Nitrobenzyl, und die katalytische Reduktion der Nitrobenzylgruppe wird beispielsweise über Palladium/Kohle bei einem Wasserstoffdruck von einer Atmosphäre durchgeführt. Man erhitzt dabei entweder in Gegenwart oder in Abwesenheit eines Lösungsmittels, beispielsweise Essigsäure. Die erhaltene Verbindung der Formel Vb kann mittels herkömmlicher Methoden, beispielsweise Destillation, Kristallisation der entsprechenden Säureadditionssalze, Chromatographie oder dergleichen, isoliert werden.

## Reaktionsschema IX

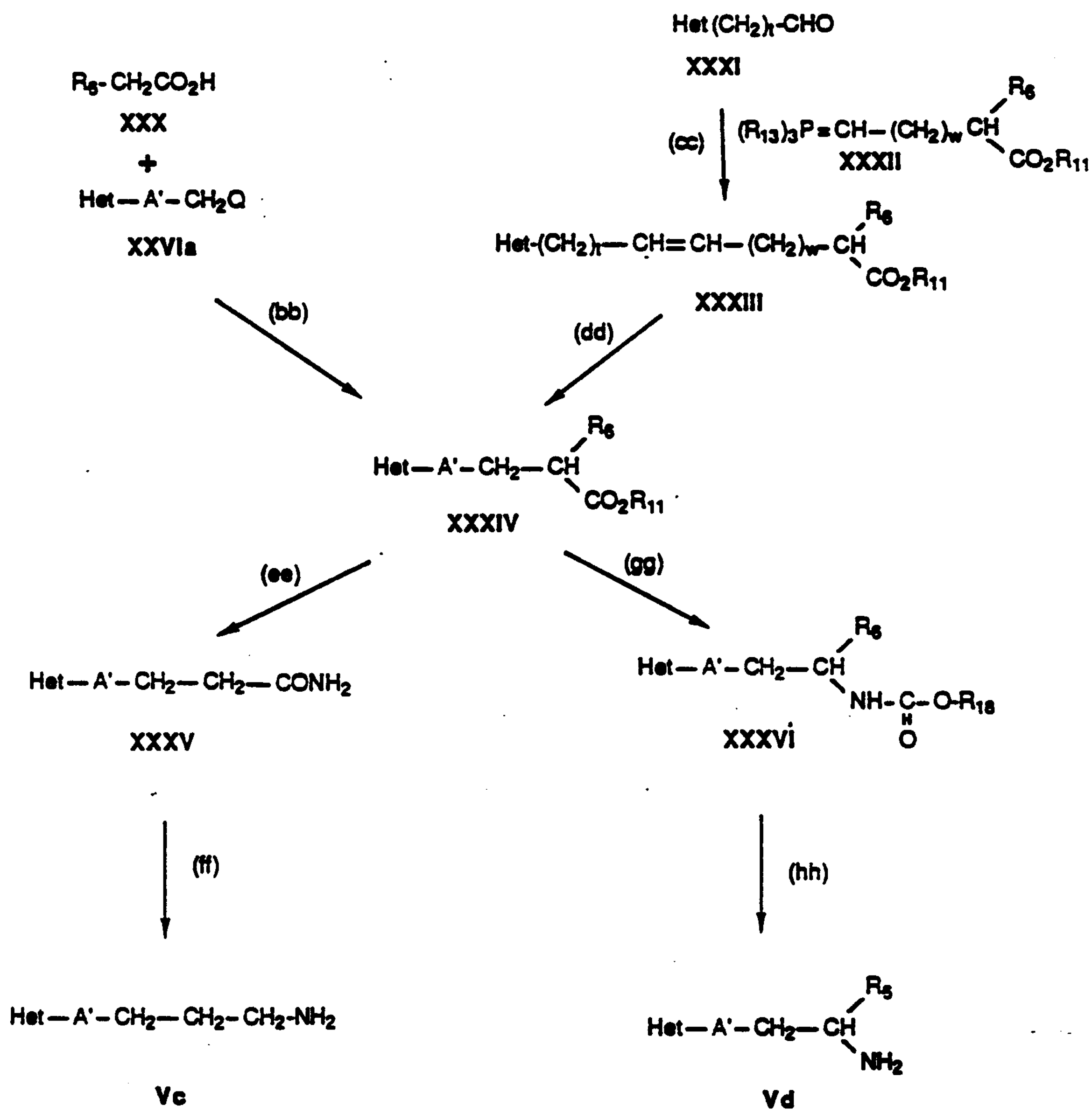


worin Het, $R_6$, $R_{13}$, A′, Q und t obige Bedeutung besitzen, und $R_{11}$ Wasserstoff oder niederes Alkyl, $R_{18}$ niederes Alkyl oder Aryl und w eine ganze Zahl von 0 bis 3 bedeuten.

In Reaktionsschema IX Stufe (bb), wird das Dilithiumsalz einer Verbindung der Formel XXX (beispielsweise durch Behandeln mit Lithiumdiisopropylamid hergestellt) in einem geeigneten inerten Lösungsmittel, beispielsweise Tetrahydrofuran, mit einer Verbindung der Formel XXVIa zu einer Verbindung der Formel XXXIV ($R_{11}$ = Wasserstoff) umgesetzt. Die erhaltene Verbindung der Formel XXXIV kann mittels herkömmlicher Methoden, beispielsweise Kristallisation, Chromatographie oder dergleichen, isoliert werden.

Verbindungen der Formel XXXIV, worin $R_6$ von Wasserstoff verschieden ist, und $R_{11}$ Wasserstoff bedeutet, können in die Enantiomeren gespaltet werden, indem man sie in Salze von chiralen, optisch einheitlichen Aminen, z.B. Cinchonin, Brucin, α-Methylbenzylamin oder dergleichen, überführt. Die reinen diasteromeren Salze erhält man dann durch fraktionierte Kristallisation aus geeigneten Lösungsmitteln, z.B. niederen Alkoholen. Die chiralen, optisch reinen Säuren der Formel XXXIV ($R_{11}$ = Wasserstoff) können mittels herkömmlicher Methoden aus den entsprechenden Salzen hergestellt werden, beispielsweise durch

Extraktion aus wässrigen sauren Lösungen.

In Stufe (cc) wird ein heteroaromatisches Carboxaldehyd der Formel XXXI mit einem (Carboxyalkyliden)triarylphosphoran der Formel XXXII in einem geeigneten Lösungsmittel, z. B. Tetrahydrofuran, Methylenchlorid, Methanol oder Dimethylsulfoxid, zu einer Verbindung der Formel XXXIII umgesetzt. Die erhaltene Verbindung der Formel XXXIII kann mittels herkömmlicher Methoden, beispielsweise mittels Kristallisation, Destillation, Chromatographie oder dergleichen, isoliert werden.

In Stufe (dd) wird eine Verbindung der Formel XXXIII über einen geeigneten Katalysator, z.B. Palladium/Kohle oder Platinoxid, in einem geeigneten Lösungsmittel, z. B einem niederen Alkohol, bei einem Wasserstoffdruck von 1 bis 5 Atmosphären hydriert, und zwar bis die theoretische Menge an Wasserstoff aufgenommen worden ist, wobei man eine Verbindung der Formel XXXIV erhält.

In Stufe (ee) wird eine Verbindung der Formel XXXIV in ein Amid der Formel XXXV übergeführt, wobei man die für die Umwandlung von Carbonsäuren und Estern in entsprechende primäre Amide herkömmlichen Methoden verwendet. Beispielsweise kann eine Verbindung der Fomel XXXIV, worin $R_{11}$ Wasserstoff bedeutet, durch Behandeln mit Thionylchlorid in ein entsprechendes Säurechlorid übergeführt werden, das dann mit überschüssigem Ammoniak in eine entsprechende Verbindung der Formel XXXV übergeführt wird. Als Alternative kann man auch eine Verbindung der Formel XXXIV, worin $R_{11}$ niederes Alkyl bedeutet, durch Behandeln mit überschüssigem Ammoniak, gegebenenfalls in Gegenwart eines Cosolvens, beispielsweise einem niederen Alkohol, bei einer Temperatur von -33 °C bis Raumtemperatur in eine entsprechende Verbindung der Formel XXXV überführen. Die Reaktion kann gegebenenfalls auch in einem Druckgefäss durchgeführt werden.

In Stufe (ff) wird eine Verbindung der Formel XXXV mit einem Reduktionsmittel, beispielsweise Boran in Tetrahydrofuran, bei einer Temperatur von Raumtemperatur bis zur Rückflusstemperatur des Lösungsmittels behandelt, und zwar während 4 bis 24 Stunden oder bis die Reduktion vollständig ist. Man erhält dabei eine Verbindung der Formel Vc. Diese kann mittels herkömmlicher Methoden isoliert werden, indem man das überschüssige Reagenz mit einem niederen Alkohol zerstört, das Reaktionsgemisch mit einer Mineralsäure, beispielsweise Salzsäure, behandelt, basisch stellt, das Lösungsmittel abdampft und das Produkt in ein geeignetes organisches Lösungsmittel, beispielsweise Methylenchlorid, extrahiert. Die Reinigung erfolgt dann durch Destillation, Chromatographie oder dergleichen.

In Stufe (gg) wird eine Säure der Formel XXXIV ($R_{11}$ = Wasserstoff), welche durch Hydrolyse des entsprechenden Esters erhalten werden kann, in Gegenwart eines niederen Alkohols den Bedingungen einer Curtius-Umlagerung unterworfen. In einer bevorzugten Ausführungsform wird eine Carbonsäure der Formel XXXIV, worin $R_{11}$ Wasserstoff bedeutet, mit einem Aequivalent von Diphenylphosphorylazid in Gegenwart eines Protonenakzeptors, beispielsweise Triethylamin oder dergleichen, und eines Ueberschusses an einem niederen Alkohol oder einem Phenol behandelt, wobei man eine Verbindung der Formel XXXVI erhält. Die erhaltene Verbindung der Formel XXXVI kann mittels herkömmlicher Methoden, beispielsweise mittels Kristallisation, Chromatographie oder dergleichen, isoliert werden.

In Stufe (hh) wird eine Verbindung der Formel XXXVI mit einem Ueberschuss an einer Mineralsäure in Wasser und gegebenenfalls einem Cosolvens, beispielsweise einem niederen Alkohol, bei einer Temperatur von Raumtemperatur bis 100 °C oder mit einer starken Base in Wasser, gegebenenfalls in Gegenwart eines Cosolvens, beispielsweise eines niederen Alkohols, bei einer Temperatur zwischen 60 °C und 100 °C behandelt, wobei man eine Verbindung der Formel Vd erhält. Diese kann mittels herkömmlicher Methoden, beispielsweise mittels Kristallisation der entsprechenden Säureadditionssalze, Destillation, Chromatographie oder dergleichen, isoliert werden.

## Reaktionsschema X

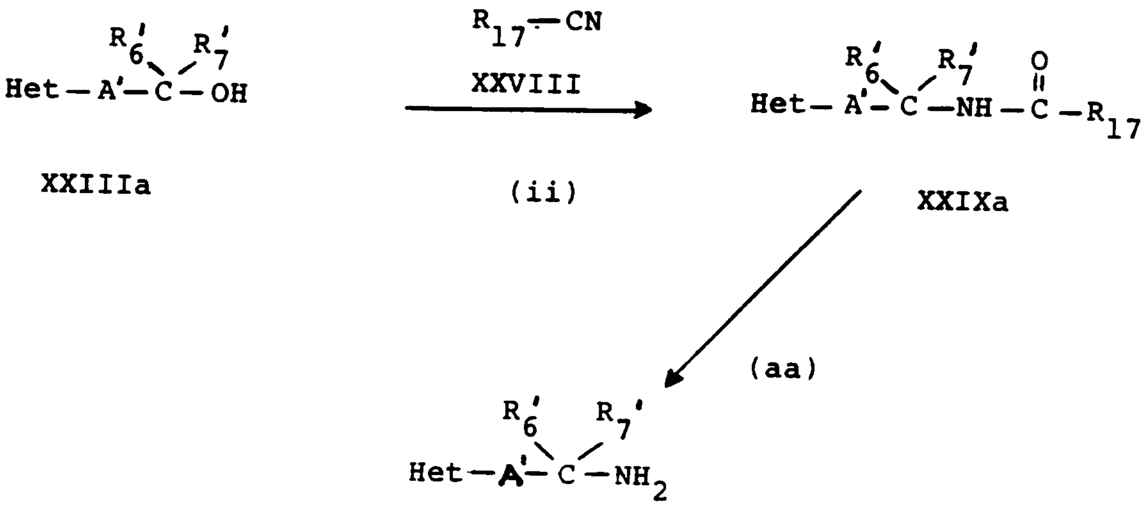


XXIIIa XXIXa Vd

worin Het, A′, R′7 und R17 obige Bedeutung besitzen, und R′6 niederes Alkyl bedeutet.

In Reaktionsschema X, Stufe (ii), wird ein Alkohol der Formel XXIIIa mit einem Nitril der Formel XXVIII umgesetzt, und zwar in Gegenwart einer Mineralsäure, z.B. Schwefelsäure, und Wasser bei einer Temperatur von -20 °C bis Raumtemperatur. Man erhält dabei eine Verbindung der Formel XXIXa, welche mittels herkömmlicher Methoden, z.B. Chromatographie, Kristallisation oder dergleichen, isoliert werden kann.

In diesem Reaktionsschema ist die Stufe (aa) identisch mit der Stufe (aa) aus Reaktionsschema VIII. Man erhält eine Verbindung der Formel Vd, welche mittels herkömmlicher Methoden, beispielsweise Chromatographie, Kristallisation ihrer Säureadditionssalze, Destillation oder dergleichen, isoliert werden kann.

## Reaktionsschema XI

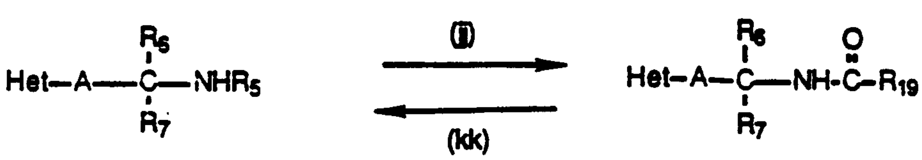


V XXXVIII

worin Het, A, $R_5$, $R_6$ und $R_7$ obige Bedeutung besitzen, und $R_{19}$ eine chirale Gruppe, beispielsweise eine verzweigte niedere Alkylgruppe oder eine niedere Alkylgruppe, welche mit einer oder zwei Gruppen ausgewählt aus Hydroxy, niederem Alkoxy, niederem Alkylcarbonyloxy, Perfluoralkyl oder Aryl, stubstituiert ist.

In Reaktionsschema XI, Stufe (jj), können diejenigen Verbindungen der Formel V, welche racemisch sind, in ihre Enantiomeren aufgespalten werden, und zwar durch Ueberführung in Amide von chiralen, optisch reinen Carbonsäuren und unter Verwendung von Verfahren, wie sie bei der Peptidherstellung üblich sind. Man kann beispielsweise ein Amin der Formel V mit einer chiralen, optisch reinen Carbonsäure, beispielsweise (R)-Mandelsäure, in Gegenwart eines geeigneten Kondensationsmittels, beispielsweise von Dicyclohexylcarbodiimid, gegebenfalls in Gegenwart eines Promoters, beispielsweise 1-Hydroxybenzotrizol, in einem polaren aprotischen Lösungsmittel, beispielsweise Dimethylformamid, zu einem Amid der Formel XXXVIII kondensieren. Die Amide der Formel XXXVIII können mittels fraktionierter Kristallisation, Chromatographie oder dergleichen in die reinen Diastereomere aufgespalten werden.

In Stufe (kk) erhält man die reinen enantiomeren Verbindungen der Formel V durch Hydrolyse der reinen diastereomeren Amide der Formel XXXVIII, beispielsweise mittels wässrigen Mineralsäure, bei einer Temperatur von 60 ° C bis 120 ° C.

## Reaktionsschema XII

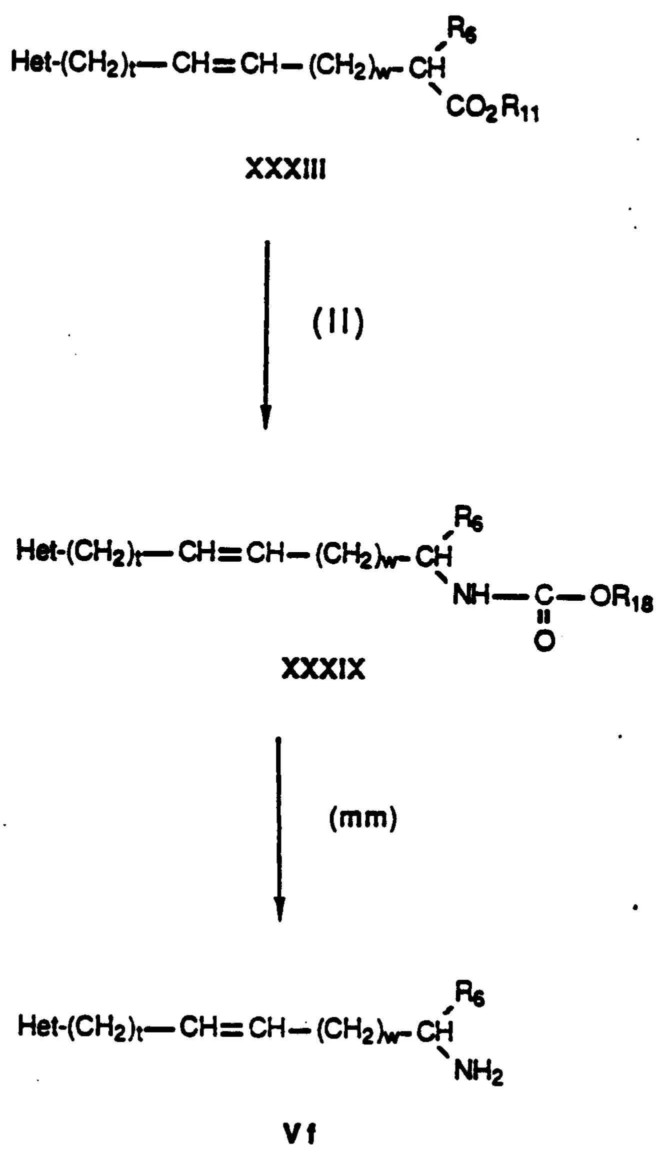


XXXIII

XXXIX

Vf

worin $R_6$, $R_{11}$, $R_{18}$, t, w und Het obige Bedeutung besitzen.

In Reaktionsschema XII, Stufe (11), wird eine Carbonsäure der Formel XXXVIII ($R_{11}$ bedeutet Wasserstoff), welche durch Hydrolyse aus dem entsprechenden Ester erhalten werden kann, mit einem Aequivalent an Diphenylphosphorylazid in Gegenwart eines Protonenakzeptors, beispielsweise von Triethylamin, und einem Ueberschuss an einem niederen Alkohol oder Phenol behandelt, wobei man eine Verbindung der Formel XXXIX erhält. Diese kann mittels herkömmlicher Methoden, beispielsweise mittels Destillation, Kristallisation, Chromatographie oder dergleichen, isoliert werden.

In Reaktionsschema XII, Stufe (mm), wird eine Verbindung der Formel XXXIX mit einem Ueberschuss an einer Mineralsäure in Wasser und gegebenenfalls einem Cosolvens, beispielsweise einem niederen Alkohol, bei einer Temperatur von Raumtemperatur bis 100 °C behandelt, wobei man eine Verbindung der Formel Vf erhält. Diese kann mittels herkömmlicher Methoden, beispielsweise mittels Chromatographie, Destillation oder dergleichen isoliert werden.

## Reaktionsschema XIII

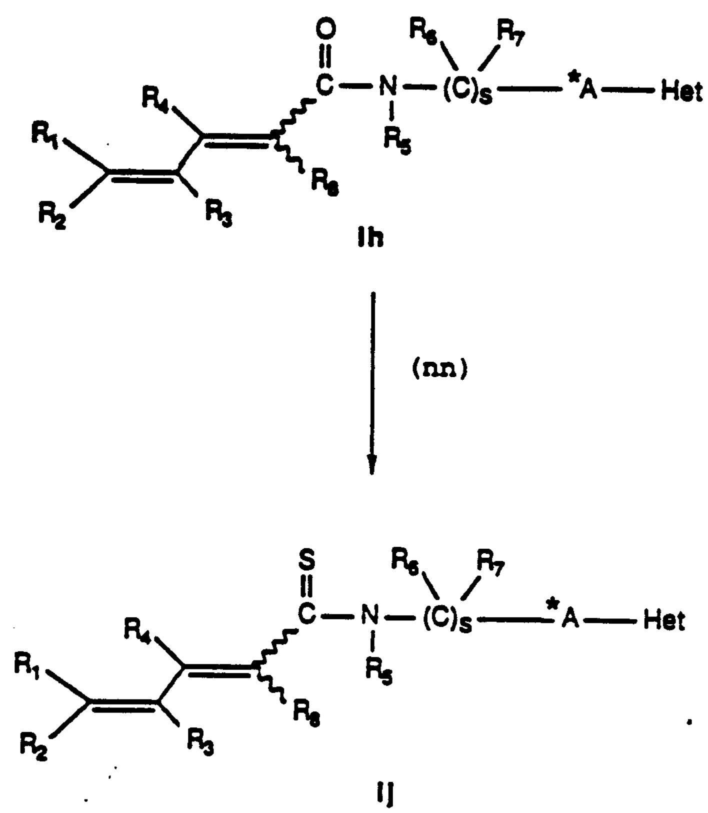


Ih

Ij

worin $R_1$, $R_2$, $R_3$, $R_4$, $R_5$, $R_6$, $R_7$, $R_8$, *A, Het und s obige Bedeutung besitzen.

In Reaktionsschema XIII, Stufe (nn), wird ein Carboxamid der Formel Ih mit Phosphorpentasulfid behandelt, und zwar in einem geeigneten Lösungsmittel, beispielsweise Methylenchlorid oder Tetrahydrofuran, und gegebenenfalls in Gegenwart eines Protonenakzeptors, beispielsweise Triethylamin, bei einer Temperatur von Raumtemperatur bis zur Rückflusstemperatur des Lösungsmittels. Die erhaltene Verbindung der Formel Ij kann mittels herkömmlicher Methoden, wie Chromatographie, Kristallisation und dergleichen, isoliert werden.

Die Verbindungen der Formel I können Säureadditionssalze mit anorganischen und organischen Säuren bilden. Sie bilden pharmazeutisch annehmbare Säureadditionssalze sowohl mit pharmazeutisch annehmbaren organischen als auch mit pharmazeutisch annehmbaren anorganischen Säuren, beispielsweise mit Halogenwasserstoffsäuren, wie Salzsäure, Bromwasserstoffsäure, Jodwasserstoffsäure, anderen Mineralsäuren, wie Schwefelsäure, Salpetersäure, Phosphorsäure, Perchlorsäure oder dergleichen, niederen Alkyl-und Aryl-sulfonsäuren, wie Ethansulfonsäure, p-Toluolsulfonsäure, Benzolsulfonsäure oder dergleichen, anderen organischen Säuren, wie Weinsäure, Maleinsäure, Zitronensäure, Salicylsäure, Ascorbinsäure und dergleichen. Pharmazeutisch nicht annehmbare Säureadditionssalze von Verbindungen der Formel I können mittels herkömmlicher metathetischer Reaktionen in pharmazeutisch annehmbare Säureadditionssalze übergeführt werden, wobei das pharmazeutisch nicht annehmbare Anion durch ein pharmazeutisch annehmbares Anion ersetzt wird. Als Alternative kann man auch ein pharmazeutisch nicht annehmbares Säureadditionssalz neutralisieren und die erhaltene freie Base mit einem Reagenz umsetzen, das ein pharmazeutisch annehmbares Säureadditionssalz liefert.

Die Verbindung der Formel I entfalten Wirkungen als PAF-Antagonisten und sind daher nützlich bei Krankheiten, welche durch überschüssiges PAF gekennzeichnet sind, oder zur Verhütung oder Behandlung von Kreislaufkrankheiten, Lungenkrankheiten, immunologischen Störungen, Entzündungen, dermatologischen Störungen, Schocks oder Transplantatabstossungen.

Die nützlichen Eigenschaften der Verbindungen der Formel I können mittels der nachfolgenden Verfahren gezeigt werden:

Binding-Test

a) Test

Der Binding-Test wird in 400 µl-Polyethylenmikrozentrifugenröhrchen (Beckmann) durchgeführt, welche 50 µl einer Mischung aus 2 Teilen Siliconol AR 200 (Serva) und 1 Teil Silicone Fluid (Arthur H. Thomas) enthalten. Man versetzt mit Puffer, Standard oder Analogen (150 µl Gesamtvolumen). Anschliessend gibt man radioaktiv markiertes $^3$H-PAF (50 µl) dazu. Durch Zugabe von 50 µl Hundeplättchen (2 x $10^7$ Plättchen) wird die Reaktion gestartet. Die Röhrchen werden verschlossen, mehrmals zwecks Durchmischung gekippt und während 10 Minuten bei Raumtemperatur inkubiert. Die Plättchen werden mittels Zentrifugation während einer Minute in einer Beckmann-Mikrofuge B getrennt. Die Spitze des Mikrozentrifugenröhrchens wird abgeschnitten, und die Plättchen werden mit 200 µl50-proz. Methanol (Burdick and Jackson) ausgewaschen. Man gibt Aquasol (NEN, 10 ml) dazu und bestimmt die Radioaktivität in der Probe mittels eines Beckmann LS 8100-Flüssigkeitsscintillationszählers (welcher mit einem Techtran-Recorder verbunden ist). Die erhaltenen Daten werden mittels eines im Hause vorhandenen Computersystems verarbeitet. Als Alternative kann man die Radioaktivität auch mittels eines Searle Mark III-Flüssigkeitsscintillationszählers bestimmten, welcher mit einem Iso-Data-Mikroprocessor verbunden ist. Die erhaltenen Resultate sind in der Tabelle I zusammengestellt.

b) Herstellung der Plättchen

Blut von anesthätisierten oder nichtanesthätisierten Hunden wird in 50 ml-Zentrifugenröhrchen aus Plastik, welche 3,8-proz. Natriumcitrat als Antikoagulans enthalten (1 Volumenteil Citrat/9 Volumenteile Blut) gegeben. Die Roten Zellen werden mittels Zentrifugation während 15 Minuten bei 600 rpm (100-125 g) bei Raumtemperatur entfernt. Ein Aliquot des überstehenden plättchenreichen Plasmas (PRP) wird zwecks Auszählung der Zellen auf die Seite gestellt und der Rest wird mit 0,15 M Zitronensäure auf pH 6,5 gestellt. Nach Zentrifugation während 10 Minuten bei 2000 rpm (1000 g) bei Raumtemperatur erhält man ein Plättchen-Pellet. Man suspendiert dieses Plättchen-Pellet einmal in PBS, das 1 mM EDTA enthält, zentrifugiert wie erwähnt, suspendiert die Plättchen in 0,1-proz. BSA-PBS und erhält auf diese Weise gewaschene Plättchen. Ein Aliquot der gewaschenen Plättchen wird ausgezählt. Für den Binding-Test werden die Plättchen auf 2 x $10^7$ Plättchen pro Versuchsröhrchen (4 x $10^8$ Plättchen pro ml) verdünnt. Die Plättchen werden mittels eines Royco Cell-Crit 921 ausgezählt.

PAF-Induzierter Bronchokonstriktionstest

Männliche Meerschweinchen (Hartley Stamm, 400-500 g) werden mit Urethan (2 g/kg, i.p.) anesthätisiert. Man kanüliert die Trachea der Versuchstiere und beatmet diese mit einem Harvard-Respirator für kleine Nagetiere (3,0 cc Hubvolumen, 40 Atemzüge pro Min.). Man misst den Trachealdruck mittels einer in die Trachea eingeführten und mit einem Statham-Pressure-Transducer verbundenen Kanüle.

Die Vena jugularis wird zwecks Verabreichung der Testverbindungen kanüliert. Die spontane Atmung wird mit Succinylcholin (1,2 mg/kg, i.v.), das man 2 Minuten vor der intravenösen Injektion von PAF verabreicht, eingestellt. Da das Propanolol die Bronchokonstriktorantwort erhöht, werden alle Versuchstiere

5 Minuten vor der Behandlung mit Propanolol (0,1 mg/kg, i.v.) behandelt.

Für den intravenösen Test verabreicht man den Meerschweinchen 5 Minuten vor der Behandlung eine Dosis von 0,1 mg/kg Propanolol intravenös. Die Testverbindung wird eine 1 Minute vor der intravenösen Behandlung mit PAF verabreicht. Die Versuchstiere werden dann mit einer intravenösen Dosis 1,0 µg/kg PAF behandelt, und die Aenderung des Trachealdruckes wird gemessen.

Beim oralen Test wird die Testverbindung mittels einer Magensonde 2 Stunden vor der Behandlung verabreicht. Propanolol oder Succinylcholin und PAF werden intravenös verabreicht, und die Aenderung im Trachealdruck wird gemessen.

Die Aenderung des Trachealdruckes wird bestimmt, indem man vom Spitzenwert nach Behandlung mit PAF den nach Verabreichen von Succinylcholin erhaltenen steady state-Grundwert abzieht. Für jede Testverbindung wird der Mittelwert berechnet und mit dem bei Kontrolltieren erhaltenen Mittelwert verglichen, wobei man die Hemmung der Bronchokonstriktion in Prozent erhält. Die Standard-Abweichung wird als Standard-Abweichung vom Mittelwert berechnet.

PAF induzierte Herabsetzung der Plättchenzahl

Männliche 500 bis 900 g schwere Hartley-Meerschweinchen erhalten ein Standard-Meerschweinchenfutter und Wasser ad libitum. PAF wird in Ethanol aufgelöst und als 2mM Vorratslösung bei -70 °C aufbewahrt. Für alle Experimente wird die Vorratslösung von PAF mit Tris-Puffer vom pH 7,4 und 0,1-proz. BSA auf 1pM verdünnt.

a) Intravenöses Verfahren

Die Meerschweinchen werden mit Urethan (1,6 g/kg, i.p.). anesthätisiert. Für die Blutentnahme wird ein Katheter (PE50) in die rechte Arteria carotis eingeführt. Zwecks Injektion der Testsubstanzen und für die Verabreichung von PAF (75 ng/kg, i.v.) wird ein zweiter Katheter (PE10) in die Vena jugularis eingeführt. Zur Kontrolle entnimmt man eine Blutprobe und zählt die Plättchen in einem Blutplättchenanalysator. Anschliessend verabreicht man PAF und entnimmt jeweils 15, 30 und 60 Sekunden später Blutproben für die Bestimmung der Anzahl Plättchen. Nach 15 Minuten werden Lösungen der Testverbindungen in DMSO in vorbestimmten Konzentrationen intravenös injiziert, wobei man jeweils Gruppen von 4 bis 6 Versuchstieren verwendet. Es wird in jeder Gruppe von Versuchstieren eine Konzentration des Antagonisten verwendet. 15 Minuten nach der Injektion der Testsubstanz werden vor und 15, 30 und 60 Sekunden nach der Behandlung mit PAF Blutproben entnommen, welche dann auf die Anzahl Plättchen untersucht werden.

b) Orales Verfahren

Die Versuchstiere werden mit Natriumpentobarbital (35 mg/kg, i.p.) anesthätisiert, worauf man die Arteria carotis und die Vena jugularis wie oben beschrieben kanüliert. Die Kanülen werden dann an der Basis des Nackens befestigt, worauf man die Tiere wieder zu sich kommen lässt. Mindestens 18 Stunden nach der Operation werden dann wache Meerschweinchen für das Experiment verwendet. Die Versuchstiere werden zweimal mit PAF behandelt (120 ng/kg, i.v.), wobei die erste Behandlung zur Festlegung einer konsistenten Antwort auf PAF und die zweite Behandlung als Kontrolle für die Behandlung mit PAF dienen. Blutproben wurden jeweils 15, 30 und 45 Sekunden nach der Behandlung mit PAF entnommen, worauf die Plättchen ausgezählt wurden. 30 Minuten nach der Kontrollbehandlung mit PAF erhalten die Tiere eine orale Dosis an Testsubstanz oder an Gummi acacium. Die Versuchstiere werden dann 1, 3, 5 und 24 Stunden nach der Verabreichung der Testsubstanz wiederum mit PAF behandelt.

Datenanalyse und Statistik

Die Aenderung in %, d.h. der Unterschied in der Anzahl Plättchen vor und nach der Behandlung mit PAF wird nach der folgenden Formel berechnet:

Aenderung in % = [#Plättchen (Kontrolle, Zeit O) - niedrigste #Plättchen (nach PAF)]:#Plättchen (Kontrolle, Zeit O).

Die Aktivität der Testsubstanz in % wird dann nach der folgenden Formel bestimmt:

Aktivität in % = [Aenderung in % (Kontrolle) - Aenderung in % (nach Testsubstanz)]: Aenderung in %

(Kontrolle).

Die statistische Signifikanz ($p<0,05$) in bezug auf die Aktivitätsdifferenz zwischen den durchschnittlichen PAf-Kontrollen und den durchschnittlichen mit Testsubstanz behandelten Versuchstieren wird mit dem gepaarten Students's t-Test bestimmt.

Die Verbindungen der Formel I besitzen ebenfalls Thromboxan-Synthase-($TXA_2$ Syn.)-hemmende Eigenschaften, welche wie folgt ermittelt werden können:

$TxA_2$-Synthase-Hemmung

Die hemmende Wirkung auf die Thromboxan-Synthase ($TXA_2$-Syn.) wird gemessen, indem man die Umwandlung von $^{14}C$-Thromboxan $A_2$ ($TXA_2$) verfolgt. Als Enzymquelle verwendet man microsomale Fraktionen von menschlichen Plättchen. Das $TXA_2$ zersetzt sich im wässrigen Inkubationsmedium rasch zu $TXB_2$. Die Menge an $TXA_2$-Syn. wird so eingestellt, dass unter den Versuchsbedingungen ungefähr 80 bis 90% des Sutstrates $PGH_2$, in Kontrollröhrchen zum Produkt umgewandelt wird. Zur Herstellung von $^{14}C$-$PGH_2$ wird $^{14}C$-AA(50-60mCi/mmole, Rose Chem.) während 1,5 Minuten bei 37°C mit Microsomen aus Samenblasen-Drüsen von Schafen inkubiert, worauf das gebildete $^{14}C$-$PGH_2$ mit Diethylether extrahiert, über Kolonnen von Sephadex LH-20 oder Kieselgel gereinigt und in Aceton bei -70°C aufbewahrt wird. Die Inkubationen werden wie folgt durchgeführt. Man gibt genügend $^{14}C$-$PGH_2$ in die Inkubationsröhrchen, um am Ende eine Substratkonzentration von 10 mmole (ca. 30'000 cpm) zu erreichen, worauf man das Aceton unter Stickstoff entfernt. Die Röhrchen werden dann in ein Eisbad gestellt, worauf man in dieser Reihenfolge 215 µl eiskalte phosphatgepufferte Kochsalzlösung, 10 µl Ethanol (Kontrolle) oder Testsubstanz in Ethanol und 25 µl der Microsomen-Suspension dazugibt und jeweils so rasch wie möglich mischt. Die Röhrchen werden dann bei 22°C während 2 Minuten inkubiert. Die Reaktion wird dann gestoppt und die radioaktiven Produkte und das nicht umgesetzte $PGH_2$ werden extrahiert und mittels Dünnschichtchromatographie analysiert. Die Menge an $^{14}C$-$PGH_2$, welche in Produkte umgewandelt worden ist, wird mittels Dünnschichtchromatographie analysiert. Die Menge an $^{14}C$-$PGH_2$, welche in Produkte umgewandelt worden ist, wurde als Mass für die $TXA_2$-Synthase-Aktivität verwendet. Inhibitoren werden anfänglich bei einer Konzentration von 100 µM getestet. Die $IC_{50}$-Werte werden mittels linearer Regressionsanalyse einer Verdünnungsreihe (jeweils 10-fache Verdünnung der Testsubstanz berechnet.

Die mit den Verbindungen der Formel I in den beschriebenen Experimenten erhaltenen Resultate werden in der nachfolgenden Tabelle I zusammengestellt:

| Verbindung | Hemmung der PAF-Bindung $IC_{50}$ (nM) | Hemmung der PAF-induzierten Bronchokonstriktion in % 1 mg/kg i.v. | 50 mg/kg p.o. | Hemmung der PAF-induzierten Reduktion der Anzahl Plättchen in % 3 mg/kg i.v. | 30 mg/kg p.o |
|---|---|---|---|---|---|
| (E)-5,5-Diphenyl-N-[4-(3-pyridinyl)butyl]-2,4-pentadienamid | 55 | 35 ± 15 | 12 ± 1 | 14 | |
| (Z)-5,5-Diphenyl-N-[4-(3-pyridinyl)butyl]-2,4-pentadienamid | 400 | | | | |
| [R,S](E)]-5,5-Diphenyl-N-[1-methyl-4-(3-pyridinyl)butyl]-2,4-pentadienamid | 100 | | | | |
| (E)-5,5-Diphenyl-2-methyl-N-[4-(3-pyridinyl)-butyl]-2,4-pentadienamid | 100 | | | | |
| (E)-5,5-bis(3-Fluorophenyl)-N-[4-(3-pyridinyl)butyl]-2,4-pentadienamid | 20 | 72 ± 16 | 37 ± 11 | 61 | 58 |
| [R,S-(E)]-5,5-bis-(3-Fluorophenyl)-N-[1-methyl-4-(3-pyridinyl)butyl]-2,4-pentadienamid | 30 | | | | |
| [R-(E)-5,5-bis(3-Fluorophenyl)-N-[1-methyl-4-(3-pyridinyl)butyl]-2,4-pentadienamid | 170 | 73 ± 6 | 91 ± 5 | 17 | |
| [S-(E)-]-5,5-bis(3-Fluorophenyl)-N-[1-methyl-4-(3-pyridinyl)butyl]-2,4-pentadienamid | 250 | 34 ± 7 | 46 ± 8 | 19 | |
| (E)-5,5-bis(3-Fluorophenyl)-N-[1,1-dimethyl-4-(3-pyridinyl)butyl]-2,4-pentadienamid | | | 11 ± 10 | | |
| (E)-5,5-bis(4-Fluorophenyl)-N-[4-(3-pyridinyl)butyl]-2,4-pentadienamid | 40 | 91 ± 3 | 97 ± 1 | 18 | |
| [R-(E)-5,5-bis(4-Fluorophenyl)-N-[1-methyl-4-(3-pyridinyl)butyl]-2,4-pentadienamid | 250 | 70 ± 9 | 53 ± 11 | 11 | |
| [S-(E)-5,5-bis(4-Fluorophenyl)-N-[1-methyl-4-(3-pyridinyl)butyl]-2,4-pentadienamid | 275 | | 44 ± 10 | | |
| (E)-5,5-bis(4-Fluorophenyl)-N-[1,1-dimethyl-4-(3-pyridinyl)butyl]-2,4-pentadienamid | | | 20 ± 14 | | |

| Verbindung | Hemmung der PAF-Bindung $IC_{50}$ (nM) | Hemmung der PAF-induzierten Bronchokonstriktion in % 1 mg/kg i.v. | Hemmung der PAF-induzierten Bronchokonstriktion in % 50 mg/kg p.o. | Hemmung der PAF-induzierten Reduktion der Anzahl Plättchen in % 3 mg/kg i.v. | Hemmung der PAF-induzierten Reduktion der Anzahl Plättchen in % 30 mg/kg p.o |
|---|---|---|---|---|---|
| (E)-5,5-bis(3-Methoxyphenyl)-N-[4-(3-pyridinyl)butyl]-2,4-pentadienamid | 2 | 69 ± 7 | 31 ± 11 | 75 | 48 |
| (Z)-5,5-bis(3-Methoxyphenyl)-N-[4-(3-pyridinyl)butyl]-2,4-pentadienamid | | | | | |
| (E)-5,5-bis(3-Methoxphenyl)-N-[6-(3-pyridinyl)hexyl]-2,4-pentadienamid | 450 | | | | |
| [R,S-(E)]-5,5-bis(3-Methoxyphenyl)-N-[1-methyl-4-(3-pyridinyl)butyl]-2,4-pentadienamid | 20 | | | | |
| [R-(E)-5,5-bis(3-Methoxyphenyl)-N-[1-methyl-4-(3-pyridinyl)butyl]-2,4-pentadienamid | 35 | 87 ± 4 | 87 ± 8 | 31 | |
| [S-(E)]-5,5-bis(3-Methoxyhenyl)-N-[1-methyl-4-(3-pyridinyl)butyl]-2,4-pentadienamid | 300 | 36 ± 4 | 10 ± 5 | 10 | |
| (E)-5,5-bis(3-Methoxyphenyl)-N-[1,1-dimethyl-4-(3-pyridinyl)butyl]-2,4-pentadienamid | | | 22 ± 10 | | |
| (E)-5,5-bis(4-Methoxphenyl)-N-[4-(3-pyridinyl)butyl]-2,4-pentadienamid | 25 | 92 ± 3 | 82 ± 12 | 40 | 45 |
| [R,S-(E)]-5,5-bis(4-Methoxyphenyl)-N-[1-methyl-4-(3-pyridinyl)butyl]-2,4-pentadienamid | | | | | |
| [R-(E)]-5,5-bis(4-Methoxyphenyl)-N-[1-methyl-4-(3-pyridinyl)butyl]-2,4-pentadienamid | 65 | 87 ± 4 | 97 ± 2 | 85 | 81 |
| [S-(E)]-5,5-bis(4-Methoxyphenyl)-N-[1-methyl-4-(3-pyridinyl)butyl]-2,4-pentadienamid | 200 | 42 ± 13 | 90 ± 6 | 42 | |
| (E)-5,5-bis(4-Methoxyphenyl)-N-[1,1-dimethyl-4-(3-pyridinyl)butyl]-2,4-pentadienamid | | | 54 ± 9 | | |
| (E)-5,5-bis(3,4-Dimethoxyphenyl)-N-[4-(3-pyridinyl)butyl]-2,4-pentadienamid | 55 | 78 ± 6 | 71 ± 2 | | |

| Verbindung | Hemmung der PAF-Bindung $IC_{50}$ (nM) | Hemmung der PAF-induzierten Bronchokonstriktion in % 1 mg/kg i.v. | Hemmung der PAF-induzierten Bronchokonstriktion in % 50 mg/kg p.o. | Hemmung der PAF-induzierten Reduktion der Anzahl Plättchen in % 3 mg/kg i.v. | Hemmung der PAF-induzierten Reduktion der Anzahl Plättchen in % 30 mg/kg p.o |
|---|---|---|---|---|---|
| [R-(E)-5,5-bis(3,4-Dimethoxphenyl)-N-[1-methyl-4-(3-pyridinyl)butyl]-2,4-pentadienamid | 50 | 99 ± 1 | 66 ± 19 | | |
| (E)-5,5-bis(3-Chlorophenyl)-N-[4-(3-pyridinyl)-butyl]-2,4-pentadienamid | 25 | 76 ± 14 | 35 ± 7 | 21 | |
| (E)-5,5-bis(4-Chlorophenyl)-N-[4-(3-pyridinyl)-butyl]-2,4-pentadienamid | 60 | 95 ± 2 | 63 ± 10 | 0 | |
| (E)-5,5-bis[2-(Trifluoromethyl)phenyl]-N-[4-(3-pyridinyl)butyl]-2,4-pentadienamid | 80 | 6 ± 10 | - | 0 | |
| (E)-5,5-bis[3-(Trifluoromethyl)phenyl]-N-[4-(3-pyridinyl)butyl]-2,4-pentadienamid | 150 | inactiv | - | 83 | |
| (E)-5,5-bis(3-Nitrophenyl)-N-[4-(3-pyridinyl)-butyl]-2,4-pentadienamid | 115 | 18 ± 2 | | | |
| (E,E)-5-(3-Methoxyphenyl)-5-phenyl-N-[4-(3-pyridinyl)butyl]-2,4-pentadienamid | 20 | | 13 ± 18 | 40 | |
| (2E,4Z)-5-(3-Methoxyphenyl)-5-phenyl-N-[4-(3-pyridinyl)butyl]-2,4-pentadienamid | 35 | | 10 ± 10 | inactiv | |
| (2E,4Z)-5-(3-Fluorophenyl)-5-(3-methoxphenyl)-N-[4-(3-pyridinyl)butyl]-2,4-pentadienamid | 40 | 90 ± 5 | 1 ± 8 | 67 (1 min) | |
| (E,E)-5-(3-Fluorophenyl)-5-(3-methoxyphenyl)-N-[4-(3-pyridinyl)butyl]-2,4-pentadienamid | 25 | 88 ± 4 | 25 ± 14 | inactiv | |
| (E)-5,5-bis[(1,1'-Biphenyl)-4-yl]-N-[4-3-pyridinyl)butyl]-2,4-pentadienamid | | | | | |
| (E)-5,5-bis(4-Methoxyphenyl)-N-[5-(2-pyridinyl)-pentyl]-2,4-pentadienamid | 1000 | 27 ± 6 | | | |
| (E)-5,5-bis(4-Methoxyphenyl)-N-[3-(4-pyridinyl)-propyl]-2,4-pentadienamid | 100 | 8 ± 12 | | | |

| Verbindung | Hemmung der PAF-Bindung $IC_{50}$ (nM) | Hemmung der PAF-induzierten Bronchokonstriktion in % 1 mg/kg i.v. | Hemmung der PAF-induzierten Bronchokonstriktion in % 50 mg/kg p.o. | Hemmung der PAF-induzierten Reduktion der Anzahl Plättchen in % 3 mg/kg i.v. | Hemmung der PAF-induzierten Reduktion der Anzahl Plättchen in % 30 mg/kg p.o |
|---|---|---|---|---|---|
| (E)-5,5-bis(4-Methoxyphenyl)-N-[4-(3-pyridinyl)-methyl)ethoxy]-2,4-pentadienamid | 25 | 48 ± 7 | | | |
| (E)-5,5-bis(4-Methoxyphenyl)-N-[3-[(3-pyridinyl)-ox]propyl]-2,4-pentadienamid | 300 | | | | |
| (E)-N-[4-(4-Isoquinolinyl)butyl]-5-5-bis-(4-methoxyphenyl)-2,4-pentadienamid | >1000 | | | | |
| (E)-N-[3-(8-Isoquinolinyl)propyl]-5,5-bis-(4-methoxyphenyl)-2,4-pentadienamid | >1000 | | | | |
| (E)-N-(5-Isoquinolinyl)-5,5-bis(4-methoxyphenyl)-2,4-pentadienamid | 600 | | | | |
| (E)-5,5-bis(4-Methoxyphenyl)-N-[4-(3-pyridinyl)-phenyl]-2,4-pentadienamid | 10 | 97 ± 1 | 84 ± 8 | | |
| (E)-5,5-bis(4-Methoxyphenyl)-N-[4-(3-pyridinyl)-methyl)phenyl]-2,4-pentadienamid | 250 | 3 ± 7 | | | |
| [R,S-(E)]-5,5-bis(4-Methoxphenyl)-N-[1-methyl-4-(6-methyl-3-pyridinyl)butyl]-2,4-pentadienamid | 700 | | | | |
| [R,S-(E)]-5,5-bis(4-Methoxyphenyl)-N-[1-methyl-4-(2-methyl-3-pyridinyl)butyl]-2,4-pentadienamid | 30 | 47 ± 10 | | | |
| [R,S-(E)]-5,5-bis(4-Methoxyphenyl)-N-[1-methyl-4-(5-ethyl-3-pyridinyl)butyl]-2,4-pentadienamid | 900 | | | | |
| [R,S-(E)]-N-[1-Ethyl-4-(3-pyridinyl)butyl]-5,5-bis-(4-methoxphenyl)-2,4-pentadienamid | 90 | 86 ± 8 | 99 ± 1 | | |
| [R-(E)]-N-[1-Ethyl-4-(3-pyridinyl)butyly-5,5-bis-(4-methoxphenyl)-2,4-pentadienamid | 120 | 99 ± 0.4 | 99 ± 1<br>85 ± 12 (8 hr) | | |
| [S-(E)-N-[1-Ethyl-4-(3-pyridinyl)butyl]-5,5-bis-(4-methoxyphenyl)-2,4-pentadienamid | 400 | 44 ± 8 | | | |

| Verbindung | Hemmung der PAF-Bindung $IC_{50}$ (nM) | Hemmung der PAF-induzierten Bronchokonstriktion in % 1 mg/kg i.v. | Hemmung der PAF-induzierten Bronchokonstriktion in % 50 mg/kg p.o. | Hemmung der PAF-induzierten Reduktion der Anzahl Plättchen in % 3 mg/kg i.v. | Hemmung der PAF-induzierten Reduktion der Anzahl Plättchen in % 30 mg/kg p.o |
|---|---|---|---|---|---|
| [R,S](E)-5,5-bis(4-Methoxyphenyl)-N-[1-propyl-4-(3-pyridinyl)butyl]-2,4-pentadienamid | >1000 | 24 ± 13 | | | |
| [R,S-(E)-5,5-bis(4-Methoxyphenyl)-N-[1-(1-methylethyl)-3-pyridinyl)butyl]-2,4-pentadienamid | 800 | 87 ± 7 | 57 ± 19 | | |
| [R,S-(E)-N-[1-Butyl-4-(3-pyridinyl)butyl]-5,5-bis-(4-methoxyphenyl)-2,4-pentadienamid | >1000 | 42 ± 7 | | | |
| [R,S-(E)-N-[1-Cyclopropyl-4-(3-pyridinyl)butyl]-5,5-bis(4-methoxyphenyl)-2,4-pentadienamid | 60 | 98 ± 1 | 99 ± 0.3 | | |
| [R,S-(E)]-N-[1-Cyclopentyl-4-(3-pyridinyl)butyl]-5,5-bis(4-methoxyphenyl)-2,4-pentadienamid | >1000 | 19 ± 14 | | | |
| [R,S-(E)]-N-[1-Cyclohexyl-4-(3-pyridinyl)butyl]-5,5-bis(4-methoxyphenyl)-2,4-pentadienamid | >1000 | 11 ± 15 | | | |
| [R,S-(E)]-N-[1-(4-Bromophenyl)-4-(3-pyridinyl)butyl]-5,5-bis(4-methoxyphenyl)-2,4-pentadienamid | >1000 | 6 ± 13 | | | |
| (E)-5,5-bis(4-Methoxphenyl)-N-[1-[3-(3-pyridinyl)-propyl]-4-(3-pyridinyl)butyl]-2,4-pentadienamid | 500 | 50 ± 15 | | | |
| (E)-5,5-bis(2-Methoxphenyl)-N-[4-(3-pyridinyl)-butyl]-2,4-pentadienamid | 40 | 40 ± 2 | | | |
| (E)-5,5-bis(4-Methylphenyl)-N-[4-(3-pyridinyl)-butyl]-2,4-pentadienamid | 60 | 95 ± 1 | 67 ± 15<br>11 ± 6 (8 hr) | | |
| [R-(E)]-5,5-bis(4-Methylphenyl)-N-[1-methyl-4-(3-pyridinyl)butyl]-2,4-pentadienamid | 250 | 97 ± 3 | 91 ± 7<br>14 ± 16 (8 hr) | | |
| [R-(E,E)]-5-(4-Methoxyphenyl)-N-[1-methyl-4-(3-pyridinyl)butyl]-5-phenyl-2,4-pentadienamid | | 89 ± 4 | 95 ± 1 | | |
| [R-(2E,4Z)-5-(4-Methoxyphenyl)-N-[1-methyl-4-(3-pyridinyl)butyl]-5-phenyl-2,4-pentadienamid | | 74 ± 11 | 0 ± 2 | | |

| Verbindung | Hemmung der PAF-Bindung $IC_{50}$ (nM) | Hemmung der PAF-induzierten Bronchokonstriktion in % 1 mg/kg i.v. | Hemmung der PAF-induzierten Bronchokonstriktion in % 50 mg/kg p.o. | Hemmung der PAF-induzierten Reduktion der Anzahl Plättchen in % 3 mg/kg i.v. | Hemmung der PAF-induzierten Reduktion der Anzahl Plättchen in % 30 mg/kg p.o |
|---|---|---|---|---|---|
| (E,E)-5-(2-Methoxyphenyl)-5-(4-methoxyphenyl)-N-[4-(3-pyridinyl)butyl]-2,4-pentadienamid | 45 | 80 ± 5 | 21 ± 7 | | |
| (2E,4Z)-5-(2-Methoxyphenyl)-5-(4-methoxyphenyl)-N-[4-(3-pyridinyl)butyl]-2,4-pentadienamid | 55 | 36 ± 6 | | | |
| (E,E)-5-(4-Methoxyphenyl)-5-(3-pyridinyl)-N-[4-(3-pyridinyl)butyl]-2,4-pentadienamid | 25 | 97 ± 0.6 | 85 ± 8 | | |
| (2E,4Z)-5-(4-Methoxyphenyl)-5-(3-pyridinyl)-N-[4-(3-pyridinyl)butyl]-2,4-pentadienamid | 50 | 78 ± 10 | 39 ± 8 | | |
| [R-(2E,4Z)-5-(4-Methoxyphenyl)-N-[1-methyl-4-(3-pyridinyl)butyl]-5-(2-thienyl)-2,4-pentadienamid | 16 | 97 ± 1 | 96 ± 2<br>27 ± 6 (6 hr) | | |
| [R-(E,E)]-5-Cyclopropyl-5-(4-methoxyphenyl)-N-[1-methyl-4-(3-pyridinyl)butyl]-2,4-pentadienamid | 50 | 35 ± 5 | | | |
| [R-(2E,4Z)-5-Cyclopropyl-5-(4-methoxyphenyl)-N-[1-methyl-4-(3-pyridinyl)butyl]-2,4-pentadienamid | 70 | 85 ± 4 | 63 ± 19 | | |
| [R-(E,E)]-5-Cyclohexyl-5-(4-methoxyphenyl)-N-[1-methyl-4-(3-pyridinyl)butyl]-2,4-pentadienamid | 20 | 73 ± 17 | 97 ± 1<br>12 ± 10 (6 hr) | | |
| [R-(2E,4Z)]-5-Cyclohexyl-5-(4-methoxyphenyl)-N-[1-methyl-4-(3-pyridinyl)butyl]-2,4-pentadienamid | | 18 ± 12 | | | |
| [R-(E,E)-5-(4-Methoxyphenyl)-N-[1-methyl-4-(3-pyridinyl)butyl]-2,4-pentadienamid | 30 | 17 ± 13 | | | |
| [R-(E,E)]-5-(4-Methoxyphenyl)-N-[1-methyl-4-(3-pyridinyl)butyl]-2,4-hexadienamid | 25 | 42 ± 16 | | | |
| [R-(E,E)]-5-(4-Methoxyphenyl)-N-[1-methyl-4-(3-pyridinyl)butyl]-2,4-heptadienamid | 30 | 72 ± 13 | 84 ± 2 | | |
| [R-(E,E)]-5-(4-Methoxyphenyl)-6-methyl-N-[1-methyl-4-(3-pyridinyl)butyl]-2,4-heptadienamid | 30 | 38 ± 11 | | | |

| Verbindung | Hemmung der PAF-Bindung $IC_{50}$ (nM) | Hemmung der PAF-induzierten Bronchokonstriktion in % 1 mg/kg i.v. | 50 mg/kg p.o. | Hemmung der PAF-induzierten Reduktion der Anzahl Plättchen in % 3 mg/kg i.v. | 30 mg/kg p.o |
|---|---|---|---|---|---|
| [R-(2E,4Z)]-5-(4-Methoxyphenyl)-6-methyl-N-[1-methyl-4-(3-pyridinyl)butyl]-2,4-heptadienamid | 40 | 89 ± 3 | 71 ± 14 | | |
| [R-(E,E)]-5-(4-Methoxyphenyl)-N-[1-methyl-4-(3-pyridinyl)butyl]-2,4-octadienamid | 50 | 86 ± 7 | 9 ± 13 | | |
| [R-(E,E)]-5-(4-Methoxyphenyl)-N-[1-methyl-4-(3-pyridinyl)butyl]-2,4-nonadienamid | 14 | 99 ± 1 | 98 ± 1 | | |
| [R,S-(E,E)]-5-(4-Methoxyhenyl)-N-[1-methyl-4-(3-pyridinyl)butyl]-2,4-nonadienamid | | | | | |
| [R-(E,E)]-5-(3-Methoxyphenyl)-N-[1-methyl-4-(3-pyridinyl)butyl]-2,4-nonadienamid | 15 | 91 ± 5 | | | |
| [R-(2E,4Z)-5-(3-Methoxphenyl)-N-[1-methyl-4-(3-pyridinyl)butyl]-2,4-nonadienamid | | | | | |
| (E,E)-5-(4-Methoxyhenyl)-N-[4-(3-pyridinyl)-butyl]-2,4-decadienamid | 15 | 99 ± 1 | 26 ± 2 | | |
| (2E,4Z)-5-(4-Methoxyphenyl)-N-[4-(3-pyridinyl)-butyl]-2,4-decadienamid | 40 | 68 ± 11 | | | |
| [R-(E,E)-5-(4-Methoxyphenyl)-N-[1-methyl-4-(3-pyridinyl)butyl]-2,4-decadienamid | 40 | 99 ± 1 | 100 ± 0.5<br>71 ± 14 (8 hr) | | |
| [S-(E,E)-5-(4-Methoxyphenyl)-N-[1-methyl-4-(3-pyridinyl)butyl]-2,4-decadienamid | 120 | 46 ± 16 | 25 ± 13 | | |
| [R-(2E,4Z)-5-(4-Methoxyphenyl)-N-[1-methyl-4-(3-pyridinyl)butyl]-2,4-decadienamid | | 57 ± 12 | | | |
| [R-(E,E)]-5-(3-Fluorophenyl)-N-[1-methyl-4-(3-pyridinyl)butyl]-2,4-decadienamid | 30 | 93 ± 0.9 | | | |
| [R-(2E,4Z)-5-(3-Fluorophenyl)-N-[1-methyl-4-(3-pyridinyl)butyl]-2,4-decadienamid | 105 | 93 ± 1 | | | |

| Verbindung | Hemmung der PAF-Bindung $IC_{50}$ (nM) | Hemmung der PAF-induzierten Bronchokonstriktion in % 1 mg/kg i.v. | Hemmung der PAF-induzierten Bronchokonstriktion in % 50 mg/kg p.o. | Hemmung der PAF-induzierten Reduktion der Anzahl Plättchen in % 3 mg/kg i.v. | Hemmung der PAF-induzierten Reduktion der Anzahl Plättchen in % 30 mg/kg p.o |
|---|---|---|---|---|---|
| [R-(E,E)]-5-(4-Fluorophenyl)-N-[1-methyl-4-(3-pyridinyl)butyl]-2,4-decadienamid | 40 | 95 ± 1 | | | |
| [R-(E,E)-5-(3,4-Dimethoxyphenyl)-N-[1-methyl-4-(3-pyridinyl)butyl]-2,4-decadienamid | 6 | 99 ± 0.4 | | | |
| [R,S-(E,E)]-5-(4-Methoxyphenyl)-N-[1-methyl-4-(2-methyl-3-pyridinyl)butyl]-2,4-decadienamid | 5 | 77 ± 5 | | | |
| [R,S-(E,E)]-5-(4-Methoxyphenyl)-N-[(Z)-1-methyl-4-(3-pyridinyl)-3-butenyl]-2,4-decadienamid | | | | | |
| [R-(E,E)]-5-(4-Methoxyphenyl)-N-[1-methyl-4-(3-pyridinyl)butyl]-2,4-undecadienamid | 9 | 100 ± 1 | 96 ± 1 | | |
| [R-(E)]-N-[1-Methyl-4-(3-pyridinyl)butyl]-5-pentyl-2,4-decadienamid | 100 | 78 ± 7 | 4 ± 4 | | |

Die Verbindungen der Formel I und die pharmazeutisch annehmbaren Säureadditionssalze davon können nach an sich bekannten Methoden verabreicht werden. Sie können entweder als Einzelsubstanz oder zusammen mit anderen pharmazeutischen Wirkstoffen verabreicht werden, wobei beispielsweise Antihistamine, Inhibitoren von Vermittlersubstanzen, Methylxanthine, Beta-Agonisten oder antiasthmatische Steroide, wie Prednison und Prednisolon, in Frage kommen. Sie können oral, parenteral, rectal oder durch Inhalation, beispielsweise in Form von Aerosolen, micropulverisierten Pulvern oder Sprühlösungen, verabreicht werden. Für die orale Verabreichung können sie in Form von Tabletten oder Kapseln, beispielsweise als Mischung mit Talk, Stärke, Milchzucker oder anderen inerten Bestandteilen, d.h. pharmazeutisch annehmbaren Trägern, oder in Form von wässrigen Lösungen, Suspensionen, Elixiren oder wässrigalkoholischen Lösungen, verabreicht werden. Sie können beispielsweise noch Zucker oder andere Süssmittel, Aromatisierungsmittel, Färbemittel, Verdickungsmittel und andere herkömmliche pharmazeutische Hilfsstoffe enthalten. Für die parenterale Verabreichung können sie in Form von Lösungen oder Suspensionen, beispielsweise als wässrige oder als Erdnussoellösung oder -suspension verabreicht werden, wobei man die für diese Verabreichungsart herkömmlichen Hilfsstoffe und Träger verwendet. Zwecks Verabreichung als Aerosole können sie in geeignete pharmazeutisch annehmbare Lösungsmittel, beispielsweise Ethylalkohol oder Mischungen von miteinander mischbaren Lösungsmitteln aufgelöst und mit einem pharmazeutisch annehmbaren Treibgas vermischt werden. Derartige Aerosole werden für die Verwendung in Druckbehälter abgefüllt, welche mit einem Aerosolventil versehen sind. Das Aerosol-Ventil ist vorzugsweise ein geeichtes Ventil, d.h. ein Ventil das nach Betätigung eine vorbestimmte Menge der Aerosol-Zusammensetzung freigibt.

Im Rahmen der voliegenden Erfindung muss berücksichtigt werden, dass die zu verabreichende Dosis einer Verbindung der Formel I oder eines Salzes davon und die Häufigkeit der Verabreichung von der Potenz und der Wirkungsdauer der zu verabreichenden Verbindung der Formel I oder des Salzes davon, vom Verabreichungswege sowie von der Stärke der Krankheit, vom Alter des zu behandelnden Säugers und dergleichen abhängt. Im Rahmen der vorliegenden Erfindung liegen orale Dosierungen von Verbindungen der Formel I oder Salzen davon im Bereich von etwa 25 bis etwa 1000 mg pro Tag, vorzugsweise zwischen etwa 25 bis etwa 250 mg, wobei diese Dosierung entweder als Einzeldosis oder in unterteilten Dosen verabreicht werden kann.

Die von der Formel I umfassten geometrisch Isomeren können mittels herkömmlicher Methoden, beispielsweise mittels Chromatographie, Kristallisation und dergleichen, aufgetrennt werden. Es wird allerdings bemerkt, dass die Auftrennung in den meisten Fällen auf einer früheren Stufe der Synthese der Verbindungen der Formel I vorgenommen wird.

Ferner werden diejenigen erfindungsgemässen Verbindungen der Formel I, worin $R_6$ und $R_7$ verschieden sind, üblicherweise als racemische Mischungen erhalten, da sie ein asymmetrisch substituiertes Kohlenstoffatom besitzen. Die Spaltung derartiger Racemate in die optisch aktiven Isomeren kann nach an sich bekannten Methoden durchgeführt werden. Einige racemische Mischungen können als Eutektika ausgefällt und danach aufgetrennt werden. Die chemische Spaltung wird allerdings bevorzugt. In diesem Falle wird die racemische Mischung durch Behandeln mit einem optisch aktiven Spaltungsmittel, z.B. mit einer optisch aktiven Base, wie di-(+)-$\alpha$-methylbenzylamin, das mit einer Carbonsäuregruppe reagieren kann, in eine Mischung von Diastereomeren übergeführt. Die so gebildeten Diastereomeren können mittels fraktionierter Kristallisation getrennt und anschliessend in die entsprechenden optischen Isomeren übergeführt werden. Die vorliegende Erfindung umfasst somit die Racemate von Verbindungen der Formel I und ihre optisch aktiven Isomeren (Enantiomeren).

Die nachfolgenden Beispiele dienen der näheren Erläuterung der vorliegenden Erfindung. Alle Temperaturen sind in Grad Celsius angegeben, sofern nichts anderes erwähnt wird.

## Teil I: Herstellung von Verbindungen der Formel I

### Beispiel 1

Zu einer gerührten Lösung von 5,5-Diphenyl-2,4-pentadiencarbonsäure (6,00 g) und Triethylamin (3,7 ml) in trockenem Tetrahydrofuran (40 ml) gibt man bei 0°C eine Lösung von Ethylchloroformat (2,37 ml) in Tetrahydrofuran (15 ml) tropfenweise so dazu, dass die Reaktionstemperatur bei 0°C gehalten werden kann. Nach Beendigung der Zugabe wird das Reaktionsgemisch während 15 Minuten bei 0°C gerührt, worauf man eine Lösung von 3-Pyridinbutanamine (3,78 g) in Tetrahydrofuran (20 ml) tropfenweise dazu gibt, wobei man die Reaktionstemperatur bei 0°C hält. Man rührt das Gemisch während 1 Stunde bei 0°C und dann während 2 Stunden bei Raumtemperatur und filtriert dann vom ausgefallenen Triethylamin-Hydrochlorid ab. Das Filtrat wird eingedampft, und der Rückstand wird zwischen 1N Salzsäure (80 ml) und Ether (80 ml) verteilt. Die etherische Phase wird verworfen; die wässrige Phase wird mit 4N Natriumhydroxid (30 ml) basisch gestellt und mit Ether (4 x 100 ml) extrahiert. Nach Eindampfen der getrockneten ($K_2CO_3$) organischen Auszüge wird der erhaltene rohe Amid mittels HPLC (Hexan/Essigester/Triethylamin; 25:75:2) gereinigt. Man erhält 3,55 g (E)-5,5-Diphenyl-N-[4-(3-pyridinyl)butyl-2,4-pentadienamid-hydrat (0,2 molar) als Oel. Elementaranalyse berechnet für $C_{26}H_{26}N_2O/0,2H_2O$: C 80,88, H 6,29, N 7,25; Gefunden: C 80,81, H 6,91, N 7,30

Beispiel 2

Eine Lösung von (E)-5,5-Diphenyl-2,4-pentadien carbonsäure-4-nitrophenylester (1,11 g) und (R,S)-α-Methyl-3-pyridinbutanamin (0,6 g) in Tetrahydrofuran (10 ml) wird während 16 Stunden bei Raumtemperatur gerührt. Das Lösungsmittel wird unter vermindertem Druck abgedampft, und eine Lösung des Rückstandes in Methylenchlorid (25 ml) wird mit 0,5N Natriumhydroxid (4x25 ml) gewaschen. Die wässerigen Phasen werden jeweils mit Methylenchlorid (2x25 ml) gewaschen. Die getrockneten ($K_2CO_3$) organischen Auszüge werden eingedampft. Das erhaltene rohe Amid wird mittels HPLC (Essigester) gereinigt. Man erhält 1,2 g [R,S-(E)]-5,5-Diphenyl-N-[1-methyl-4-(3-pyridinyl)butyl-2,4-pentadienamid. Durch Kristallisieren aus Essigester/Hexan erhält man 0,98 g des Amides mit einem Schmelzpunkt von 124,5-125,5°C. Elementaranalyse berechnet für $C_{27}H_{28}N_20$: C 81,78, H 7,12, N 7,06; Gefunden: C 81,84, H 7,18, N 7,08.

Beispiel 3

Wie im Beispiel 2 wird (E)-5,5-Diphenyl-2-methyl-2,4-pentadien- carbonsäure-4-nitrophenylester (1,6 g) während 18 Stunden bei 25 °C mit 3-Pyridinbutanamin (0,63 g) umgesetzt. Das nach dem üblichen Aufarbeiten erhaltene rohe Amid wird mittels HPLC (Essigester) gereinigt und dann aus Ether kristallisiert. Man erhält 1,42 g (E)-5,5-Diphenyl-2-methyl-N-[4-(3-pyridinyl)butyl]-2,4-pentadien -amid mit einem Schmelzpunkt von 93-94°C. Elementaranalyse für : $C_{27}H_{28}N_20$: C 81,78, H 7,12 N 7,06; Gefunden: C 81,74, H 7,02, N 7,01.

Beispiel 4

Wie im Beispiel 2 wird eine Lösung von (E)-5,5-bis(3-Fluorphenyl-2,4-pentadiencarbonsäure-4-nitrophenylester (25,85 g) und 3-Pyridinbutanamin (10 g) in Tetrahydrofuran (100 ml) während 18 Stunden bei Raumtemperatur gerührt und dann in üblicher Weise aufgearbeitet. Das Rohprodukt wird mittels HPLC (Essigester) gereinigt und dann aus Essigester/Hexan kristallisiert. Man erhält 23,9 g (E)-5,5-bis(3-Fluorphenyl)-N-[4-(3-pyridinyl)butyl]-2,4-pentadienamid mit einem Schmelzpunkt von 103-104° C. Elementaranalyse für $C_{26}H_{24}F_2N_20$: C 74,62, H 5,78, F 9,08, N 6,69; Gefunden: C 74,35, H 5,99, F 8,93, N 6,59.

Beispiel 5

Wie im Beispiel 2 wird eine Lösung von (E)-5,5-bis(3-Fluorphenyl)-2,4-pentadiencarbonsäure-4-nitrophenyl ester (6,34 g) und (R,S)-α-Methyl-3-pyridinbutanamin (2,8 g) in Tetrahydrofuran (100 ml) während 18 Stunden bei Raumtemperatur gerührt und dann in üblicher Weise aufgearbeitet. Das rohe Amid wird mittels HPLC (Essigester) gereinigt und dann aus Essigester/ Hexan kristallisiert. Man erhält 3,35 g [R,S-(E)]-5,5-bis(3-Fluorphenyl)-N-[1-methyl-4-(3-pyridinyl)butyl]2,4-pentadienamid mit einem Schmelzpunkt von 103-106°C.

Elementaranalyse für $C_{27}H_{26}F_2N_20$: C 74,98, H 6,06, F 8,79, N 6,48; Gefunden: C 74,50, H 5,99, F 8,66, N 6,15.

Beispiel 6

Wie im Beispiel 2 wird eine Lösung von (E)-5,5-bis(3-Fluorphenyl)-2,4-pentadiencarbonsäure-4-nitrophenyl ester (2,04 g) und (R)-α-Methyl-3-pyridinbutanamin (0,82 g) in Tetrahydrofuran (15 ml) während 18 Stunden bei Raumtemperatur gerührt und dann in üblicher Weise aufgearbeitet. Das rohe Amid wird mittels HPLC (Essigester) gereinigt und dann aus Essigester/Hexan kristallisiert. Man erhält 1,8 g [R-(E)]-5,5-bis(3-Fluorphenyl)-N-[1-methyl-4-(3-pyridinyl)butyl]-2,4-pentadienamid mit einem Schmelzpunkt von 137-139°C; $[\alpha]_D^{25}$ +30. 64° (C 1,0; MeOH). Elementaranalyse für $C_{27}H_{26}F_2N_20$: C 74,98, H 6,06, F 8.79, N 6,48; Gefunden: C 74,96, H 6,17, F 8,48, N 6,44.

Beispiel 7

Wie im Beispiel 2 wird eine Lösung von (E)-5,5-bis(3-Fluorphenyl)-2.4-pentadiencarbonsäure-4-nitrophenyl ester (0,815 g) und (S)-α-Methyl-3-pyridinbutanamin (0,33 g) in Tetrahydrofuran (10 ml) während 18 Stunden bei Raumtemperatur gerührt und dann in üblicher Weise aufgearbeitet. Das rohe Amid wird mittels HPLC (Essigester) gereinigt und dann aus Essigester/Hexan kristallisiert. Man erhält 0,8 g [S-(E)]-5,5-bis(3-Fluorphenyl)-N-[1-methyl-4-(3-pyridinyl)butyl] 2,4-pentadienamid mit einem Schmelzpunkt von 135-136°C;

$[\alpha]_D^{25}$ -29,5° (c 1,0; MeOH). Elementaranalyse für $C_{27}H_{26}F_2N_20$ : C 74,98, H 6,06, F 8,79, N 6.48; Gefunden: C 75,06, H 6.03, F 8,46, N 6,49.

Beispiel 8

Wie im Beispiel 2 wird eine Lösung von (E)-5,5-bis(3-Fluorphenyl) -2,4-pentadiencarbonsäure-4-nitrophenylester (1,63 g) und α,α-Dimethyl-3-pyridinbutanamin (0,9 ml) in Tetrahydrofuran (20 ml) während 29 Stunden unter Rückfluss gerührt und dann in üblicher Weise aufgearbeitet. Das rohe Amid wird aus Essigester/Hexan kristallisiert. Man erhält 1,6 g (E)-5,5-bis(3-Fluorphenyl)-N-[1,1-dimethyl-4-(3-pyridinyl)-butyl]-2,4-pentadienamid mit einem Schmelzpunkt von 135-136° C. Elementaranalyse für $C_{28}H_{28}F_2N_20$ : C 75,32, H 6.32, F 8,51, N 6,27; Gefunden: C 75,30, H 6, 49, F 8,21, N 6,30.

Beispiel 9

Wie im Beispiel 2 wird eine Lösung von (E)-5,5-bis(4-Fluorphenyl)-2,4-pentadiencarbonsäure-4-nitrophenyl ester (15 g) und 3-Pyridinbutamin (5,53 g) in Tetrahydrofuran (60 ml) während 1,5 Stunden bei Raumtemperatur gerührt und dann in üblicher Weise aufgearbeitet. Das rohe Amid wird aus Essigester/Hexan (2x) kristallisiert. Man erhält 14,45 g (E)-5,5-bis(4-Fluorphenyl-N-[4-(3-pyridinyl)butyl]-2,4-pentadienamid mit einem Schmelzpunkt von 155-156° C. Elementaranalyse für $C_{26}H_{24}F_2N_20$: C 74,62, H 5,78, F 9,08, N 6.69; Gefunden: C 74,55, H 5,87, F 9,33, N 6,60

Beispiel 10

Wie im Beispiel 2 wird eine Lösung von (E)-5,5-bis(4-Fluorphenyl)-2,4-pentadiencarbonsäure-4-nitrophenyl ester (2,04 g) und (R)-α-Methyl-3-pyridinbutanamin (0,82 g) in Tetrahydrofuran (15 ml) während 18 Stunden bei Raumtemperatur gerührt und dann in üblicher Weise aufgearbeitet. Das rohe Amid wird mittels HPLC (Essigester) gereinigt und dann aus Ether/Hexan kristallisiert. Man erhält 1,8 g [R-(E)]-5,5-bis(4-Fluorphenyl)-N-[1-methyl-4-(3-pyridinyl)butyl]-2,4-pentadienamid mit einem Schmelzpunkt von 160-162° C. Eine Probe wird aus Essigester/Hexan umkristallisiert, wobei man eine analytisch reine Probe mit einem Schmelzpunkt von 160,5-161,5° C erhält; $[\alpha]_D^{25}$ +32,15° (C 1,0; MeOH). Elementaranalyse für $C_{27}H_{26}F_2N_20$: C 74,98, H 6,06, F 8,79, N 6,48; Gefunden: C 74,87, H 6,08, F 8,49, N 6,46.

Beispiel 11

Wie im Beispiel 2 wird eine Lösung von (E)-5,5-bis(4-Fluorphenyl)-2,4-pentadiencarbonsäure-4-nitrophenyl ester (0,815 g) und (S)-α-Methyl-3-pyridinbutanamin (0.33 g) in Tetrahydrofuran (10 ml) während 18 Stunden bei Raumtemperatur gerührt und dann in üblicher Weise aufgearbeitet. Das rohe Amid wird mittels HPLC (Essigester) gereinigt und dann aus Essigester/Hexan kristallisiert. Man erhält 0,8 g [S-(E)]-5,5-bis(4-Fluorphenyl)-N-[1-methyl-4-(3-pyridinyl)butyl]-2,4-pentadienamid mit einem Schmelzpunkt von 155-156° C; $[\alpha]_D^{25}$ -30,2° (C 1,0; (MeOH). Elementaranalyse für $C_{27}H_{26}F_2N_20$: C 74,98, H 6,06, F 8,79, N 6,48; Gefunden: C 75,06, H 6,03, F 8,46, N 6,49.

Beispiel 12

Wie im Beispiel 2 wird eine Lösung von (E)-5,5-bis(4-Fluorphenyl)-2,4-pentadiencarbonsäure-4-nitrophenyl ester (1,63 g) und α,α-Dimethyl-3-pyridinbutanamin (0,9 ml) in Tetrahydrofuran (20 ml) während 24 Stunden unter Rückfluss gerührt. Das Rohprodukt wird in üblicher Weise isoliert und dann aus Essigester kristallisiert. Man erhält 1,6 g (E)-5,5-bis(4-Fluorphenyl)-N-[1,1-dimethyl-4-(3-pyridinyl)butyl]-2,4-pentadienamid mit einem Schmelzpunkt von 163,5-164,5° C. Elementaranalyse für $C_{28}H_{28}F_2N_20$: C 75,32, H 6,32, F 8,51, N 6,27; Gefunden: C 75,23, H 6,48, F 8,39, N 6,22.

Beispiel 13

Wie im Beispiel 2 wird eine Lösung von (E)-5,5-bis(3-Methoxyphenyl)-2,4-pentadiencarbonsäure-4-nitrophenyl ester (32,36 g) und 3-Pyridinbutanamin (11,4 g) in Tetrahydrofuran (120 ml) während 18 Stunden bei Raumtemperatur gerührt und dann in üblicher Weise aufgearbeitet. Das Rohprodukt wird mittels HPLC (Essigester) gereinigt und dann mit Ether/Hexan behandelt. Man erhält 29,6 g (E)-5,5-bis(3-Methoxyphenyl)-N-[4-(3-pyridinyl)butyl]-2,4-pentadienamid mit einem Schmelzpunkt von 84-86° C. Nach

Umkristallisieren des Produktes aus Essigester/Ether erhält man 29,0 g des Amides mit einem Schmelzpunkt von 86-88 °C. Elementaranalyse für $C_{28}H_{30}N_20$: C 75,99, H 6,83, N 6,33; Gefunden: C 75,85, H 6,84, N 6,27.

Beispiel 14

Wie im Beispiel 2 wird eine Lösung von (E)-5,5-bis(3-Metoxyphenyl)-2,4-pentadiencarbonsäure-4-nitrophenyl ester (3,0 g) und 3-Pyridinhexanamin (1,25 g) in Tetrahydrofuran (20 ml) während 1,5 Stunden bei Raumtemperatur gerührt und dann in üblicher Weise aufgearbeitet. Das Produkt wird mittels HPLC (Essigester) gereinigt und dann mit Ether behandelt. Man erhält 2,5 g (E)-5,5-bis(3-Methoxyphenyl)-N-[6-(3-pyridinyl)hexyl]-2,4-pendadienamid mit einem Schmelzpunkt von 66-70 °C. Durch Umkristallisieren einer Probe aus Essigester/Hexan erhält man reines Amid mit einem Schmelzpunkt von 71-72 °C. Elementaranalyse für $C_{30}H_{34}N_2O_3$: C 76,57, H 7,28, N 5,98; Gefunden: C 76,39, H 7,28, N 5,90.

Beispiel 15

Wie im Beispiel 2 wird eine Lösung von (E)-5,5-bis(3-Methoxyphenyl)-2,4-pentadien carbonsäure-4-nitrophenylester (3,1 g) und (R,S)-α-Methyl-3-pyridinbutanamin (1,4 g) in Tetrahydrofuran (25 ml) während 18 Stunden bei Raumtemperatur gerührt und dann in üblicher Weise aufgearbeitet. Das Rohprodukt wird mittels HPLC (Essigester) gereinigt und dann aus Ether/Hexan kristallisiert. Man erhält 2,6 g [R,S-(E)]-5,5-bis(3-Methoxyphenyl)-N-[1-methyl-4-(3-pyridinyl)-butyl]-2,4-pentadienamid mit einem Schmelzpunkt von 95-97 °C. Eine Umkristallisation aus den gleichen Lösungsmitteln änderte den Schmelzpunkt nicht. Elementaranalyse für $C_{29}H_{32}N_2O_3$: C 76,29, H 7,06, N 6,13; Gefunden: C 75,99, H 6,98, N 5,87.

Beispiel 16

Wie im Beispiel 2 wird eine Lösung von (E)-5,5-bis(3-Methoxyphenyl)-2,4-pendadien carbonsäure-4-nitrophenylester (2,16 g) und (R)-α-Methyl-3-pyridinbutanamin (0,82 g) in Tetrahydrofuran (15 ml) während 67 Stunden bei Raumtemperatur gerührt. Das rohe Amid wird in üblicher Weise isoliert und mittels HPLC (Essigester) gereinigt und dann aus Essigester/Hexan kristallisiert. Man erhält 1,9 g [R-(E)]-5,5-bis(3-Methoxyphenyl)-N-[1-methyl-4-(3-pyridinyl) butyl]-2,4-pentadienamid mit einem Schmelzpunkt von 109-110 °C; $[\alpha]_D^{25}$ +27,7 ° (C 1,0; MeOH). Elementaranalyse für $C_{29}H_{32}N_2O_3$: C 76,29, H 7,07, N 6,13; Gefunden: C 76,41, H 6,86, N 6,11.

Beispiel 17

Wie im Beispiel 2 wird eine Lösung von (E)-5,5-bis(3-Methoxyphenyl)-2,4-pentadien carbonsäure-4-nitrophenylester (0,863 g) und (S)-α-Methyl-3-pyridinbutanamin (0,33 g) in Tetrahydrofuran (10 ml) während 18 Stunden bei Raumtemperatur gerührt und dann in üblicher Weise aufgearbeitet. Das rohe Amid wird mittels HPLC (Essigester) gereinigt und dann aus Essigester/Hexan kristallisiert. Man erhält 0,8 g [S-(E)]-5,5-bis(3-Methoxyphenyl)-N-[1-methyl-4-(3-pyridinyl)butyl]-2,4-pentadienamid mit einem Schmelzpunkt von 105-108 °C; $[\alpha]_D^{25}$ -26.2 °C (C 1,0; MeOH). Elementaranalyse für $C_{29}H_{32}N_2O_3$: C 76,29, H 7,06, N 6,14; Gefunden: C 76,33, H 7,08, N 6,12.

Beispiel 18

Wie im Beispiel 2 wird eine Lösung von (E)-5,5-bis(3-Methoxyphenyl)-2,4-pentadien carbonsäure-4-nitrophenylester (1,73 g) und α,α-Dimethyl-3-pyridinbutanamin (0,9 ml) in Tetrahydrofuran (20 ml) während 30 Stunden unter Rückfluss gerührt. Nach dem üblichen Aufarbeiten wird das Produkt aus Essigester/Hexan kristallisiert. Man erhält 1,6 g (E)-5,5-bis(3-Methoxyphenyl)-N-[1,1-dimethyl-4-(3-pyridinyl)butyl]-2,4-pentadienamid mit einem Schmelzpunkt von 120-121 °C. Elementaranalyse für $C_{30}H_{34}N_2O_3$: C 76,57, H 7,28, N 5.95; Gefunden: C 76,67, H 7,44, N 5,88.

Beispiel 19

Wie im Beispiel 2 wird eine Lösung von (E)-5,5-bis(4-Methoxyphenyl)-2,4-pentadien carbonsäure-4-nitrophenylester (4,3 g) und 3-Pyridinbutanamin (1.51 g) in Tetrahydrofuran (20 ml) während 1,5 Stunden bei Raumtemperatur gerührt und dann in üblicher Weise aufgearbeitet. Das rohe Amid wird aus

Essigester/Hexan kristallisiert. Man erhält 3,6 g (E)-5,5-bis(4-Methoxyphenyl)-N-[4-(3-pyridinyl)butyl]-2,4-penta-dienamid mit einem Schmelzpunkt von 76-77°C. Elementaranalyse für $C_{28}H_{30}N_2O_3$: C 75,99, H 6,83, N 6,33; Gefunden: C 76,36, H 6,97, N 6,35.

Beispiel 20

Wie im Beispiel 2 wird eine Lösung von (E)-5,5-bis(4-Methoxyphenyl)-2,4-pentadien carbonsäure-4-nitrophenylester (2,16 g) und (R,S)-α-Methyl-3-pyridinbutanamin (0,82 g) in Tetrahydrofuran (15 ml) über Nacht bei Raumtemperatur gerührt und dann in üblicher Weise aufgearbeitet. Das rohe Amid wird mittels HPLC (Essigester) gereinigt und dann aus Benzol lyophilisiert. Man erhält 2,3 g [R,S-(E)]-5,5-bis(4-Methoxyphenyl)-N-[1-methyl-4-(3-pyridinyl) butyl]-2,4-pentadienamid-hydrat (0,25 molar) als amorphen Festkörper. Elementaranalyse für $C_{29}H_{32}N_2O_3/0,25\ H_2O$: C 75,54, H 7,10, N 6,07; Gefunden: C 75,51, H 6,94, N 6,04.

Beispiel 21

Wie im Beispiel 2 wird eine Lösung von (E)-5,5-bis(4-Methoxyphenyl)-2,4-pentadiencarbonsäure-4-nitrophenyl ester (2,16 g) und (R)-α-Methyl-3-pyridinbutanamin (0,82 g) in Tetrahydrofuran (15 ml) während 18 Stunden bei Raumtemperatur gerührt und dann in üblicher Weise aufgearbeitet. Das Rohprodukt wird mittels HPLC (Essigester) gereinigt und dann aus Benzol lyophilisiert. Man erhält 2,3 g [R-(E)]-5,5-bis(4-Methoxyphenyl)-N-[1-methyl-4-(3-pyridinyl)butyl]-2,4-pentadienamid-hydrat (0,4 molar) als amorphen Festkörper mit einem Schmelzpunkt von 50-60°C; $[\alpha]_D^{25}$ +27,7° (C 1,0; MeOH). Elementaranalyse für $C_{29}H_{32}N_2O_3/0,4\ H_2O$: C 75,10, H 7,13, N 6,04; Gefunden: C 75,03, H 7,10, N 5,89.

Beispiel 22

Wie im Beispiel 2 wird ein Lösung von (E)-5,5-bis(4-Methoxyphenyl)-2,4-pentadien carbonsäure-4-nitrophenylester (0,863 g) und (S)-α-Methyl-3-pyridinbutanamin (0,33 g) in Tetrahydrofuran (10 ml) während 18 Stunden bei Raumtemperatur gerührt und dann in üblicher Weise aufgearbeitet. Das rohe Amid wird mittels HPLC (Essigester) gereinigt und dann aus Benzol lyophilisiert. Man erhält 0,9 g [S-(E)]-5,5-bis(4-Methoxyphenyl)-N-[1-methyl-4-(3-pyridinyl)butyl]-2,4-pentadienamid als amorphen Festkörper mit einem Schmelzpunkt von 60-70°C; $[\alpha]_D^{25}$ +27,7° (C 1,0; MeOH). Elementaranalyse für $C_{29}H_{32}N_2O_3$: C 76,29, H 7,06, N 6,14; Gefunden: C 76,00, H 7,20, N 6,03.

Beispiel 23

Wie im Beispiel 2 wird eine Lösung von (E)-5,5-bis(4-Methoxyphenyl)-2,4-pentadien carbonsäure-4-nitrophenylester (1,73 g) und α,α-Dimethyl-3-pyridinbutanamine (0,9 5 ml) in Tetrahydrofuran (20 ml) während 20 Stunden bei Raumtemperatur und dann während weiterer 24 Stunden unter Rückfluss gerührt, um die Reaktion zu vervollständigen. Nach dem üblichen Aufarbeiten wird das rohe Amid aus Essigester/Hexan kristallisiert (2x). Man erhält 1,55 g (E)-5,5-bis(4-Methoxyphenyl)-N-[1,1-dimethyl-4-(3-pyridinyl)butyl]-2,4-pentadienamid mit einem Schmelzpunkt von 144-145°C. Elementaranalyse für $C_{30}H_{34}N_2O_3$: C 76,57, H 7,28, N 5,95; Gefunden: C 76,70, H 7,48, N 6,20.

Beispiel 24

Wie im Beispiel 2 wird eine Lösung von (E)-5,5-bis(3,4-Dimethoxyphenyl)-2,4-pentadien carbonsäure-4-nitrophenylester (4,9 g) und 3-Pyridinbutanamin (1,5 g) in Tetrahydrofuran (25 ml) während 17 Stunden bei Raumtemperatur gerührt und dann in üblicher Weise aufgearbeitet. Das rohe Amid wird aus Essigester/Hexan kristallisiert. Man erhält 3,6 g (E)-5,5-bis(3,4-Dimethoxyphenyl)-N-[4-(3-pyridinyl)butyl]-2,4-pentadienamid mit einem Schmelzpunkt von 145-146°C. Elementaranalyse für $C_{30}H_{34}N_2O_5$: C 71,69, H 6,82, N 5,57; Gefunden: C 71,33, H 6,93, N 5,72.

Beispiel 25

Wie in Beispiel 2 wird eine Lösung von (E)-5,5-bis(3,4-Dimethoxyphenyl)-2,4-pentadien carbonsäure-4-nitrophenylester (2,46 g) und (R)-α-Methyl-3-pyridinbutanamin (0,82 g) in Tetrahydrofuran (15 ml) über Nacht bei Raumtemperatur gerührt und dann in üblicher Weise aufgearbeitet. Das rohe Amid wird mittels

HPLC (Essigester) gereinigt und dann aus Ether kristallisiert. Man erhält 2,3 g [R-(E)] 5,5-bis(3,4-Dimethoxyphenyl)-N-[1-methyl-4-(3-pyridinyl)butyl]-2,4-pentadienamid mit einem Schmelzpunkt von 140-141 °C. Eine Probe wird aus Essigester umkristallisiert, wobei man eine analytisch reine Probe mit einem Schmelzpunkt von 140,5-141,5 °C erhält; $[\alpha]_D^{25}$ +26,01 ° (C 1,0; MeOH). Elementaranalyse für $C_{31}H_{36}N_2O_5$: C 72,07, H 7,02, N 5,42; Gefunden: C 71,88, H 7,19, N 5,36.

Beispiel 26

Wie in Beispiel 2 wird eine Lösung von (E)-5,5-bis(3-Chlorphenyl)-2,4-pentadiencarbonsäure-4-nitrophenyl ester (6,4 g) und 3-Pyridinbutanamin (2,7 g) in Tetrahydrofuran (75 ml) während 40 Minuten bei 40 °C gerührt und dann in üblicher Weise aufgearbeitet. Das rohe Amid wird mittels HPLC (Essigester) gereinigt und dann aus Essigester/Hexan kristallisiert. Man erhält 5,1 g (E)-5,5-bis(3-Chlorphenyl)-N-[4-(3-pyridinyl)-butyl]-2,4-pentadien amid mit einem Schmelzpunkt von 100-102 °C. Elementaranalyse für $C_{26}H_{24}Cl_2N_2O$: C 69,18, H 5,36, Cl 15,71, N 6,21; Gefunden: C 69,20, H 5,50, Cl 15,61, N 6,19.

Beispiel 27

Wie in Beispiel 2 wird eine Lösung von (E)-5,5-bis(4-Chlorphenyl)-2,4-pentadiencarbonsäure-4-nitrophenyl ester (6,35 g) und 3-Pyridinbutanamin (2,7 g) in Tetrahydrofuran (75 ml) während 2 Stunden bei 25 °C gerührt und dann in üblicher Weise aufgearbeitet. Das Rohprodukt wird mittels HPLC (Essigester) gereinigt und dann aus Essigester/Hexan kristallisiert. Man erhält 5,3 g (E)-5,5-bis(4-Chlorphenyl)-N-[4-(3-pyridinyl)-butyl]-2,4-pentadienamid mit einem Schmelzpunkt von 146-147 °C. Elementaranalyse für $C_{26}H_{24}Cl_2N_2O$: C 69,18, H 5,36, Cl 15,71, N 6,21; Gefunden: C 69.00, H 5,57, Cl 15,71, N 6,21.

Beispiel 28

Wie in Beispiel 2 wird eine Lösung von (E)-5,5-bis[2-(Trifluormethyl)phenyl]-2,4-pentadien carbonsäure-4-nitrophenylester (1,45 g) und 3-Pyridinbutanamin (0,44 g) in Tetrahydrofuran (10 ml) während einer Stunde bei 25 °C gerührt und dann in üblicher Weise aufgearbeitet. Das Rohprodukt wird auf eine mit Methylenchlorid hergestellte Kieselgelsäule (12 g) gegeben, worauf das Amid mit Essigester eluiert wird. Das erhaltene Material wird aus Essigester/Hexan kristallisiert. Man erhält 1,01 g (E)-5,5-bis[2-(Trifluormethyl)phenyl]-N-[4-(3-pyridinyl)butyl]-2,4-pentadienamid mit einem Schmelzpunkt von 110-112 °C. Elementaranalyse für $C_{28}H_{24}F_6N_2O$: C 64,86, H 4,67, F 21,98, N 5,40; Gefunden: C 64,74, H 4,94, F 21,82, N 5,37.

Beispiel 29

Wie in Beispiel 2 wird eine Lösung von (E)-5,5-bis[3-(Trifluormethyl)phenyl]-2,4-pentadien carbonsäure-4-nitrophenylester (4,8 g) und 3-Pyridinbutanamin (1,5 g) in Tetrahydrofuran (20 ml) während 2 Stunden bei Raumtemperatur gerührt und dann in üblicher Weise aufgearbeitet. Das Rohprodukt wird mittels HPLC (Essigester) gereinigt und dann aus Ether/Hexan kristallisiert. Man erhält 3,2 g (E)-5,5-bis[3-(Trifluormethyl)-phenyl]-N-[4-(3-pyridinyl)butyl]-2,4-pentadienamid mit einem Schmelzpunkt von 89-90 °C. Elementaranalyse für $C_{28}H_{24}F_6N_2O$: C 64,86, H 4,67, F 21,98, N 5,40; Gefunden: C 64,82, H 4,72, F 22,19, N 5,38.

Beispiel 30

Wie in Beispiel 2 wird eine Lösung von (E)-5,5-bis(3-Nitrophenyl)-2,4-pentadiencarbonsäure-4-nitrophenyl ester (0,950 g) und 3-Pyridinbutanamin (0,34 g) in Tetrahydrofuran (10 ml) während 1 Stunde bei Raumtemperatur gerührt. Das nach dem üblichen Aufarbeiten erhaltene rohe Amid wird aus Essigester/Hexan kristallisiert. Man erhält 0,82 g (E)-5,5-bis(3-Nitrophenyl)-N-[4-[3-pyridinyl)butyl)-2,4-pentadienamid mit einem Schmelzpunkt von 158-159 °C. Elementaranalyse für $C_{26}H_{24}N_4O_5$: C 66,09, H 5,12, N 11,86; Gefunden C 65,91, H 5,34, N 11,82.

Beispiel 31

Wie in Beispiel 2 wird eine Lösung von (2E,4E)-5-(3-Methoxyphenyl)-5-phenyl-2,4-pentadien carbonsäure-4-nitrophenylester (2,96 g) und 3-Pyridinbutanamin (1,107 g) in Tetrahydrofuran (15 ml) während 1 Stunde bei Raumtemperatur gerührt. Nach dem üblichen Aufarbeiten wird das rohe Amid mittels

HPLC (Essigester) gereinigt und dann aus Essigester/Hexan kristallisiert. Man erhält 2,5 g (2E,4E)-5-3-(Methoxyphenyl)-5-phenyl-N-[4-(3-pyridinyl)butyl]-2,4-pentadienamid mit einem Schmelzpunkt von 66-68 °C. Elementaranalyse für $C_{28}H_{28}N_2O_2$: C 78,61, H 6,84, N 6,79; Gefunden: C 78,42, H 6,88, N 6,79.

Beispiel 32

Wie in Beispiel 2 wird eine Lösung von (2E,4E)-5-(3-Fluorphenyl)-5-(3-methoxyphenyl)-2,4-pentadien carbonsäure-4-nitrophenylester (1,8 g) und 3-Pyridinbutanamin (0,650 g) in Tetrahydrofuran (20 ml) während 17 Stunden bei Raumtemperatur gerührt. Das in üblicher Weise isolierte rohe Amid wird mittels HPLC (Essigester) gereinigt und dann aus Ether/Hexan kristallisiert. Man erhält 1,6 g (2E,4E)-5-(3-Fluorphenyl)-5-(3-methoxyphenyl)-N-[4-(3-pyridinyl)butyl]-2,4-pentadienamid mit einem Schmelzpunkt von 55-60 °C. Eine Probe wird aus Essigester/ Hexan umkristallisiert, wobei man eine analytisch reine Probe mit einem Schmelzpunkt von 58-60 °C erhält. Elementaranalyse für $C_{27}H_{27}FN_2O$: C 75,33, H 6,32, F 4,41, N 6,51; Gefunden: C 75,31, H 6,34, F 4,38, N 6,47.

Beispiel 33

Wie in Beispiel 2 wird eine Lösung von (E)-5,5-bis[(1,1′-Biphenyl)-4-yl]-2,4-pentadien carbonsäure-4-nitrophenylester (0,880 g) und 3-Pyridinbutanamin (0,251 g) in Tetrahydrofuran (5 ml) während 1 Stunde bei Raumtemperatur gerührt und dann in üblicher Weise aufgearbeitet. Das Rohprodukt wird über Kieselgel (7g) chromatographiert, wobei man mit Essigester eluiert. Man erhält 0,65 g eines Oeles, das man aus Benzol lyophilisiert. Man erhält (E)-5,5-bis[(1,1′-Biphenyl)-4-yl]-N-[4-(3-pyridinyl)butyl]-2,4-pentadienamid-Hydrat (0,15 molar) als amorphes Pulver. Elementaranalyse für $C_{38}H_{34}N_2O/O.15H_2O$: C 84,93, H 6,43, N 5,21; Gefunden: C 84,68, H 6,38, N 5,28.

Beispiel 34

Wie in Beispiel 1 wird das aus (Z)-5,5-Diphenyl-2,4-pentadien carbonsäure (2,75 g), Triethylamin (1,68 ml) und Ethylchloroformat (1,08 ml) in Tetrahydrofuran (35 ml) hergestellte gemischte Anhydrid mit 3-Pyridinbutanamin (1,73 g) behandelt. Nach dem üblichen Aufarbeiten wird das rohe Amid mittels HPLC (Hexan/Essigester/Triethylamin; 27:75:2) gereinigt. Man erhält 2,5 g (Z)-5,5-Diphenyl-N-[4-(3-pyridinyl)butyl]-2,4-pentadienamid. Nach Kristallisieren aus Ether erhält man 2,07 g des Amids mit einem Schmelzpunkt von 87-89 °C. Elementaranalyse für $C_{26}H_{26}N_2O$: C 81,64, H 6,85, N 7,32; Gefunden: C 81,59, H 6,99, N 7,36.

Beispiel 35

Wie in Beispiel 2 wird eine Lösung von (Z)-5,5-bis(3-Methoxyphenyl)-2,4-pentadien carbonsäure-4-nitrophenylester (1,2 g) und 3-Pyridinbutanamin (0,42 g) in Tetrahydrofuran (7 ml) während einer Stunde bei Raumtemperatur gerührt. Das nach dem üblichen Aufarbeiten isolierte rohe Amid ist noch mit einer kleinen Menge des entsprechenden (E)-Isomeren verunreinigt. Diese Mischung wird mittels HPLC (Essigester) aufgetrennt und das gewünschte Produkt wird aus Ether (2x) kristallisiert. Man erhält 0,84 g (Z)-5,5-bis(3-Methoxyphenyl)-N-[4-(3-pyridinyl)butyl]-2,4-pentadienamid mit einem Schmelzpunkt von 69-73 °C. Elementaranalyse für $C_{28}H_{30}N_2O_3$: C 75,99, H 6,83, N 6,33; Gefunden: C 75,79, H 6,70, N 6,39.

Beispiel 36

Wie in Beispiel 2 wird eine Lösung von (E)-5,5-bis(4-Methoxyphenyl)-2,4-pentadien carbonsäure-4-nitrophenylester (1,72 g) und 2-Pyridinpentanamin (0,657 g) in Tetrahydrofuran (25 ml) während 17 Stunden bei Raumtemperatur gerührt und dann in üblicher Weise aufgearbeitet. Das Rohprodukt wird mittels HPLC (Essigester) gereinigt und dann aus Essigester kristallisiert. Man erhält 4,6 g (E)-5,5-bis(4-Methoxyphenyl)-N-[5-(2-pyridinyl)pentyl]-2,4-pentadienamid mit einem Schmelzpunkt von 99-100 °C. Elementaranalyse für $C_{29}H_{32}N_2O_3$: C 76,29, H 7,06, N 6,14; Gefunden: C 76,57, H 7,06, N 6,14.

Beispiel 37

Wie in Beispiel 2 wird eine Lösung von (E)-5,5-bis(4-Methoxyphenyl)-2,4-pentadien carbonsäure-4-nitrophenylester (0,647 g) und 4-Pyridinpropanamin (0,204 g) in Tetrahydrofuran (5 ml) während 3 Stunden

bei Raumtemperatur gerührt. Nach dem üblichen Aufarbeiten wird das Rohprodukt mittels HPLC (Essigester) gereinigt und dann aus Essigester kristallisiert. Man erhält 0,595 g (E)-5,5-bis(4-Methoxyphenyl)-N-[3-(4-pyridinyl)propyl]-2,4-pentadienamid mit einem Schmelzpunkt von 90-92° C. Elementaranalyse für $C_{27}H_{28}N_2O_3$: C 75,68, H 6,59, N 6,54; Gefunden: C 75,65, H 6,74, N 6,58.

Beispiel 38

Wie in Beispiel 2 wird eine Lösung von (E)-5,5-bis(4-Methoxyphenyl)-2,4-pentadien carbonsäure-4-nitrophenylester (1,1 g) und 2-[(3-Pyridinyl)methoxy]ethanamin (0,43 g) in Tetrahydrofuran (20 ml) während 17 Stunden bei Raumtemperatur gerührt und dann in üblicher Weise aufgearbeitet. Das Rohprodukt wird mittels HPLC (Essigester) gereinigt und dann aus Benzol lyophilisiert. Man erhält 0,903 g (E)-5,5-bis(4-Methoxyphenyl)-N-[2-(3-pyridinylmethoxy)ethyl]-2,4-pentadienamid als amorphen Festkörper. Elementaranalyse für $C_{27}N_{28}N_2O_4$: C 72,95, H 6,35, N 6,30; Gefunden: C 72,64, H 6,59, N 5,99.

Beispiel 39

Wie in Beispiel 2 wird eine Lösung von (E)-5,5-bis(4-Methoxyphenyl)-2,4-pentadien carbonsäure-4-nitrophenylester (1,0 g) und 3-(3-Pyridinyloxy)propanamin (0,4 g) in Tetrahydrofuran (10 ml) über Nacht bei Raumtemperatur gerührt. Nach dem üblichen Aufarbeiten wird das Rohprodukt aus Essigester/Hexan kristallisiert. Man erhält 0,85 g (E)-5,5-bis(4-Methoxyphenyl)-N-(3-[(3-pyridinyl)oxy]propyl]-2,4-pentadienamid mit einem Schmelzpunkt von 146-147° C. Elementaranalyse für $C_{27}H_{28}N_2O_4$: C 72,95, H 6,35, N 6,39; Gefunden: C 72,85, H 6,50, N 6,35.

Beispiel 40

Wie in Beispiel 2 wird eine Lösung von (E)-5,5-bis(4-Methoxyphenyl)-2,4-pentadien carbonsäure-4-nitrophenylester (0,841 g) und 4-Isochinolinbutanamin (0,39 g) in Tetrahydrofuran (8 ml) während 17 Stunden bei Raumtemperatur gerührt und dann in üblicher Weise aufgearbeitet. Das Rohprodukt wird mittels HPLC (Essigester/Hexan; 3:1) gereinigt und dann aus Essigester/Hexan kristallisiert. Man erhält 0,284 g (E)-N-[4-(4-Isochinolinyl)butyl]-5,5-bis(4-methoxyphenyl)-2,4-pentadienamid mit einem Schmelzpunkt von 91-93° C. Elementaranalyse für $C_{32}H_{32}N_2O_3$: C 78,02, H 6,55, N 5,69; Gefunden: C 78,01, H 6,45, N 5,97.

Beispiel 41

Wie in Beispiel 2 wird eine Lösung von (E)-5,5-bis(4-Methoxyphenyl)-2,4-pentadien carbonsäure-4-nitrophenylester (0,24 g) und 8-Isochinolinpropanamin (0,103 g) in Tetrahydrofuran (5 ml) während 17 Stunden bei Raumtemperatur gerührt und dann in üblicher Weise aufgearbeitet. Das rohe Amid wird mittels HPLC (Essigester) gereinigt und dann aus Essigester/Hexan kristallisiert. Man erhält 0,181 g (E)-N-[3-(8-Isochinolinyl)propyl]-5,5-bis(4-methoxyphenyl)-2,4-pentadienamid mit einem Schmelzpunkt von 115-118° C. Elementaranalyse für $C_{31}H_{30}N_2O_3$: C 77,80, H 6,32, N 5,85; Gefunden: C 77,61, H 6,23, N 5,76.

Beispiel 42

Oxalylchlorid (0,5 ml) wird zu einer Suspension von (E)-5,5-bis(4-Methoxyphenyl)-2,4-pentadiencarbonsäure (0,776 g) in Toluol (5 ml) gegeben, und das Reaktionsgemisch wird während 30 Minuten bei Raumtemperatur gerührt. Nach Abdampfen des Lösungsmittels unter vermindertem Druck wird das Säurechlorid in Methylenchlorid (10 ml) gelöst und die erhaltene Lösung wird bei 5° C tropfenweise zu einer gerührten Lösung von 3-(4-Aminophenyl)pyridin (0,426 g) und Triethylamin (0,87 ml) in Methylenchlorid (20 ml) gegeben. Man lässt über Nacht bei Raumtemperatur reagieren und wäscht dann die Reaktionslösung mit 0,5N NaOH-Lösung und mit Kochsalzlösung. Durch Eindampfen der getrockneten ($Na_2SO_4$) organischen Phase erhält man einen Schaum, den man aus Aceton/ Essigester kristallisiert. Man erhält 0,7 g (E)-5,5-bis-(4-Methoxyphenyl)-N-[4-(3-pyridinyl)phenyl]-2,4-pentadienamid mit einem Schmelzpunkt von 210-212° C. Elementaranalyse für $C_{30}H_{26}N_2O_3$: C 77,90, H 5,67, N 6,06; Gefunden: C 77,85, H 5,91, N 5,94.

Beispiel 43

Wie in Beispiel 42 wird das aus Oxalylchlorid (0,5 ml) und (E)-5,5-bis(4-Methoxyphenyl)-2,4-pentadiencarbonsäure (0,815 g) hergestellte Säurechlorid in Methylenchlorid (10 ml) aufgelöst, und diese Lösung wird bei 5°C tropfenweise zu einer gerührten Lösung von 3-(4-Aminobenzyl)pyridin (0,461 g) und Triethylamin (0,87 ml) in Methylenchlorid (20 ml) gegeben. Man lässt über Nacht bei Raumtemperatur reagieren, arbeitet in der üblichen Weise auf und kristallisiert das rohe Amid aus Aceton/Essigester. Man erhält 0,74 g (E)-5,5-bis(4-Methoxyphenyl)-N-[4-(3-Pyridinylmethyl)phenyl]-2,4-pentadienamid mit einem Schmelzpunkt von 185-186°C. Elementaranalyse für $C_{31}H_{28}N_2O_3$: C 78,13, H 5,92, N 5,88; Gefunden: C 77,78, H 6,13, N 5,71.

Beispiel 44

Wie in Beispiel 42 wird das aus Oxalylchlorid (0,35 ml) und (E)-5,5-bis(4-Methoxyphenyl)-2,4-pentadiencarbonsäure (0,5 g) hergestellte Säurechlorid in Methylenchlorid (10 ml) gelöst und diese Lösung wird bei 7°C tropfenweise zu einer gerührten Lösung von 5-Aminoisochinolin (0,22 g) und Triethylamin (0,55 ml) in Methylenchlorid (5 ml) gegeben. Man lässt über Nacht bei Raumtemperatur reagieren und arbeitet dann in der üblichen Weise auf. Das rohe Amid wird mittels Säulenchromatographie (Essigester/Methylenchlorid; 1:1) gereinigt und dann aus Essigester kristallisiert. Man erhält 0,22 g (E)-N-(5-Isochinolinyl)-5,5-bis(4-methoxyphenyl)-2,4-pendatienamid mit einem Schmelzpunkt von 197-199°C. Elementaranalyse für $C_{28}H_{24}N_2O_3$: C 77,04, H 5,54, N 6,42; Gefunden: C 76,24, H 5,42, N 6,23.

Beispiel 45

Wie in Beispiel 2 wird eine Lösung von (E)-5,5-bis(4-Methoxyphenyl)-2,4-pentadiencarbonsäure-4-nitrophenyl ester (1,93 g) und (R,S)-6,$\alpha$-Dimethyl-3-pyridinbutanamin (0,8 g) in Tetrahydrofuran (15 ml) über Nacht bei Raumtemperatur gerührt und dann in üblicher Weise aufgearbeitet. Das rohe Amid wird mittels HPLC (Essigester) gereinigt und dann aus Benzol lyophilisiert. Man erhält 1,34 g [R,S-(E)]-5,5-bis(4-Methoxyphenyl)-N-[1-methyl-4-(6-methyl-3-pyridinyl)butyl]-2,4-pentadienamid-Hydrat (0,5 molar) als amorphen Festkörper. Elementaranalyse für $C_{30}H_{34}N_2O_3/0,5H_2O$: C 75,13, H 7,36, N 5,84, $H_2O$ 1,87; Gefunden: C 74,81, H 7,39, N 5,78, $H_2O$ 2,21.

Beispiel 46

Wie in Beispiel 2 wird eine Lösung von (E)-5,5-bis(4-Methoxyphenyl)-2,4-pentadien carbonsäure-4-nitrophenylester (1,11 g) und (R,S)-2,$\alpha$-Dimethyl-3-pyridinbutanamin (0,46 g) in Tetrahydrofuran (10 ml) über Nacht bei Raumtemperatur gerührt. Nach dem üblichen Aufarbeiten wird das rohe Amid mittels HPLC (Essigester) gereinigt und dann aus Benzol lyophilisiert. Man erhält 1,0 g [R,S-(E)]-5,5-bis(4-Methoxyphenyl)-N-[1-methyl-4-(2-methyl-3-pyridinyl)butyl]-2,4-pentadienamid als amorphen Festkörper. Elementaranalyse für $C_{30}H_{34}N_2O_3$: C 76,57, H 7,28, N 5,35; Gefunden: C 76,41, H 7,18, N 6,01.

Beispiel 47

Wie in Beispiel 2 wird eine Lösung von (E)-5,5-bis(4-Methoxyphenyl)-2,4-pentadien carbonsäure-4-nitrophenylester (1,88g) und (R,S)-6-Ethyl-$\alpha$-methyl-3-pyridinbutanamin (0,84 g) in Tetrahydrofuran (10 ml) über Nacht bei Raumtemperatur gerührt und dann in üblicher Weise aufgearbeitet. Das rohe Amid wird mittels HPLC (Essigester) gereinigt und dann aus Benzol lyophilisiert. Man erhält 1,1 g [R,S-(E)]-5,5-bis(4-Methoxyphenyl)-N-[1-methyl-4-(6-ethyl-3-pyridinyl)butyl]-2,4-pentadienamid-Hydrat (0,66 molar) als amorphen Festkörper. Elementaranalyse für $C_{31}H_{36}N_2O_3/0.66H_2O$: C 74,99, H 7,58, N 5,67, $H_2O$ 2,39; Gefunden: C 75,19, H 7,66, N 5,70, $H_2O$ 2,69.

Beispiel 48

Wie in Beispiel 2 wird eine Lösung von (E)-5,5-bis(4-Methoxyphenyl)-2,4-pentadien carbonsäure-4-nitrophenylester (2,0 g) und (R,S)-$\alpha$-Ethyl-3-pyridinbutanamin (0,83 g) in Tetrahydrofuran (15 ml) während 18 Stunden bei Raumtemperatur gerührt und dann in üblicher Weise aufgearbeitet. Das erhaltene Material wird mittels HPLC (Essigester) gereinigt und dann aus Benzol lyophilisiert. Man erhält 1,7 g [R,S-(E)]-N-[1-Ethyl-4-(3-pyridinyl)butyl]-5,5-bis(4-methoxy-phenyl)-2,4-pentadienamid. Elementaranalyse für $C_{30}H_{34}N_2O_3$:

C 76,57, H 7,28, N 5,95; Gefunden: C 76,24, H 7,38, N 5,90.

Beispiel 49

Wie in Beispiel 2 wird eine Lösung von (E)-5,5-bis(4-Methoxyphenyl)-2,4-pentadien carbonsäure-4-nitrophenylester (1,22 g) und (R)-α-Ethyl-3-pyridinbutanamin (0,5 g) in Tetrahydrofuran (15 ml) während 20 Stunden bei 50°C gerührt und dann in üblicher Weise aufgearbeitet. Das rohe Amid wird mittels HPLC (Essigester) gereinigt und dann aus Benzol lyophilisiert. Man erhält 1,05 g [R-(E)]-N-[1-Ethyl-4-(3-pyridinyl)-butyl]-5,5-bis(4-methoxyphenyl)2,4-pentadienamid als amorphen Festkörper. Elementaranalyse für $C_{30}H_{34}N_2O_3$: C 76,57, H 7,28, N 5,95; Gefunden: C 76,02, H 7,16, N 5,81.

Beispiel 50

Wie in Beispiel 2 wird eine Lösung von (E)-5,5-bis(4-Methoxyphenyl)-2,4-pentadien carbonsäure-4-nitrophenylester (1,68 g) und (S)-α-Ethyl-3-pyridinbutanamin (0,687 g) in Tetrahydrofuran (15 ml) während 20 Stunden bei 50 °C gerührt und dann in üblicher Weise aufgearbeitet. Das Amid wird mittels HPLC (Essigester gereinigt und dann aus Benzol lyophilisiert. Man erhält 1,655 g [S-(E)]-N-[1-Ethyl-4-(3-pyridinyl)-butyl]-5,5-bis(4-methoxyphenyl)-2,4-pentadienamid als amorphes Pulver. Elementaranalyse für $C_{30}H_{24}N_2O_3$: C 76,57, H 7,28, N 5,95; Gefunden: C 76,08, H 7,22, N 5,85.

Beispiel 51

Wie in Beispiel 2 wird eine Lösung von (E)-5,5-bis(4-Methoxyphenyl)-2,4-pentadien carbonsäure-4-nitrophenylester (2,16 g) und (R,S)-α-Propyl-3-pyridinbutanamin (0,962 g) in Tetrahydrofuran (20 ml) während 17 Stunden bei 50°C gerührt und dann in üblicher Weise aufgearbeitet. Das erhaltene rohe Material wird mittels HPLC (Essigester) gereinigt und dann aus Benzol lyophilisiert. Man erhält 2,1 g [R,S-(E)]-5,5-bis(4-Methoxyphenyl)-N-[1-propyl-4-(3-pyridinyl)butyl]-2,4-pentadienamid als glasigen Festkörper. Elementaranalyse für $C_{31}H_{36}N_2O_3$; C 76,83, H 7,49, N 5,78; Gefunden: C 76,59, H 7,42, N 5,76.

Beispiel 52

Wie in Beispiel 2 wird eine Lösung von (E)-5,5-bis(4-Methoxyphenyl)-2,4-pentadien carbonsäure-4-nitrophenylester (2,16 g) und (R,S)-α-(1-Methylethyl)-3-pyridinbutanamin (0,962 g) in Tetrahydrofuran (25 ml) während 20 Stunden bei 50°C gerührt und dann in üblicher Weise aufgearbeitet. Das rohe Amid wird mittels HPLC (Essigester) gereinigt und dann aus Benzol lyophilisiert. Man erhält 2,1 g [R,S-(E)]-5,5-bis(4-Methoxyphenyl)-N-[1-(1-methylethyl)-4-(3-pyridinyl)butyl]-2,4-pentadienamid als amorphes Pulver. Elementaranalyse für $C_{31}H_{36}N_2O_3$: C 76,83, H 7,49, N 5,78; Gefunden: C 76,72, H 7,63, N 5,76.

Beispiel 53

Wie in Beispiel 2 wird eine Lösung von (E)-5,5-bis(4-Methoxyphenyl)-2,4-pentadien carbonsäure-4-nitrophenylester (2,16 g) und (R,S)-α-Butyl-3-pyridinbutamin (1,03 g) in Tetrahydrofuran (20 ml) während 20 Stunden bei 50°C gerührt und dann in üblicher Weise aufgearbeitet. Das Amid wird mittels HPLC (Essigester) gereinigt und dann aus Benzol lyophilisiert. Man erhält 2,15 g [R,S-(E)]-N-[1-Butyl-4-(3-pyridinyl)butyl]-5,5-bis(4-methoxy-phenyl)-2,4-pentadienamid als amorphen Festkörper. Elementaranalyse für $C_{32}H_{38}N_2O_3$: C 77,08, H 7,68, N 5,62; Gefunden: C 76,88, H 7,64, N 5,66.

Beispiel 54

Wie in Beispiel 2 wird eine Lösung von (E)-5,5-bis(4-Methoxyphenyl)-2,4-pentadien carbonsäure-4-nitrophenylester (2,16 g) und (R,S)-α-Cyclopropyl-3-pyridinbutanamin (1,03 g) in Tetrahydrofuran (20 ml) während 20 Stunden bei 50°C gerührt und dann in üblicher Weise aufgearbeitet. Das rohe Material wird mittels HPLC (Essigester) gereinigt und dann aus Benzol lyophilisiert. Man erhält 2,1 g [R,S-(E)]-N-[1-Cyclopropyl-4-(3-pyridinyl)butyl]-5,5-bis(4-methoxyphenyl)-2,4-pentadienamid als amorphes Pulver. Elementaranalyse für $C_{31}H_{34}N_2O_3$: C 77,15, H 7,10 N 5,80; Gefunden: C 76,71, H 7,08, N 5,76.

Beispiel 55

Wie in Beispiel 2 wird eine Lösung von (E)-5,5-bis(4-Methoxyphenyl)-2,4-pentadien carbonsäure-4-nitrophenylester (1,64 g) und (R,S)-α-Cyclopentyl-3-pyridinbutanamin (0,832 g) in Tetrahydrofuran (15 ml) während 17 Stunden bei 50°C gerührt und dann in üblicher Weise aufgearbeitet. Das Amid wird mittels HPLC (Essigester) gereinigt und dann aus Benzol lyophilisiert. Man erhält 1,7 g [R,S-(E)]-N-[1-Cyclopentyl-4-(3-pyridinyl)butyl]-5,5-bis(4-methoxyphenyl)-2,4-pentadienamid als amorphen Festkörper. Elementaranalyse für $C_{33}H_{38}N_2O_3$: C 77,61, H 7,50, N 5,49; Gefunden: C 77,52, H 7,56, N 5,46.

Beispiel 56

Wie in Beispiel 2 wird eine Lösung von (E)-5,5-bis(4-Methoxyphenyl)-2,4-pentadien carbonsäure-4-nitrophenylester (2,16 g) und (R,S)-α-Cyclohexyl-3-pyridinbutanamin (1,16 g) in Tetrahydrofuran (20 ml) während 20 Stunden bei 50°C gerührt. Nach dem üblichen Aufarbeiten wird das Produkt mittels HPLC (Essigester) gereinigt und dann aus Benzol lyophilisiert. Man erhält 2,2 g [R,S-(E)]-N-[1-Cyclohexyl-4-(3-pyridinyl)butyl]-5,5-bis(4-methoxyphenyl)-2,4-pentadienamid als amorphes Pulver. Elementaranalyse für $C_{34}H_{40}N_2O_3$: C 77,83, H 7,68, N 5,34; Gefunden: C 77,39, H 7,65, N 5,24.

Beispiel 57

Wie in Beispiel 2 wird eine Lösung von (E)-5,5-bis(4-Methoxyphenyl)-2,4-pentadiencarbonsäure-4-nitrophenylester (1,47 g) und (R,S)-α-(4-Bromphenyl)-3-pyridinbutanamin (1,04 g) in Tetrahydrofuran (20 ml) während 17 Stunden bei 50°C gerührt. Nach dem üblichen Aufarbeiten wird das Amid mittels HPLC (Essigester) gereinigt und dann aus Ether kristallisiert. Man erhält 1,2 g [R,S-(E)]-N-[1-(4-Bromphenyl)-4-(3-pyridinyl)butyl]-5,5-bis(4-methoxyphenyl)-2,4-pentadienamid mit einem Schmelzpunkt von 145-146°C. Elementaranalyse für $C_{34}H_{33}BrN_2O_3$: C 68,34, H 5,57, Br 13,37, N 4,69; Gefunden: C 68,44, H 5,54, Br 13,39, N 4,76.

Beispiel 58

Wie in Beispiel 2 wird eine Lösung von (E)-5,5-bis(4-Methoxyphenyl)-2,4-pentadiencarbonsäure-4-nitrophenyl ester (1,94 g) und α-[3-(3-Pyridinyl)propyl]-3-pyridinbutanamin (1,21 g) in Tetrahydrofuran (25 ml) während 20 Stunden bei 50°C gerührt und dann in üblicher Weise aufgearbeitet. Das rohe Amid wird mittels HPLC (Esigester/Triethylamin; 19:1) gereinigt und dann aus Benzol lyophilisiert. Man erhält 1,95 g (E)-5,5-bis(4-Methoxyphenyl)-N-[1-[3-(3-pyridinyl)propyl]-4-(3-pyridinyl)butyl]-2,4-pentadienamid als glasigen Festkörper. Elementaranalyse für $CH_{36}H_{39}N_3O_3$: C 76,98, H 7,00, N 7,48; Gefunden: C 76,41, H 7,17, N 7,63.

Beispiel 59

Wie in Beipiel 2 wird eine Lösung von (E)-5,5-bis(2-Methoxyphenyl)-2,4-pentadiencarbonsäure-4-nitrophenyl ester (2,1 g) und 3-Pyridinbutanamin (0,9 g) in Tetrahydrofuran (20 ml) über Nacht bei Raumtemperatur gerührt und dann in üblicher Weise aufgearbeitet. Das rohe Amid wird mittels HPLC (Essigester) gereinigt und dann aus Ether kristallisiert. Man erhält 1,8 g (E)-5,5-bis(2-Methoxyphenyl)-N-[4-(3-pyridinyl)-butyl]-2,4-pentadienamid mit einem Schmelzpunkt von 103,5-104,5°C. Elementaranalyse für $C_{28}H_{30}N_2O_3$: C 75,99, H 6,83, N 6,33; Gefunden: C 76,18, H 6,92, N 6,40.

Beispiel 60

Wie in Beispiel 2 wird eine Lösung von (E)-5,5-bis(4-Methylphenyl)-2,4-pentadiencarbonsäure-4-nitrophenyl ester (2,5 g) und 3-Pyridinbutanamin (1,08 g) in Tetrahydrofuran (25 ml) über Nacht bei Raumtemperatur gerührt und dann in üblicher Weise aufgearbeitet. Das Amid wird mittels HPLC (Essigester) gereinigt und dann zweimal aus Ether kristallisiert. Man erhält 1,85 g (E)-5,5-bis(4-Methylphenyl)-N-[4-(3-pyridinyl)butyl]-2,4-pantadienamid mit einem Schmelzpunkt von 144,5-145,5°C. Elementaranalyse für $C_{28}H_{30}N_2O$: C 81,91, H 7,37, N 6,82; Gefunden: C 81,65, H 7,38, N 6,78.

Beispiel 61

Wie in Beispiel 2 wird eine Lösung von (E)-5,5-bis-(4-Methylphenyl)-2,4-pentadiencarbonsäure-4-nitrophe nylester (2,0 g) und (R)-α-Methyl-3-pyridinbutanamin (0,95 g) in Tetrahydrofuran (20 ml) über Nacht bei Raumtemperatur gerührt und dann in üblicher Weise aufgearbeitet. Das rohe Amid wird zuerst aus Ether und dann aus Essigester/Hexan kristallisiert. Man erhält 1,4 g [R-(E)]-5,5-bis(4-Methylphenyl)-N-[1-methyl-4-(3-pyridinyl)butyl]-2,4-pentadienamid mit einem Schmelzpunkt von 105-108 °C. Elementaranalyse für $C_{29}H_{32}N_2O$: C 82,04, H 7,60, N 6,60; Gefunden: C 81,73, H 7,75, N 6,58.

Beispiel 62

Wie in Beispiel 2 wird eine Lösung von (E,E)-5-(4-Methoxyphenyl)-5-phenyl-2,4-pentadiencarbonsäure-4-nitrophenylester (1,8 g) und (R)-α-Methyl-3-pyridinbutanamin (0,85 g) in Tetrahydrofuran (20 ml) über Nacht bei Raumtemperatur gerührt und dann in üblicher Weise aufgearbeitet. Das Rohprodukt wird mittels HPLC (Essigester) gereinigt und dann aus Benzol lyophilisiert. Man erhält 1,8 g [R-(E,E)]-5-(4-Methoxyphenyl)-N-[1-methyl-4-(3-pyridinyl)butyl]-5-phenyl-2,4-pentadienamid als amorphen Festkörper Elementaranalyse für $C_{28}H_{30}N_2O_2$ : C 78,84, H 7,09, N 6,57; Gefunden: C 78,59, H 7,27, N 6,27.

Beispiel 63

Wie in Beispiel 2 wird eine Lösung von (2E,4Z)-5-(4-Methoxyphenyl)-5-phenyl-2,4-pentadiencarbonsäure-4-nitrophenylester (1,63 g) und (R)-α-Methyl-3-pyridinbutanamin (0,758 g) in Tetrahydrofuran (20 ml) über Nacht bei Raumtemperatur gerührt und dann in üblicher Weise aufgearbeitet. Das Rohprodukt wird mittels HPLC (Essigester) gereinigt und zuerst aus Ether und dann aus Essigester/Hexan kristallisiert. Man erhält 1,03 g [R-(2E,4Z)]-5-(4-Methoxyphenyl)-N-[1-methyl-4-(3-pyridinyl)butyl]5-phenyl-2,4-pentadienamid mit einem Schmelzpunkt von 129,5-131 °C. Elementaranalyse für $C_{28}H_{30}N_2O_2$: C 78,84, H 7,09, N 6,57; Gefunden: C 78,01, H 7,30, N 6,67.

Beispiel 64

Wie in Beispiel 2 wird eine Lösung von (E,E)-5-(2-Methoxyphenyl)-5-(4-Methoxyphenyl)-2,4-pentadiencarbonsäure-4-nitrophenylester (1,5 g) und 3-Pyridinbutanamin (0,64 g) in Tetrahydrofuran (15 ml) über Nacht bei Raumtemperatur gerührt. Nach dem üblichen Aufarbeiten wird das Rohprodukt aus Essigester kristallisiert. Man erhält 1,25 g (E,E)-5-(2-Methoxyphenyl)-5-(4-methoxyphenyl)-N-[4-(3-pyridinyl)-butyl]-2,4-pentadienamid mit einem Schmelzpunkt von 164-165 °C. Elementaranalyse für $C_{28}H_{30}N_2O_3$: C 75,99, H 6,83, N 6,33; Gefunden: C 76,21, H 6,93, N 6,39.

Beispiel 65

Wie in Beispiel 2 wird eine Lösung von (2E,4Z)-5-(2-Methoxyphenyl)-5-(4-methoxyphenyl)-2,4-pentadiencarbonsäure-4-nitrophenylester (0,82 g) und 3-Pyridinbutanamin (0,33 g) in Tetrahydrofuran (7 ml) über Nacht bei Raumtemperatur gerührt. Nach dem üblichen Aufarbeiten wird das Rohprodukt mittels HPLC (Essigester) gereinigt und dann aus Benzol lyophilisiert. Man erhält 0,81 g (2E,4Z)-5-(2-Metoxyphenyl)-5-(4-methoxyphenyl)-N-[4-(3-pyridinyl) butyl]-2,4-pentadienamid als amorphen Festkörper. Elementaranalyse für $C_{28}H_{30}N_2O_3$: C 75,99, H 6,83, N 6,33; Gefunden: C 75,99, H 6,99, N 6.30.

Beispiel 66

Wie in Beispiel 2 wird eine Lösung von (E,E)-5-(4-Methoxyphenyl)-5-(3-pyridinyl)-2,4-pentadiencarbonsäure-4-nitrophenylester (1,61 g) und 3-Pyridinbutanamin (0,61 g) in Tetrahydrofuran (20 ml) über Nacht bei Raumtemperatur gerührt. Nach dem üblichen Aufarbeiten wird das Rohprodukt aus Essigester kristallisiert. Man erhält 1,05 g (E,E)-5-(4-Methoxyphenyl)-5-(3-pyridinyl)-N-[4-(3-pyridinyl)butyl]-2,4-pentadienamid mit einem Schmelzpunkt von 121-123 °C. Elementaranalyse für $C_{26}H_{27}N_3O_2$: C 75,52, H 6,58, N 10,16; Gefunden: C 75,48, H 6,71, N 10,26.

Beispiel 67

Wie in Beispiel 2 wird eine Lösung von (2E,4Z)-5-(4-Methoxyphenyl)-5-(3-pyridinyl)-2,4-pentadiencarbonsäure-4-nitrophenylester (2,01 g) und 3-Pyridinbutanamin (0,75 g) in Tetrahydrofuran (25 ml) während 17 Stunden bei Raumtemperatur gerührt. Nach dem üblichen Aufarbeiten wird das Rohprodukt aus Essigester kristallisiert. Man erhält 1,65 g (2E,4Z)-5-(4-Methoxyphenyl)-5-(3-pyridinyl)-N-[4-(3-pyridinyl) butyl]-2,4-pentadienamid mit einem Schmelzpunkt von 108-110 ° C. Durch Umkristallisieren einer Probe aus dem gleichen Lösungsmittel erhält man eine analytisch reine Probe mit einem Schmelzpunkt von 109-110 ° C. Elementaranalyse für $C_{26}H_{27}N_3O_2$: C 75,52, H 6,58, N 10,16; Gefunden: C 75,43, H 6,65, N 10,19.

Beispiel 68

Wie in Beispiel 2 wird eine Lösung von (E,E)-5-Cyclopropyl-5-(4-methoxyphenyl)-2,4-pentadiencarbonsäure-4nitrophenylester (1,1 g) und (R)-α-Methyl-3-pyridinbutanamin (0,5 g) in Tetrahydrofuran (15 ml) während 2 Tagen bei Raumtemperatur gerührt. Nach dem üblichen Aufarbeiten wird das Amid mittels HPLC (Essigester) gereinigt und dann aus Ether kristallisiert. Man erhält 0,99 g [R-(E,E)]-5-Cyclopropyl-5-(4-methoxyphenyl)-N-[1-methyl-4-(3-pyridinyl)butyl]-2,4-pentadienamid mit einem Schmelzpunkt von 111-113 ° C. Elementaranalyse für $C_{25}H_{30}N_2O_2$: C 76,89, H 7,74, N 7,17; Gefunden: C 76,84, H 7,92, N 7,18.

Beispiel 69

Wie in Beispiel 2 wird eine Lösung von (2E,4Z)-5-Cyclopropy-5-(4-methoxyphenyl)-2,4-pentadiencarbonsäure-4-nitrophenylester (1,1 g) und (R)-α-Methyl-3-pyridinbutanamin (0,5 g) in Tetrahydrofuran (15 ml) während 2 Tagen bei Raumtemperatur gerührt. Nach dem üblichen Aufarbeiten wird das Rohprodukt mittels HPLC (Essigester) gereinigt und dann aus Ether kristallisiert. Man erhält 1,02 g [R-(2E,4Z)]-5-Cyclopropyl-5-(4-methoxyphenyl)-N-[1-methyl-4-(3-pyridinyl)butyl]-2,4-pentadienamid mit einem Schmelzpunkt von 99-100 ° C. Elementaranalyse für $C_{25}H_{30}N_2O_2$: C 76,89, H 7,74, N 7,17; Gefunden: C 76,84, H 7,94, N 7,14.

Beispiel 70

Wie in Beispiel 2 wird eine Lösung von (E,E)-5-Cyclohexyl-5-(4-methoxyphenyl)-2,4-pentadiencarbonsäure-4-nitrophenylester (1,22 g) und (R)-α-Methyl-3-pyridinbutanamin (0,5 g) in Tetrahydrofuran (15 ml) während 2 Tagen bei Raumtemperatur gerührt. Nach dem üblichen Aufarbeiten wird das rohe Amid aus Essigester/Hexan kristallisiert. Man erhält 0,8 g [R-(E,E)]-5-Cyclohexyl-5-(4-methoxyphenyl)-N-[1-methyl-4-(3-pyridinyl)butyl]-2,4-pentadienamid mit einem Schmelzpunkt von 142,5-143,5 ° C. Elementaranalyse für $C_{28}H_{36}N_2O_2$: C 77,74, H 8,39, N 6,48; Gefunden: C 77,57, H 8,28, N 6,54.

Beispiel 71

Wie in Beispiel 2 wird eine Lösung von (2E,4Z)-5-Cyclohexyl-5(4-methoxyphenyl)-2,4-pentadiencarbonsäure-4-nitrophenylester (1,83 g) und (R)-α-Methyl-3-pyridinbutanamin (0,85 g) in Tetrahydrofuran (20 ml) über Nacht bei Raumtemperatur gerührt. Nach dem üblichen Aufarbeiten wird das Rohprodukt aus Ether kristallisiert. Man erhält 1,68 g [R-(2E,4Z)]-5-Cyclohexyl-5(4-methoxyphenyl)-N-[1-methyl-4-(3-pyridinyl)butyl]-2,4-pentadienamid mit einem Schmelzpunkt von 147-148,5 ° C. Elementaranalyse für $C_{28}H_{36}N_2O_2$: C 77,74, H 8,39, N 6,48; Gefunden: C 77,54, H 8,31, N 6,51.

Beispiel 72

Wie in Beispiel 2 wird eine Lösung von (E,E)-5-(4-Methoxyphenyl)-2,4-pentadiencarbonsäure-4-nitrophenylester (0,98 g) und (R)-α-Methyl-3-pyridinbutanamin (0,5 g) in Tetrahydrofuran (20 ml) während 8 Stunden unter Rückfluss gerührt. Nach dem üblichen Aufarbeiten wird das Rohprodukt mit Ether behandelt. Durch Kristallisieren des erhaltenen Festkörpers aus Essigester erhält man 0,85 g [R-(E,E)]-5-(4-Methoxyphenyl)-N-[1-methyl-4-(3-pyridinyl)-butyl]-2,4-pentadienamid mit einem Schmelzpunkt von 186,5-187,5 ° C. Elementaranalyse für $C_{22}H_{26}N_2O_2$: C 75,40, H 7,48, N 7,99; Gefunden C 75,73, H 7,38, N 8,00.

Beispiel 73

Wie in Beispiel 2 wird eine Lösung von (E,E)-5-(4-Methoxyphenyl)-2,4-hexadiencarbonsäure-4-nitrophenylester (1,02 g) und (R)-α-Methyl-3-pyridinbutanamin (0,5 g) in Tetrahydrofuran (20 ml) während 17 Stunden bei Raumtemperatur gerührt. Nach dem üblichen Aufarbeiten wird das Rohprodukt aus Essigester/Hexan kristallisiert. Man erhält 0,6 g [R-(E,E)]-5-(4-Methoxyphenyl)-N-[1-methyl-4-(3-pyridinyl)-butyl]-2,4-hexadienamid mit einem Schmelzpunkt von 107,5-108,5°C. Elementaranalyse für $C_{23}H_{28}N_2O_2$: C 75,79, H 7,74, N 7,69; Gefunden: C 75,74, H 7,58, N 7,72.

Beispiel 74

Wie in Beispiel 2 wird eine Lösung von (E,E)-5-(4-Methoxyphenyl)-6-methyl-2,4-heptadiencarbonsäure-4-nitrophenylester (1,1 g) und (R)-α-Methyl-3-pyridinbutanamin (0,5 g) in Tetrahydrofuran (15 ml) während 2 Tagen bei Raumtemperatur gerührt. Nach dem üblichen Aufarbeiten wird das Rohprodukt mittels HPLC (Essigester) gereinigt und dann aus Benzol lyophilisiert. Man erhält 0,9 g [R-(E,E)]-5-(4-Methoxyphenyl)-6-methyl-N-[1methyl-4-(3-pyridinyl)butyl]-2,4-heptadienamid als Oel. Elementaranalyse für $C_{25}H_{32}N_2O_2$: C 76,50, H 8,22, N 7,14; Gefunden: C 76,76, H 8,29, N 7,14.

Beispiel 75

Wie in Beispiel 2 wird eine Lösung von (2E,4Z)-5-(4-Methoxyphenyl)-6-methyl-2,4-heptadiencarbonsäure-4-nitrophenylester (1,65 g) und (R)-α-Methyl-3-pyridinbutanamin (0,85 g) in Tetrahydrofuran (20 ml) über Nacht bei Raumtemperatur gerührt. Nach dem üblichen Aufarbeiten wird das Rohprodukt mittels HPLC (Essigester) gereinigt und aus Ether/Hexan kristallisiert. Man erhält 1,5 g [R-(E,E)-]-5-(4-Methoxyphenyl)-6-methyl-N-[1-methyl-4-(3-pyridinyl)-butyl]-2,4-heptadienamid mit einem Schmelzpunkt von 83-4,5°C. Elementaranalyse für $C_{25}H_{32}N_2O_2$: C 76,50, H 8,22, N 7,14; Gefunden: C 77,02, H 8,33, N 7,17.

Beispiel 76

Wie in Beispiel 2 wird eine Lösung von (E,E)-5-(4-Methoxyphenyl)-2,4-heptadiencarbonsäure-4-nitrophenylester (1,06 g) und (R)-α-Methyl-3-pyridinbutanamin (0,5 g) in Tetrahydrofuran (15 ml) während 2 Tagen bei Raumtemperatur gerührt. Nach dem üblichen Aufarbeiten wird das Rohprodukt aus Ether kristallisiert. Man erhält 0,778 g [R-(E,E)]-5-(4-Methoxyphenyl)-N-[1-methyl-4-(3-pyridinyll)-butyl]-2,4 heptadienamid mit einem Schmelzpunkt von 107-109°. Elementaranalyse für $C_{24}H_{30}N_2O_2$: C 76,16, H 7,99, N 7,40; Gefunden: C 75,78, H 8,15, N 7,15.

Beispiel 77

Wie in Beispiel 2 wird eine Lösung von (E,E)-5-(4-Methoxyphenyl)-2,4-octadiencarbonsäure-4-nitrophenyles ter (1,1 g) and (R)-α-Methyl-3-pyridinbutanamin (0,5 g) in Tetrahydrofuran (15 ml) während 42 Stunden bei Raumtemperatur gerührt. Nach dem üblichen Aufarbeiten wird das rohe Amid aus Ether kristallisiert. Man erhält 0,835 g [R-(E,E)]-5-(4-Methoxyphenyl)-N-[1-methyl-4-(3-pyridinyl)-butyl]-2,4-octadienamid. Elementaranalyse für $C_{25}H_{32}N_2O_2$:C 75,49, H 8,22, N 7,14; Gefunden: C 75,95, H 8,29, N 7,00.

Beispiel 78

Wie in Beispiel 2 wird eine Lösung von (E,E)-5-(4-Methoxyphenyl)-2,4-nonadiencarbonsäure-4-nitrophenylester (1,14 g) und (R)-α-Methyl-3-pyridinbutanamin (0,5 g) in Tetrahydrofuran (15 ml) während 42 Stunden bei Raumtemperatur gerührt. Nach dem üblichen Aufarbeiten wird das Rohprodukt mittels HPLC (Essigester) gereinigt und dann aus Ether kristallisiert. Man erhält 0,826 g [R-(E,E)]-5-(4-Methoxyphenyl)-N-[1-methyl-4(3-pyridinyl)butyl]-2,-4-nonadienamid mit einem Schmelzpunkt von 89-91°C. Elementaranalyse für $C_{26}H_{34}N_2O_2$: C 76,81, H 8,43, N 6,89; Gefunden: C 76,90, H 9,63, N 6,92.

Beispiel 79

Wie in Beispiel 2 wird eine Lösung von (E,E)-5-(3-Methoxyphenyl)-2,4-nonadiencarbonsäure-4-nitrophenylester (1,62 g) und (R)-α-Methyl-3-pyridinbutanamin (0,75 g) in Tetrahydrofuran (15 ml) während 42

Stunden bei Raumtemperatur gerührt. Nach dem üblichen Aufarbeiten wird das Rohprodukt mittels HPLC (Essigester) gereinigt und dann aus Ether kristallisiert. Man erhällt 1,32 g [R-(E,E)]-5-(3-Methoxyphenyl)-N-[1-methyl-4-(3-pyridinyl)butyl]-2,4-nonadienamid mit einem Schmelzpunkt von 72-73,5 ° C. Elementaranalyse für $C_{26}H_{34}N_2O_2$: C 76,81, H 8,43, N 6,89; Gefunden: C 76,66, H 8,57, N 6,82.

Beispiel 80

Wie in Beispiel 2 wird eine Lösung von (2E,4Z)-5-(4-Methoxyphenyl)-2,3-nonadiencarbonsäure-4-nitrophenylester (1,62 g) und (R)-α-Methyl-3-pyridinbutanamin (0,75 g) in Tetrahydrofuran (15 ml) während 2 Tagen bei Raumtemperatur gerührt. Nach dem üblichen Aufarbeiten wird das Rohprodukt mittels HPLC (Essigester) gereinigt und dann aus Benzol lyophilisiert. Man erhält 1,45 g [R-(2E,4Z)]-5-(4-Methoxyphenyl)-N-[1-methyl-4-(3-pyridinyl)butyl]-2,4-nonadienamid als Oel.

Beispiel 81

Wie in Beispiel 2 wird eine Lösung von (E,E)-5-(4-Methoxyphenyl)-2,4-decadiencarbonsäure-4-nitrophenylester (2,5 g) und 3-Pyridinbutanamin (1,12 g) in Tetrahydrofuran (25 ml) über Nacht bei Raumtemperatur gerührt. Nach dem üblichen Aufarbeiten wird das Rohprodukt mittels HPLC (Essigester) gereinigt und dann aus Ether/Hexan kristallisiert. Man erhält 1,3 g (E,E)-5-(4-Methoxyphenyl)-N-[4-(3-pyridinyl)butyl]-2,4-decadienamid mit einem Schmelzpunkt von 83,5-84,5 ° C. Elementaranalyse für $C_{26}H_{34}N_2O_2$: C 76,81, H 8,43, N 6,89; Gefunden: C 77,05, H 8,52, N 6,87.

Beispiel 82

Wie in Beispiel 2 wird eine Lösung von (2E,4Z)-5-(4-Methoxyphenyl)-2,4-decadiencarbonsäure-4-nitrophenylester (2 g) und 3-pyridinbutanamin (0,9 g) in Tetrahydrofuran (20 ml) über Nacht bei Raumtemperatur gerührt. Nach dem üblichen Aufarbeiten wird das Rohprodukt mittels HPLC (Essigester) gereinigt und der erhaltene Festkörper wird mit Ether behandelt. Man erhält 1,45 g (E,E)-5-(4-Methoxyphenyl)-N-[4-(3-pyridinyl)butyl]-2,4-decadien amid. Eine Probe davon wird aus Essigester/Hexan umkristallisiert, wobei man eine analytische reine Probe mit einem Schmelzpunkt von 81,5-82,5 ° C erhält. Elementaranalyse für $C_{26}H_{34}N_2O_2$: C 76,81, H 8,43, N 6,89; Gefunden: C 77,76, H 8,56, N 6,74.

Beispiel 83

Wie in Beispiel 2 wird eine Lösung von (E,E)-5-(4-Methoxyphenyl)-2,4-decadiencarbonsäure-4-nitrophenylester (13,2 g) und (R)-α-Methyl-3-pyridinbutanamin (5,6 g) in Tetrahydrofuran (100 ml) während 2 Tagen bei Raumtemperatur gerührt. Nach dem üblichen Aufarbeiten wird das Amid mittels HPLC (Essigester) gereinigt und dann aus Essigester/Hexan umkristallisiert. Man erhält 12,5 g [R-(E,E)]-5-(4-Methoxyphenyl)-N-[1-methyl-4-(3-pyridinyl)butyl]-2,4-decadienamid mit einem Schmelzpunkt von 88-89 ° C; $[\alpha]_D^{RT}$ -28,65 ° (c 1,0;MeOH). Elementaranalyse für $C_{27}H_{36}N_2O_2$: C 77,10, H 8,63, N 6,66; Gefunden: C 77,21, H 8,72, N 6,65.

Beispiel 84

Wie in Beispiel 2 wird eine Lösung von (E,E)-5-(4-Methoxyphenyl)-2,4-decadiencarbonsäure-4-nitrophenylester (7,9 g) und (S)-α-Methyl-3-pyridinbutanamin (3,35 g) in Tetrahydrofuran (75 ml) während 17 Stunden bei Raumtemperatur und dann während 3 Stunden unter Rückfluss gerührt. Nach dem üblichen Aufarbeiten wird das rohe Amid aus Essigester/Hexan umkristallisiert. Man erhält 7,19 g [S-(E,E)]-5-(4-Methoxyphenyl)-N-[1-methyl-4-(3-pyridinyl)butyl]-2,4-decadienamid mit einem Schmelzpunkt von 88-89 ° C; $[\alpha]_D^{RT}$ +28,6 ° (c 1,0; MeOH). Elementaranalyse für $C_{27}H_{36}N_2O_2$: C 77,10, H 8,63, N 6,66; Gefunden: C 77,31, H 8,67, N 6.70.

Beispiel 85

Wie in Beispiel 2 wird eine Lösung von (2E,4Z)-5-(4-Methoxyphenyl)-2,4-decadiencarbonsäure-4-nitrophenylester (1,8 g) und (R)-α-Methyl-3-pyridinbutanamin (0,94 g) in Tetrahydrofuran (20 ml) über Nacht bei Raumtemperatur gerührt. Nach dem üblichen Aufarbeiten wird das Rohprodukt mittels HPLC (Essigester) gereinigt und dann aus Benzol lyophilisiert. Man erhält 1,8 g [R-(2E,4Z)]-5-(4-Methoxyphenyl)-N-[1-methyl-4-(3-pyridinyl)butyl]-2,4-decadienamid als Semi-Festkörper. Elementaranalyse für $C_{27}H_{36}N_2O_2$: C 77,10, H

8,63, N 6,66; Gefunden: C 76,62, H 8,60, N 6,64.

Beispiel 86

Wie in Beispiel 2 wird eine Lösung von (E,E)-5-(3-Fluorphenyl)-2,4-decadiencarbonsäure-4-nitrophenylester (1,53 g) und (R)-α-Methyl-3-pyridinbutanamin (0,75 g) in Tetrahydrofuran (100 ml) während 2 Tagen bei Raumtemperatur gerührt. Nach dem üblichen Aufarbeiten wird das Amid mittels HPLC (Essigester) gereinigt und dann aus Ether kristallisiert. Man erhält 1,2 g [R-(E,E)]-5-(3-Fluorphenyl)-N-[1-methyl-4-(3-pyridinyl)butyl]-2,4-decadienamid mit einem Schmelzpunkt von 87-88° C. Elementaranalyse für $C_{26}H_{33}FN_2O$: C 76,42, H 8,14, F 4,65, N 6,86; Gefunden: C 76,46, H 8,45, F 4,48, N 6,86.

Beispiel 87

Wie in Beispiel 2 wird eine Lösung von (2E,4Z)-5-(3-Fluorphenyl)-2,4-decadiencarbonsäure-4-nitrophenylester (1,53 g) und (R)-α-Methyl-3-pyridinbutanamin (0,75 g) in Tetrahydrofuran (20 ml) während 2 Tagen bei Raumtemperatur gerührt. Nach dem üblichen Aufarbeiten wird das Rohprodukt mittels HPLC (Essigester) gereinigt und dann aus Ether/Hexan kristallisiert. Man erhält 1,1 g [R-(2E,4Z)]-5-(3-Fluorphenyl)-N-[1-methyl-4-(3-pyridinyl)butyl]-2,4-decadienamid mit einem Schmelzpunkt von 71-73° C. Elementaranalyse für $C_{26}H_{33}FN_2O$: C 76,43, H 8,14, F 4,65, N 6,86; Gefunden: C 76,71, H 8,14, F 4,57, N 6,89.

Beispiel 88

Wie in Beispiel 2 wird eine Lösung von (E,E)-5-(4-Fluorphenyl)-2,4-decadiencarbonsäure-4-nitrophenylester (1,53 g) und (R)-α-Methyl-3-pyridinbutanamin (0,75 g) in Tetrahydrofuran (20 ml) während 2 Tagen bei Raumtemperatur gerührt. Nach dem üblichen Aufarbeiten wird das Amid mittels HPLC (Essigester) gereinigt und dann aus Ether kristallisiert. Man erhält 1,34 g [R-(E,E)]-5-(4-Fluorphenyl)-N-[1-methyl-4-(3-Pyridinyl)butyl]-2,4-decadienamid mit einem Schmelzpunkt von 76-77° C. Elementaranalyse für $C_{26}H_{33}FN_2O$: C 76,43, H 8,14, F 4,65, N 6,86; Gefunden: C 76,53, H 8,33, F 4,33, N 6,92.

Beispiel 89

Wie in Beispiel 2 wird eine Lösung von (E,E)-5-(3,4-Dimethoxyphenyl)-4-decadiencarbonsäure-4-nitrophenyl-ester und (R)-α-Methyl-3-pyridinbutanamin (0,75 g) in Tetrahydrofuran (20 ml) während 2 Tagen bei Raumtemperatur gerührt. Nach dem üblichen Aufarbeiten wird das Rohprodukt zunächst mittels HPLC (Essigester) gereinigt und dann aus Ether kristallisiert. Man erhält 1,52 g [R-(E,E)]-5-(3,4-Dimethoxyphenyl)-N-[1-methyl-4-(3-pyridinyl)butyl-2,4-decadienamid mit einem Schmelzpunkt von 67-69° C. Elementaranalyse für $C_{28}H_{38}N_2O_3$: C 74,63, H 8,50, N 6,22; Gefunden: C 74,68, H 8,64, N 6,26.

Beispiel 90

Wie in Beispiel 2 wird eine Lösung von (E,E)-5-(4-Methoxyphenyl)-2,4-decadiencarbonsäure-4-nitrophenylester (1,1 g) und (R,S)-2,α-Dimethyl-3-pyridinbutanamin (0,5 g) in Tetrahydrofuran (12 ml) über Nacht bei Raumtemperatur gerührt. Nach dem üblichen Aufarbeiten wird das Amid mittels Chromatographie über Kieselgel (Essigester) gereinigt und dann aus Benzol lyophilisiert. Man erhält 0,95 g [R,S-(E,E)]-5-(4-Methoxyphenyl)-N-[1-methyl-4-(2-methyl-3-pyridinyl)butyl]-2,4-decadienamid (0,5 molar) als glasigen Festkörper. Elementaranalyse für $C_{28}H_{38}N_2O_2/0,5H_2O$: C 75,81, H 8,86, N 6,31; Gefunden: C 75,59, H 8,75, N 6,42.

Beispiele 91

Wie in Beispiel 2 wird eine Lösung von (E,E)-5-(4-Methoxyphenyl)-2,4-decadiencarbonsäure-4-nitrophenylester (1,72 g) und [(R,S)-(Z)]-5-(3-Pyridinyl)-4-penten-2-amin (0,811 g) in Tetrahydrofuran (20 ml) während 2 Tagen bei Raumtemperatur gerührt. Nach dem üblichen Aufarbeiten wird das Rohprodukt zunächst mittels HPLC (Essigester) gereinigt und dann aus Ether kristallisiert. Man erhält 1,32 g [R,S-(E,E)]-5-(4-Methoxyphenyl)-N-[(Z)-1-methyl-4-(3-pyridinyl)-3-butenyl]-2,4-decadienamid mit einem Schmelzpunkt von 79-81° C. Elementaranalyse für $C_{27}H_{34}N_2O_2$: C 77,47, H 8,19, N 6,69; Gefunden: C 77,67, H 8,20, N 6.80.

Beispiel 92

Wie in Beispiel 2 wird eine Lösung von (E,E)-5-(4-Methoxyphenyl)-2,4-undecadiencarbonsäure-4-nitrophenylester (1,78 g) und (R)-α-Methyl-3-pyridinbutanamin (0,85 g) in Tetrahydrofuran (20 ml) über Nacht bei Raumtemperatur gerührt. Nach dem üblichen Aufarbeiten wird das Rohprodukt mittels HPLC (Essigester/Hexan; 9:1) gereinigt und dann aus Ether/Hexan kristallisiert. Man erhält 0,84 g [R-(E,E)]-5-(4-Methoxyphenyl)-N-[1-methyl-4-(3-pyridinyl)-butyl]-2,4-undecadienamid mit einem Schmelzpunkt von 71,5-73 ° C. Elementaranalyse für $C_{28}H_{38}N_2O_2$: C 77,38, H 8,81, N 6,44; Gefunden: C 77,69, H 9,06, N 6,56.

Beispiel 93

Wie in Beispiel 2 wird eine Lösung von (E)-5-Pentyl-2,4-decadiencarbonsäure-4-nitrophenylester (1,08 g) und (R)-α-Methyl-3-pyridinbutanamin (0,5 g) in Tetrahydrofuran (15 ml) über Nacht bei Raumtemperatur gerührt. Nach dem üblichen Aufarbeiten wird das Rohprodukt mittels HPLC (Essigester/Hexan; 7:3) gereinigt. Man erhält 0,9 g [R-(E)]-N-[1-Methyl-4-(3-pyridinyl)butyl]-5-pentyl-2,4-decadienamid als Oel.

Beispiel 94

In Analogie zu den Angaben in Beispiel 2 erhält man aus (E,E)-5-(4-Methoxyphenyl)-2,4-decadiencarbonsäure-4-nitrophenylester und (S)-α,2-Dimethyl-3-pyridinbutanamin das [S-(E,E)]-5-(4-Methoxyphenyl)-N-[1-methyl-4-(2-methyl-3-pyridinyl)butyl]-2,4-decadienamid mit einem Schmelzpunkt von 87-89 ° C (Essigester/Hexan), $[\alpha]_D$ + 29,07 ° (c 0,9734; EtOH).

Beispiel 95

In Analogie zu den Angaben in Beispiel 2 erhält man aus (E,E)-5-(4-Methoxyphenyl)-2,4-decadiencarbonsäure-4-nitrophenyles ter und (R)-α,2-Dimethyl-3-pyridinbutanamin das [R-(E,E)]-5-(4-Methoxyphenyl)-N-[1-methyl-4-(2-methyl-3-pyridinyl)butyl]-2,4-decadienamid mit einem Schmelzpunkt von 87-89 ° C (Essigester/ Hexan), $[\alpha]_D$ -26,53 ° (c 1,0098; EtOH).

Beispiel 96

Zu einer Lösung von (E,E)-5-(4-Methoxyphenyl)-N-[4-(3-pyridinyl)butyl]-2,4-decadienamid (4,88 g) in trockenem Tetrahydrofuran (110 ml) in einem Ultraschallbad wird Phosphorpentasulfid (2,67 g) dazugegeben. Die Mischung wird während 1 Stunde gerührt, worauf man die überstehende Lösung vom gelben Festkörper, der sich gebildet hat, abdekantiert und den Festkörper mit 4 x 100 ml Portionen an Methylenchlorid behandelt. Die vereinigten Auszüge werden mit 1N Natriumhydroxid (3x 100 ml) und mit Kochsalzlösung gewaschen, getrocknet ($K_2CO_3$) und eingedampft. Man erhält 2,75 g des rohen Thioamides. Durch Kristallisation des Rohproduktes aus Essigester/Hexan (2x) und aus 2-Propanol/Hexan erhält man 1,31 g (E,E)-5-(4-Methoxyphenyl)-N-[4-(3-pyridinyl)butyl]-2,4-decadienthioamid als gelben Festkörper mit einem Schmelzpunkt von 121-122 ° C.

Beispiel 97

Wie in Beispiel 2 wird eine Lösung von (E,E)-5-(3-Methoxyphenyl)-5-phenyl-2,4-pentadiencarbonsäure-4-nitrophenylester (3,3 g) und 3-pyridinbutanamin (1,24 g) in Tetrahydrofuran (20 ml) während 1 Stunde bei Raumtemperatur gerührt. Nach dem üblichen Aufarbeiten wird das rohe Amid mittels HPLC (Essigester) gereinigt und aus Essigester kristallisiert. Man erhält 2,7 g (2E,4Z)-5-(3-Methoxyphenyl)-5-phenyl-N-[4-(3-pyridinyl)butyl]-2,4-pentadienamid mit einem Schmelzpunkt von 89-91 ° C. Eine Portion wird aus dem gleichen Lösungsmittel umkristallisiert, wobei man das reine Amid mit einem Schmelzpunkt von 90-92 ° C erhält. Elementaranalyse für $C_{27}H_{28}N_2O_2$: C 78,61. H 6,84, N 6,79; Gefunden: C 78,88, H 6,87, N 6,66.

Beispiel 98

Wie in Beispiel 2 wird eine Lösung von (2E,4Z)-5-(3-Fluorphenyl)-5-(3-methoxyphenyl)-2,4-pentadiencarbonsäure-4-nitrophenylester (3,4 g) und 3-Pyridinbutanamin (1,23 g) in Tetrahydrofuran (20 ml) über Nacht bei Raumtemperatur gerührt und dann in üblicher Weise aufgearbeitet. Das rohe Amid wird mittels HPLC (Essigester) gereinigt und aus Essigester/Hexan kristallisiert. Man erhält 2,6 g (2E,4Z)-5-(3-Fluorphenyl)-5-(3-methoxyphenyl)-N-[4-(3-pyridinyl)butyl]-2,4-pentadienamid mit einem Schmelzpunkt von 96-98 ° C. Eine Portion wird aus Essigester/Hexan umkristallisiert, wobei man eine analytisch reine Probe mit einem Schmelzpunkt von 98-100 ° C erhält. Elementaranalyse für $C_{27}H_{27}FN_2O_2$: C 75,33, H 6,32, F 4,41, N 6,51; Gefunden: C 75,05, H 6,28, F 4,42, N 6,41.

## Teil II: Herstellung von pharmazeutischen Präparaten

[R-(E,E)]-5-(4-Methoxyphenyl)-N-[1-methyl-4-(3-pyridinyl)butyl]-2,4-decadienamid kann als Wirkstoff in den nachfolgenden pharmazeutischen Präparaten verwendet werden:

### Beispiel A

Inhalations-Aerosol-Formulierung (Lösung)

| | Bestandteile | %Gew/Gew |
|---|---|---|
| 1. | Wirkstoff | 1,0 |
| 2. | Aethylalkohol | 30,0 |
| 3. | Ascorbinsäure | 0,5 |
| 4. | Freon 12 | 54,8 |
| 5. | Freon 114 | 13,7 |
| | TOTAL | 100 % |

Herstellungsverfahren:

1) Löse die Bestandteile 1 und 3 in Bestandteil 2.
2) Fülle die Lösung aus der Stufe 1 in geeignete Glasbehälter und verschliesse diese mit einem Ventil.
3) Fülle unter Druck eine Mischung (80:20) der Bestandteile 4 und 5 in diese Behälter.

Anmerkung: Man kann ein geeignetes Ventil verwenden, das bei Betätigung ein Volumen von 25-100 Mikroliter freigibt.

### Beispiel B

Inhalations-Aerosol-Formulierung (Suspension)

| | Bestandteile | % Gew/Gew |
|---|---|---|
| 1. | Wirkstoff | 1,0 |
| 2. | Sorbitantrioleat | 0,5 |
| 3. | Freon 12 | 64,0 |
| 4. | Freon 11 | 18,5 |
| 5. | Freon 114 | 16,0 |
| | TOTAL | 100 % |

Herstellungsverfahren:

1) Mische die Bestandteile 1 und 2 zu 4 und homogenisiere.
2) Fülle die konzentrierte Suspension aus Stufe 1 in geeignete Behälter und verschliesse diese mit einem Ventil.
3) Fülle unter Druck eine Mischung (80:20) der Bestandteile 3 und 5 ein.

Anmerkung: Man kann ein geeignetes Ventil verwenden, das bei Betätigung ein Volumen von 25-100 Mikroliter abgibt.

Beispiel C

Tablettenformulierung (Feuchtgranulation)

| | Bestandteile | mg/Tablette | |
|---|---|---|---|
| | | 100 mg | 500 mg |
| 1. | Wirkstoff | 100 | 500 |
| 2. | Lactose | 30 | 150 |
| 3. | Vorgelatinisierte Stärke | 6 | 30 |
| 4. | Microkristalline Zelluose | 30 | 150 |
| 5. | Magnesiumstearat | 1 | 6 |
| | TOTAL | 167 | 836 |

Herstellungsverfahren:

1) Mische Bestandteile 1, 2, 3 und 4 und granuliere mit Wasser.
2) Trockne das Granulat bei 50 ° C.
3) Mahle das Granulat in einer geeigneten Mahlapparatur.
4) Gib Bestandteil 5 dazu und mische während 3 Minuten. Verpresse auf einer geeigneten Presse.

Beispiel D

Kapselformulierung

| | Bestandteile | mg/Kapsel | |
|---|---|---|---|
| | | 100 mg | 500 mg |
| 1. | Wirkstoff | 100 | 500 |
| 2. | Maisstärke | 8 | 40 |
| 3. | Modifizierte Stärke | 4 | 20 |
| 4. | Talk | 4 | 20 |
| 5. | Magnesiumstearat | 1 | 2 |
| | TOTAL | 117 | 582 |

Herstellungsverfahren:

1) Mische Bestandteile 1, 2, und 3 und granuliere mit Wasser. Trockne über Nacht bei 45 ° C.
2) Mahle in einer geeigneten Apparatur unter Verwendung eines geeigneten Siebes.
3) Gib die Bestandteile 4 und 5 dazu und mische während 5 minuten.
4) Fülle in geeignete Kapseln ab.

Beispiel E

Kapselformulierung

| | Bestandteile | mg/Kapsel | | | |
|---|---|---|---|---|---|
| 1. | Wirkstoff | 0,01 | 0,5 | 5,0 | 25,0 |
| 2. | Lactose (Hydratisiert) | 168,99 | 168,5 | 159,0 | 123,0 |
| 3. | Maisstärke | 20,0 | 20,0 | 25,0 | 35,0 |
| 4. | Talk | 10,0 | 10,0 | 10,0 | 15,0 |
| 5. | Magnesiumstearat | 1,0 | 1,0 | 1,0 | 2,0 |
| | TOTAL | 200,0 | 200,0 | 200,0 | 200,0 |

Herstellungsverfahren;

1) Mische die Bestandteile 1, 2 und 3 in einem geeigneten Mixer während 30 Minuten.
2) Gib die Bestandteile 4 und 5 dazu und mische während 3 Minuten.
3) Fülle in geeignete Kapseln ab.

Beispiel F

Tablettenformulierung (Feuchtgranulation)

| | Bestandteile | mg/Tablette | | | |
|---|---|---|---|---|---|
| 1. | Wirkstoff | 0,01 | 0,5 | 5,0 | 25,0 |
| 2. | Lactose DTG | 106,99 | 106,5 | 102,0 | 118,0 |
| 3. | Avicel PH 102 | 15,0 | 15,0 | 15,0 | 25,0 |
| 4. | Modifizierte Stärke | 7,0 | 7,0 | 7,0 | 10,0 |
| 5. | Magnesiumstearat | 1,0 | 1,0 | 1,0 | 2,0 |
| | TOTAL | 130,0 | 130,0 | 130,0 | 180,0 |

Herstellungsverfahren:

1) Löse Bestandteil 1 in einem geeigneten Lösungsmittel, wie Alkohol.
2) Sprühe die Lösung aus Stufe 1 über Bestandteil 2, trockne.
3) Gib die Bestandteile 3 und 4 dazu und mische während 10 Minuten.
4) Gib das Magnesiumstearat dazu, mische während 3 Minuten und verpresse.

Beispiel G

Creme (0,5%)

Nachfolgend wird die quantitative Zusammensetzung der Formulierung gegeben:

| Bestandteile | g/kg | Vernünftige Variationen |
|---|---|---|
| Wirkstoff | 5,150 | ---- |
| Glycerylmonostearat S.E. (Arlacel 165) | 100,00 | 80-120 |
| Polysorbat 60 (Tween 60) | 20,00 | 15-25 |
| Cetyl-Alkohol | 50,00 | 40-60 |
| Petrolatum | 70,00 | 50-90 |
| Methylparaben | 1,50 | 1,25-1,75 |
| Propylparaben | 0,50 | 0,4-0,6 |
| Propylenglycol | 200,00 | 150-250 |
| Gereinigtes Wasser | 568,05 | 475-575 |
| TOTAL | 1'015,20 | |

Teil III: Herstellung von Verbindungen der Formel V.

Beispiel 1

a) In einer inerten Atmosphäre werden bei Raumtemperatur 26 g bis(Triphenylphosphin)-palladiumdichlorid und 2.28 g Kupfer(I)jodid zu einer gerührten Lösung von 311,2 g (R,S)-4-Pentin- 2-ol, 556.8 g 3-Brompyridin und 665 ml Triethylamin in 1,8 1 Methylenchlorid gegeben. Die Reaktion ist leicht exotherm, und nach Rühren während 75 Min. wird die Rückflusstemperatur ereicht. Nach Beendigung des leichten Siedens (40 Min.) wird das Reaktionsgemisch noch während 5 Stunden erwärmt. Nach Abkühlen wird über Nacht bei Raumtemperatur gerührt, worauf man mit 1 l Wasser und 500 g Eis und anschliessend mit 420 ml conc. Salzsäure (HCl) versetzt und noch während mehrerer Minuten rührt. Nach Trennen der Phasen wird die wässrige Phase mit Methylenchlorid extrahiert (4 x 1 l). Die organischen Phasen werden anschliessend mit 1 l 1N HCl gewaschen, bevor sie verworfen werden. Die ursprüngliche wässrige Phase wird mit 500 ml 10N Natronlauge (NaOH) behandelt und die zweite wässrige Phase wird mit 200 ml 10N NaOH basisch gestellt, bevor sie nacheinander mit Methylenchlorid (1 x 2 l; 3 x 1 l) extrahiert werden. Die vereinigten organischen Auszüge werden über Kaliumcarbonat ($K_2CO_3$) getrocknet und unter vermindertem Druck zum konstanten Gewicht eingedampft. Man erhält 477,3 g rohes (R,S)-α-Methyl-4-(3-pyridinyl-3-butin-1-ol als bernsteinfarbenes Oel.

b) Das rohe (R,S)-α-Methyl-4-(3-pyridinyl)-3-butin-1-ol(477,3 g) wird bei Raumtemperatur und Atmosphärendruck in 3,51 Ethanol über 20 g Platinoxid hydriert. Nach Beendigung der Wasserstoffaufnahme wird der Katalysator abfiltriert, und das Lösungsmittel wird unter vermindertem Druck abgedampft. Das verbeibende Oel wird in einem Kugelrohr-Apparat destilliert (115-120°C/0.1 mm (13,3 Pa)). Man erhält 420,5 g (R,S)-α- Methyl-3-pyridinbutanol.

c) Eine gerührte Lösung von 218,6 g Oxalylchlorid in 1.5 l trockenem Methylenchlorid wird unter Argon auf -75°C abgekühlt. Anschliessend wird eine Mischung von 141 g trockenem Dimethylsulfoxid in 200 ml Methylenchlorid über einen Zeitraum von 75 Min. so dazu getropft, dass die Reaktionstemperatur nicht über -72°C steigt. Man rührt die Mischung während 10 Min. bei -75°C und versetzt dann über einen Zeitraum von 55 Min. tropenweise mit einer Lösung von 271,5 g (R,S)-α-Methyl- 3-pyridinbutanol in 125 ml Methylenchlorid, und zwar so dass die Reaktionstemperatur unterhalb -70°C gehalten werden kann. Nach Beendigung der Zugabe rührt man noch während weiteren 30 Min. bei -75°C und versetzt dann über einen Zeitraum von 65 Min. mit 520 ml Triethylamin, wobei man die Reaktionstemperatur zwischen -65°C und -70°C hält. Anschliessend wird das Kühlbad entfernt. Man lässt die Reaktionstemperatur während 1 Stunde auf Raumtemperatur ansteigen, versetzt dann mit 1 l Wasser und trennt die Phasen. Die wässrige Phase wird mit Methylenchlorid (2 x 800 ml) extrahiert und die organische Phase und die Auszüge werden ihrerseits mit 800 ml 1,5N NaOH und mit 800 ml 10%-Natriumchloridlösung (NaCl) gewaschen. Die vereinigten organischen Auszüge werden getrocknet ($K_2CO_3$) und eingedampft. Man erhält 266 g rohes Keton. Das Produkt wird destilliert, wobei man 248.6 g 5-(3-Pyridinyl)-2-pentanon erhält (Sdp. 100-102°C/0.2mm (26,7 Pa)).

d) Eine Mischung von 248,5 g 5-(3-Pyridinyl)-2-pentanon, 95,85 g Natriumcyanoborhydrid und 1170 g Ammoniumacetat in 5,3 1 trockenem Methanol wird während 8 Tagen bei Raumtemperatur gerührt. 3 l Methanol werden dann unter vermindertem Druck abgedampft (interne Temperatur ca. 30 ° C). Man kühlt die Reaktionsmischung in einem Eisbad und versetzt über einen Zeitraum von 2 Stunden tropfenweise mit 3,8 l 6N HCl. Man rührt die Mischung über Nacht bei Raumtemperatur und stellt sie durch Zugabe von 2 l 12.5N NaOH stark basisch und extrahiert sie mit Methylenchlorid (1 x 2 l; 2 x 1 l). Die vereinigten Auszüge werden getrocknet ($K_2CO_3$) und eingedampft. Man erhält 244 g eines hellbraunen Oeles, das destilliert wird. Man erhält 205 g (R,S)-α-Methyl-3-pyridinbutanamin (Sdp. 95-100 ° C/0,15 mm (20 Pa)).

Beispiel 2

a) In einer inerten Atmosphäre wird eine Lösung von 2.42 ml Methansulfonylchlorid in 10 ml Methylenchlorid über einen Zeitraum von 10 Min. zu einer gerührten Mischung von 5,0 g (R,S)-α-Methyl-3-pyridinbutanol und 6,2 ml Triethylamin in 50 ml trockenem Methylenchlorid, welche bei -40 ° C gehalten wird, gegeben. Nach 30 Min. wird das Reaktionsgemisch auf 0 ° C erwärmt, worauf man ein kleines Stück Eis dazu gibt und die Mischung während weiterer 15 Min. in einem Eisbad rührt. Die Lösung wird dann nacheinander mit Wasser (3 x 15 ml), 1N NaOH (2 x 15 ml) und Kochsalzlösung (10 ml) gewaschen. Die getrocknete ($K_2CO_3$) organische Phase wird eingedampft. Man erhält 7,2 g (R,S)-Methansulfonsäure-5-(3-pyridinyl)-2-pentylester.
b) Eine Mischung von 2,5 g (R,S)-Methansulfonsäure-5-(3-pyridinyl)-2-pentylester, 0,832 g Natriumazid, 1,5 ml Wasser und 15 ml Dimethylformamid wird unter Argon während 150 Min. bei 50 ° C gerührt. Die abgekühlte Lösung wird mit 40 ml Wasser vedünnt und mit Methylenchlorid (3 x 30 ml) extrahiert. Die Extrakte werden mit Wasser (2 x 20 ml) gewaschen und dann vereinigt, getrocknet ($K_2CO_3$) und eingedampft. Man erhält 1,76 g (R,S)-3-(4-Azidopentyl)pyridin als Oel.
c) Eine Lösung von 0,9 g (R,S)-3-(4-Azidopentyl)pyridin in 25 ml Ethanol wird bei 50 psi (345 kPa) über 0.05 g 10% Pd/C hydriert. Nach 105 Min. wird der Katalysator abfiltriert, und das Lösungsmittel wird unter vermindertem Druck eingedampft. Man erhält 0.69 g eines farblosen Oeles. Durch evaporative Destillation des Rohproduktes erhält man 0,57 g (R,S)-α-Methyl-3-Pyridinbutanamin.

Beispiel 3

a) Eine Lösung von 86,3 g (R,S)-4-Brom-2-methylbutansäureethylester und 104,9 g Triphenylphosphin in 600 ml Toluol wird während 4 Tagen unter Rückfluss gerührt. Im Verlauf der Reaktion fällt das gebildete Phosphoniumbromid in Form eines Oeles aus. Nach Abkühlen der Reaktionsmischung wird die überstehende Toluolphase abdekantiert, worauf man 500 ml frisches Toluol dazu gibt. Das Reaktionsgemisch wird während 30 Min. unter Rückfluss gerührt, abgekühlt, worauf man die Toluolphase erneut abdekantiert. Man wiederholt diesen Schritt noch ein weiteres Mal und trocknet dann das verbleibende Oel im Vakuum. Man erhält 187 g (R,S)-3-(Ethoxycarbonyl)butyltriphenylphosphoniumbromid als viskoses Oel.
b) Eine gerührte Lösung von 10,56 g Natriumhydrid (60% Dispersion in Oel) in 1000 ml trockenem Dimethylsulfoxid wird bis zu Beendigung der Wasserstoffentwicklung (30 Min.) auf 70 ° C erhitzt. Die Lösung wird dann auf 0 ° C abgekühlt und mit einer Lösung von 103.7 g (R,S)-3-(Ethoxycarbonyl)butyl-tri-phenylphosphoniumbromid in 200 ml Dimethylsulfoxid versetzt. Man rührt das Reaktionsgemisch noch während 30 Min. bei Raumtemperatur, versetzt dann mit einer Lösung von 20,8 ml 3-Pyridincarboxaldehyd in 100 ml Tetrahydrofuran und rührt noch über Nacht bei Raumtemperatur. Die Mischung wird mit Eiswasser verdünnt und mit Methylenchlorid extrahiert (6 x 150 ml). Die vereinigten organischen Auszüge werden dann mit 4 x 400 ml 0.5N HCl extrahiert. Die sauren Auszüge werden mit 125 ml Triethylamin basisch gestellt und mit Methylenchlorid extrahiert (5 x 150 ml). Die getrockneten ($K_2CO_3$) Auszüge werden eingedampft, wobei man 34 g Rohprodukt erhält. Man reinigt dieses Material zunächst mittels HPLC (Aether/Hexan; 3:2) und erhält 11.9 g einer Mischung der (Z)- und (E)-Isomeren (4:1). Eine weitere Auftrennung dieser Mischung mittels HPLC unter Rezyclisierung liefert 6.77 g [(R,S)-Z]- 2-Methyl-5-(3-pyridinyl)-4-pentensäure-ethylester und 3 g einer Mischung aus [(R,S)-Z]-2-Methyl-5-(3-pyridinyl)-4-pentensäure-ethylester und des entsprechenden (E)-Isomeren.
c) Eine Lösung von 5,5 g einer Mischung (1:1) von [R,S)-E]- und [(R,S)-Z]-2-Methyl-5-(3-pyridinyl)-4-pentensäure-ethylester in 100 ml Ethanol wird über 0.4 g 10% Palladium/Kohle (Pd/C) hydriert. Nach Beendigung der Wasserstoffaufnahme wird der Katalysator abfiltriert und das Lösungsmittel wird abgedampft. Man erhält 5,33 g (R,S)-α-Methyl-3-pyridinpentancarbonsäure-ethylester.
d) Eine Mischung aus 5.3 g (R,s)-α-Methyl-3-pyridinpentancarbonsäure-ethylester, 35 ml 1N NaOH und 35 ml Methanol wird während 3 Stunden unter Rückfluss gerührt. Anschliessend wird das meiste

Methanol unter vermindertem Druck abgedampft. Die Lösung wird mit 100 ml Wasser verdünnt und mit Methylenchlorid extrahiert (3 x 35 ml). Die wässrige Phase wird dann mit 35 ml 1N HCl neutralisiert und mit Methylenchlorid extrahiert (3 x 30 ml). Die Auszüge werden über Natriumsulphat ($Na_2SO_4$) getrocknet und eingedampft. Man ehält 3.47 g (R,S)-α-Methyl-3-pyridinpentancarbonsäure.

e) Wie in Beispiel 11b) werden 1,93 g (R,S)-α-Methyl-3-pyridinpentancarbonsäure mit 2,21 ml Diphenylphosphorylazid in 10 ml t-Butanol enthaltend 1,4 ml Triethylamin behandelt. Man erhält 2,45 g Rohprodukt, das mittels HPLC (Essigester) gereinigt wird. Man erhält 2.2 g (R,S)-[1-Methyl 4-(3-pyridinyl)butyl]carbaminsäure-1,1-dimethylethylester als farbloses Oel.

f) Wie in Beispiel 11c) werden 2,1 g (R,S)-[1-Methyl-4-(3-pyridinyl)butyl]carbaminsäure-1,1-dimethylethylester in 25 ml 1N HCl hydrolysiert. Nach üblichem Aufarbeiten und evaporativer Destillation des Produktes (95-100 ° C/0,2mm (26,7 Pa)) erhält man 1,25 g (R,S)-α-Methyl-3-pyridinbutanamin.

Beispiel 4

a) Man versetzt eine Mischung von 10,6 g (R,S)-α-Methyl-3-pyridinbutanol in 30 ml Acetonitril mit 20 ml Schwefelsäure. Man rührt während 2 Stunden bei 50 ° C und giesst dann auf eine eine Mischung aus 500 g Eis und 400 ml 4N NaoH und extrahiert mit Methylenchlorid (2 x 150 ml). Nach Eindampfen der getrockneten ($K_2CO_3$) Auszüge erhält man 6 g Rohprodukt, das man mittels HPLC (Methanol/Essigester; 1:49) reinigt und dann mit Aether behandelt. Man erhält 2,9 g (R,S)-N-[1-Methyl-4-(3-pyridinyl] butyl]-acetamid mit einem Schmelzpunkt von 70-71,5 ° C.

b) Eine Lösung von 2,06 g (R,S)-N-[1-Methyl-4-(3-pyridinyl)butyl]acetamid in 50 ml 6N HCl wird während 22 Stunden unter Rückfluss gerührt. Die abgekühlte Mischung wird in einer Argonatmosphäre durch vorsichtige Zugabe von 30 ml 10N NaOH basisch gestellt und mit Methylenchlorid extrahiert (2 x 75 ml). Die Auszüge werden mit Kochsalzlösung gewaschen, vereinigt, getrocknet ($K_2CO_3$) und eingedampft. Man erhält 1.43 g (R,S)-α-Methyl-3-pyridinbutanamin.

Beispiel 5

aa) Eine Lösung von 281,5 g 1,3-Dicyclohexylcarbodiimid in 400 ml Dimethylformamid wird unter Rühren zu einer Lösung von 204 g (R,S)-α-Methyl-3-pyridinbutanamin, 198,4 g (R)-Mandelsäure und 209,75 g 1-Hydroxybenzotriazol in 1400 ml Dimethylformamid gegeben, wobei man die Temperatur durch gelegentliches Kühlen mit einem Trockeneis/Acetonbad bei -10 ° C hält. Man rührt während 4 Stunden bei -5 ° C, dann über Nacht bei Raumtemperatur und kühlt dann für 2 Stunden wieder auf 0 ° C ab. Die ausgefallenen Festkörper werden abfiltriert und nacheinander mit kaltem Dimethylformamid (2 x 150 ml) und Essigester (2 x 300 ml) gewaschen. Dieses Material, eine Mischung von 1,3-Dicyclohexylharnstoff (DCU) und dem weniger löslichen (R*,R)-Mandelsäureamid, wird in 2 l 1N HCl suspendiert und während 3 Stunden bei Raumtemperatur gerührt. Das unlösliche Material (DCU) wird abfiltriert und mit 200 ml verdünnter HCl und Wasser gewaschen. Das Filtrat wird basisch gestellt, und das ausgefallene Material wird abfiltriert, mit Wasser gewaschen und im Vakuum getrocknet. Man erhält 64,4 g [R-(R*,R*)]-α-Hydroxy-N-[1-methyl-4-(3-pyridinyl)butyl]benzolacetamid, das (R*,R)-Mandelsäureamid, mit einem Schmelzpunkt von 144-146 ° C; $[\alpha]_D^{25}$ -27,8 ° (c 1,0; MeOH).

Die ursprünglichen Mutterlaugen und Waschlösungen werden unter vermindertem Druck zur Trockne eingedampft, und der Rückstand wird in 2 l 1,5N NaOH suspendiert. Man extrahiert mit Methylenchlorid (1 x 2 l, 2 x 1 l), wäscht die organischen Auszüge nacheinander mit 1N NaOH (2 x 800 ml) und 1N HCl (1 x 1,5 l, 2 x 750 ml), stellt die vereinigten wässrigen Phasen mit 350 ml 10N NaOH basisch und extrahiert mit Methylenchlorid (1 x 2 l, 2 x 1 l). Die Auszüge werden getrocknet ($K_2CO_3$) und eingedampft. Man erhält 280 g Mandelsäureamid, das mit dem (S*,R)-Diastereomeren angereichert ist (ungefähr 3:2). Der Rückstand wird 3x aus 2-Propanol kristallisiert, und man erhält 74,1 g des weniger löslichen (R*,R)-Mandelsäureamides mit einem Schmelzpunkt von 144-146 ° C.

Die Mutterlaugen der beiden letzten Kristallisationen werden vereinigt und eingedampft. Der Rückstand wird 2x aus 2-Propanol kristallisiert, wobei man eine zusätzliche Portion von 7.2 g des (R*,R)-Diastereomeren mit einem Schmelzpunkt von 143-145 ° C erhält. Die gesamt Ausbeute an [R-(R*,-R*)]-α-Hydroxy-N-[1-methyl-4-(3-pyridinyl)butyl]benzolacetamid beträgt 145,6 g (78,5 %).

Alle verbleibenden Mutterlaugen werden vereinigt und eingedampft. Nach Trocknen erhält man 198 g Mandelsäureamid, das mit dem (S*,R)-Diastereoisomeren angereichert ist.

Dieses Material wird für die weitere Verarbeitung, insbesondere als Quelle für (S)-α-Methyl-4-pyridinbutanamin, auf die Seite gestellt.

ab) Eine Lösung von 70.5 g 5-(3-pyridinyl)-2-pentanon und 53,5 g (R)-(+)-α-Methylbenzylamin in 700 ml Toluol, enthaltend 1.8 g p-Toluolsulfonsäure, wird während 17 Stunden unter Rückfluss erhitzt. Das gebildete Reaktionswasser wird mittels einem Dean-Stark-Apparat entfernt. Die abgekühlte Lösung wird dann über 70 g Raney-Nickel bei Raumtemperatur und 50 psi (345 kPa) hydriert. Nach Aufnahme von etwa 50% der theoretisch berechneten Menge an Wasserstoff kommt die Reaktion praktisch zum Stillstand. Der verbrauchte Katalysator wird entfernt und durch 70 g frisches Raney-Nickel ersetzt, worauf die Hydrierung bis zur Beendigung der Wasserstoffaufnahme fortgesetzt wird. Man entfernt den Katalysator mittels Filtration, wäscht das Filtrat mit 250 ml 1N Natriumhydroxidlösung, trocknet und dampft ein. Man erhält 106 g eines Oeles. Gemäss HPLC-Analyse enthält das Produkt als Hauptkomponente ungefähr 68% [R-(R*,-R)]-N-[1-Methyl-(3-pyridinyl)butyl]-α-methylbenzylamin zusammen mit etwa 13% des entsprechenden (S*,R)-Diastereomeren.

Die obige Mischung (105 g) wird in 1 l Ethanol über 21 g 20-Proz. $Pd(OH)_2$ auf Kohle während 51 Stunden hydrogenolysiert (50°C, 25 psi (122 kPa)). Nach Abfiltrieren des Katalysators wird das Lösungsmittel abgedampft und der Rückstand wird destilliert. Man erhält 33,6 g α-Methyl-3-pyridinbutanamin, dass mit dem (R)-Enantiomeren angereichert ist.

Zu einer gekühlten (-5°C) Lösung von 32 g des obigen angereicherten Amines, 33 g 1-Hydroxybenzotriazol und 31,22 g (R)-Mandelsäure in 350 ml Dimethylformamid, gibt man eine Lösung von 44,26 g 1,3-Dicyclohexylcabodiimid in 150 ml Dimethylformamid, und rührt die erhaltene Mischung während 18 Stunden bei -5°C. Nach Abfiltrieren des ausgefallenen Dicyclohexylharnstoffes wird das Filtrat eingedampft und der Rückstand in 300 ml kalter 2N Natronlauge suspendiert. Die erhaltenen Festkörper werden abfiltriert, mit verdünnert Natronlauge und Wasser gewaschen und dann in 500 ml 2N Salzsäure aufgelöst. Die saure Lösung wird mit Methylenchlorid extrahiert (3 x 150 ml), um neutrale Verunreinigungen zu entfernen, und dann mit 10N Natronlauge basisch gestellt und mit Methylenchlorid extrahiert (6 x 300 ml). Die getrockneten Auszüge werden eingedampft, wobei man 55 g eines Festkörpers erhält. Durch Kristallisieren dieses Produktes aus Ethanol erhält man 25,6 g [R-(R*,R)-α-Hydroxy-N-[1-methyl-4-(3-pyridinyl)-butyl]benzolacetamid mit einem Schmelzpunkt von 141-143°C. Aus den Mutterlaugen erhält man weitere 1,3 g des Produktes mit einem Schmelspunkt von 141-143°C.

b) Eine Lösung von 145 g [R-(R*,R*)]-α-Hydroxy-N-[1-methyl-4-(3-pyridinyl)butyl]benzolacetamid in 900 ml 6N HCl wird mit 80 ml conc. HCl behandelt und dann während 2 Tagen unter Rückfluss erhitzt. Nachdem man den grössten Teil des Lösungsmittels unter vermindertem Druck abgedampft hat, wird der Rückstand mit 10N NaOH unter einer Argonatmosphäre basisch gestellt und mit Methylenchlorid extrahiert (1 x 1,2 l, 2 x 600 ml). Die getrockneten ($K_2CO_3$) Auszüge werden eingedampft, und das Rohprodukt wird destilliert. Man erhält 78,5 g (R)-α- Methyl-3-pyridinbutanamin (Sdp. 95°C/0.2 mm (26,7 Pa)).

Beispiel 6

aa) Zu einer eiskalten Lösung von 2,0 g (S)-(+)-Mandelsäure und 1,82 ml Triethylamin in 20 ml trockenem Dimethylformamid gibt man 2,82 ml Diphenylphosphorylazid. Die Mischung wird während 30 Min. bei 0°C gerührt, worauf man 2.15 g (R,S)-α-Methyl-3-pyridinbutanamin dazu gibt. Man rührt das Reaktionsgemisch über Nacht bei Raumtemperatur, verdünnt dann mit 130 ml Essigester, wäscht mit Wasser (4 x 50 ml), trocknet ($K_2CO_3$) und dampft ein. Der Rückstand wird aus Essigester (3x) kristallisiert, wobei man 0.45 g [S-(S*,-S)]-α-Hydroxy-N-[1-methyl-4-(3-pyridinyl)butyl]benzolacetamid mit einem Schmelzpunkt von 142-145°C erhält.

Die absolute Konfiguration dieses Produktes wird mittels Röntgenstrahlanalyse ermittelt.

ab) Eine Lösung von 160 g rohem [S-(R*,R*)]-α-Hydroxy-N-[1-methyl-4-(3-pyridinyl)butyl]benzolacetamid in 800 ml 6N HCl wird mit 85 conc. HCl behandelt und dann Wie in Beispiel 5b) beschrieben über Nacht unter Rückfluss erhitzt. Das rohe Amin (ungefähr 85 g) wird in üblicher Weise isoliert und destilliert. Man erhält 75,4 g (S)-α-Methyl-3-pyridinbutanamin (Sdp. 89-91°C/0,15 mm (20 Pa)).

Unter den in Beispiel 5aa) beschriebenen Bedingungen werden 76.2 g des Amines in Gegenwart von 105.2 g 1,3-Dicyclohexylcarbodiimid und 78,4 g 1-Hydroxybenzotriazol in 1 l Dimethylformamid mit 74,2 g (S)-Mandelsäure umgesetzt. Nach ähnlichem Aufarbeiten erhält man 109 g [S-(S*,S*)]-α-Hydroxy-N-[1-methyl-4-(3-pyridinyl)butyl]benzolacetamid mit einem Schmelzpunkt von 143-145°C, $[\alpha]_D^{25}$ +27,8° (c 1,0; MeOH).

b) Wie in Beispiel 5b) wird eine Lösung von 16,3 g [S-(S*,S*)]-α-Hydroxy-N-[1-methyl-4-(3-pyridinyl)-butyl]benzolacetamid in 160 ml 6N HCl während 22 Stunden unter Rückfluss erhitzt. Das nach dem üblichen Aufarbeiten erhaltene Rohprodukt wird destilliert. Man erhält 8,3 g (S)-α-Methyl-3-pyridinbutanamin (Sdp. 85-87°C/0,1 mm(13,3 Pa)).

Beispiel 7

a) Unter den in Beispiel 1a) beschriebenen Bedingungen werden 395 g 3-Brompyridin und 259,3 g (R,S)-5-Hexin-3-ol in Gegenwart von 418 ml Triethylamin, 17,56 g bis(Triphenylphosphin)palladiumdichlorid und 1,7 g Kupfer(I)jodid in 1,5 l Methylenchlorid miteinander umgesetzt. Nach üblichem Aufarbeiten erhält man 361,5 g rohes (R,S)-α-Ethyl-4-(3-pyridinyl)-3-butin-1-ol als braunes Oel.

b) Wie in Beispiel 1b) werden 361,5 g rohes (R,S)-α-Ethyl-4-(3-pyridinyl)-3-butin-1-ol über 15 g Platinoxid in 3 l Ethanol bei Raumtemperatur und Atmosphärendruck hydriert. Nach Destillation des erhaltenen Produktes erhält man 357 g (R,S)-α-Ethyl-3-pyridinbutanol (Sdp. 120-130° C/0.1 mm (13,3 Pa)) als farbloses Oel.

c) Wie in Beispiel 1c) gibt man 356,6 g (R,S)-α-Ethyl-3-pyridinbutanol zu einer Mischung, welche Wie beschrieben aus 211,7 g Oxalylchlorid und 170 g Dimethylsulfoxid in 1,75 l Methylenchlorid hergestellt worden ist. Nach Zugabe von 630 ml Triethylamin wird das Reaktionsgemisch in üblicher Weise aufgearbeitet. Man erhält 347,6 g Rohprodukt, das destilliert wird. Man erhält 327,9 g 5-(3-Pyridinyl)-3-hexanon (Sdp. 110-115° C/0,1mm (13,3 Pa)).

d) Wie in Beispiel 1d) beschrieben werden 372,9 g 6-(3-Pyridinyl)-3-hexanon mit 116,5 g Natriumcyanoborhydrid und 1426 g Ammoniumacetat in 6,5 l trockenem Methanol während 3 Tagen bei Raumtemperatur umgesetzt, worauf man 4,5 l 6N HCl dazu gibt und die Mischung über Nacht rührt. Nach Destillation des Rohproduktes erhält man 289,4 (R,S)-α-Ethyl-3-pyridinbutanamin (Sdp. 95-100° C/0,1 mm(13,3 Pa)).

Beispiel 8

a) Wie in Beispiel 5aa) wird eine Lösung von 367,4 g 1,3-Dicyclohexylcarbodiimid in 500 ml Dimethylformamid unter Rühren zu einer Lösung von 289 g (R,S)-α-Ethyl-3-pyridinbutanamin, 259 g (R)-Mandelsäure und 274 g 1-Hydroxybenzotriazol in 1,7 l Dimethylformamid gegeben, wobei die Temperatur während der Zugabe bei -10° C gehalten wird. Man rührt während 3 Stunden bei -5° C, dann über Nacht bei Raumtemperatur und kühlt die Mischung während 2 Stunden auf 0° C ab. Der ausgefallene Festkörper wird abfiltriert und nacheinander mit kaltem Dimethylformamid (3 x 50 ml) und Essigester (3 x 200 ml) gewaschen. Der Rückstand, eine Mischung aus 1,3-Dicyclohexylharnstoff (DCU) und dem weniger löslichen (R*,R)-Mandelsäureamid, wird in 1N HCl (2 l) suspendiert, worauf man während 4 Stunden bei Raumtemperatur rührt. Das unlösliche Material (DCU) wird abfiltriert und mit 200 ml verdünnter HCl und mit Wasser gewaschen. Das Filtrat wird basisch gestellt, und das erhaltene kristalline Material wird abfiltriert, mit Wasser gewaschen und im Vakuum getrocknet. Man erhält 195,4 g of [R-(R*,R*)]-α-Hydroxy-N-[1-ethyl-4-(3-pyridinyl)butyl]benzolacetamid mit einem Schmelzpunkt von 161,5-163° C; $[\alpha]_D^{25}$ -14,9° (c 1,0; MeOH).

Die Mutterlaugen und Waschlösungen werden zur Trockne eingedampft und wie in Beispiel 5aa) aufgearbeitet. Das Rohprodukt wird mit heissem Hexan (1 l) behandelt und der Festkörper wird abfiltriert. Man erhält 265 g Mandelsäureamid, das mit dem besser löslichen (S*,R)-Diastereomeren angereichert ist (>7:1). Durch fraktionierte Kristallisation dieses Materials aus 2-Propanol erhält man 147 g [S-(R*,R*)]-α-Hydroxy-N-[1-ethyl-4-(3-pyridinyl)butyl]benzolacetamid mit einem Schmelzpunkt von 122-124° C; $[\alpha]_D^{25}$ 41.2° (c 1.0; MeOH).

b) Wie in Beispiel 5b) wird eine Lösung von 195 g [R-(R*,R*)]-α-Hydroxy-N-[1-ethyl-4-(3-pyridinyl)butyl]-benzolacetamid in 1,1 l 6N HCl mit 104 ml conc. HCl behandelt und dann während 2 Tagen unter Rückfluss erhitzt. Das nach dem üblichen Aufarbeiten erhaltene rohe Amin wird destilliert. Man erhält 109 g (R)-α-Ethyl-3-pyridinbutanamin (Sdp. 105° C/0,2mm (26,7 Pa)) $[\alpha]_D^{25}$ -11,9° (c 1,0; MeOH).

c) Wie in Beispiel 5b) wird eine Lösung von 31.2 g [S-(R*,R*)]-α-Hydroxy-N-[1-ethyl-4-(3-pyridinyl)butyl]-benzolacetamid in 175 ml 6N HCl mit 16 ml conc. HCl behandelt und dann während 42 Stunden unter Rückfluss erhitzt. Durch übliches Aufarbeiten erhält man 16,4 g (S)-α-Ethyl-3-pyridinbutanamin (Sdp. 95-98° C/0,1 mm (13,3 Pa)); $[\alpha]_D^{25}$ +11,75° (c 1.0; MeOH).

Beispiel 9

a) Eine Suspension von 7.48 g 5-Hydroxy-2-methyl-pyridin und 24.4 g bis(Trifluormethansulphonyl)-phenylimid in 25 ml Methylenchlorid wird in einem Eisbad gekühlt und mit 10 ml trockenem Triethylamin versetzt. Nach einer Stunde bei 0° C wird die Mischung während 18 Stunden bei Raumtemperatur gerührt und dann nacheinander mit 1N NaOH (2 x 50 ml) und mit halb-gesättigter $K_2CO_3$-Lösung gewaschen. Durch Eindampfen der getrockneten ($K_2CO_3$) Lösung erhält man ein gelbes Oel, woraus

man durch Destillation 14,48 g Trifluormethansulfonsäure-6-methyl-3-pyridinylester (Sdp. 65-70° C/0,1 mm (13,3 Pa)) erhält.

b) Eine Lösung von 28 g Trilfuomethansulfonsäure-6-methyl-3-pyridinylester und 14.4 (R,S)-4-pentin-2-ol und 110 ml Triethylamin in 350 ml trockenem Dimethylformamid wird mittels Argon Sauerstoff-frei gemacht, worauf man 2,4 g bis(Triphenylphospin)palladiumdichlorid dazu gibt. Nach Rühren der Mischung während 3 Stunden bei 90° C wird abgekühlt, mit 300 ml 6N HCl sauer gestellt und mit Aether extrahiert. Die wässrige Phase wird mit Natriumhydroxidlösung basisch gestellt und mit Essigester extrahiert. Der organische Auszug wird mit Kochsalzlösung gewaschen, getrocknet ($K_2CO_3$) und eingedampft. Das erhaltene Oel wird destilliert, wobei man 10.8 g (R,S)-5-(6-Methyl-3-pyridinyl)-4-pentin-2-ol erhält (Sdp. 123-130° C/0,02 mm (2,67 Pa)).

c) Wie in Beispiel 1b) werden 10,3 g (R,S)-5-(6-Methyl-3-pyridinyl)-4-pentin-2-ol über 1,1 g 10-proz. Pd/C in 135 ml Ethanol bei Raumtemperatur und Atmosphärendruck hydriert. Nach dem üblichen Aufarbeiten wird das erhaltene gelbe Oel mittels HPLC (Essigester) gereinigt, wobei man 10,45 g (R,S)-α,6-Dimethyl-3-pyridinbutanol erhält. Eine Portion davon wird bei 105-110° C/0,1 mm (13,3 Pa) destilliert, wobei man eine analytisch reine Probe erhält.

d) Unter ähnlichen Bedinungen Wie in Beispiel 1c) beschrieben werden 10,25 g (R,S)-α,6-Dimethyl-3-pyridinbutanol in 50 ml Methylenchlorid zu einer aus 8,58 g Oxalylchlorid und 9,27 g Dimethylsulfoxid in 225 ml Methylenchlorid hergestellten Mischung gegeben. Nach Zugabe von 36,8 ml Triethylamin wird in üblicher Weise aufgearbeitet. Das Rohprodukt wird mittels HPLC (Essigester/Hexan; 1:1) gereinigt. Das erhaltene Material wird destilliert und man erhält 7,.4 g 5-(6-Methyl-3-pyridinyl)-2-pentanon (Sdp. 88-92° C/0,.05 mm (6,7 Pa)).

e) Wie in Beispiel 1d) werden 7,27 g 5-(6-Methyl-3-pyridinyl)-2-pentanon während 3 Tagen bei Raumtempertur mit 2.71 g Natriumcyanoborhydrid und 31,1 g Ammoniumacetat in 115 ml Methanol umgesetzt. Das Reaktionsgemisch wird dann mit 110 ml 6N HCl versetzt, und die Mischung wird während 90 Min. unter Rückfluss erhitzt, abgekühlt und in üblicher Weise aufgearbeitet. Durch Destillation des Rohproduktes erhält man 4,31 g (R,S)-6,α-Dimethyl-3-pyridinbutanamin (Sdp. 98-101° C/0,1 mm (13,3 Pa)).

Beispiel 10

a) Eine entgaste Lösung von 15,0 g 2,5-Dibrompyridin, 9,0 ml Trimethylsilylacetylen und 0,27 g Kupfer-(I)jodid in 200 ml Triethylamin wird mit 1,0 g bis(Triphenylphosphin)palladiumdichlorid behandelt. Die anfänglich exotherme Reaktion wird mittels eines Eisbades kontrolliert und dann über Nacht bei Raumtemperatur gerührt. Man verdünnt mit 400 ml Aether und wäscht die Mischung nacheinander mit Wasser (4 x 75 ml) und mit Kochsalzlösung (75 ml), trocknet ($K_2CO_3$) und dampft ein. Das verbleibende dunkle Oel wird über ein Kieselgelpolster filtriert (Aether) und dann mittels HPLC (Aether/Hexan; 1:49) gereinigt. Durch Kristallisieren des erhaltenen Materials aus Hexan erhält man 11.86 g 5-Brom-2-[(2-Trimethylsilyl)ethinyl]pyridin mit einem Schmelzpunkt von 56-59° C.

b) Eine Lösung von 9.78 g 5-Brom-2-[(2-Trimethylsilyl)ethinyl]pyridin und 0,19 g Kupfer(I)Jodid in 150 ml Triethylamin und 50 ml Methylenchlorid wird mittels Argon Sauerstofffrei gemacht, worauf man mit 3,4 g (R,S)-4-pentin-2-ol und 0,7 g bis(Triphenylphosphin)palladiumdichlorid versetzt. Man rührt die dunkle Mischung über Nacht bei Raumtemperatur, enfernt die Lösungsmittel unter vermindertem Druck und löst den Rückstand in Aether auf. Diese Lösung wird mit Wasser und Kochsalzlösung gewaschen und dann getrocknet ($K_2CO_3$) und eingedampft. Das Rohprodukt wird über ein Kieselgelpolster filtriert (Essigester/Hexan; 1:10) und dann mittels HPLC (Essigester) gereinigt. Man erhält (R,S)-5-[6-[2-Trimethylsilyl)ethinyl]-3-pyridinyl]-4-pentin-2-ol mit einem Schmelzpunkt von 79-80° C.

c) Eine Lösung von 8,51 g 5-[6-[2-Trimethylsilyl)ethinyl]-3-pyridinyl]-4-pentin-2-ol in 60 ml Methanol und 15 ml 2,5N NaOH wird während 1 Stunde gerührt und dann mit 300 ml Essigester verdünnt. Die abgetrennte organische Phase wird nacheinander mit Wasser und Kochsalzlösung gewaschen, getrocknet ($K_2CO_3$) und eingedampft. Der Rückstand wird über 0,7 g 10-Proz. Pd/C in 150 ml Ethanol bei Raumtemperatur und Atmosphärendruck hydriert. Nach dem üblichen Aufarbeiten wird das Rohprodukt destilliert, wobei man 5,61 g (R,S)-6-Ethyl-3-pyridin-α-methylbutanol (Sdp. 110-115° C/0,1 mm (13,3 Pa)) erhält.

d) Wie in Beispiel 1c) erhält man ausgehend von 4,4 g (R,S)-6-Ethyl-3-pyridin-α-methylbutanol 4,9 g 5-(6-Ethyl-3-pyridinyl)-2-pentanon mit einem Siedepunkt von 107-110° C/0,1 mm (13,3 Pa)).

e) Wie in Beispiel 1d) erhält man ausgehend von 4,75 g 5-(6-Ethyl-3-pyridinyl)-2-pentanon 2.44 g (R,S)-6-Ethyl-α-methyl-3-pyridinbutanamin mit einem Siedepunkt von 101-104° C/0,15 mm (20 Pa).

Beispiel 11

a) In einer inerten Atmosphäre werden 33 ml 1,6M Butyllithium in Hexan zu einer gerührten Lösung von 7,4 ml Diisopropylamin in 20 ml trockenem Tetrahydrofuran gegeben, das vorgängig während 30 Min. auf -78° C abgekührt worden ist, worauf man über einen Zeitraum von 3 Min. mit einer Lösung von 2,5 g Cyclopropanessigsäure in 10 ml trockenem Tetrahydrofuran versetzt. Man lässt die Reaktionsmischung auf Raumtemperatur kommen und erwärmt dann während 1 Stunde auf 50° C um die Bildung des Dianions zu vervollständigen. Die Mischung wird dann erneut auf -78° C abgekühlt, und eine Lösung von 7,67 g 3-(3-Brompropyl)pyridin (aus dessen HBr-Salz frisch hergestellt) in 20 ml Tetrahydrofuran wird dazugegeben. Man lässt das Reaktionsgemisch auf Raumtemperatur kommen und erwärmt dann während 7 Stunden auf 50° C. Man entfernt die Lösungsmittel im Vakuum, löst den Rückstand in 100 ml 1N HCl auf und extrahiert mit Methylenchlorid (3 x 50 ml). Die organischen Auszüge werden jeweils mit 2 x 25 ml-Portionen 1N HCl gewachen worauf die wässrigen Auszüge mit 17 ml 10N NaOH basisch gestellt und mit Methylenchlorid (3 x 100 ml) extrahiert werden, um Ausgangsbromid zu entfernen. Die wässrige Phase wird dann durch Zugabe von 3 ml Essigsäure angesäuert und mit Methylenchlorid extrahiert (1 x 150 ml; 2 x 100 ml). Die Auszüge werden mit Kochsalzlösung gewaschen, vereinigt, getrocknet ($Na_2SO_4$) und eingedampft. Man erhält 4,6 g (R,S)-α-Cyclopropyl-3-pyridinpentancarbonsäure als farblosen Festkörper. Eine Portion wird aus Aether/Hexan kristallisiert, wobei man eine analytisch reine Probe mit einem Schmelzpunkt von 82-84° C erhält.

b) Eine Lösung von 4,2 g (R,S)-α-Cyclopropyl-3-pyridinpentancarbonsäure, 5,8 g Diphenylphosphorylazid und 3 ml Triethylamin in 40 ml t-Butanol wird unter Argon und Rückfluss über Nacht gerührt. Man entfernt das Lösungsmittel unter vermindertem Druck, löst den Rückstand in 100 ml Methylenchlorid und wäscht 2 x mit 50 ml-Portionen 1N NaOH. Die wässrigen Auszüge werden ihrerseits mit 50 ml Methylenchlorid gewaschen. Die vereinigten organischen Auszüge werden dann getrocknet ($K_2CO_3$) und eingedampft, wobei man 5,6 g eines Oeles erhält. Das rohe Carbamat wird mittels HPLC (Essigester) gereinigt, und man erhält 4,8 g (R,S)-[1-Cyclopropyl-4-(3-pyridinyl)butyl]carbaminsäure-1,1-dimethylethylester als farbloses Oel.

c) Eine Lösung von 4,4 g (R,S)-[1-Cyclopropyl-4-(3-pyridinyl)butyl]carbaminsäure-1,1-dimethylethylester in 50 ml 1N HCl wird während 75 Min. auf einem Dampfbad erwärmt, worauf man abkühlt und mit 50 ml Aether extrahiert. Unter Argon wird die wässrige Phase mit 6 ml 10N NaOH behandelt und 2 x mit 50 ml-Portionen Methylenchlorid extrahiert. Nach Eindampfen der getrockneten ($K_2CO_3$) Auszüge erhält man 2,8 g (R,S)-α-Cyclopropyl-3-pyridinbutanamin als farbloses Oel.

Beispiel 12

a) Wie in Beispiel 11a) werden 2.04 g Pentancarbonsäure mit 2 Aequivalenten Lithiumdiisopropylamid (LDA) behandelt und dann mit 4,0 g 3-(3-Brompropyl)pyridin umgesetzt. Nach dem Aufarbeiten wird das Rohprodukt (3.5 g) aus Aether/Hexan kristallisiert. Man erhält 2,7 g (R,S)-α-Propyl-3-pyridinpentancarbonsäure, mit einem Schmelzpunkt von 55-57° C.

b) Wie in Beispiel 11b) werden 2,5 g (R,S)-α-Propyl-3-pyridinpentancarbonsäure mit 2,45 ml Diphenyphosphorylazid in 25 ml t-Butanol, das 1,6 ml Triethylamin enthält, umgesetzt, wobei man 3,1 g des rohen Carbamates erhält. Nach Reinigung dieses Materials mittels HPLC (Essigester) erhält man 2,75 g (R,S)-[1-Propyl-4-(3-pyridinyl)-butyl]carbaminsäure-1,1-dimethylethyester als Oel.

c) Wie in Beispiel 11c) erhält man durch Hydrolyse von 1.8 g (R,S)-[1-Propyl-4-(3-pyridinyl)-butyl]-carbaminsäure-1,1-dimethylethyester in 25 ml 1N HCl nach dem üblichen Aufarbeiten 1.15 g (R,S)-α-Propyl-3-pyridinbutanamin. Eine Portion davon wird in einem Kugelrohrapparat destilliert (110° C/0.1 mm (13,3 Pa)), wobei man eine analytisch reine Probe erhält.

Beispiel 13

a) Wie in Beispiel 11a) werden 2,04 g Isovaleriansäure mit 2 Aequivalenten LDA behandelt und dann mit 4,0 g 3-(3-Brompropyl)pyridin umgesetzt. Das erhaltene Rohprodukt wird aus Aether/Hexan kristallisiert. Man erhält 2,8 g (R,S)-α-(1-Methylethyl)-3-pyridinpentancarbonsäure mit einem Schmelzpunkt von 52-55° C. Durch Umkristallisieren einer Portion aus den gleichen Lösungsmitteln erhält man eine analytisch reine Probe mit einem Schmelzpunkt von 54-56° C.

b) Wie in Beispiel 11b) erhält man aus 2.3 g (R,S)-α-(1-Methylethyl)-3-pyridin-pentancarbonsäure durch Umsetzen mit 2.3 ml Diphenylphosporylazid in 25 ml t-Butanol, das 1.5 ml Triethylamin enthält, 2,8 g des Produktes. Durch Reinigung des rohen Esters mittels HPLC (Essigester) erhält man 2.5 g (R,S)-[1-(1-Methylethyl)-4-(3-pyridinyl)butyl]carbaminsäure-1,1-dimethylethylester als Oel.

c) Wie in Beispiel 11c) erhält man durch Hydrolyse von 1.7 g (R,S)-[1-(1-Methylethyl)-4-(3-pyridinyl)-butyl]carbaminsäure-1,1-dimethylethylester in 25 ml 1N HCl 1,1 g (R,S)-α-(1-Methylethyl)-3-pyridinbutanamin. Eine Probe davon wird in einem Kugelrohrapparat destilliert, wobei man eine analytisch reine Probe mit einem Siedepunkt von 110-115 ° C/0,1 mm (13,3 Pa) erhält.

Beispiel 14

a) Wie in Beispiel 11a) werden 2,32 g Hexancarbonsäure mit 2 Aequivalenten LDA behandelt und dann mit 4,0 g 3-(3-Brompropyl)pyridin umgesetzt. Nach dem üblichen Aufarbeiten erhält man 4 g (R,S)-α-Butyl-3-pyridinpentancarbonsäure als Oel.

b) Wie in Beispiel 11b) erhält man aus 3,7 g (R,S)-α-Butyl-3-pyridinpentancarbonsäure durch Umsetzen mit 3,4 ml Diphenylphosphorylazid in 25 ml t-Butanol, das 2,2 ml Triethylamin enthält, 4,4 g rohes Carbamat. Durch Reinigung mittels HPLC (Essigester) erhält man 3,6 g (R,S)-[1-Butyl-4-(3-pyridinyl)-butyl]carbaminsäure-1,1-dimethylethylester als Oel.

c) Wie in Beispiel 11c) erhält man durch Hydrolyse von 2.2 g (R,S)-[1-Butyl-4-(3-pyridinyl)butyl]-carbaminsäure-1,1-dimethylethylester in 25 ml 1N HCl nach dem üblichen Aufarbeiten 1.35 g (R,S)-α-Butyl-3-pyridinbutanamin. Eine Portion davon wird in einem Kugelrohrapparat destilliert, wobei man eine analytisch reine Probe mit einem Siedepunkt von 115 ° C/0,1 mm (13,3Pa) erhält.

Beispiel 15

a) Wie in Beispiel 11a) werden 5,56 g Cyclopentanessigsäure mit 2 Aequivalenten LDA behandelt und dann mit 4,0 g 3-(3-Brompropyl)pyridin umgesetzt. Das Rohprodukt (4 g) wird aus Aether/Hexan kristallisiert, wobei man 3,1 g (R,S)-α-Cyclopentyl-3-pyridinpentancarbonsäure mit einem Schmelzpunkt von 95-97 ° C erhält.

b) Wie in Beispiel 11b) erhält man aus 2,8 g (R,S)-α-Cyclopentyl-3-pyridinpentancarbonsäure durch Behandeln mit 2.5 ml Diphenylphosphorylazid in 25 ml t-Butanol, das 1.58 ml Triethylamin enthält, 3,4 g des Produktes. Durch Reinigung mittels HPLC (Essigester) erhält man 2.5 g (R,S)-[1-Cyclopentyl-4-(3-pyridinyl)butyl]carbaminsäure-1,1-dimethylethylester als Oel.

c) Wie in Beispiel 11c) erhält man durch Hydrolyse von 1.6 g (R,S)-[1-Cyclopentyl-4-(3-pyridinyl)butyl]-carbaminsäure-1,1-dimethylethylester in 25 ml 1N HCl 1,05 g (R,S)-α-(1-Cyclopentyl)-3-pyridnbutanamin. Eine Probe davon wird destilliert, wobei man eine analytisch reine Probe mit einem Sdp. von 125-130 ° C/0,1 mm (13,3 Pa) erhält.

Beispiel 16

a) Wie in Beisiel 11a) werden 2,8 g Cyclohexanessigsäure mit 2 Aequivalenten LDA behandelt und dann mit 4,0 g 3-(3-Brompropyl)pyrdin umgesetzt. Das Rohprodukt (3,8 g) wird aus Aether/Hexan kristallisiert. Man erhält 2.7 g (R,S)-α-Cyclohexyl-3-pyridinpentancarbonsäure mit einem Schmelzpunkt von 92-93 ° C.

b) Wie in Beispiel 11b) erhält man aus 2,5 g (R,S)-α-Cyclohexyl-3-pyridinpentancarbonsäure durch Umsetzen mit 2,68 g Diphenylphosphorylazid in 25 ml t-Butanol, das 0.97 g Triethylamin enthält, 3,1 g Rohprodukt. Durch Reinigung mittels HPLC (Essigester) erhält man 2,8g (R,S)-[1-Cyclohexyl4-(3-pyridinyl)butyl]carbaminsäure-1,1-dimethylethylester. Dieses Material wird aus Aether/Hexan kristallisiert, wobei man eine analytisch reine Probe mit einem Schmelpunkt von 64-66 ° C erhält.

c) Wie in Beispiel 11c) erhält man durch Hydrolyse von 1.9 g (R,S)-[1-Cyclohexyl-4-(3-pyridinyl)butyl]-carbaminsäure-1,1-dimethylethylester in 25 ml 1N HCl 1,25 g (R,S)-α-(1-Cyclohexyl)-3-pyridinbutanamin. Durch Destillation einer Portion dieses Materials in einem Kugelrohrapparat erhält man eine analytisch reine Probe mit einem Sdp. von 140-145 ° C/0,1 mm (13,3 Pa).

Beispiel 17

a) Wie in Beispiel 11a) werden 7,16 g 3-Pyridinpentancarbonsäure mit 2 Aequivalenten LDA behandelt und dann mit 8,8 g 3-(3-Brompropyl)pyridin umgesetzt. Nach üblichem Aufarbeiten erhält man 8,9 g eines orange-farbenen Oels, das hauptsächlich aus α-[3-(3-Pyridinyl)propyl]-3-pyridinpentancarbonsäure besteht, das mit einer kleinen Menge des Ausgangsmaterials 3-Pyridinpentancarbonsäure, kontaminiert ist.

b) Wie in Beispiel 11b) erhält man aus 8,8 g α-[3-(3-Pyridinyl)propyl]-3-pyridinpentancarbonsäure durch Umsetzen mit 9,1 g Diphenylphosphorylazid in 75 ml t-Butanol, das 4,1 ml Triethylamin enthält, 11,1 g des Produktes. Durch Reinigung mittels HPLC (Essigester/methanol; 13:1) erhält man 5,5 g [1-[3-(3-Pyridinyl)propyl]-4-(3-pyridinyl)butyl]carbaminsäure-1,1-dimethylethylester als Oel.
c) Wie in Beispiel 11c) erhält man durch Hydrolyse von 5,4 g [1-[3-(3-Pyridinyl)propyl]-4-(3-pyridinyl)-butyl]carbaminsäure-1,1-dimethylethylester in 55 ml 1N HCl 3,15 g α-[3-(3-Pyridinyl)propyl]-3-pyridinbutanamin.

Beispiel 18

a) Wie in Beispiel 11a) werden 2,15 g p-Bromphenylessigsäure mit 2 Aequivalenten LDA behandelt und dann mit 2,0 g 3-(3-Brompropyl)pyridin umgesetzt. Das Rohprodukt wird aus Aether/Hexan kristallisiert, wobei man 1.46 g (R,S)-α-(4-Bromphenyl)-3-pyridinpentancarbonsäure mit einem Schmelzpunkt von 123-126 °C erhält.
b) Wie in Beispiel 11b) erhält man aus 1,45 g (R,S)-α-(4-Bromphenyl)-3-pyridinpentancarbonsäure durch Behandeln mit 0,94 ml Diphenylphosphorylazid in 10 ml t-Butanol, das 0,44 g Triethylamin enthält, 1,7 g rohes (R,S)-[1-(4-Bromphenyl)-4-(3-pyridinyl)butyl]carbaminsäure-1,1-dimethylethylester als Oel.
c) Wie in Beispiel 11c) erhält man durch Hydrolyse von 1,7 g (R,S)-[1-(4-Bromphenyl)-4-(3-pyridinyl)-butyl]carbaminsäure-1,1-dimethylethylester in 15 ml 1N HCl 1,1 g (R,S)-α-(4-Bromphenyl)-3-pyridinbutanamin.

Beispiel 19

a) Eine Lösung von 9,95 g [(R,S)-Z]-2-Methyl-5-(3-pyridinyl)-4-pentencarbonsäure-ethylester in 75 ml 1N NaOH und 75 ml Methanol wird während 3 Tagen unter Rückfluss erhitzt, worauf man das meiste Methanol unter vermindertem Druck entfernt. Man extrahiert die erhaltene Lösung mit Methylenchlorid (3 x 50 ml), neutralisiert die wässrige Phase mit 75 ml 1N HCl und extrahiert mit Methylenchlorid (4 x 40 ml). Die getrockenten ($Na_2SO_4$) Auszüge werden eingedampft, wobei man 7,28 g eines Festkörpers erhält, den man aus Aether/Hexan kristallisiert. Man erhält 6,12 g [(R,S)-Z]-2-Methyl-5-(3-pyridinyl)-4-pentencarbonsäure mit einem Schmelzpunkt von 79-82 °C.
b) Wie in Beispiel 11b) werden 2.87 g [(R,S)-Z]-2-Methyl-5-(3-pyridinyl)-4-pentencarbonsäure mit 3,31 ml Diphenylphosphorylazid in 30 ml t-Butanol, das 2,1 ml Triethylamin enthält, umgesetzt. Das Produkt wird in üblicher Weise isoliert, wobei man 3,88 g [(R,S)-Z]-[1-Methyl-4-(3-pyridinyl)-3-butenyl]carbaminsäure-1,1-dimethylethylester als Oel erhält.
c) Wie in Beispiel 11c) erhält man durch Hydrolyse von 3,88 g [(R,S)-Z]-[1-Methyl-4-(3-pyridinyl)-3-butenyl]carbaminsäure-1,1-dimethylethylester in 50 ml 1N HCl nach dem üblichen Aufarbeiten und Destillation des Produktes 1,7 g [(R,S)-Z]-1-Methyl-4-(3-pyridinyl)-3-butenamin als Oel mit einem Sdp. von 100-120 °C/0.1 mm (13,3 Pa).

Beispiel 20

a) Eine Suspension von 7.0 g Natriumhydrid (60-Proz. Dispersion in Oel) in 75 ml trockenem Dimethylsulfoxid wird während 45 Min. bei 75 °C unter Argon gerührt, wobei die Bildung von Wasserstoff nach dieser Zeit beendet war. Nach Abkühlen der Lösung versetzt man mit 61 g Methyltriphenylphoshoniumbromid und rührt die Mischung während 30 Min. bei Raumtemperatur, wonach man mit 25 g 5-(3-Pyridinyl)-2-pentanon in 125 ml Dimethylsulfoxid versetzt. Die Reaktionsmischung wird dann bei Raumtemperatur gerührt. Man gibt dann 1 l 1N Salzsäure dazu, filtriert das ausgefallene Triphenylphosphinoxid ab und stellt das Filtrat mit 110 ml 10N Natriumhydroxid basisch. Man extrahiert das Produkt mit Methylenchlorid (4 x 300 ml), wäscht die Auszüge mit Kochsalzlösung, vereinigt sie, trocknet ($K_2CO_3$) und dampft ein. Man erhält 25 g Rohprodukt, das man mittels HPLC (Essigester/Hexan; 1:1) reinigt. Man erhält 17.5 g 3-(4-Methyl-4-pentenyl)pyridin als farbloses Oel.
b) Eine Mischung von 22,7 g 3-(4-Methyl-pentenyl)pyridin und 22.8 g 2-Nitrobenzolacetonitril in 80 ml Essigsäure wird auf 12-13 °C abgekühlt und dann über einen Zeitraum von 6 Min. tropfenweise mit 16 ml Schwefelsäure versetzt. Man rührt das Reaktionsgemisch während 2 Stunden bei Raumtemperatur, entfernt dann die Essigsäure im Vakuum, versetzt mit 1 l Wasser und extrahiert die Mischung mit Methylenchlorid, um neutrale Verunreinigungen zu entfernen. Die wässrige Phase wird mit 10N Natriumhydroxid basisch gestellt und mit Methylenchlorid (4 x 200 ml) extrahiert. Die getrockneten ($K_2CO_3$) Auszüge werden eingedampft, wobei man 35.6 g N-[1,1-Dimethyl-(3-pyridinyl)butyl]-2-nitrobenzolaceta-

mid erhält. Eine Portion davon wird aus Essigester/Hexan kristallisiert, wobei man eine analytisch reine Probe mit einem Schmelzpunkt von 117-118,5 ° C erhält.

c) Eine Lösung von 35.2 g N-[1,1-Dimethyl-(3-pyridinyl)butyl]-2-nitrobenzolacetamid in 250 ml Essigsäure wird über 3.5 g 10-Proz. Pd/C bei Raumtemperatur und Atmosphärendruck hydriert. Die Reaktion verläuft exotherm und stoppt nach der Aufnahme der theoretischen Menge an Wasserstoff (7,5 l) aprupt ab. Der Katalysator wird abfiltriert, und das Filtrat wird während 90 Min. unter Rückfluss erhitzt. Man kühlt die Lösung ab, versetzt mit 10 ml conc. HCl und entfernt das Lösungsmittel unter vermindertem Druck. Der Rückstand wird in 1 l Wasser aufgenommen. Man extrahiert mit Essigester (4 x 200 ml) um das Nebenprodukt Oxindol zu entfernen. Die wässrige Phase wird mit 10N NaOH basisch gestellt und mit Methylenchlorid extrahiert. Nach Eindampfen der getrockneten ($K_2CO_3$) Auszüge erhält man 15 g Produkt. Dieses Material wird in einem Kugelrohrapparat destilliert, und man erhält 14,3 g α,α-Dimethyl-3-pyridinbutanamin mit einem Sdp. von 95 ° C/0,1 mm (13,3 Pa).

Beispiel 21

a) In einer inerten Atmosphäre werden 10,7 g 4-Pyridincaboxaldehyd zu einer gerührten Lösung von 35,1 g (Carbomethoxy)methylentriphenylphosphoran in 250 ml Methanol gegeben. Nach 90 Min. wird das Lösungsmittel unter vermindertem Druck abgedampft, und der Rückstand wird mit Aether/Hexan behandelt. Der erhaltene Festkörper (Triphenylphosphinoxid) wird abfiltriert, und das Filtrat wird eingedampft. Man erhält 18 g einer Mischung aus (E)- und (Z)-3-(4-Pyridiny)-2-propencarbonsäure-methylester, das noch mit einer kleinen Menge an Triphenylphosphinoxid kontaminiert ist.

Die rohe Mischung (18 g) wird über 1.6 g 10-Proz. Pd/C in 200 ml Methanol bei Atmosphärendruck und Raumtemperatur hydriert. Nach Beendiugng der Wasserstoffaufnahme wird der Katalysator abfiltriert und das Lösungsmittel im Vakuum abgedampft. Man erhält 16 g rohen 4-Pyridinpropancarbonsäure-methylester.

Der rohe Ester wird in 250 ml 7.1M methanolischer Ammoniaklösung aufgelöst, worauf man während 65 Stunden bei Raumtemperatur rührt. Nach entfernen des Lösungsmittels und des überschüssigen Ammoniaks mittels Destillation unter vermindertem Druck wird der Rückstand in 150 ml 1N HCl gelöst. Man extrahiert mit Methylenchlorid, um Triphenylphosphinoxid zu entfernen. Die wässrige Phase wird mit 40 ml 4N NaOH basisch gestellt und dann mi Essigester (5 x 300 ml) extrahiert. Die getrockneten ($Na_2SO_4$) Auszüge werden eingedampft, wobei man 6 g Amid erhält. Die wässrige Phase wird zu Trockne eingedampft und der Rückstand wird mit Tetrahydrofuran (4 x 100 ml) behandelt. Durch Eindampfen der Tetrahydrofuranauszüge erhält man weitere 5 g des Amides. Die beiden Portionen des Rohproduktes werden vereinigt und mit Aether behandelt. Nach Abfiltrieren des Festkörpers erhält man 10,7 g 4-Pyridinpropanamid mit einem Schmelzpunkt von 164-166 ° C.

b) Ein 1M Lösung von $BH_3$ in Tetrahydrofuran (192 ml) wird über einen Zeitraum von 10 Min. unter Rühren zu einer Suspension von 7.2 g 4-Pyridinpropanamid in 50 ml Tetrahydrofuran bei 0-5 ° C gegeben. Nach Entfernen des Kühlbades wird während 17 Stunden unter Rückfluss erhitzt, worauf man das Lösungsmittel im Vakuum abdampft. Man löst den Rückstand in 160 ml 3,5N HCl, erhitzt über Nacht auf einem Dampfbad, kühlt ab, stellt mit überschüssigem 10N NaOH basisch und extrahiert dann mit Methylenchlorid. Die getrockneten ($K_2CO_3$) Auszüge werden eingedampft. Man erhält 6.98 g eines bernsteinfarbenen Oeles, das man im Vakuum destilliert. Man erhält 4,3 g 4-Pyridinpropanamin mit einem Sdp. von 100-110 ° C/0,2 mm (26,7 Pa).

Beispiel 22

a) Wie in Beispiel 1a) werden 15,8 g 2-Brompyridin und 8,4 g 4-Pentin-1-ol in 125 ml Methylchlorid in Gegenwart von 4,2 ml Triethylamin, 2,1 g bis(Triphenylphosphin)palladiumdichlorid und 0,135 g Kupfer-(I)jodid miteinander umgesetzt. Nach 48 Stunden unter Rückfluss wird das Reaktionsgemisch in üblicher Weise aufgearbeitet. Durch Destillation des Rohproduktes erhält man 8,8 g 5-(2-Pyridinyl)-4-pentin-1-ol mit einem Sdp. von 115-120 ° C/0,25 mm (33,3 Pa).

b) 5-(2-Pyridinyl)-4-pentin-1-ol (8,8 g) wird über 1,0 g 10-Proz. Pd/C in 125 ml Ethanol bei Raumtemperatur und Atmosphärendruck hydriert. Nach Beendigung der Wasserstoffaufnahme wird der Katalysator abfiltriert und das Lösungsmittel wird unter vermindertem Druck abgedampft. Das verbleibende Oel wird in einem Kugelrohrapparat destilliert (115-120 ° C/0.1 mm (13,3 Pa)), wobei man 8,4 g 2-Pyridinpentanol erhält.

c) Eine Lösung von 4,1 ml Thionylchlorid in 30 ml Methylenchlorid wird über einen Zeitraum von 10 Min. unter Rühren zu einer bei -5 ° C gehaltenen Lösung von 6,65 g 2-Pyridinpentanol in 60 ml Methylenchlorid gegeben. Nach Beendigung der Zugabe wird die Mischung während 17 Stunden bei Raumtemperatur gerührt auf 5°C abgeschreckt und über einen Zeitraum von 10 Min. tropfenweise mit 150 ml 1N NaOH versetzt. Die Schichten werden getrennt, und die wässrige Phase wird mit 75 ml Methylenchlorid extrahiert. Die organischen Phasen werden mit Kochsalzlösung gewaschen, vereinigt, getrocknet ($K_2CO_3$) und eingedampft. Man erhält 7,4 g 2-(5-Chlorpentyl)pyridin als Oel.

d) Eine Mischung von 6,35 g 2-(5-Chlorpentyl)pyridin, 7,7 g Kaliumphthalimid, 5,2g Natriumjodid und 3,7 g Natriumcarbonat in 50 ml Dimethylformamid wird während 20 Stunden bei 50°C gerührt. Man entfernt das Lösungsmittel unter vermindertem Druck, nimmt den Rückstand in 100 ml Wasser auf und extrahiert mit Methylenchlorid (1 x 250 ml; 1 x 150 ml). Die organischen Auszüge werden mit Kochsalzlösung gewaschen, vereinigt, getrocknet ($K_2CO_3$) und im Vakuum eingedampft. Man erhält 10.1 g eines orangefarbenen Oeles. Durch Reinigung des Rohproduktes mittels HPLC (Essigester/ Hexan) erhält man 6,7 g 1-[5-(2-Pyridinyl)pentyl]-1H-isoindol-1,3-(2H)-dion.

e) Eine Lösung von 6,5 g 1-[5-(2-Pyridinyl)pentyl]-1H-isoindol-1,3-(2H)-dion und 1,15 ml Hydrazin-hydrat in 35 ml Ethanol wird während 90 Min. unter Rückfluss erhitzt. Das abgekühlte Reaktionsgemisch wird mit 10 ml 6N HCl behandelt, worauf der Festkörper abfiltriert und mit 20 ml 0,5N HCl gewaschen wird. Das Filtrat wird konzentriert, um das Ethanol zu entfernen, mit 10N NaOH basisch gestellt und dann mit Methylenchlorid extrahiert. Der organsiche Auszug wird mit Kochsalzlösung gewaschen, getrocknet ($K_2CO_3$) und eingedampft. Man erhält 3,4 g eines gelben Oeles, dass man destilliert (105-110°C/0,01 mm (1,33 Pa)). Man erhält 2,4 g 2-Pyridinpentanamin.

Beispiel 23

a) In einer inerten Atmosphäre werden 0,068 g bis (Triphenylphosphin)palladiumdichlorid und 0,013 g Kupfer(I)jodid unter Rühren zu einer sauerstofffreien Lösung von 1 g 8-Bromisochinolin, 0,56 ml Propargylalkohol und 2 ml Triethylamin in 25 ml Methylenchlorid gegeben. Die Mischung wird während 2 Stunden bei Raumtemperatur und dann während Stunden unter Rückfluss gerührt. Das abgekühlte Reaktionsgemisch wird filtriert, und das Filtrat wird in Vakuum eingedampft. Das verbleibende Oel wird mittels HPLC (Essigester/Toluol; 2:3) gereinigt Man erhält 0,4g 3-(8-Isochinolinyl)-2-propin-1-ol mit einem Schmelzpunkt von 138-139°C.

b) Eine Lösung von 0,4 g 3-(8-Isochinolinyl)-2-propin1-ol in einer Mischung aus 10 ml Ethanol und 5 ml Methanol wird über 0,06 g 10-Proz. Pd/C bei Raumtemperatur und Atmosphärendruck während 22 Stunden und dann während 20 Stunden bei 50 psi (345 kPa) hydriert. Nach Entfernen des Katalystors durch Filtration wird das Filtrat eingedampft. Das erhaltene Oel wird mittels HPLC (Methanol/Chloroform; 1:19) gereinigt und aus Essigester/Hexan kristallisiert, wobei man 0,136 g 8-Isochinolinpropanol mit einem Schmelzpunkt von 66-69°C erhält.

c) Eine Lösung von 0,142 g 8-Isochinolinpropanol in 3 ml Chloroform wird zu einer Lösung von 0,085 ml Thionylchlorid in 1 ml Chloroform gegeben, worauf das Reaktionsgemisch während 3 Stunden unter Rückfluss gerührt wird. Man wäscht das abgekühlte Gemisch mit $NaHCO_3$-Lösung und mit Kochsalzlösung, trocknet ($Na_2SO_4$) und dampft ein. Das erhaltene Oel wird zusammen mit 0,281 g Kaliumphthalimid und 0,126 g Kaliumjodid in 3 ml trockenem Methylformamid während 90 Min. bei 130°C gerührt. Nach Abdampfen des Lösungsmittels wird der Rückstand zwischen Methylenchlorid und Wasser verteilt. Die getrocknete ($Na_2SO_4$) organische Phase wird eingedampft und das Rohprodukt wird mittels HPLC (Essigester/Toluol; 1:4) gereinigt. Durch Kristallisieren aus Aether erhält man 0.177 g [3-(8-Isochinolinyl)-propyl]-1H-isoindol-1,3-(2H)-dion mit einem Schmelzpunkt von 135-140°C.

d) Man versetzt eine Lösung von 0,174 g 3-(8-Isochinolinyl)propyl]-1H-isoindol-1,3-(2H)-dion in 8 ml Ethanol unter Rückfluss mit 0,12 ml Hydrazin-hydrat und rührt das Reaktionsgemisch während 5,5 Stunden unter Rückfluss. Man entfernt das Lösungsmittel unter vermindertem Druck und behandelt den Rückstand mit Chloroform. Der Chloroformauszug wird zu einem Oel konzentriert, das man durch eine kurze Kieselgelsäure (Chloroform/methanol/Triethylamin; 1:4:15) laufen lässt. Man erhält 0,103 g 8-Isochinolinpropanamin als Oel.

Beispiel 24

a) In einer inerten Amtosphäre gibt man 0,268 g bis(Triphenylphosphin)palladiumdichlorid und 0,072 g Kupfer(I)jodid unter Rühren zu einer sauerstofffreien Lösung von 5 g 4-Bromisochinolin, 3.02 g 3-Butin-1-ol und 10 ml Triethylamin in 20 ml Methylenchlorid. Das Reaktionsgemisch wird während 1 Stunde bei Raumtemperatur und dann währen 18 Stunden unter Rückfluss gerührt. Die abgekühlte Mischung wird filtriert und das Filtrat wird mit Wasser gewaschen. Die getrocknete ($Na_2SO_4$) organische Phase wird im

Vakuum eingedampft, und das erhaltene Oel wird mittels HPLC (Essigester/Toluol; 2:3) gereinigt. Man erhält 3,4 g 4-(4-Isochinolinyl)-3-butin-1-ol als Oel.

b) Eine Lösung von 3.4 g 4-(4-Isochinolinyl)-3-bytin-1-ol in 35 ml Ethanol wird über 0,35 g 10-Proz. Pd/C bei Raumtemperatur und Atmosphärendruck während 5 Stunden hydriert. Der Katalysator wird dann abfiltriert, und das Filtrat wird eingedampft. Man erhält 3,3 g (E)-4-(4-Isochinolinyl)-3-buten-1-ol als Oel.

c) Eine Lösung von 3,3 g (E)-4-(4-Isochinolinyl)-3-buten-1-ol in 15 ml trockenem Chloroform wird zu einer kalten Lösung von 1,8 ml Thionylchlorid in 5 ml trockenem Chloroform gegeben. Nach 15 Min. wird das Kühlbad entfernt, und das Reaktionsgemisch wird während 1 Stunde bei Raumtemperatur und dann während 3 Stunden unter Rückfluss gerührt. Die abgekühlte Mischung wird mit $NaHCO_3$-Lösung und mit Kochlsalzlösung gewaschen, getrocknet ($Na_2SO_4$) und eingedampft. Der Rückstand wird mittels Chromatographie über Kieselgel (Essigester/Toluol; 3:17) gereinigt. Man erhält 2,2 g (E)-4-(4-Chlor-1-butenyl)-isochinolin als Oel.

d) Eine Mischung aus 2,2 g (E)-4-(4-Chlor-1-butenyl)isochinolin, 3,8 g Kaliumphthalimid und 1,7 g Kaliumjodid in 20 ml trockenem Dimethylformamid wird während 5 Stunden bei 130°C gehalten. Nach Eindampfen des Lösungsmittels wird der Rückstand zwischen Methylenchlorid und Wasser verteilt. Die getrocknete ($Na_2SO_4$) organische Phase wird eingedampft, und das Rohprodukt wird mittels Chromatographie an Kieselgel (Essigester/Toluol; 3:7) gereinigt. Man erhält 1,75 g (E)-1-[4-(4-Isochinolinyl)-3-butenyl]-1H-isoindol-1,3-(2H)-dion mit einem Schmelzpunkt von 135-140°C.

e) Zu einer Lösung von 1,75 g (E)-1-[4-(4-Isochinolinyl)-3-butenyl]-1H-isoindol-1,3-(2H)dion in 80 ml Ethanol gibt man unter Rückfluss 1,1 ml Hydrazin-hydrat und rührt das Reaktionsgemisch noch während 17 Stunden unter Rückfluss. Das Lösungsmittel wird im Vakuum abgedampft, und der Rückstand wird mit Chloroform behandelt. Nach Eindampfen des Auszuges erhält man 1 g (E)-4-(4-Isochinolinyl)-3-buten-1-amin als Oel.

f) Eine Lösung von 1 g (E)-4-(4-Isochinolinyl)-3-buten-1-amin in 20 ml Ethanol wird über 0,12 g 10-Proz. Pd/C bei Raumtemperatur und Atmosphärendruck hydriert. Nach 8 Stunden wird der Katalysator abfiltriert und das Filtrat wird eingedampft. Man erhält 0,893 g 4-Isochinolinbutanamin.

Beispiel 25

aa) Wie in Beispiel 5aa) erhält man ausgehend von (S)-Mandelsäure und (R,S)-α,2-Dimethyl-3-pyridinbutanamin nach Umkristallisieren aus Isopropanol [S-(R*,R*)]-α-Hydroxy-N-[1-methyl-4-(2-methyl-3-pyridinyl)butyl]benzolacetamid mit einem Schmelzpunkt von 133-134°C, $[\alpha]_D$ +30,50° (c 0,986; EtOH).

ab) Wie in Beispiel 5aa) erhält man ausgehend von (R)-Mandelsäure und (R,S)-α,2-Dimethyl-3-pyridinbutanamin nach Umkristallisieren aus Isopropanol [R-(R*,S*)]-α-Hydroxy-N-[1-methyl-4-(2-methyl-3-pyridinyl)butyl]benzolacetamid mit einem Schmelzpunkt von 133-134°C, $[\alpha]_D$ -30,24° (c 1,068; EtOH).

b) Wie in Beispiel 5b) erhält man ausgehend von [R-(R*,S*)]-α-Hydroxy-N-[1-methyl-4-(2-methyl-3-pyridinyl)butyl]benzolacetamid nach Kugelrohrdestillation bei 105-110°C (Luftbadtemperatur)/0,1 mm (13,3 Pa) (S)-α,2-Dimethyl-3-pyridinbutanamin-hydrat (0,1 molar), $[\alpha]_D$ +3,18° (c 1,4125; EtOH).

c) Wie in Beispiel 5b) erhält man ausgehend von [S-(R*,R*)]-α-Hydroxy-N-[1-methyl-4-(2-methyl-3-pyridinyl)butyl]benzolacetamid nach Kugelrohrdestillation bei 105-110°C (Luftbadtemperatur)/0,15 mm (20 Pa) (R)-α,2-Dimethyl-3-pyridinbutanamin, $[\alpha]_D$ -4,18° (c 1,0265; EtOH).

Teil IV: Herstellung von Verbindungen der Formel IX

Beispiel 1

Eine Lösung von frisch destilliertem Diisopropylamin (193.2 ml) in trockenem Tetrahydrofuran (1 l) wird unter Argon in einem Aceton/Trockeneis-Bad auf -5°C gekühlt. Die ganze Reaktion wird unter wasserfreien Bedingungen durchgefünrt. Eine 1,6M Lösung von n-Butyllithium in Hexan (862 ml) wird so dazu gegeben, dass die Reaktionstemperatur -5°C nicht übersteigt. Nach beendeter Zugabe wird die Lösung während 15 Min. bei 5°C gerührt, dann auf -78°C abgekühlt und über einen Zeitraum von 10 Min. tropfenweise mit Acetaldehyd N-tert-butylimin (88,5 ml) versetzt. Man rührt während 30 Min. bei -78°C, versetzt langsam mit Diethylchlorphosphonat (101,2 ml), wobei die Temperatur unterhalb -65°C gehalten wird, und rührt die gelbe Lösung während einer Stunde bei -78°C. Das Kühlbad wird dann entfernt und man lässt die Reaktionsmischung während 45 Min. auf -10°C erwärmen. Dann gibt man Benzophenon (109,3 g) dazu und rührt die Mischung über Nacht bei Raumtemperatur. Nach Entfernen des Lösungsmittels im Vakuum wird der Rückstand in eine Lösung von Oxalsäure-dihydrat (175 g) in Wasser (1,5 l) aufgenommen, worauf Toluol (1,5 l) dazugegeben wird. Die Mischung wird unter Argon und über Nacht stark gerührt. Die

Schichten werden dann getrennt, und die organische Phase wird nacheinander mit 5-Proz. Oxalsäure, Kochsalzlösung, gesättigter Natriumbicarbonatlösung und Kochsalzlösung gewaschen. Die wässrigen Phasen werden mit 500 ml Toluol gewaschen, worauf die vereinigten organische Auszüge getrocknet ($MgSO_4$), mit Aktivkohle behandelt und auf ungefähr 250 ml eingedampft werden. Das Konzentrat wird auf eine kurze Kieselgelsäure gegeben (500 g), welche mit Methylenchlorid hergestellt worden ist, und das Produkt wird mit dem gleichen Lösungsmittel eluiert (7 x 400 ml-Fraktionen). Die geeigneten Fraktionen werden vereinigt und eingedampft, und der Rückstand wird in warmem Hexan (1,2 l) aufgelöst. Die erhaltene Lösung wird unter leichtem Rühren langsam auf 5 ° C abgekühlt, und das ausgefallene kristalline Material wird abfiltriert. Man erhält 109,3 g 3,3-Diphenyl-2-propenal mit einem Schmelzpunkt von 45-46,5 ° C. Durch Eindampfen der Mutterlaugen erhält man eine zweite Portion von unreinem 3,3-Diphenyl-2-propenal, das nach zwei Umkristallisationen aus Hexan 10,1 g Produkt mit einem Schmelzpunkt von 44,5-46 ° C liefert.

Beispiel 2

Man verfährt wie in Beispiel 1 und verwendet die folgenden Reagenzien: 2,2'-Difluorbenzophenon (6,0 g), Diisopropylamin (28,8 ml), 1,6M n-Butyllithium in Hexan (129 ml), Acetalde hyd-N-tert-butylimin (13,2 ml), Diethylchlorophosphonat (14,85 ml) und Tetrahydrofuran (100 ml). Die Reaktion wird in üblicher Weise aufgearbeitet, wobei jedoch die Oxalsäure-katalysierte Hydrolyse langsam verläuft und das Reaktionsgemisch während 1 Stunde auf 85 ° C erwärmt werden muss, um die Reaktion zu vervollständigen. Das als organge-farbenes Oel erhaltene Rohprodukt (5,8 g) wird aus Hexan kristallisiert und man erhält 4,6 g 3,3-bis(2-Fluorphenyl)-2-propenal mit einem Schmelzpunkt von 75-77 ° C.

Beispiel 3

Man verfährt wie in Beispiel 1 und verwendet die folgenden Reagenzien: 3,3'-Difluorbenzophenon (19,8 g), Diisopropylamin (32,2 ml), 1,55M n-Butyllithium in Hexan (148 ml), Acetaldehyd N-tert-butylimin (14,75 ml), Diethylchlorophosphonat (16,6 ml) und Tetrahydrofuran (200 ml). Nach üblichem Aufarbeiten erhält man 23 g Material, das man aus Hexan kristallisiert. Man erhält 17,4 g 3,3-bis(3-Fluorphenyl)-2-propenal mit einem Schmelzpunkt von 51-53 ° C. Durch Umkristallisieren einer kleinen Portion aus dem gleichen Lösungsmittel erhält man eine analytisch reine Probe mit einem Schmelzpunkt von 52-54 ° C.

Beispiel 4

Man verfährt wie in Beispiel 1 und verwendet die folgenden Reagenzien: 4,4'-Difluorbenzophenon (22,4 g), Diisopropylamin (32,2 ml), 1,55M n-Butyllithium in Hexan (148 ml), Acetaldehyd-N-tert-butylimin (14,75 ml), Diethylchlorophosphonat (16,6 ml) und Tetrahydrofuran (200 ml). Nach üblichem Aufarbeiten erhält man 26 g eines organgefarbenen Oeles, das man aus 2-Propanol kristallisiert. Man erhält 19,3 g 3,3-bis(4-Fluorphenyl)-2-propenal mit einem Schmelzpunkt von 56-58 ° C. Eine kleine Portion wird aus Pentan umkristallisiert, wobei man eine analytisch reine Probe mit einem Schmelzpunkt von 58-59.5 ° C erhält.

Beispiel 5

Man verfährt wie in Beispiel 1, wobei die Reaktion jedoch nach 30 Stunden aufgearbeitet wird. Man verwendet die folgenden Reagenzien: 3,3'-Dimethoxybenzophenon (48,5 g), Diisopropylamin (64,4 ml), 1,55M n-Butyllithium in Hexan (286 ml), Acetaldehyd-N-tert-butylimin (29,5 ml), Diethylchlorophosphonat (33,2 ml) und Tetrahydrofuran (400 ml). Die Oxalsäure-katalysiert Hydrolyse wurde während 2 Stunden bei 85 ° C durchgeführt. Nach dem Aufarbeiten erhält man 53 g von im wesentlichen reinem 3,3-bis(3-Methoxyphenyl)-2-propenal als schwachgelbes Oel.

Beispiel 6

Man verfährt wie in Beispiel 1, wobei die Reaktionszeit jedoch auf 68 Stunden ausgedehnt wird. Man verwendet die folgenden Reagenzien: 4,4'Dimethoxybenzophenon (24,2 g), Diisopropylamin (32,2 ml), 1,55M n-Butyllithium in Hexan (148 ml), Acetaldehyd-N-tert-butylimin (14,75 ml), Diethylchlorophosphonat (16,6 ml) und Tetrahydrofuran (200 ml). Nach dem üblichen Aufarbeiten erhält man 25,6 g eines organgefarbenen Oels, das man aus Aether/Hexan kristallisiert. Man erhält 22,7 g 3,3-bis(4-Dimethoxyphenyl)-2-propenal als gelben Festkörper mit einem Schmelzpunkt von 55-57 ° C. Aus dem gleichen Lösungsmittelsystem erhält man eine analytisch reine Probe mit einem Schmelzpunkt von 56-57 ° C.

Beispiel 7

Man verfährt wie in Beispiel 1 und verwendet die folgenden Reagenzien: 3,3',4,4'-Tetramethoxybenzophenon (32,2 g), Diisopropylamin (32,2 ml), 1,6M n-Butyllithium in Hexan (144 ml), Acetaldehyd-N-tert-butylimin (14,75 ml), Diethylchlorophosphonat (16,6 ml) und Tetrahydrofuran (300 ml). Am Ende der Reaktion wird unlösliches Material (8 g), das als Ausgangsbenzophenon identifiziert wird, abfiltriert. Nach üblichem Aufarbeiten erhält man 22 g eines gelborangen Festkörpers, der aus Aether kristallisiert wird. Man erhält 19.6 g 3,3-bis(3,4-Dimethoxyphenyl)-2-propenal mit einem Schmelzpunkt von 129-130°C.

Beipsiel 8

Man verfährt wie in Beispiel 1 und verwendet die folgenden Reagenzien: 3,3'-Dichlorbenzophenon (25,1 g), Diisopropylamin (32,2 ml), 1,6M n-Butyllithium in Hexan (144 ml), Acetaldehyd-N-tert-butylimin (14.75 ml), Diethylchlorophosphonat (16,6 ml) und Tetrahydrofuran (200 ml). Nach dem üblichen Arbeiten erhält man 24,0 g 3,3-bis(3-Chlorphenyl)-2-propenal als orange-farbenes Oel, das im wesentlichen rein ist.

Beispiel 9

Man verfährt wie in Beispiel 1 und verwendet die folgenden Reagenzien: 4,4'-Dichlorbenzophenon (25,1 g), Diisopropylamin (32,2 ml), 1,6M n-Butyllithium in Hexan (144 ml), Acetaldehyd -N-tert-butylimin (14,75 ml), Diethylchlorophosphonat (16,6 ml) und Tetrahydrofuran (200 ml). Nach üblichem Aufarbeiten erhält man 23,8 g im wesentlichen reines 3,3-bis(4-Chlorphenyl)-2-propenal als orange farbenes Oel.

Beispiel 10

a) Zu einer gerührten Lösung von n-Butyllithium in Hexan (1.6M; 100 ml) gibt man unter Argon und bei -78°C tropfenweise eine Lösung von 3-Bromlenzotrilfuorid (36 g) in trockenem Aether (75 ml) über einen Zeitraum von 15 Min. Nach Beendigung der Zugabe wird die Lösung während 45 Min. bei -78°C gerührt und dann über eine Zeitraum von 20 Min. mit einer Lösung von α,α,α-Trifluor-m-toluonitril (25,7 g) in trockenem Aether versetzt. Man lässt die dunkelrote Mischung während 30 Min. bei -78°C reagieren, entfernt dann das Kühlbad und versetzt sofort rasch mit 6N-Salzsäure (100 ml). Das Reaktionsgemisch entfärbt und es bildet sich ein dichter Niederschlag, wobei die Reaktionstemperatur rasch auf 0-5°C steigt. Man gibt Wasser (200 ml) und Aether (200 ml) dazu, erwärmt das Gemisch auf 35°C und rührt bei dieser Temperatur bis der Niederschlag aufgelöst ist (2 Stunden). Nach dem Abkühlen werden die Phasen getrennt und die wässrige Phase wird mit Aether (200 ml) gewaschen. Die organischen Auszüge werden ihrerseits mit gesättigter Natriumlicarbonatlösung gewaschen, vereinigt, getrocknet ($MgSO_4$) und eingedampft. Der feste Rückstand wird in heissem Hexan (200 ml) aufgelöst und man lässt die Mischung über Nacht bei 0-5°C stehen. Das erhaltene farblose kristalline Material wird abfiltriert, mit Hexan gewaschen und getrocknet. Man erhält 43.9 g 3,3'-bis(Trilfuormethyl)benzophenon mit einem Schmelzpunkt von 98,5-99,5°C.

b) Man verfährt wie in Beispiel 1 und verwendet die folgenden Reagenzien: 3,3'bis(Trilfuormethyl)-benzophenon 31.8 g), Diisopropylamin (32,2 ml), 1,6M n-Butyllithium in Hexan (144 ml), Acetaldehyd-N-tert-butylimin (14,75 ml), Diethylchlorophosphonat (16.6 ml) und Tetrahydrofuran (200 ml). Nach dem üblichen Aufarbeiten wird das Produkt mittels HPLC gereinigt. Man erhält 30,8 g 3,3'-bis(Trilfuormethyl)-phenyl]-2-propenal als Oel.

Beispiel 11

Man verfährt wie in Beispiel 1 und verwendet die folgenden Reagenzien: 3,3'-Dinitrobenzophenon (27,3 g), Diisopropylamin (32,2 ml), 1,55M n-Butyllithium in Hexan (148 ml), Acetaldehyd-N-tert-Butylimin (14,75 ml), Diethylchlorophosphonat (16,6 ml) und Tetrahydrofuran (200 ml). Nach dem üblichen Aufarbeiten erhät man einen braunen Festkörper, den man aus 2-Propanol kristallisiert. Man erhält 23 g des leicht verunreinigten Aldehydes. Nach Umkristallisieren aus Methylenchlorid/Hexan erhält man 20,6 g 3,3'-bis(3-Nitrophenyl)-2-propenal mit einem Schmelzpunkt von 131-136°C. Durch Umkristallisieren einer kleinen Portion erhält man eine analytisch reine Probe mit einem Schmelzpunkt von 134-136°C.

Beispiel 12

Man verfährt wie in Beispiel 1 und verwendet die folgenden Reagenzien: 3-Methoxybenzophenon (17,0 g), Diisopropylamin (32,2 ml), 1,55M n-Butyllithium in Hexan (148 ml), Acetaldehyd N-Tert-butylimin (14,75 ml), Diethylchlorophosphonat (16,6 ml) und Tetrahydrofuran (200 ml). Das nach üblichem Aufarbeiten erhaltene Rohprodukt wird mittels HPLC gereinigt. Man erhält 13,0 g einer Mischung (5:4) aus (E)- und (Z)-3-(3-Methoxyphenyl)-3-phenyl-2-propenalals Oel.

Beispiel 13

a) Eine Lösung von 3-Methoxybenzoylchlorid (301 g) in trockenem Tetrahydrofuran (600 ml) wird unter Rühren und unter Argon zu einer Lösung von 2-Mercaptopyridin (200,5 g) und Triethylamin (345 ml) in trockenem Tetrahydrofuran (1,2 l) gegeben. Unter Verwendung eines Eiswasser-Bades wird die Reaktionstemperatur bei 0-5°C gehalten. Das Kühlbad wird dann entfernt und nach 45 Min. bei Raumtemperatur wir das Reaktionsgemisch filtriert, um Triethylaminhydrochlorid zu entfernen. Das Filtrat und die Waschlösungen werden zur Trockne eingedampft, und der Rückstand wird zwischen 1N Natriumhydroxyd (250 ml) und Aether (2 l) verteilt. Die organische Phase wird abgetrennt, mit 1N Natriumhydroxyd (2 x 250 ml) und Kochsalzlösung gewaschen und eingedampft. Das verbleibende Oel (428,8 g) wird in einem Kugelrohr-Apparat destilliert (180-200°C/0,05mm (6,7 Pa)). Man erhält 417,3 g S-(2-Pyridyl)-3-methoxybenzolthionat.

b) Eine Lösung von 3-Fluorphenylmagnesiumlromid - hergestellt aus Magnesium (3,2 g) und 3-Bromfluorbenzol (22,25 g) in Tetrahydrofuran (55 ml) - wird tropfenweise zu einer gekühlten (-5°C) Lösung von S-(2-Pyridyl)-3-methoxybenzolthionat (30,66 g) in Tetrahydrofuran gegeben und zwar so, dass die Reaktionstemperatur 5°C nicht übersteigt. Die Mischung wird während 15 Min. bei 0°C gerührt und dann während 30 Min. bei Raumtemperatur. Nach Zugabe von Wasser (5 ml) wird das Lösungsmittel unter vermindertem Druck abgedampft, und der Rückstand wird in einer Mischung aus 1N Salzsäure (300 ml) und Aether (300 ml) aufgenommen. Die abgetrennte organische Phase wird mit 1N Salzsäure (2 x 100 ml), 1N Natriumhydroxyd (3 x 150 ml) und mit Kochsalzlösung gewaschen. Die wässrigen Phasen werden nacheinander mit Aether (2 x 200 ml) gewaschen, und die vereinigten aetherischen Auszüge werden getrocknet ($MgSO_4$) und eingedampft. Man erhält 30 g eines schwach gelben Festkörpers. Dieses rohe Material wird aus Hexan kristallisiert. Man erhält 22,1 g 3-Fluor-3′-methoxybenzophenon mit einem Schmelzpunkt von 56-57°C. Durch Eindampfen der Mutterlauge erhält man eine weitere Portion von 3,4 g dieses Produktes mit einem Schmelzpunkt von 55-57°C.

c) Man verfährt wie in Beispiel 1 und verwendet die folgenden Reagenzien: 3-Fluor-3′-methoxybenzophenon (23 g), Diisopropylamin (32,2 ml), 1,55M n-Butyllithium in Hexan (148 ml), Acetaldehyd N-Tert-butylimin (14,75 ml), Diethylchlorophosphonat (16,6 ml) und Tetrahydrofuran (200 ml). Nach dem üblichen Aufarbeiten erhält man 30 g eines Oeles, das mittels HPLC (Methylenchlorid/Hexan; 8:3) gereinigt wird. Man erhält 25,2 g 3-(4-Fluorphenyl)-3-(3-methoxyphenyl)-2-propenal als Mischung (1:1) der entsprechenden (E)- und (Z)-Isomeren.

Beispiel 14

Eine gerührte Lösung von Diisopropylamin (4,02 ml) und Acetonitril in trockenem Tetrahydrofuran (30 ml) wird unter Argon tropfenweise mit 1,6M n-Butyllithium in Hexan (18 ml) behandelt. Die Reaktionstemperatur wird während der Zugabe und für 15 Min. danach bei -5°C gehalten. Anschliessend wird auf -78°C gekühlt und eine Lösung von 2,2′-Bis(trifluormethyl)benzophenon (8,0 g) in Tetrahydrofuran wird so zugegebenen, dass die Reaktionstemperatur -65°C nicht übersteigt. Die Mischung wird während 10 Min. bei -78°C gerührt, worauf man über einen Zeitraum von 30 Min. langsam auf Raumtemperatur erwärmen lässt. Das Lösungsmittel wird unter vermindertem Druck abgedampft, und der Rückstand wird mit Wasser (100 ml) behandelt, worauf der erhaltene Festkörper abfiltriert, mit Wasser gewaschen und getrocknet wird. Durch Kristallisieren des Festkörpers aus Methylenchlorid/Hexan erhält man 7,72 g 3-Hydroxy-3,3-bis[2-(trifluormethyl)-phenyl]propannitril mit einem Schmelzpunkt von 168-170°C. Durch Umkristallisieren einer Probe aus dem gleichen Lösungsmittelsystem erhält man eine analytisch reine Probe mit einem Schmelzpunkt von 171-172°C.

Man kühlt Schwefelsäure (25 ml) auf -5°C ab und gibt portionenweise das obige Carbinol 3-Hydroxy-3,3-bis[2-(trifluormethyl)-phenyl]propanitril (7,2 g) dazu. Nach beendeter Zugabe wird das Kühlbad entfernt und die Suspension wird solange gerührt, bis der gesamte Festkörper in Lösung gegangen ist (30 Min.). Das Reaktionsgemisch wird dann auf Eis gegossen (300 g) und das erhaltene ölige Gemisch wird mit

Toluol (2 x 100 ml) extrahiert. Die organischen Auszüge werden mit Wasser (2 x 100 ml) gewaschen, vereinigt, getrocknet ($MgSO_4$) und eingedampft. Man erhält 6,6 g eines Oels, das man aus 2-Propanol kristallisiert. Man erhält 5,8 g 3,3-Bis[2-(trifluormethyl)phenyl]-2-propennitril mit einem Schmelzpunkt von 65-68 ° C. Durch Umkristallisieren einer Portion aus 2-Propanol erhält man analytisch reines Nitril mit einem Schmelzpunkt von 67-68 ° C.

Eine Lösung des obigen Nitrils (5,6 g) in trockenem Toluol, wird unter Argon auf -40 ° C gekühlt, worauf man über einen Zeitraum von 15 Min. eine 1,5M Lösung von Diisobutylaluminiumhydrid in Toluol (12 ml) dazugibt und das Reaktionsgemisch auf Raumtemperatur erwärmen lässt. Nach 30 Min. wird die Lösung in einem Eisbad abgekühlt und tropfenweise mit einer 2N Schwefelsäurelösung (50 ml) versetzt. Die saure Mischung wird während 2 Std. bei 25 ° C gerührt, worauf man die wässrige Phase abtrennt und mit Toluol (50 ml) extrahiert. Die organischen Auszüge werden mit Wasser gewaschen, vereinigt, getrocknet ($MgSO_4$) und eingedampft. Man erhält 5,2 g im wesentlichen reines 3,3-Bis[2-(trifluormethyl)phenyl]-2-propenal als Oel.

Beispiel 15

Zu einer Suspension von 4,4′-Diphenylbenzophenon (15,5 g) und Acetonitril in trockenem Benzol (200 ml) gibt man rasch tropfenweise eine Suspension von Natriumamid (2,3 g) in trockenem Benzol (20 ml) dazu. Die erhaltene Mischung wird während 60 Min. unter Rückfluss gerührt, abgekühlt und vorsichtig mit Eis (50 g) behandelt. Die Phasen werden getrennt, und die wässrige Phase wird mit Toluol extrahiert. Die organischen Auszüge werden mit Wasser gewaschen, vereinigt, getrocknet ($MgSO_4$) und eingedampft. Man erhält 13,5 g 3,3-Bis[(1,1′-biphenyl)-4-yl]-3-hydroxypropannitril als Oel.

Das rohe 3,3-Bis[(1,1-biphenyl)-4-yl]-3-hydroxypropannitril (13,5 g; 0,036 mol) wird in Trifluoressigsäure (30 ml) gelöst, und die Lösung wird während 10 Min. auf 65 ° C erwärmt. Nach Abdampfen des Lösungsmittels im Vakuum wird der Rückstand mit Wasser behandelt, und der erhaltene Festkörper wird in Methylenchlorid gelöst. Die getrocknete ($MgSO_4$) Lösung wird eingedampft, wobei man 12,2 g 3,3-Bis[(1,1-biphenyl)-4-yl]-2-propennitril als Oel erhält.

Eine Lösung des obigen Nitrils (12,2 g) in trockenem Toluol (75 ml) wird auf -40 ° C gekühlt und wird während der tropfenweisen Zugabe einer 1,5M Lösung von Diisobutylaluminiumhydrid in Toluol (25 ml) bei dieser Temperatur gehalten. Man lässt die Reaktion auf Raumtemperatur erwärmen, kühlt nach 30 Min. auf 10 ° C ab und versetzt tropfenweise unter starkem Rühren mit 2N Schwefelsäure (100 ml). Die Mischung wird während 30 Min. bei Raumtemperatur gerührt, worauf die Phasen getrennt und die wässrige Phase mit Toluol (50 ml) extrahiert wird. Die vereinigten organischen Auszüge werden gewaschen, getrocknet ($MgSO_4$) und eingedampft. Man erhält 12 g des rohen Aldehydes, dass mittels HPLC (Toluol) gereinigt wird. Man erhält 3,8 g 3,3-Bis[(1,1′-biphenyl)-4-yl]-2-propenal. Eine Probe wird aus Methylenchlorid/Essigester kristallisiert, und man erhält analytisch reines Aldehyd mit einem Schmelzpunkt von 163-164 ° C.

Beispiel 16

Man verfährt wie in Beispiel 1, wobei jedoch die Reaktion nach 30 Std. aufgearbeitet wird. Man verwendet die folgenden Reagenzien: 4,4′-Dimethylbenzophenon (21 g), Diisopropylamin (32,2 ml), 1,55M n-Butyllithium in Hexan (143 ml), Acetaldehyd N-Tert-butylimin (14,75 ml), Diethylchlorophosphonat (17,6 ml) und Tetrahydrofuran (200 ml). Man arbeitet in üblicher Weise auf, wobei jedoch die Oxalsäure-katalysierte Hydrolyse über Nacht bei Raumtemperatur durchgeführt wird. Man erhält 23 g Rohprodukt, das aus Hexan kristallisiert wird. Man erhält 18,1 g 3,3-Bis(4-methylphenyl)-2-propenal mit einem Schmelzpunkt von 92-93,5 ° C.

Beispiel 17

Man verfährt wie in Beispiel 1, wobei man jedoch die Reaktion nach 17 Std. aufarbeitet. Man verwendet die folgenden Reagenzien: 2,4′-Dimethoxybenzophenon (21,8 g), Diisopropylamin (32,2 ml), 1,55M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, wobei jedoch die Oxalsäure-katalysierte Hydrolyse über Nacht bei Raumtemperatur durchgeführt wird. Man erhält 24 g Rohprodukt bestend aus (E)-3-(2-Methoxyphenyl)-3-(4-methoxyphenyl)-2-propenal und (Z)-3-(2-Methoxyphenyl)-3-(4-methoxyphenyl)-2-propenal in einem E/Z-Verhältnis von etwa 7:3. Durch dreimaliges Umkristallisieren dieses Materials aus Aether/Hexan erhält man 10 g (E) -3-(2-Methoxyphenyl)-3-(4-methoxyphenyl)-2-propenal mit einem

Schmelzpunkt von 92-94°C. Eine Probe wird aus Aether/Hexan umkristallisiert und liefert analytisch reines Material mit einem Schmelzpunkt von 93-94°C.

Die Mutterlage aus der ersten Kristallisation wird eingedampft und mittels HPLC Aether/Hexan; 1:3; 3 Recyclisierungen) aufgetrennt. Durch Eindampfen der Fraktionen, welche das weniger polare Aldehyd enthalten, erhält man 3 g eines Oeles, dass man aus Aether/Hexan kristallisiert. Man erhält 2,2 g (Z)-3-(2-methoxyphenyl)-3-(4-methoxyphenyl)-2-propenal. Durch Umkristallisieren einer Probe aus den gleichen Lösungsmitteln erhält man analytisch reines Material mit einem Schmelzpunkt von 110°C (wird bei etwa 78°C weich).

Beispiel 18

Man verfährt wie in Beispiel 1, arbeitet die Reaktion jedoch nach 40 Std. auf. Man verwendet die folgenden Reagenzien: 4-Methoxybenzphenon (21,2 g), Diisopropylamin (32,2 ml), 1,55M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse über Nacht bei Raumtemperatur durch. Man erhält 24,2 g Rohprodukt bestehend aus (E)-3-(4-Methoxyphenyl)-3-phenyl-2-propenal und (Z)-3-(4-Methoxyphenyl)-3-phenyl-2-propenal in einem E/Z-Verhältnis von etwa 1:1. Durch Auftrennen dieses Produktes mittels HPLC (Aether/Hexan; 1:7; 3 Recyclisierungen) erhält man 7,3 g der weniger polaren Komponente (Z)-3-(4-Methoxyphenyl)-3-phenyl-2-propenal als Oel, sowie 8,7 g der polareren Komponente (E)-3-(4-Methoxyphenyl)-3-phenyl-2-propenal als Oel.

Beispiel 19

Man verfährt wie in Beispiel 1, arbeitet jedoch die Reaktion nach 17 Stunden auf. Man verwendet die folgenden Reagenzien: 3-(4-Methoxybenzoyl)pyridin (22 g), Diisopropylamin (32,2 ml), 1,55M n-Butyllithium in Hexan (148 ml), Acetaldehyd N-Tert-Butylimin (14,75 ml), Diethylchlorophosphonat (16,6 ml) und Tetrahydrofuran (200 ml). Man arbeitet in üblicher Weise auf, führt jedoch die Hydrolyse des Imins durch Behandeln mit 2N HCl (300 ml) während 9 Std. bei 70°C durch. Nach Basischstellen und Extraktion erhält man 24 g Rohprodukt, das aus einer Mischung aus (E)-3-(4-Methoxyphenyl)-3-(3-pyridinyl)-2-propenal und (Z)-3-(4-Methoxyphenyl)-3-(3-pyridinyl)-2-propenal besteht. Reinigung des rohen Gemisches mittels HPLC (Aether; 3 Recyclisierungen) liefert 5,45 g (E)-3-(4-Methoxyphenyl)-3-(3-pyridinyl)-2-propenal und 11 g (Z)-3-(4-Methoxyphenyl)-3-(3-pyridinyl)2-propenal als Oele.

Beispiel 20

Man verfährt wie in Beispiel 1, arbeitet die Reaktion jedoch nach 3 Tagen auf. Man verwendet die folgenden Reagenzien: 4-Methoxybenzaldehyd (13,5 g), Diisopropylamin (32,2 ml), 1,6M n-Butyllithium in Hexan (143 ml), Acetaldehyd N-Tert-butylimin (14,75 ml), Diethylchlorophosphonate (16,6 ml) und Tetrahydrofuran (200 ml). Man arbeitet in üblicher Weise auf, führt jedoch die Oxalsäure-katalysierte Hydrolyse über Nacht bei Raumtemperatur und dann während 2 Std. bei 85°C durch. Man erhält 12,5 g Rohprodukt, das aus Aether/Hexan kristallsisiert wird. Man erhält 10 g (E)-3-(4-Methoxyphenyl)-2-propenal mit einem Schmelzpunkt von 58,5-60°C.

Beispiel 21

Man verfährt wie in Beispiel 1, arbeitet die Reaktion jedoch nach 3 Tagen auf. Man verwendet die folgenden Reagenzien: 4'-Methoxyacetophenon (15 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse über Nacht bei Raumtemperatur durch. Man erhält 16,3 g Rohprodukt bestehend aus (E)-3-(4-Methoxyphenyl)-2-butenal und (Z)-3-(4-Methoxyphenyl)-2-butenal in einem E/ Z-Verhältnis von etwa 3:2. Das Material wird in Methylenchlorid (400 ml) enthaltend Trifluoressigsäure (20 ml) aufgelöst, und die Lösung wird über Nacht bei Raumtemperatur gerührt. Anschliessend wird sie nacheinander mit Wasser (3 x 250 ml), 2N NaOH (100 ml) und mit Kochsalzlösung gewaschen. Die getrocknete ($MgSO_4$) organische Phase wird eingedampft und der Rückstand wird durch eine kurze Kieselgelsäule (150 g), welche mit Methylenchlorid hergestellt worden ist, filtriert. Man erhält eine Mischung aus (E) -3-(4-Methoxyphenyl)-2-butenal und (Z)-3-(4-Methoxyphenyl)-2-butenal in einem E/Z-Verhältnis von etwa 3:1. Das Gemisch wird mittels HPLC

(Aether/Hexan; 1:3) aufgetrennt. Man erhält 2,85 g (Z)-3-(4-Methoxyphenyl)-2-butenal als Oel und 8,6 g kristallines (E)-3-(4-Methoxyphenyl)-2-butenal. Eine Portion des (E)-Isomeren wird aus Aether/Hexan kristallisiert, wobei man eine analytisch reine Probe mit einem Schmelzpunkt von 42,5-45 ° C erhält.

Beispiel 22

Man verfährt wie in Beispiel 1, arbeitet die Reaktion jedoch nach 3 Tagen auf. Man verwendet die folgenden Reagenzien: 4'-Methoxypropiophenon (16,4 g), Diisopropylamin (32,2 ml),1,6M n-Butyllithium in Hexan (143 ml), Acetaldehyd N-Tert-Butylimin (14,75 ml), Diethylchlorophosphonate (16,6 ml) und Tetrahydrofuran (200 ml). Man arbeitet in üblicher Weise auf, führt jedoch die Oxalsäure-katalisierte Hydrolyse während 3 Tagen bei Raumtemperatur durch. Man erhält 21,4 g Rohprodukt bestehend aus (E)-3-(4-Methoxyphenyl)-2-pentenal und (Z)-3-(4-Methoxyphenyl)-2-pentenal in einem E/Z-Verhältnis von etwa 1:1. Das Gemisch wird mittels HPLC (Aether/Hexan; 1:7,5) aufgetrennt. Man erhält 7 g (Z)-3-(4-Methoxyphenyl)-2-pentenal als Oel sowie 7,1 g (E)-3-(4-Methoxyphenyl)-2-pentenal als Oel.

Beispiel 23

Man verfährt wie in Beispiel 1, arbeitet jedoch die Reaktion nach 3 Tagen auf. Man verwendet die folgenden Reagenzien: 1-(4-Methoxyphenyl)-2-methylpropanon (17,8 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt die Oxalsäure-katalysierte Hydrolyse jedoch über Nacht bei Raumtemperatur durch. Man erhält 21,3 g Rohprodukt bestehend aus (E)- und (Z)-3-(4-Methoxyphenyl)-4-methyl-2-pentenal in einem Verhältnis von ungefähr 2:3.

Beispiel 24

Man verfährt wie in Beispiel 1, arbeitet die Reaktion jedoch nach 3 Tagen auf. Man verwendet die folgenden Reagenzien: 4'-Methoxybutyrophenon (17,8 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse während 3 Tagen bei Raumtemperatur durch. Man erhält 21,1 g Rohprodukt bestehend aus (E)-3-(4-Methoxyphenyl)-2-hexenal und (Z)-3-(4-Methoxyphenyl)-2-hexenal in einem E/Z-Verhältnis von etwa 3:4. Das Gemisch wird mittels HPLC (Aether/Hexan; 1:7,5) aufgetrennt. Man erhält 8,2 g (Z)-3-(4-Methoxyphenyl)-2-hexenal als Oel und 6,0 g (E)-4-(4-Methoxyphenyl)-2-hexenal als Oel.

Beispiel 25

Man verfährt wie in Beispiel 1, arbeitet die Reaktion jedoch nach 3 Tagen auf. Man verwendet die folgenden Reagenzien: 4'-Methoxyvalerophenon (19,2 g), Diisopropylamin (32,2 ml), 1,55 M n-Butyllithium in Hexan (148 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse über Nacht bei Raumtemperatur durch. Man erhält 22,1 g Rohprodukt bestehend aus (E)-3-(4-Methoxyphenyl)-2-heptenal und (Z)-3-(4-Methoxyphenyl)-2-heptenal in einem E/Z-Verhältnis von ungefähr 4:3. Das Gemisch wird mittels HPLC (Aether/Hexan; 1:7,5; 3 Recyclisierungen) aufgetrennt. Man erhält 7,5 g (Z)-3-(4-Methoxyphenyl)-2-heptenal als Oel und 10 g (E)-3-(4-Methoxyphenyl)-2-heptenal als Oel.

Beispiel 26

Man verfährt wie in Beispiel 1, arbeitet die Reaktion jedoch nach 3 Tagen auf. Man verwendet die folgenden Reagenzien: 3'-Methoxyvalerophenon (20,6 g), Diisopropylamin (32,2 ml), 1,6M n-Butyllithium in Hexan (144 ml), Acetaldehyd N-Tert-butylimin (14,75 ml), Diethylchlorophosphonat (16,6 ml) und Tetrahydrofuran (200 ml). Man arbeitet in üblicher Weise auf, führt die Oxalsäure katalysierte Hydrolyse jedoch während mehrerer Tage bei Raumtemperatur durch. Man erhält 24,3 g eines Oeles bestehend aus einer Mischung (4:3) aus (E)-3-(3-Methoxyphenyl)-2-heptenal und (Z)-3-(3-Methoxyphenyl)-2-heptenal.

Beispiel 27

Man verfährt wie in Beispiel 1, arbeitet die Reaktion jedoch nach 5 Tagen auf. Man verwendet die folgenden Reagenzien: 4'-Methoxyhexanophenon (20,6 g), Diisopropylamin (32,2 ml), 1,55M n-Butyllithium in Hexan (148 ml), Acetaldehyd N-Tert-butylimin (14,75 ml), Diethylchlorophosphonat (16,6 ml) und Tetrahydrofuran (200 ml). Man arbeitet in üblicher Weise auf, führt die Oxalsäure-katalysierte Hydrolyse jedoch über Nacht bei Raumtemperatur aus. Man erhält 23,3 g Rohprodukt bestehend aus (E)-3-(4-Methoxyphenyl)-2-octenal und (Z)-3-(4-Methoxyphenyl)-2-octenal in einem E/ Z-Verhältnis von etwa 3:2. Das Gemisch wird mittels HPLC (Aether/Hexan; 1:7) aufgetrennt, wobei man 8,1 g (Z)-3-(4-Methoxyphenyl)-2-octenal als Oel und 9 g (E)-3-(4-Methoxyphenyl)-2-octenal als Oel erhält.

Beispiel 28

Wie in Beispiel 14 werden Acetonitril (30 ml) und eine Lösung von 4'-Methoxyhexanophenon (93,2 g) in trockenem Tetrahydrofuran (250 ml) nacheinander zu einer auf -70 ° C gekühlten Lösung von Lithiumdiisopropylamid gegeben, welche aus Diisopropylamin (72,5 ml), 2,5M n-Butyllithium und Hexan (207 ml) in Tetrahydrofuran (250 ml) hergestellt worden ist. Man rührt die Mischung während 15 Min. bei -70 ° C, entfernt dann das Kühlbad und lässt auf -40 ° C erwärmen. Anschliessend wird eine Lösung von Essigsäure (60 g) in Wasser (60 ml) rasch dazugetropft. Man lässt auf Raumtemperatur erwärmen und arbeitet in üblicher Weise auf. Man erhält 112,8 g eines Oeles, das aus Aether/Hexan kristallisiert wird. Man erhält 107,8 g 3-Hydroxy-3-(4-methoxyphenyl)octannitril als farblosen Festkörper mit einem Schmelzpunkt von 45 -4 7 ° C.

Eine Lösung des obigen Carbinols (107,8 g) in Methylenchlorid (540 ml), enthaltend Trifluoressigsäure (21,6 ml), wird während 7 Std. unter Rückfluss erhitzt und dann über Nacht bei Raumtemperatur stehen gelassen. Die Lösung wird nacheinander mit Wasser (250 ml), 1N NaOH-Lösung (500 ml) und mit Kochsalzlösung (100 ml) gewaschen. Nach Waschen der wässrigen Phasen mit Methylenchlorid (2 x 100 ml), werden die vereinigten organischen Auszüge getrocknet ($MgSO_4$) und eingedampft. Man erhält 99,2 g rohes (E)-3-(4-Methoxyphenyl)-2-octennitril als Oel.

Eine Lösung des rohen Nitrils (99,2 g) in trockenem Toluol (960 ml) wird wie in Beispiel 14 mit einer 1,5M-Lösung von Diisobutylaluminiumhydrid in Toluol (385 ml) bei -40 ° C behandelt. Man rührt während 60 Min. bei -40 ° C und arbeitet dann in üblicher Weise auf. Man erhält 100,2 g rohes (E)-3-(4-Methoxyphenyl)-2-octenal als schwach gelbes Oel.

Beispiel 29

Man verfährt wie in Beispiel 1, arbeitet die Reaktion jedoch nach mehreren Tagen auf. Man verwendet die folgenden Reagenzien: 4'-Fluorhexanophenon (19,4 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse während 3 Tagen bei Raumtemperatur durch. Man erhält 23,7 g Rohprodukt bestehend aus den (E)- und (Z)-Isomeren in einem E/Z-Verhältnis von etwa 1:1. Die Mischung wird mittels HPLC (Aether/Hexan; 1:7) aufgetrennt. Man erhält 6,5 g (Z) -3-(3-Fluorphenyl)-2-octenal als Oel und 6,0 g (E)-3-(3-Fluorphenyl)-2-octenal als Oel.

Beispiel 30

Man verfährt wie in Beispiel 1, arbeitet die Reaktion jedoch nach mehreren Tagen auf. Man verwendet die folgenden Reagenzien: 4'-Fluorhexanophenon (19,4 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse während 3 Tagen bei Raumtemperatur durch. Man erhält 21,5 g Rohprodukt bestehend aus den (E)- und (Z)-Isomeren in einem E/Z-Verhältnis von etwa 1:1. Das Gemisch wird mittels HPLC (Aether/Hexan; 1:7) aufgetrennt. Man erhält 8,3 g (Z) -3 -(4-Fluorphenyl)-2-octenal als Oel und 8,7 g (E)-3-(4-Fluorphenyl)-2-octenal als Oel.

Beispiel 31

Man verfährt wie in Beispiel 1, arbeitet die Reaktion jedoch nach 3 Tagen auf. Man verwendet die folgenden Reagenzien: 3',4'-Dimethoxyhexanophenon (23,6 g), Diisopropylamin (32,2 ml), 1,6M n-Butyllithium in Hexan (144 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse während mehreren Tagen bei Raumtemperatur durch. Man erhält 25,5 g eines orange-farbenen Oels bestehend aus einer Mischung (ungefährt 4:3) aus (E)-3-(3,4-Dimethoxyphenyl)-2-octenal und (Z)-3-(3,4-Dimethoxyphenyl)-2-octenal.

Beispiel 32

Man verfährt wie in Beispiel 1, wobei die Reaktion jedoch nach 3 Tagen aufgearbeitet wird. Man verwendet die folgenden Reagenzien: 4'-Methoxyheptanophenon (22 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, wobei jedoch die Oxalsäure-katalysierte Hydrolyse über Nacht bei Raumtemperatur durchgeführt wird. Man erhält 27 g Rohprodukt bestehend aus den (E)- und (Z)-Isomeren in einem E/Z-Verhältnis von etwa 2:3. Dieses Material wird in Methylenchlorid (500 ml), enthaltend Trifluoressigsäure (20 ml) gelöst, und die Lösung wird über Nacht bei Raumtemperatur gerührt. Man wäscht dann nacheinander mit Wasser (3 x 200 ml), 1N NaOH (2 x 200 ml) und Kochsalzlösung. Die getrocknete ($MgSO_4$) organische Phase wird eingedampft, und der Rückstand wird über eine Kieselgelsäule (150 g), welche in Methylenchlorid hergestellt worden ist, gelassen. Man erhält 22,3 g einer Mischung aus (E)-3-(4-Methoxyphenyl)-2-nonenal und (Z)-3-(4-Methoxyphenyl)-2-nonenal in einem E/Z-Verhältnis von etwa 11:9.

Beispiel 33

Man verfährt wie in Beispiel 1, arbeitet die Reaktion jedoch nach 3 Tagen auf. Man verwendet die folgenden Reagenzien: 4'-Methoxynonanophenon (24,8 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt die Oxalsäure-katalysierte Hydrolyse jedoch über Nacht bei Raumtemperatur durch. Man erhält 31 g Rohprodukt bestehend aus den (E)- und (Z)-Isomeren in einem E/Z-Verhältnis von 2:3. Dieses Material wird in Methylenchlorid (500 ml), das Trifluoressigsäure (20 ml) enthält, aufgelöst. Die Lösung wird über Nacht bei Raumtemperatur gerührt und dann nacheinander mit Wasser (3 x 200 ml), 1N NaOH (2 x 200 ml) und Kochsalzlösung gewaschen. Die getrocknete ($MgSO_4$) organische Phase wird eingedampft, und der Rückstand wird durch eine kurze Kieselgelsäule (150 g), welche in Methylenchlorid hergestellt worden ist, gelassen. Man erhält 26 g einer Mischung aus (E)-3-(4-Methoxyphenyl)-2-undecenal und (Z)-3-(4-Methoxyphenyl)-2-undecenal in einem E/Z-Verhältnis von etwa 3:2.

Beispiel 34

Man verfährt wie in Beispiel 1, arbeitet die Reaktion jedoch nach 3 Tagen auf. Man verwendet die folgenden Reagenzien: 4'-Methoxytridecanophenon (30,4 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt die Oxalsäurekatalysierte Hydrolyse jedoch während mehrerer Tage bei Raumtemperatur durch. Man erhält 32 g einer Mischung (ungefähr 1:1) aus (E)-3-(4-Methoxyphenyl)-2-pentadecenal und (Z)-3-(4-Methoxyphenyl)-2-pentadecenal.

Beispiel 35

Man verfährt wie in Beispiel 1, arbeitet die Reaktion jedoch nach 3 Tagen auf. Man verwendet die folgenden Reagenzien: Cyclopropyl 4-Methoxyphenylketon (17,6 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt die säurekatalysierte Hydrolyse jedoch während 3 Tagen bei Raumtemperatur durch. Man erhält 21,1 g einer Mischung (ungefähr 2:3) aus (E)- und (Z)-Aldehyd. Dieses Material wird mittels HPLC (Aether/Hexan; 1:7,5) gereinigt. Man erhält 7,2 g (Z)-3-Cyclopropyl-3-(4-methoxyphenyl)-2-propenal und 10,3 g einer Mischung aus den isomeren Aldehyden,

in welchem das (E)-3-Cyclopropyl-3-(4-methoxyphenyl)-2-propenal (7:3) überwiegt.

Beispiel 36

Man verfährt wie in Beispiel 1, arbeitet die Reaktion jedoch nach 3 Tagen auf. Man verwendet die folgenden Reagenzien: Cyclohexyl 4-Methoxyphenylketon (21,8 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse während 3 Tagen bei Raumtemperatur durch. Man erhält 25 g einer Mischung (ungefährt 9:11) der (E)- und (Z)-Aldehyde. Dieses Material wird mittels HPLC (Aether/Hexan; 1:7; 3 Recyclisierungen) gereinigt. Man erhält 5,9 g (E)-3-Cyclohexyl-3-(4-methoxyphenyl)-2-propenal als Oel, sowie 7,3 g (Z)-3-Cyclohexyl-3-(4-methoxyphenyl)-2-propenal.

Beispiel 37

Man verfährt wie in Beispiel 1, arbeitet die Reaktion jedoch nach 3 Tagen auf. Man verwendet die folgenden Reagenzien: 6-Undecanon (17 g), Diisopropylamin (32,2 ml), 1,6M n-Butyllithium in Hexan (143 ml), Acetaldehyd N-Tert-butylimin (14,75 ml), Diethylchlorophosphonat (16,6 ml) und Tetrahydrofuran (200 ml). Man arbeitet in üblicher Weise auf, führt jedoch die Oxalsäure-katalysierte Hydrolyse über Nacht bei Raumtemperatur durch. Man erhält den rohen Aldehyd, den man mittels HPLC (Aether/Hexan; 1:16,6) reinigt. Man erhält 15,5 g 3-Pentyl-2-octenal als Oel.

Beispiel 38

a) Wie in Beispiel 14 wird eine Lösung von 2,2'-Dimethoxybenzophenon (24,2 g) und Acetonitril (6 ml) in Tetrahydrofuran (120 ml) zu einer auf -70 °C gekühlten Lösung von Lithiumdiisopropylamid - hergestellt aus Diisopropylamin (16 ml) und 1,55M n-Butyllithium in Hexan (74 ml) - in trockenem Tetrahydrofuran (120 ml) gegeben. Man hält das Reaktiongemisch während 15 Min. bei -70 °C, lässt es dann auf Raumtemperatur erwärmen und arbeitet in üblicher Weise auf. Man erhält 26,4 g $\beta$-Hydroxy-$\beta$-(2-methoxyphenyl)-2-methoxybenzolpropannitril mit einem Schmelzpunkt von 170-180 °C. Eine Probe wird aus Methanol umkristallisiert, wobei man analytisch reines Material mit einem Schmelzpunkt von 181-182 °C erhält.

b) Man gibt Thionylchlorid (4,5 ml) zu einer gerührten Suspension von $\beta$-Hydroxy-$\beta$-(2-methoxyphenyl)-2-methoxybenzolpropannitril (13,45 g) in trockenem Pyridin (70 ml), welche während der Zugabe bei 10 °C gehalten wird. Man rührt während 2 Std. bei Raumtemperatur, verdünnt dann mit Eiswasser und extrahiert mit Methylenchlorid. Die organische Phase wird mit 3N HCl (1 x 150 ml; 1 x 20 ml) und Kochsalzlösung gewaschen, getrocknet ($MgSO_4$) und eingedampft. Der Rückstand wird aus Isopropanol/Hexan kristallisiert. Man erhält 9,0 g 3,3-Bis(2-methoxyphenyl)-2-propennitril mit einem Schmelzpunkt von 99-100 °C. Durch Umkristallisieren einer Portion aus dem gleichen Lösungsmittelgemisch erhält man analytisch reines Material mit einem Schmelzpunkt von 100,5-101,5 °C.

c) Wie in Beispiel 14 wird eine Lösung von 3,3-Bis(2-methoxyphenyl)-2-propennitril (7,3 g) in Toluol (70 ml) bei -40 °C mit einer Lösung von 1,5M Diisobutylaluminiumhydrid in Toluol (21 ml) behandelt. Nach 15 Min. bei -40 °C lässt man das Reaktionsgemisch auf Raumtemperatur erwärmen und arbeitet 30 Min. später in üblicher Weise auf. Durch Kristallisieren des Rohproduktes auf Aether/Hexan erhält man 7,2 g 3,3-Bis(2-methoxyphenyl)-2-propenal mit einem Schmelzpunkt von 59,5-61,5 °C.

Teil V: Herstellung von Verbindungen der Formel II.

Beispiel 1

Ein Lösung von 3,3-Diphenyl-2-propenal (18,73 g) und (Carbethoxymethylene)triphenylphosphoran (33,1 g) in Ethanol (100 ml) wird während 1 Std. bei Raumtemperatur gerührt Man dampft das Lösungsmittel im Vakuum ab und erhitzt den erhaltenen Festkörper in einer Mischung aus Aether (125 ml) und Hexan (250 ml) während 10 Min. unter Rückfluss. Die Mischung wird auf Raumtemperatur abgekühlt, und das ausgefallene Triphenylphosphinoxid (25 g) wird abfiltriert und mit Aether/Hexan (1:2; 2 x 50 ml) gewaschen. Die Filtrate werden eingedampft, und der Rückstand wird über eine kurze Kieselgelsäule (200 g), welche in Methylenchlorid hergestellt worden ist, gelassen, um restliches Triphenylphosphinoxid und Ausgangsylid zu entfernen. Nach Eindampfen der geeigneten Eluate wird das erhaltene Estergemisch mittels HPLC

(Methylenchlorid/Hexan; 1:1) aufgetrennt. Die Fraktionen, welche das weniger polare Material enthalten, werden vereinigt und eingedampft. Man erhält 4,2 g (Z) -5,5-Diphenyl-2,4-pentadiencarbonsäureaethylester als Oel, das beim Stehen kristallisiert, Schmelzpunkt 36-37 ° C.

Durch Eindampfen der Fraktionen, welche den polareren Ester enthalten, erhält man 20,2 g (E)-5,5-Diphenyl-2,4-pentadiencarbonsäureaethylester. Durch Kristallisieren einer Portion aus Pentan erhält man eine analytisch reine Probe mit einem Schmelzpunkt von 34-35 ° C.

Eine Lösung von (Z) -5,5-Diphenyl-2,4-pentadiencarbonsäureaethylester (3,5 g) in Methanol (20 ml) wird mit 4N Kaliumhydroxidlösung (5 ml) behandelt, und die Mischung wird während 30 Min. unter Rückfluss gerührt. Das meiste Methanol wird unter vermindertem Druck abgedampft, und die erhaltene Lösung wird auf Eis gegossen, das 3N Salzsäure (10 ml) enthält. Der erhaltene Festkörper wird abfiltriert, mit Wasser gewaschen, getrocknet und aus Methylenchlorid/Hexan kristallisiert. Man erhält 3,05 g (Z) -5,5-Diphenyl-2,4-pentadiencarbonsäure mit einem Schmelzpunkt von 175-176,5 ° C.

Eine Lösung von (E) -5,5-Diphenyl-2,4-pentadiencarbonsäureaethylester (15 g) in Methanol (80 ml) wird mit 4N Kaliumhydroxidlösung (20 ml) behandelt, und die Mischung wird während 45 Min. unter Rückfluss gerührt. Das meiste Methanol wird unter vermindertem Druck abgedampft, und die erhaltene Lösung wird auf Eis gegossen, das 3N Salzsäure (30 ml) enthält. Der ausgefallene Festkörper wird abfiltriert, mit Wasser gewaschen und getrocknet. Durch Kristallisieren des Rohproduktes aus 2-Propanol erhält man 11,7 g (E)-5,5-Diphenyl-2,4-pentadiencarbonsäure mit einem Schmelzpunkt von 193-194 ° C. Umkristallisation aus dem gleichen Lösungsmittel beeinflusst den Schmelzpunkt nicht.

## Beispiel 2

Wie in Beispiel 1 wird eine Lösung von 3,3-Diphenyl-2-propenal (2,08 g) in Aethanol (25 ml) mit Carbethoxyaethyliden) triphenylphosphoran (4,0 g) während 30 Min. bei Raumtemperatur umgesetzt. Die Reaktion wird in üblicher Weise aufgearbeitet, und man erhält 2,8 g (E)-5,5-Diphenyl-2-methyl-2,4-pentadiencarbonsäuremethylester als einziges Produkt. Der rohe Ester (2,8 g) in Methanol (25 ml) wird mit 2N Kaliumhydroxid (10 ml) behandelt, und die Mischung wird solange unter Rückfluss erhitzt, bis das Oel in Lösung gegangen ist. Anschliessend wird abgekühlt und bei Raumtemperatur über Nacht stehengelassen. Man verdünnt das Reaktionsgemisch mit Wasser (20 ml) und dampft das meiste Methanol unter vermindertem Druck ab. Die Lösung wird mit 2N Salzsäure (12 ml) sauer gestellt und das ausgefallene Material wird abfiltriert, mit Wasser gewaschen und getrocknet. Die rohe Säure wird mit heissem Hexan behandelt, worauf man 2,08 g (E)-5,5-Diphenyl-2-methyl-2,4-pentadiencarbonsäure mit einem Schmelzpunkt von 184-186 ° C erhält. Eine Probe wird aus 2-Propanol kristallisiert, wobei man eine analytisch reine Probe mit einem Schmelzpunkt von 185-186,5 ° C erhält.

## Beispiel 3

Wie in Beispiel 1 wird 3,3-Bis(2-fluorphenyl)-2-propenal (1,85 g) mit (Carbethoxymethylen)-triphenylphosphoran (2,8 g) in Methanol umgesetzt. Das nach dem üblichen Aufarbeiten erhaltene Estergemisch wird mittels HPLC (Methylenchlorid/ Hexan; 1:2) aufgetrennt. Man erhält 0,4 g (Z)-5,5-Bis-(2-fluorphenyl)-2,4-pentadiencarbonsäureaethylester als Oel und 1,7 g (E)-5,5-Bis-(2-fluorphenyl)-2,4-pentadiencarbonsäure als Oel.

Die Hydrolyse von (Z) -5,5-Bis(2-fluorphenyl)-2,4-pentadiencarbonsäureaethylester (0,4 g) in Methanol (5 ml) wird unter Verwendung von 4N Natriumhydroxid (0,6 ml) durchgeführt. Nach Ansäuern wird das Produkt mit Methylenchlorid (2 x 30 ml) extrahiert. Die vereinigten und getrockneten Auszüge werden eingedampft, und der Rückstand wird aus Aether/Hexan kristallisiert. Man erhält 0,35 g (Z)-5,5-Bis(2-fluorphenyl)-2,4-pentadiencarbonsäure mit einem Schmelzpunkt von 147-148 ° C.

Die Hydrolyse von (E)-5,5-Bis(2-fluorphenyl)-2,4-pentadiencarbonsäureaethylester (1,7 g) in Methanol (15 ml) wird unter Verwendung von 4N Natriumhydroxid (2,5 ml) durchgeführt. Nach Ansäuern wird das Produkt mit Methylenchlorid (2 x 50 ml) extrahiert. Die vereinigten Auszüge werden getrocknet und eingedampft, und der Rückstand wird aus Aether/Hexan kristallisiert. Man erhält 1,45 g (E)-5,5-Bis(2-fluorphenyl)-2,4-pentadiencarbonsäure mit einem Schmelzpunkt von 159-160 ° C.

## Beispiel 4

Wie in Beispiel 1 wird 3,3-Bis( 3-fluorphenyl)-2-propenal (17,8 g) mit (Carbethoxymethylen)-triphenylphosphoran (26,2 g) in Tetrachlorkohlenstoff (200 ml) über Nacht bei Raumtemperatur umgesetzt. Das nach dem üblichen Aufarbeiten erhaltene Rohprodukt (23 g) besteht vorwiegend aus (E)-5,5-Bis(3-

fluorphenyl-2,4-pentadiencarbonsäureaethylester. Eine gerührte Lösung dieses Materials (23 g) in heissem Methanol (200 ml) wird in einer Portion mit 2N Natriumhydroxidlösung (75 ml) behandelt. Nach 10 Min. unter Rückfluss wird das Reaktionsgemisch homogen, und das meiste Methanol wird abdestilliert. Die abgekühlte Lösung wird mit Wasser (300 ml) verdünnt, worauf man mit Methylenchlorid (3 x 200 ml) extrahiert. Die organischen Auszüge werden verworfen und die wässrige Phase wird in einem feinen Strahl zu einer gerührten Mischung aus 2N Salzsäure (200 ml) und Eis (300 g) gegeben. Der erhaltene Niederschlag wird abfiltriert, mit Wasser gewaschen und getrocknet. Durch Behandeln der rohen Säure mit heissem Hexan (150 ml) erhält man 18,2 g (E)-5,5-Bis(3-fluorphenyl-2,4-pentadiencarbonsäure mit einem Schmelzpunkt von 162-164 ° C. Aus 2-Propanol erhält man eine analytisch reine Probe mit einem Schmelzpunkt von 163,5-165 ° C.

Die Mutterlaugen aus der Behandlung der rohen (E)-Säure werden eingedampft, wobei man 1,2 g eines Festkörpers erhält, welcher mit dem (Z)-Isomeren angereichert ist. Dieses Material wird dreimal aus Aether/Hexan kristallisiert. Man erhält 0,62 g (Z)-5,5-Bis-(3-fluorphenyl)-2,4-pentadiencarbonsäure mit einem Schmelzpunkt von 141,5-143 ° C.

Beispiel 5

Wie in Beispiel 1 wird 3,3-Bis(4-fluorphenyl)-2-propenal (9,8 g) mit (Carbethoxymethylen)-triphenylphosphoran (14,3 g) in Aethanol (20 ml) während 30 Min. bei Raumtemperatur umgesetzt. Das nach dem üblichen Aufarbeiten erhaltene Estergemisch wird mittels HPLC (Methylenchlorid/Hexan; 1:1) aufgetrennt. Man erhält 2,0 g (Z)-5,5-Bis(4-fluorphenyl)-2,4-pentadiencarbonsäureaethylester als Oel und 9,3 g (E)-5,5-Bis(4-fluorphenyl)-2,4-pentadiencarbonsäureaethylester. Eine Portion des (E)-Isomeren wird aus Hexan kristallisiert, wobei man den reinen Ester mit einem Schmelzpunkt von 71-72,5 ° C erhält.

Eine Lösung von (E)-5,5-Bis(4-fluorphenyl)-2,4-pentadiencarbonsäureaethylester (8,8 g) in Methanol (30 ml) wird mit 2N Natriumhydroxidlösung (20 ml) behandelt, und die Mischung wird während 15 Min. unter Rückfluss erhitzt. Das nach dem üblichen Aufarbeiten erhaltene Rohprodukt wird aus Methylenchlorid/Hexan kristallisiert, wobei man 7,5 g (E)-5,5-Bis(4-fluorphenyl)-2,4-pentadiencarbonsäure mit einem Schmelzpunkt von 186-188 ° C erhält. Durch Umkristallisieren einer Portion aus Aether/Hexan erhält man eine analytisch reine Probe mit einem Schmelzpunkt von 187-188 ° C.

Beispiel 6

Wie in Beispiel 1 wird 3,3-Bis( 3-methoxyphenyl)-2-propenal (24,8 g) mit (Carbethoxymethylen)-trimethylphosphoran (33 g) in Aethanol (100 ml) während 30 Min. bei 50 ° C umgesetzt. Das nach dem üblichen Aufarbeiten erhaltene Estergemisch wird mittels HPLC (Hexan/Aether; 9:1) aufgetrennt. Man erhält 2,3 g (Z)-5,5-Bis(3-methoxyphenyl)-2,4-pentadiencarbonsäureaethylester als Oel und 19,8 g (E)-5,5-Bis(3-methoxyphenyl)-2,4-pentadiencarbonsäureaethylester.

Eine Mischung aus (E)-5,5-Bis(3-methoxyphenyl)-2,4-pentadiencarbonsäureaethylester (19,8 g), Methanol (200 ml) und 2N Natriumhydroxidlösung (40 ml) wird während 30 Min. unter Rückfluss erhitzt. Das meiste Methanol wird im Vakuum abdestilliert, und die erhaltene Lösung wird mit Wasser verdünnt (300 ml), mit 2N Salzsäure (50 ml) angesäuert, worauf der erhaltene Festkörper abfiltriert wird. Das getrocknete Material (17,8 g) wird aus Methylenchlorid/Hexan kristallisiert. Man erhält 15,9 g (E)-5,5-Bis(3-methoxyphenyl)-2,4-pentadiencarbonsäure mit einem Schmelzpunkt von 179-180,5 ° C.

Auf ähnliche Weise wird (Z)-5,5-Bis(3-methoxyphenyl)-2,4-pentadiencarbonsäureaethylester (2,2 g) in Methanol (15 ml) unter Verwendung von 2N Natriumhydroxidlösung (5 ml) hydrolysiert. Durch übliches Aufarbeiten erhält man 2,0 g (Z)-5,5-Bis(3-methoxyphenyl)-2,4-pentadiencarbonsäure. Durch Kristallisieren einer Portion auf Aether erhält man eine analytisch reine Probe mit einem Schmelzpunkt von 178-180 ° C.

Beispiel 7

Eine Mischung aus 3,3-Bis(4-methoxyphenyl)-2-propenal (22,7 g) und (Carbethoxymethylen)-triphenylphosphoran (30 ml) in Tetrachlorkohlenstoff (200 ml) wird über Nacht bei Raumtemperatur gerührt und dann während 5 Std. unter Rückfluss erhitzt, um die Reaktion zu vervollständigen. Das Rohprodukt (28,4 g), das man nach dem üblichen Aufarbeiten erhält, besteht im wesentlichen aus (E)-5,5-Bis(4-methoxyphenyl)-2,4-pentadiencarbonsäureaethylester. Der rohe Ester (28,4 g) wird zusammen mit einer Mischung aus Methanol (200 ml) und 2N Natriumhydroxidlösung (100 ml) während 30 Min. unter Rückfluss erhitzt, worauf das meiste Methanol abdestilliert wird. Die Mischung wird abgekühlt, mit Wasser (300 ml) verdünnt, mit Methylenchlorid (3 x 400 ml) extrahiert und dann auf eine gerührte Mischung aus konzentrierter Salzsäure (50 ml), Wasser (200 ml) und Eis (200 g) gegossen. Der gelbe Niederschlag wird abfiltriert, mit Wasser gewaschen und getrocknet. Dieses Material wird mit Aether (3 x 100 ml) behandelt, wobei man 21,5 g (E)-5,5-Bis(4-methoxyphenyl)-2,4-pentadiencarbonsäure mit einem Schmelzpunkt von 187-189 ° C erhält. Durch Kristallisieren aus Methylenchlorid/Hexan erhält man die reine Säure mit einem Schmelzpunkt von 189,5-190,5 ° C.

Beispiel 8

Eine Mischung aus 3,3-Bis( 3,4-dimethoxyphenyl)-2-propenal (19,6 g) und (Carbomethoxymethylen)-triphenylphosphoran (22 g) in Benzol (150 ml) wird über Nacht bei Raumtemperatur gerührt. Das Lösungsmittel wird unter vermindertem Druck abgedampft und der Rückstand (45 g), der im wesentlichen aus Triphenylphosphinoxid und (E)-5,5-Bis(3,4-dimethoxyphenyl)-2,4-pentadiencarbonsäureaethylester besteht, wird in einer Mischung aus Methanol (100 ml) und 2N Natriumhydroxidlösung (50 ml) aufgenommen. Man erhitzt während 30 Min. unter Rückfluss, dampft das Methanol im Vakuum ab, verdünnt das Konzentrat mit Wasser (100 ml) und filtriert das ausgefallene Triphenylphosphinoxid ab. Das basische Filtrat wird zunächst mit Aether extrahiert, um neutrale Verunreinigungen zu entfernen, und dann auf 1N Salzsäure (300 ml) gegossen und mit Methylenchlorid (3 x 150 ml) extrahiert. Die getrockneten ($MgSO_4$) Auszüge werden eingedampft, und der Rückstand wird mit Aether behandelt. Man erhält 18,7 g (E)-5,5-Bis(3,4-dimethoxyphenyl)-2,4-pentadiencarbonsäure mit einem Schmelzpunkt von 174-175,5 ° C. Durch Kristallisieren aus Methylenchlorid/2-Propanol erhält man eine analytisch reine Probe mit einem Schmelzpunkt von 174-175,5 ° C.

Beispiel 9

Wie in Beispiel 1 wird 3,3-Bis( 3-chlorphenyl)-2-propenal (14,6 g) mit (Carbethoxymethylen)-triphenylphosphoran (19,15 g) in Aethanol (50 ml) während 30 Min. bei Raumtemperatur umgesetzt. Das nach dem üblichen Aufarbeiten erhaltene Estergemisch wird mittels HPLC (Methylenchlorid/Hexan; 1:2) aufgetrennt. Man erhält 3,2 g (Z)-5,5-Bis(3-chlorphenyl)-2,4-pentadiencarbonsäureaethylester als Oel und 11,2 g (E)-5,5-Bis(3-chlorphenyl)-2,4-pentadiencarbonsäureaethylester. Eine Portion des (E)-Isomeren wird aus 2-Propanol kristallisiert und hat dann einen Schmelzpunkt von 68-6 9 ° C.

(E)-5,5-Bis(3-chlorphenyl)-2,4-pentadiencarbonsäureaethylester (10,5 g) in Methanol (30 ml) wird 2N Natriumhydroxidlösung (20 ml) behandelt, worauf man das Gemisch während 15 Min. unter Rückfluss erhitzt. Die rohe Säure, welche man nach dem üblichen Aufarbeiten erhält, wird aus Methylenchlorid/Hexan kristallisiert, wobei man 8,95 g (E)-5,5-Bis(3-chlorphenyl)-2,4-pentadiencarbonsäure mit einem Schmelzpunkt von 167-168 ° C erhält. Durch Umkristallisieren aus Aether/Hexan erhält man eine analytisch reine Probe mit einem Schmelzpunkt von 167-168 ° C.

Durch Hydrolyse von (Z)-5,5-Bis(3-chlorphenyl)-2,4-pentadiencarbonsäureaethylester (3,2 g) unter den gleichen Bedingungen erhält man 2,5 g rohes (Z)-5,5-Bis(3-chlorphenyl)-2,4-pentadiencarbonsäure mit einem Schmelzpunkt von 142-145 ° C. Eine Portion dieses Materials wird aus Aether/Hexan kristallisiert. Man erhält eine analytisch reine Probe mit einem Schmelzpunkt von 145-146 ° C.

Beispiel 10

Wie in Beispiel 1 wird 3,3-Bis(4-chlorphenyl)-2-propenal (11,08 g) mit (Carbethoxymethylen)-triphenylphosphoran (14,3 g) in Aethanol (30 ml) während 30 Min. bei Raumtemperatur umgesetzt. Das nach dem üblichen Aufarbeiten erhaltene Estergemisch wird mittels Kristallisation aus Aether/Hexan gereinigt. Man erhält 8,1 g (E)-5,5-Bis(4-chlorphenyl)-2,4-pentadiencarbonsäureaethylester mit einem Schmelzpunkt von 98-100 ° C. Ein Portion des (E)-Isomeren wird aus Hexan kristallisiert. Man erhält den reinen Ester mit einem Schmelzpunkt von 98,5-100 ° C.

Eine Mischung aus (E)-5,5-Bis(chlorphenyl)-2,4-pentadiencarbonsäureaethylester (7,5 g) in Methanol (30 ml) und 2N Natriumhydroxidlösung (15 ml) wird während 30 Min. unter Rückfluss erhitzt. Das nach dem üblichen Aufarbeiten erhaltene Rohprodukt wird aus Aether/Hexan kristallisiert. Man erhält 6,55 g (E)-5,5-Bis(4-chlorphenyl)-2,4-pentadiencarbonsäure mit einem Schmelzpunkt von 213-215 ° C. Durch Umkristallisieren einer Portion aus 2-Propanol erhält man eine analytisch reine Probe mit einem Schmelzpunkt von 214-215 ° C.

Die Mutterlaugen aus der Isolierung des (E)-Esters werden eingedampft und mittels HPLC (Methylenchlorid/Hexan; 1:2) gereinigt. Man erhält 0,9 g (Z)-5,5-Bis(4-chlorphenyl)-2,4-pentadiencarbonsäureaethylester als Oel. Durch Verseifung des (Z)-Esters (0,9 g) unter den üblichen Bedingungen erhält man

0,76 g der rohen (Z)-5,5-Bis(4-chlorphenyl)-2,4-pentadiencarbonsäure. Durch 2 Kristallisationen dieses Materials aus Methylenchlorid/Hexan erhält man die reine (Z)-Säure mit einem Schmelzpunkt von 215-217 ° C.

Beispiel 11

Eine Lösung von 3,3-Bis[2-(trifluormethyl)phenyl]-2-propenal (1,76 g) und (Carbethoxymethylen)-triphenylphosphoran (2,5 g) in Methylenchlorid (30 ml) wird während 1 Std. unter Rückfluss gerührt. Durch übliches Aufarbeiten erhält man 2,4 g Rohprodukt, das man mittels HPLC (Hexan/Methylenchlorid; 2:1) auftrennt. Man erhält 1,6 g (E)-5,5-Bis[2-(trifluormethyl)phenyl]-2,4-pentadiencarbonsäureaethylester, sowie eine geringe Menge (0,2 g) des (Z)-Isomeren.

Eine Lösung der Hauptkomponente (1,6 g) in Methanol (10 ml) wird mit 10N Natriumhydroxidlösung (0,5 ml) behandelt, und die Mischung wird während 20 Min. unter Rückfluss gerührt. Nach Abdampfen des Methanols im Vakuum wird der Rückstand zwischen 1N Salzsäure (20 ml) und Methylenchlorid (40 ml) verteilt. Die organische Phase wird mit Kochsalzlösung gewaschen, getrocknet ($MgSO_4$) und eingedampft. Man erhält 1,4 g der Säure, welche aus Methylenchlorid/Hexan kristallisiert wird. Man erhält 1,14 g (E)-5,5-Bis[2-(trifluormethyl)phenyl]-2,4-pentadiencarbonsäure mit einem Schmelzpunkt von 162-164 ° C. Aus dem gleichen Lösungsmittelsystem erhält man eine analytisch reine Probe mit einem Schmelzpunkt von 164-165 ° C.

Beispiel 12

Eine Lösung von 3,3-Bis[3-(trifluormethyl)phenyl]-2-propenal (18,5 g) und (Carbethoxymethylen)-triphenylphosphoran (19,15 g) in Methanol (100 ml) wird während einer 1 Std. bei Raumtemperatur gerührt. Durch übliches Aufarbeiten erhält man eine Mischung der Isomeren Ester, welche mittels HPLC (Hexan/Methylenchlorid; 2:1) aufgetrennt wird. Man erhält 2,8 g (Z)-5,5-Bis[3-(trifluormethyl)phenyl]-2,4-pentadiencarbonsäureaethylester und 16,8 g (E)-5,5-Bis[3-(trifluormethyl)phenyl]-2,4-pentadiencarbonsäureaethylester. Eine Portion des (E)-Isomeren wird aus Hexan kristallisiert, wobei man eine analytisch reine Probe mit einem Schmelzpunkt von 53-55 ° C erhält.

Eine Mischung des (E)-Isomeren (16,2 g) in Methanol (100 ml) und 1N Natriumhydroxidlösung (50 ml) wird während 30 Min. unter Rückfluss gerührt. Nach Abdampfen des Methanols im Vakuum wird das Konzentrat auf eine gerührte Mischung aus Eis und 6N Salzsäure (20 ml) gegossen. Der Festkörper wird abfiltriert, mit Wasser gewaschen, getrocknet und aus Methylenchlorid/Hexan kristallisiert. Man erhält 12,1 g (E)-5,5-Bis[3-(trifluormethyl)phenyl]-2,4-pentadiencarbonsäure mit einem Schmelzpunkt von 153-155 ° C. Aus dem gleichen Lösungsmittelgemisch erhält man eine analytisch reine Probe mit einem Schmelzpunkt von 154-155 ° C.

Das (Z)-Isomere (2,8 g) wird auf die gleiche Weise verseift. Die erhaltene rohe Säure wird mittels HPLC (Aether/methylenchlorid; 1:4) gereinigt, wobei man 1,3 g (Z)-5,5-Bis[3-(trifluormethyl)phenyl]-2,4-pentadiencarbonsäure erhält. Dieses Material wird zweimal aus Aether/Hexan kristallisiert. Man erhält die reine Säure mit einem Schmelzpunkt von 110-112 ° C.

Beispiel 13

Wie in Beispiel 1 wird eine Lösung von 3,3-Bis(3-nitrophenyl)-2-propenal (7,9 g) und (Carbethoxymethylen)triphenylphosphoran (9,75 g) in Aethanol während 1 Std. bei Raumtemperatur gerührt. Durch übliches Aufarbeiten erhält man ein Gemisch der Ester, das man aus Methylenchlorid/Hexan kristallisiert. Man erhält 6,7 g (E)-5,5-Bis(3-nitrophenyl)-2,4-pentadiencarbonsäureaethylester mit einem Schmelzpunkt von 121-123 ° C. Durch Umkristallisieren aus dem gleichen Lösungsmittel erhält man eine analytisch reine Probe mit einem Schmelzpunkt von 122-123 ° C.

Das aus den vereinigten Mutterlaugen erhaltene Material wird mittels HPLC (Methylenchlorid/Hexan; 2:1) aufgetrennt, wobei man eine zusätzliche Portion von 0,9 g des (E)-Esters und 1,6 g des Isomeren (Z)-Esters erhält. Der letztere wird aus Methylenchlorid/Hexan kristallisiert. Man erhält 1,35 g (Z)-5,5-Bis(3-nitrophenyl)-2,4-pentadiencarbonsäureaethylester mit einem Schmelzpunkt von 105-106 ° C.

Eine Mischung aus (E)-5,5-Bis(3-nitrophenyl)-2,4-pentadiencarbonsäureaethylester (6,1 g), Methanol (25 ml) und 2N Natriumhydroxidlösung (12,5 ml) wird während 15 Min. unter Rückfluss gerührt, dann mit Wasser (50 ml) verdünnt und im Vakuum konzentriert, um das Methanol zu entfernen. Die basische Lösung wird auf eine Mischung aus Eis und 2N Salzsäure (20 ml) gegossen, und der erhaltene Festkörper wird abfiltriert und getrocknet. Man erhält 5,5 g (E) -5,5-Bis(3-nitrophenyl)-2,4-pentadiencarbonsäure mit einem

Schmelzpunkt von 234-237 ° C. Durch Umkristallisieren einer Portion aus Chloroform/Aethanol erhält man die Säure als 0,25 molares Hydrat mit einem Schmelzpunkt von 237-238 ° C.

Beispiel 14

Eine Lösung einer Mischung aus (E)- und (Z)-3-(3-Methoxyphenyl)-3-phenyl-2-propenal und (Carbethoxymethylen)triphenylphosphoran (19,1 g) in Aethanol (150 ml) wird während 2 Std. bei 25 ° C gerührt. Durch übliches Aufarbeiten erhält man 16 g einer Mischung der 4 möglichen Isomeren Ester. 2 Isomeren, der (2Z,4Z)- und der (2Z,4E)-Ester, sind in geringfügiger Menge vorhanden, währenddem der (2E,4E)- und der (2E,4Z)-Ester die Hauptkomponenten in der Mischung sind. Durch Reinigung der Mischung mittels HPLC [(Methylenchlorid/Hexan; 4:1) und (Aether/Hexan; 1:12; 2 Recyclisierungen)] erhält man 2,1 g einer Mischung der in geringerer Menge vorhandenen Isomeren und 5,2 g (2E,4E)-5-(3-Methoxyphenyl)-5-phenyl-2,4-pentadiencarbonsäureaethylester als Oel und 5,1 g (2E,4Z)-5-(3-Methoxyphenyl)-5-phenyl-2,4-pentadiencarbonsäureaethylester.

Die Verseifung des (2E,4Z)-Esters (4,6 g) wird in Methanol (30 ml), das 2N Natriumhydroxid (10 ml) enthält, unter den üblichen Bedingungen durchgeführt. Durch übliches Aufarbeiten erhält man die rohe (2E,4Z)-5-(3-Methoxyphenyl)-5-phenyl-2,4-pentadiencarbonsäure, welche aus Methylenchlorid/Hexan kristallisiert wird. Man erhält 3,7 g der Säure mit einem Schmelzpunkt von 150-151 ° C.

Auf die gleiche Weise wird der (2E,4E)-Ester (5,2 g) in die (2E, 4E)-5-(3-Methoxyphenyl)-5-phenyl-2,4-pentadiencarbonsäure übergeführt. Durch Kristallisieren aus 2-Propanol/ Hexan erhält man die reine Säure mit einem Schmelzpunkt von 134-136 ° C.

Beispiel 15

Eine Lösung eines Gemisches der (E)- und (Z)-3-(3-Fluorphenyl)-3-(3-methoxyphenyl)-2-propenal (25 g) und (Carbomethoxymethylen)triphenylphosphoran (35 g) in Tetrachlorkohlenstoff wird während 16 Std. bei Raumtemperatur gerührt. Durch übliches Aufarbeiten erhält man 28,4 g einer Mischung der 4 möglichen Isomeren Ester. Durch eine vorläufige grobe Auftrennung mittels HPLC ( Aether/ Hexan; 1:9) erhält man 2,8 g einer Mischung bestehend aus den (2Z,4Z)- und (2Z,4E)-Estern, sowie 23,5 g einer Mischung bestehend aus den (2E,4E)- und (2E,4Z)-Estern.

Eine Portion (12 g) der Hauptkomponente wird mittels HPLC (Aether/Hexan; 1:8; 3 Recyclisierungen) weiter gereinigt. Man erhält 3,6 g (2E,4Z)-5-(3-Fluorphenyl)-5-(3-methoxyphenyl)-2,4-pentadiencarbonsäuremethylester, 5,5 g eines Gemische und 2,4 g (2E,4E)-5-(3-Fluorphenyl)-5-(3-methoxyphenyl)-2,4-pentadiencarbonsäuremethylester.

Der (2E,4Z)-Ester (3,6 g) wird in Methanol (25 ml) das 1N Natriumhydroxid (25 ml) enthält, unter den üblichen Bedingungen verseift. Durch Kristallisieren der rohen Säure aus Aether/Hexan erhält man 2,9 g (2E,4Z)-5-(3-Fluorphenyl)-5-(3-methoxyphenyl)-2,4-pentadiencarbonsäure mit einem Schmelzpunkt von 151-153 ° C. Durch Umkristallisieren aus Essigester/Hexan erhält man eine analytisch reine Probe mit einem Schmelzpunkt von 152,5-153,5 ° C.

Unter den gleichen Bedingungen wird der (2E,4E)-Ester (2,4 g) verseift, wobei man 2,0 g der rohen (2E, 4E)-Säure erhält. Durch Kristallisation der rohen Säure aus Aether/Hexan erhält man 1,75 g der reinen (2E, 4E)-5-(3-Fluorphenyl)-5-(3-methoxyphenyl)-2,4-pentadiencarbonsäure mit einem Schmelzpunkt von 156-157 ° C.

Beispiel 16

Wie in Beispiel 1 wird 3,3-Bis[(1,1′-biphenyl)-4-yl]-2-propenal (2 g) mit (Carbethoxymethylen)-triphenylphosphoran (2 g) in Methanol (30 ml) während 60 Min. bei Raumtemperatur umgesetzt. Das nach dem üblichen Aufarbeiten erhaltene Estergemisch wird mittels HPLC (Methylenchlorid/Hexan; 1:1) aufgetrennt. Man erhält 0,3 g (Z)-Ester und 1,8 g (E)-Ester. Der (E)-Ester wird aus 2-Propanol kristallisiert, und man erhält 1,5 g Material mit einem Schmelzpunkt von 143-145 ° C. Dieses Material wird in üblicher Weise hydrolysiert, und man erhält 1,3 g (E)-5,5-Bis[(1,1′-biphenyl)-4-yl]-2,4-pentadiencarbonsäure, welche aus 2-Propanol kristallisiert wird und 1,1 g (E)-Säure als 0,2 molares Hydrat mit einem Schmelzpunkt von 253-255 ° C liefert.

Beispiel 17

Wie in Beispiel 1 wird 3,3-Bis(4-methylphenyl)-2-propenal (9,45 g) mit (Cabomethoxymethylen)-triphenylphosphoran (15,3 g) in Tetrachlorkohlenstoff (95 ml) während 3 Tagen bei Raumtemperatur umgesetzt. Das Lösungsmittel wird unter vermindertem Druck abgedampft, und der Rückstand wird zusammen mit einer Mischung aus Methanol (40 ml) und 2N NaOH während 45 Min. unter Rückfluss gerührt. Die abgekühlte Lösung wird mit Wasser (300 ml) verdünnt, mit Methylenchlorid (3 x 100 ml) extrahiert, um nicht-saures Material zu entfernen, mit 6N HCl (50 ml) angesäuert und mit Methylenchlorid extrahiert. Die getrockneten ($MgSO_4$) Auszüge werden eingedampft, und das Rohprodukt wird aus 2-Propanol kristallisiert. Man erhält 7,5 g (E)-5,5-Bis(4-methylphenyl)-2,4-pentadiencarbonsäure mit einem Schmelzpunkt von 224,5-226,5 ° C.

Beispiel 18

a) Wie in Beispiel 1 wird 3,3-Bis(2-methoxyphenyl)-2-propenal (7,1 g) mit (Carbomethoxymethylen)-triphenylphosphoran (9,7 g) in Tetrachlorkohlenstoff (60 ml) und Methylenchlorid (20 ml) während 3 Tagen bei Raumtemperatur und dann über Nacht unter Rückfluss umgesetzt. Der rohe Ester wird in üblicher Weise isoliert und aus 2-Propanol/Hexan kristallisiert. Man erhält 6,5 g (E)-5,5-Bis(2-methoxyphenyl)-2,4-pentadiencarbonsäuremethylester mit einem Schmelzpunkt von 100-102 ° C. Durch Umkristallisieren einer Portion aus dem gleichen Lösungsmittelgemisch erhält man eine analytisch reine Probe mit einem Schmelzpunkt von 101-102 ° C.

b) Wie in Beispiel 1 wird 5,5-Bis(2-methoxyphenyl)-2,4-pentadiencarbonsäuremethylester (6,3 g) in einem Gemisch aus Methanol (50 ml) und 1N NaOH (50 ml) unter Rückfluss verseift. Nach 45 Min. wird die rohe Säure in üblicher Weise isoliert und aus 2-Propanol kristallisiert. Man erhält 5,1 g (E)-5,5-Bis(2-methoxyphenyl)-2,4-pentadiencarbonsäure mit einem Schmelzpunkt von 196-197 ° C.

Beispiel 19

a) Wie in Beispiel 1 wird (E)-3-(2-Methoxyphenyl)-3-(4-methoxyphenyl)-2-propenal (5 g) mit (Carbomethoxymethylen)triphenylphosphoran (6,83 g) in einer Mischung aus Tetrachlorkohlenstoff (50 ml) und Methylenchlorid (25 ml) während 4 Tagen bei Raumtemperatur umgesetzt. Der rohe Ester wird in üblicher Weise isoliert und aus 2-Propanol/Hexan kristallisiert. Man erhält 4,6 g (E,E)-5-(2-Methoxyphenyl)-5-(4-methoxyphenyl)-2,4-pentadiencarbonsäuremethylester. Durch Umkristallisieren einer Portion aus dem gleichen Lösungsmittelgemisch erhält man eine analytisch reine Probe mit einem Schmelzpunkt von 99,5-102,5 ° C.

b) Wie in Beispiel 1 wird (E,E)-5-(2-Methoxyphenyl)-5-(4-methoxyphenyl)-2,4-pentadiencarbonsäuremethylester (4,3 g) in einem Gemisch aus Methanol (35 ml) und 1N NaOH (35 ml) unter Rückfluss verseift. Nach 1 Std. wird die Reaktion in üblicher Weise aufgearbeitet und die rohe Säure wird aus 2-Propanol kristallisiert. Man erhält 3,6 g (E,E)-5-(2-Methoxyphenyl)-5-(4-methoxyphenyl)-2,4-pentadiencarbonsäure mit einem Schmelzpunkt von 210,5-211,5 ° C.

Beispiel 20

a) Wie in Beispiel 1 wird (Z)-3-(2-Methoxyphenyl)-3-(4-methoxyphenyl)-2-propenal (2 g) mit (Carbomethoxymethylen)triphenylphosphoran (2,73 g) in Methylenchlorid (10 ml) während 3 Tagen bei Raumtemperatur umgesetzt. Der rohe Ester wird in üblicher Weise isoliert und mittels HPLC (Aether/Hexan; 1:3) gereinigt. Man erhält 1,8 g (2E,4Z)-5-(2-Methoxyphenyl)-5-(4-methoxyphenyl)-2,4-pentadiencarbonsäuremethylester als Oel.

b) Wie in Beispiel 1 wird (2E,4Z)-5-(2-Methoxyphenyl-5-(4-methoxyphenyl)-2,4-pentadiencarbonsäuremethylester (1,8 g) in einem Gemisch aus Methanol (15 ml) und 1N NaOH (15 ml) unter Rückfluss verseift. Nach 1 Std. wird die Reaktion in üblicher Weise aufgearbeitet und die rohe Säure wird aus 2-Propanol kristallisiert. Man erhält 1,1 g (2E,4Z)-5-(2-Methoxyphenyl)-5-(4-methoxyphenyl)-2,4-pentadiencarbonsäure. Durch Umkristallisieren einer Portion aus 2-Propanol/Hexan erhält man eine analytisch reine Probe mit einem Schmelzpunkt von 159-161 ° C.

Beispiel 21

a) Wie in Beispiel 1 wird (E)-3-(4-Methoxyphenyl)-3-(3-pyridinyl)-2-propenal (5,25 g) mit (Carbomethoxymethylen)triphenylphosphoran (7,4 g) in Methylenchlorid (40 ml) während 17 Std. bei Raumtemperatur umgesetzt. Der rohe Ester wird in üblicher Weise isoliert. Man erhält 4,6 g des Esters. Eine Portion davon wird zweimal aus Aether/Hexan kristallisiert. Man erhält eine analytisch reine Probe von (E,E)-5-(4-Methoxyphenyl)-5-(3-pyridinyl)-2,4-pentadiencarbonsäuremethylester mit einem Schmelzpunkt von 75-76° C.

b) Wie in Beispiel 1 wird (E,E)-5-(4-Methoxyphenyl)-5-(3-pyridinyl)-2,4-pentadiencarbonsäuremethylester (0,295 g) in einem Gemisch aus Methanol (1,5 ml) und 1N NaOH (1,5 ml) unter Rückfluss verseift. Nach Neutralisieren der abgekühlten Lösung mit IN HCl (1,5 ml) wir die rohe Säure abfiltriert und aus Methanol kristallisiert. Man erhält 0,235 g (E,E)-5-(4-Methoxyphenyl)-5-(3-pyridinyl)-2,4-pentadiencarbonsäure als 0,33 molares Hydrat mit einem Schmelzpunkt von 173-175° C.

Beispiel 22

a) Wie in Beispiel 1 wird (Z)-3-(4-Methoxyphenyl)-3-(3-pyridinyl)-2-propenal (9,9 g) mit (Carbomethoxymethylen)triphenylphosphoran (14,4 ml) in Methylenchlorid (75 ml) während 3 Tagen bei Raumtemperatur umgesetzt. Der rohe Ester wird in üblicher Weise isoliert und mittels HPLC (Aether/Hexan; 13:7) gereinigt und aus Aether/Hexan kristallisiert. Man erhält 7,275 g (2E,4Z)-5-(4-Methoxyphenyl)-5-(3-pyridinyl)-2,4-pentadiencarbonsäuremethylester mit einem Schmelzpunkt von 99-100,5° C.

b) Wie in Beispiel 1 wird (2E,4Z)-5-(4-Methoxyphenyl)-5-(3-pyridinyl)-2,4-pentadiencarbonsäuremethylester (6,2 g) in einem Gemisch aus Methanol (30 ml) und 1N NaOH (30 ml) unter Rückfluss verseift. Nach 1 Std. wird die Reaktionsmischung abgekühlt und durch Zugabe von 1N HCl (30 ml) neutralisiert. Der erhaltene kristalline Festkörper wird abfiltriert und mit Wasser gut gewaschen. Man erhält 5,8 g (2E,4Z)-5-(4-Methoxyphenyl)-5-(3-pyridinyl)-2,4-pentadiencarbonsäure mit einem Schmelzpunkt von 235-238° C. Eine Probe wird aus Methanol umkristallisiert und hat dann einen Schmelzpunkt von 238-240° C.

Beispiel 23

a) Wie in Beispiel 1 wird (Z)-3-(4-Methoxyphenyl)-3-phenyl-2-propenal (7,3 g) mit (Carbomethoxymethylen)triphenylphosphoran (11,9 g) in Tetrachlorkohlenstoff (50 ml) und Methylenchlorid (10 ml) über Nacht bei Raumtemperatur umgesetzt. Der rohe Ester wird in üblicher Weise isoliert und mittels HPCL (Aether/Hexan; 1:7,5) gereinigt. Man erhält 6,4 g (2E,4Z)-5-(4-Methoxyphenyl)-5-phenyl-2,4-pentadiencarbonsäuremethylester als Oel.

b) Wie in Beispiel 1 wird (2E, 4Z)-5-(4-Methoxyphenyl)-5-phenyl-2,4-pentadiencarbonsäuremethylester (6,4 g) in einem Gemisch aus Methanol (30 ml) und 2N NaOH (23 ml) unter Rückfluss verseift. Nach 2,5 Std. wird die Reaktion in üblicher Weise aufgearbeitet, und die rohe Säure wird aus 2-Propanol kristallisiert. Man erhält 4,25 g (2E,4Z)-5-(4-Methoxyphenyl)-5-phenyl-2,4-pentadiencarbonsäure. Eine Probe davon wird aus 2-Propanol umkristallisiert und hat dann einen Schmelzpunkt von 184-185° C.

Beispiel 24

a) Wie in Beispiel 1 wird eine Mischung (7:3) aus (E)- und (Z)-3-Cyclopropyl-3-(4-methoxyphenyl)-2-propenal (10,3 g) mit (Carbomethoxymethylen)triphenylphosphoran (19 g) in Tetrachlorkohlenstoff (70 ml) und Methylenchlorid (15 ml) während 4 Tagen bei Raumtemperatur umgesetzt. Die rohe Mischung der (E,E)- und (2E,4Z)-Ester wird in übli cher Weise isoliert und mittels HPLC (Aether/Hexan; 1:9; 2 Recyclisierungen) gereinigt. Man erhält 6,8 g (E,E)-5-Cyclopropyl-5-(4-methoxyphenyl)-2,4-pentadiencarbonsäuremethylester und 3,1 g (2E,4Z)-5-Cyclopropyl-5-(4-methoxyphenyl)-2,4-pentadiencarbonsäuremethylester als Oele.

b) Wie in Beispiel 1 wird (E,E)-5-Cyclopropyl-5-(4-methoxyphenyl)-2,4-pentadiencarbonsäuremethylester (6,8 g) in einem Gemisch aus Methanol (30 ml) und 2N NaOH (30 ml) unter Rückfluss verseift. Nach 2,5 Std. wird die abgekühlte Reaktion in üblicher Weise aufgearbeitet, und die rohe Säure wird aus 2-Propanol/ Hexan kristallisiert. Man erhält 5,6 g (E,E)-5-Cyclopropyl-5-(4-methoxyphenyl)-2,4-pentadiencarbonsäure mit einem Schmelzpunkt von 158-160° C.

c) Wie in Beispiel 1 wird (2E,4Z)-5-Cyclopropyl-5-(4-methoxyphenyl)-2,4-pentadiencarbonsäuremethylester (3,1 g) in einem Gemisch aus Methanol (15 ml) und 2N NaOH (15 ml) unter Rückfluss verseift.

Nach 3 Std. wird die Reaktion in üblicher Weise aufgearbeitet, und die rohe Säure wird aus 2-Propanol/Hexan kristallisiert. Man erhält 2 g (2E,4Z)-5-Cyclopropyl-5-(4-methoxyphenyl)-2,4-pentadiencarbonsäuremit einem Schmelzpunkt von 154-155,5° C.

Beispiel 25

a) Wie in Beispiel 1 wird (E)-3-Cyclohexyl-3-(4-methoxyphenyl)-2-propenal (5,6 g) mit (Carbomethoxymethylen)triphenylphosphoran (8,5 g) in Tetrachlorkohlenstoff (35 ml) und Methylenchlorid (8 ml) über Nacht bei Raumtemperatur umgesetzt. Der Ester wird in üblicher Weise isoliert und gereinigt. Man erhält 6,1 g (E,E)-5-Cyclohexyl-5-(4-methoxyphenyl)-2,4-pentadiencarbonsäuremethylester als Oel.

b) Wie in Beispiel 1 wird (E,E)-5-Cyclohexyl-5-(4-methoxyphenyl)-2,4-pentadiencarbonsäuremethylester (5,9 g) mit einem Gemisch aus Methanol (20 ml) und 2N NaOH (20 ml) unter Rückfluss verseift. Nach 2 Std. wird die Reaktion in üblicher Weise aufgearbeitet und die rohe Säure wird aus Cyclohexan/Hexan kristallisiert. Man erhält 3,37 g (E,E)-5-Cyclohexyl-5-(4-methoxyphenyl)-2,4-pentadiencarbonsäure mit einem Schmelzpunkt von 130,5-131,5° C.

Beispiel 26

a) Wie in Beispiel 1 wird (Z)-3-Cyclohexyl-3-(4-methoxyphenyl)-2-propenal (7,3 g) mit (Carbomethoxymethylen)triphenylphosphoran (11 g) in Tetrachlorkohlenstoff (40 ml) und Methylenchlorid (4 ml) über Nacht bei Raumtemperatur umgesetzt. Der rohe Ester wird in üblicher Weise isoliert und mittels HPLC (Aether/Hexan; 1:7) gereinigt. Man erhält 5,7 g (2E,4Z)-5-Cyclohexyl-5-(4-methoxyphenyl)-2,4-pentadiencarbonsäuremethylester. Durch Kristallisteren einer Portion aus Hexan erhält man eine analytisch reine Probe mit einem Schmelzpunkt von 70,5-72° C.

b) Wie in Beispiel 1 wird (2E,4Z)-5-Cyclohexyl-5-(4-methoxyphenyl)-2,4-pentadiencarbonsäuremethylester (5,2 g) in Methanol (20 ml) und 2N NaOH (20 ml) unter Rückfluss verseift. Nach 2,5 Std. wird das Rohprodukt in üblicher Weise isoliert, und aus 2-Propanol/ Hexan kristallisiert. Man erhält 3,5 g (2E,4Z)-5-Cyclohexyl-5-(4-methoxyphenyl)-2,4-pentadiencarbonsäure mit einem Schmelzpunkt von 185-186° C.

Beispiel 27

Wie in Beispiel 1 wird (E)-3-(4-Methoxyphenyl)-2-propenal (9,5 g) mit (Carbomethoxymethylen)-triphenylphosphoran (21,7 g) in Tetrachlorkohlenstoff (80 ml) und Methylenchlorid (15 ml) während 3 Tagen bei Raumtemperatur umgesetzt. Die Reaktion wird in üblicher Weise aufgearbeitet, und der rohe Ester wird in einem Gemisch aus Methanol (60 ml) und 1N NaOH (120 ml) während 15 Min. verseift. Die abgekühlte Lösung wird mit Methanol (300 ml) verdünnt, während mehrerer Minuten gerührt, und dann wird der Festkörper abfiltriert. Man erhält 9,15 g (E,E)-5-(4-Methoxyphenyl)-2,4-pentadiencarbonsäure als Natriumsalz. Eine Lösung dieses Salzes in einem Gemisch aus Wasser (130 ml) und Methanol (50 ml) wird unter Rückfluss mit 6N HCl (9 ml) sauer gestellt. Der Festkörper wird abfiltriert. Man erhält 7,0 g (E,E)-5-(4-Methoxyphenyl)-2,4-pentadiencarbonsäure. Durch Kristallisieren einer Probe aus 2-Propanol erhält man eine analytisch reine Probe mit einem Schmelzpunkt von 133,5-135° C.

Beispiel 28

a) Wie in Beispiel 1 wird (E)-3-(4-Methoxyphenyl)-2-butenal (8 g) mit (Carbomethoxymethylen)-triphenylphosphoran (17 g) in Tetrachlorkohlenstoff (70 ml) und Methylenchlorid (15 ml) über Nacht bei Raumtemperatur umgesetzt. Der Ester wird in üblicher Weise isoliert und aus Hexan kristallisiert. Man erhält 7,2 g (E,E)-5-(4-Methoxyphenyl)-2,4-hexadiencarbonsäuremethylester mit einem Schmelzpunkt von 84-86° C.

b) Wie in Beispiel 1 wird (E,E)-5-(4-Methoxyphenyl)-2,4-hexadiencarbonsäuremethylester (6,8 g) in einem Gemisch aus Methanol (30 ml) und 2N NaOH (30 ml) unter Rückfluss verseift. Nach 2,5 Std. wird die Reaktion in üblicher Weise aufgearbeitet. Man erhält 6,1 g (E,E)-5-(4-Methoxyphenyl)-2,4-hexadiencarbonsäure. Durch Kristallisieren einer Portion aus 2-Propanol erhält man eine analytisch reine Probe mit einem Schmelzpunkt von 174-177° C.

Beispiel 29

a) Wie in Beispiel 1 wird (E)-3-(4-Methoxyphenyl)-2-pentenal (7,1 g) mit (Carbomethoxymethylen)-triphenylphosphoran (14 g) in Tetrachlorkohlenstoff (70 ml) und Methylenchlorid (50 ml) über Nacht bei Raumtemperatur umgesetzt. Der Ester wird in üblicher Weise isoliert. Man erhält 8 g (E,E)-5-(4-Methoxyphenyl)-2,4-heptadiencarbonsäuremethylester als Oel.

b) Wie in Beispiel 1 wird (E,E)-5-(4-Methoxyphenyl)-2,4-heptadiencarbonsäuremethylester (8 g) in einem Gemisch aus Methanol (30 ml) und 2N NaOH (30 ml) unter Rückfluss verseift. Nach 2,5 Std. wird die Reaktion in üblicher Weise aufgearbeitet. Die erhaltene Säure wird aus 2-Propanol/Hexan kristallisiert. Man erhält 5,4 g (E,E)-5-(4-Methoxyphenyl)-2,4-heptadiencarbonsäure mit einem Schmelzpunkt von 135,5-137 ° C.

Beispiel 30

a) Wie in Beispiel 1 wird ein Gemisch (1:1) aus (E)- und (Z)-(4-Methoxyphenyl)-4-methyl-2-pentenal (9,85 g) mit (Carbomethoxymethylen)triphenylphosphoran (18,1 g) in Tetrachlorkohlenstoff (75 ml) und Methylenchlorid (15 ml) während 2 Tagen bei Raumtemperatur umgesetzt. Die Mischung der Ester wird in üblicher Weise isoliert und mittels HPLC (Aether/Hexan; 1:9) aufgetrennt. Man erhält 4,2 g (E,E)-5-(4-Methoxyphenyl)-6-methyl-2,4-heptadiencarbonsäuremethylester und 4,4 g (2E,4Z)-5-(4-Methoxyphenyl)-6-methyl-2,4-heptadiencarbonsäuremethylester als Oele. Eine Probe des (E)-Isomeren wird aus Hexan kristallisiert und hat dann einen Schmelzpunkt von 65-66 ° C.

b) Wie in Beispiel 1 wird (E,E)-5-(4-Methoxyphenyl)-6-methyl-2,4-heptadiencarbonsäuremethylester (4 g) in einem Gemisch aus Methanol (15 ml) und 2N NaOH (15 ml) unter Rückfluss verseift. Nach 2 Std. wird die Reaktion in üblicher Weise aufgearbeitet, und die rohe Säure wird aus Cyclohexan/Hexan kristallisiert. Man erhält 2,8 g (E,E)-5-(4-Methoxyphenyl)-6-methyl-2,4-heptadiencarbonsäure mit einem Schmelzpunkt von 140,5-141,5 ° C.

c) Wie in Beispiel 1 wird (2E,4Z)-5-(4-Methoxyphenyl)-6-methyl-2,4-heptadiencarbonsäuremethylester (4,2 g) in einem-Gemisch aus Methanol (15 ml) und 2N NaOH (15 ml) unter Rückfluss verseift. Nach 2 Std. wird die Reaktion in üblicher Weise aufgearbeitet. Man erhält 3,8 g (2E,4Z)-5-(4-Methoxyphenyl)-6-methyl-2,4-heptadiencarbonsäure. Eine Portion wird aus Cyclohexan/Hexan kristallisiert und hat dann einen Schmelzpunkt von 131,5-133 g.

Beispiel 31

a) Wie in Beispiel 1 wird (E)-3-(4-Methoxyphenyl)-2-hexenal (6 g) mit (Carbomethoxymethylen)-triphenylphosphoran (11 g) in Tetrachlorkohlenstoff (70 ml) und Methylenchlorid (15 ml) während 4 Tagen bei Raumtemperatur umgesetzt. Der Ester wird in üblicher Weise isoliert. Man erhält 6,7 g (E E)-5-(4-Methoxyphenyl)-2,4-octadiencarbonsäuremethylester als Oel.

b) Wie in Beispiel 1 wird (E,E)-5-(4-Methoxyphenyl)-2,4-octadiencarbonsäuremethylester (6,7 g) in einem Gemisch aus Methanol (30 ml) und 2N NaOH (30 ml) unter Rückfluss verseift. Nach 3 Std. wird die Reaktion in üblicher Weise aufgearbeitet, und die rohe Säure wird aus 2-Propanol/Hexan kristallisiert. Man erhält 4,85 g (E,E)-5-(4-Methoxyphenyl)-2,4-octadiencarbonsäure mit einem Schmelzpunkt von 151-152,5 ° C.

Beispiel 32

a) Wie in Beispiel 1 wird (E)-3-(4-Methoxyphenyl)-2-heptenal (10 g) mit (Carbomethoxymethylen)-triphenylphosphoran (17 g) in Tetrachlorkohlenstoff (70 ml) und Methylenchlorid (15 ml) während 4 Tagen bei Raumtemperatur umgesetzt. Der Ester wird in üblicher Weise isoliert und mittels HPLC (Ether/Hexan; 1:9) gereinigt. Man erhält 8 g (E,E)-5-(4-Methoxyphenyl)-2,4-nonadiencarbonsäuremethylester als Oel.

b) Wie in Beispiel 1 wird (E,E)-5-(4-Methoxyphenyl)-2,4-nonadiencarbonsäuremethylester (8 g) in einem Gemisch aus Methanol (30 ml) und 2N NaOH (30 ml) unter Rückfluss verseift. Nach 2,5 Stunden wird die Reaktion in üblicher Weise aufgearbeitet, und das Rohprodukt wird aus 2-Propanol/Hexan kristallisiert. Man erhält 6,2 g (E,E)-5-(4-Methoxyphenyl)-2,4-nonadiencarbonsäure mit einem Schmelzpunkt von 138-139,5 ° C.

Beispiel 33

a) Wie in Beispiel 1 wird ein Gemisch (3:4) aus (E)- und (Z)-3-(3-Methoxyphenyl)-2-heptenal (24,3 g) mit (Carbomethoxymethylen)triphenylphosphoran (27 g) in Methylenchlorid (150 ml) während 2 Tagen bei Raumtemperatur umgesetzt. Das Estergemisch wird in üblicher Weise isoliert und mittels HPLC (Ether/Hexan; 1:7,5) aufgetrennt. Man erhält 7,77 g (E,E)-5-(3-Methoxyphenyl)-2,4-nonadiencarbonsäuremethylester und 11,67 g (2E,4Z)-5-(3-Methoxyphenyl)-2,4-nonadiencarbonsäuremethylester als Oele.
b) Wie in Beispiel 1 wird (E,E)-5-(3-Methoxyphenyl)-2,4-nonadiencarbonsäuremethylester (6,41 g) in einem Gemisch aus Methanol (30 ml) und 2N NaOH (30 ml) unter Rückfluss verseift. Nach 3 Stunden wird die Reaktion in üblicher Weise aufgearbeitet, und die rohe Säure wird aus Hexan kristallisiert. Man erhält 4,86 g (E,E)-5-(3-Methoxyphenyl)-2,4-nonadiencarbonsäure mit einem Schmelzpunkt von 77,5-78,5° C.
c) Wie in Beispiel 1 wird (2E,4Z)-5-(3-Methoxyphenyl)-2,4-nonadiencarbonsäuremethylester (11 g) in einem Gemisch aus Methanol (30 ml) und 2N NaOH (30 ml) unter Rückfluss verseift. Nach 2 Stunden wird die Reaktion in üblicher Weise aufgearbeitet. Man erhält 7,87 g (2E,4Z)-5-(3-Methoxyphenyl)-2,4-nonadiencarbonsäure. Eine Portion wird aus Ether/Hexan kristallisiert und hat dann einen Schmelzpunkt von 106,5-107,5° C.

Beispiel 34

a) Wie in Beispiel 1 wird (E)-3-(4-Methoxyphenyl)-2-octenal (6 g) mit (Carbomethoxymethylen)-triphenylphosphoran (9,5 g) in Tetrachlorkohlenstoff (60 ml) während 4 Tagen bei Raumtemperatur umgesetzt. Der Ester wird in üblicher Weise isoliert und mittels HPLC (Ether/Hexan; 1:7,5) gereinigt. Man erhält 6,1 g (E,E)-5-(4-Methoxyphenyl)-2,4-decadiencarbonsäuremethylester als Oel.
b) Wie in Beispiel 1 wird (E,E)-5-(4-Methoxyphenyl)-2,4-decadiencarbonsäuremethylester (6,1 g) in einem Gemisch aus Methanol (25 ml) und 2N NaOH (25 ml) unter Rückfluss verseift. Nach 45 Minuten wird die Reaktion in üblicher Weise aufgearbeitet, und die rohe Säure wird aus 2-Propanol kirstallisiert. Man erhält 4,7 g (E,E)-5-(4-Methoxyphenyl)-2,4-decadiencarbonsäure mit einem Schmelzpunkt von 126-127,5° C.

Beispiel 35

a) Wie in Beispiel 1 wird (Z)-3-(4-Methoxyphenyl)-2-octenal (5,5 g) mit (Carbomethoxymethylen)-triphenylphosphoran (8,7 g) in Tetrachlorkohlenstoff (55 ml) während 4 Tagen bei Raumtemperatur umgesetzt. Das Rohprodukt wird in üblicher Weise isoliert und mittels HPLC (Ether/Hexan; 3:17) gereinigt. Man erhält 3,6 g (2E,4Z)-5-(4-Methoxyphenyl)-2,4-decadiencarbonsäuremethylester als Oel.
b) Wie in Beispiel 1 wird (2E,4Z)-5-(4-Methoxyphenyl)-2,4-decadiencarbonsäuremethylester (3,6 g) in einem Gemisch aus Methanol (40 ml) und 1N NaOH (40 ml) unter Rückfluss verseift. Nach 45 Minuten wird die Säure in üblicher Weise isoliert und aus Ether/Hexan kristallisiert. Man erhält 3,0 g (2E,4Z)-5-(4-Methoxyphenyl)-2,4-decadiencarbonsäure mit einem Schmelzpunkt von 86,5-87,5° C.

Beispiel 36

a) Wie in Beispiel 1 wird (E)-3-(3-Fluorphenyl)-2-octenal (5,43 g) mit (Carbomethoxymethylen)-triphenylphosphoran (9 g) in Methylenchlorid (50 ml) während 4 Tagen bei Raumtemperatur umgesetzt. Der Ester wird in üblicher Weise isoliert und mittels HPLC (Ether/Hexan; 1:9) gereinigt. Man erhält 6,04 g (E,E)-5-(3-Fluorphenyl)-2,4-decadiencarbonsäuremethylester als Oel.
b) Wie in Beispiel 1 wird (E,E)-5-(3-Fluorphenyl)-2,4-decadiencarbonsäuremethylester (5,54 g) in einem Gemisch aus Methanol (30 ml) und 2N NaOH (30 ml) unter Rückfluss verseift. Nach 45 Minuten wird die Reaktion in üblicher Weise aufgearbeitet, und die rohe Säure wird aus Hexan kristallisiert. Man erhält 3,85 g (E,E)-5-(3-Fluorphenyl)-2,4-decadiencarbonsäure mit einem Schmelzpunkt von 84-85,5° C.

Beispiel 37

a) Wie in Beispiel 1 wird (Z)-3-(3-Fluorphenyl)-2-octenal (6,2 g) mit (Carbomethoxymethylen)-triphenylphosphoran (10,5 g) in Methylenchlorid (50 ml) während 4 Tagen bei Raumtemperatur umgesetzt. Der Ester wird in üblicher Weise isoliert, und man erhält 6,6 g (2E,4Z)-5-(3-Fluorphenyl)-2,4-decadiencarbonsäuremethylester als Oel.

b) Wie in Beispiel 1 wird (2E,4Z)-5-(3-Fluorphenyl)-2,4-decadiencarbonsäuremethylester (6,1 g) in einem Gemisch aus Methanol (40 ml) und 2N NaOH (30 ml) unter Rückfluss verseift. Nach 1 Stunde wird die rohe Säure in üblicher Weise isoliert und aus Ether/Hexan kristallisiert. Man erhält 4,1 g (2E,4Z)-5-(3-Fluorphenyl)-2,4-decadiencarbonsäure mit einem Schmelzpunkt von 100,5-101 ° C.

Beispiel 38

a) Wie in Beispiel 1 wird (E)-3-(4-Fluorphenyl)-2-octenal (7,4 g) mit (Carbomethoxymethylen)-triphenylphosphoran (13,5 g) in Methylenchlorid (60 ml) während 4 Tagen bei Raumtemperatur umgesetzt. Der Ester wird in üblicher Weise isoliert und mittels HPLC (Ether/Hexan; 1:9) gereinigt. Man erhält 7,04 g (E,E)-5-(4-Fluorphenyl)-2,4-decadiencarbonsäuremethylester als Oel.
b) Wie in Beispiel 1 wird (E,E)-5-(4-Fluorphenyl)-2,4-decadiencarbonsäuremethylester (6,5 g) in einem Gemisch aus Methanol (30 ml) und 2N NaOH (30 ml) unter Rückfluss verseift. Nach 2 Stunden wird die rohe Säure in üblicher Weise isoliert und aus Hexan kristallisiert. Man erhält 4,99 g (E,E)-5-(4-Fluorphenyl)-2,4-decadiencarbonsäure mit einem Schmelzpunkt von 116,5-117,5 ° C.

Beispie 39

a) Wie in Beispiel 1 wird eine Mischung (4:3) von (E)- und (Z)-3-(3,4-Dimethoxyphenyl)-2-octenal (25,5 g) mit (Carbomethoxymethylen)triphenylphosphoran (37 g) in Methylenchlorid (150 ml) während 2 Tagen bei Raumtemperatur umgesetzt. Man arbeitet in üblicher Weise auf und erhält 29,7 g Rohprodukt. Eine Portion davon (14,5 g) wird mittels HPLC (Ether/Hexan; 3:17) aufgetrennt. Man erhält 5,26 g (E,E)-5-(3,4-Dimethoxyphenyl)-2,4-decadiencarbonsäuremethylester und 6,2 g (2E,4Z)-5-(3,4-Dimethoxyphenyl)-2,4-decadiencarbonsäuremethylester als Oele.
b) Wie in Beispiel 1 wird (E,E)-5-(3,4-Dimethoxyphenyl)-2,4-decadiencarbonsäuremethylester (4,9 g) in einem Gemisch aus Methanol (30 ml) und 2N NaOH (30 ml) unter Rückfluss verseift. Nach 3 Stunden wird die Reaktion in üblicher Weise aufgearbeitet, und die rohe Säure wird aus Ether/Hexan kristallisiert. Man erhält 3,27 g (E,E)-5-(3,4-Dimethoxyphenyl)-2,4-decadiencarbonsäure mit einem Schmelzpunkt von 98-99,5 ° C.

Beispiel 40

a) Wie in Beispiel 1 wird eine Mischung (11:9) aus (E)- und (Z)-3-(4-Methoxyphenyl)-2-noneal (22,3 g) mit (Carbomethoxymethylen)triphenylphosphoran (37 g) in Tetrachlorkohlenstoff (150 ml) und Methylenchlorid (10 ml) während 4 Tagen bei Raumtemperatur umgesetzt. Das Rohprodukt wird in üblicher Weise isoliert und mittels HPLC (Ether/Hexan; 1:7,5; 4 Recyclisierungen) aufgetrennt. Man erhält 11 g (E,E)-5-(4-Methoxyphenyl)-2,4-undecadiencarbonsäuremethylester und 8,6 g (2E,4Z)-5-(4-Methoxyphenyl)-2,4-undecadiencarbonsäuremethylester als Oele.
b) Wie in Beispiel 1 wird (E,E)-5-(4-Methoxyphenyl)-2,4-undecadiencarbonsäuremethylester (5,9 g) in einem Gemsich aus Methanol (30 ml) und 2N NaOH (30 ml) unter Rückfluss verseift. Nach 3 Stunden wird die Reaktion in üblicher Weise aufgearbeitet, und die rohe Säure wird aus Hexan kristallisiert. Man erhält 3,27 g (E,E)-5-(4-Methoxyphenyl)-2,4-undecadiencarbonsäure mit einem Schmelzpunkt von 88-89,5 ° C.

Beispiel 41

Wie in Beispiel 1 wird 3-Pentyl-2-octenal (7,85 g) mit (Carbomethoxymethylen)triphenylphosphoran (15,3 g) in Tetrachlorkohlenstoff (100 ml) und Methlyenchlorid (50 ml) während 3 Tagen bei Raumtemperatur umgesetzt. Durch übliches Aufarbeiten erhält man 10,1 g 5-Pentyl-2,4-decadiencarbonsäuremethylester, welcher dann in einem Gemisch aus Methanol (40 ml) und 2N NaOH (30 ml) unter Rückfluss verseift wird. Nach 1 Stunde wird die Säure in üblicher Weise isoliert. Man erhält 8,15 g 5-Pentyl-2,4-decadiencarbonsäure als Oel.

Beispiel 42

a) Wie in Beispiel 1 wird (E)-3-(4-Methoxyphenyl)-3-phenyl-2-propenal (8,7 g) mit (Carbomethoxymethylen)triphenylphosphoran (14,2 g) in Tetrachlorkohlenstoff (50 ml) und Methylenchlorid (5 ml) über Nacht bei Raumtemperatur umgesetzt. Der rohe Ester wird in üblicher Weise isoliert und

mittels HPLC (Ether/Hexan; 3:17) gereinigt. Man erhält 8,6 g (E,E)-5-(4-Methoxyphenyl)-5-Phenyl-2,4-pentadiencarbonsäuremethylester. Durch Kristallisieren einer Portion aus Essigester/Hexan erhält man eine analytisch reine Probe mit einem Schmelzpunkt von 88-89,5 ° C.

b) Wie in Beispiel 1 wird (E,E)-5-(4-Methoxyphenyl)-5-phenyl-2,4-pentadiencarbonsäuremethylester (8,35 g) in einem Gemisch aus Methanol (35 ml) und 2N NaOH (30 ml) unter Rückfluss verseift. Nach 2,5 Stunden wird die rohe Säure in üblicher Weise isoliert und aus 2 Propanol kristallisiert. Man erhält 5,2 g (E,E)-5-(4-Methoxyphenyl)-5-phenyl-2,4-pentadiencarbonsäure mit einem Schmelzpunkt von 209-211 ° C.

## Teil VI: Herstellurg von Verbindungen der Formel IV.

### Beispiel 1

Eine Lösung von (E) -5,5-Diphenyl-2-methyl-2,4-pentadiencarbonsäure (1,85 g) und 4-Nitrophenol (1,12 g) in Methylenchlorid (25 ml) wird bei 0-5 ° C in einem Eiswasserbad gerührt und dann mit 1,3-Dicyclohexylcarbodiimid (DCC; 1,44 g) versetzt. Das Gemisch wird während 30 Minuten bei 0-5 ° C und dann während 1 Stunde bei Raumtemperatur gerührt. Nach dem Abfiltrieren des aufgefallenen Dicyclohexylharnstoffes wird das konzentrierte Filtrat durch eine kurze Kieselgelsäule (ungefähr 25 g), welche in Methylenchlorid/Hexan (3:2) hergestellt worden ist, filtriert. Die geeigneten Fraktionen werden vereinigt und eingedampft. Man erhält 2,4 g Ester. Durch kristallisieren dieses Materials aus 2-Propanol erhält man 1,64 g (E)-5,5-Diphenyl-2-methyl-2,4-pentadiencarbonsäure-4-nitrophenylester mit einem Schmelzpunkt von 139-141 ° C.

### Beispiel 2

Wie in Beispiel 1 wird (E)-5,5-Diphenyl-2,4-pentadiencarbonsäure (1 g) und 4-Nitrophenol (0,7 g) in Methylenchlorid(10 ml) mit DCC (0,824 g) behandelt. Die Mischung wird während 10 min. bei 0 - 5 ° C und dann während 45 Minuten bei Raumtemperatur gerührt. Nach dem üblichen Aufarbeiten wird der Ester aus 2-Propanol kristallisiert. Man erhält 1,1 g (E)-5,5-Diphenyl-2,4-pentadiencarbonsäure-4-nitrophenylester mit einem Schmelzpunkt von 113-115 ° C.

### Beispiel 3

Wie in Beispiel 1 wird (E)-5,5-bis(3-Fluorphenyl)-2,4-pentadiencarbonsäure (20 g) und 4-Nitrophenol (11,2 g) in Methylenchlorid (250 ml) mit DCC (14,7 g) behandelt. Die Mischung wird während 60 Minuten bei 0-5 ° C und dann während 3 Stunden bei Raumtemperatur gerührt. Nach dem üblichen Aufarbeiten wird der Ester aus Methylenchlorid/Hexan kristallisiert. Man erhält 26,1 g (E)-5,5-bis(3-Fluorphenyl-2,4-pentadiencarbonsäure-4-nitrophenylester mit einem Schmelzpunkt von 129-130 ° C. Durch Umkristallisieren einer Portion aus dem gleichen Lösungsmittelsystem erhält man eine analytisch reine Probe mit einem Schmelzpunkt von 129,5-130,5 ° C.

### Beispiel 4

Wie in Beispiel 1 wird (E)-5,5-bis(4-Fluorphenyl)-2,4-pentadiencarbonsäure (23,75 g) und 4-Nitrophenol (12,7 g) in Methylenchlorid (250 ml) mit DCC (17,12 g) behandelt. Die Mischung wird während 60 Minuten bei 0-5 ° C und dann während 1 Stunde bei Raumtemperatur gerührt. Nach dem üblichen Aufarbeiten wird das Rohprodukt aus 2-Propanol kristallisiert. Man erhält 26,5 g (E)-5,5-bis(4-Fluorphenyl)-2,4-pentadiencarbonsäure-4-nitrophenylester mit einem Schmelzpunkt von 112-114 ° C. Durch Umkristallisieren einer Portion aus dem gleichen Lösungsmittel erhält man eine analytsich reine Probe mit einem Schmelzpunkt von 112,5-114 ° C.

### Beispiel 5

Wie in Beispiel 1 wird (E)-5,5-bis(3-Methoxyphenyl)-2,4-pentadiencarbonsäure (49,6 g) und 4-Nitrophenol (25 g) in Methylenchlorid (600 ml) mit DCC (33 g) behandelt. Die Mischung wird während 1 Stunde bei 0-5 ° C und dann während 2 Stunden bei Raumtemperatur gerührt. Nach dem üblichen Aufarbeiten wird das Rohprodukt (67 g) aus 2-Propanol/Ether/Hexan kristallisiert. Man erhält 61 g (E)-5,5-bis(3-Methoxyphenyl)-2,4-pentadiencarbonsäure-4-nitrophenyl ester mit einem Schmelzpunkt von 95-97 ° C. Durch Umkristallisieren einer Portion aus Methylenchlorid/Hexan erhält man eine analytisch reine Probe mit einem Schmelzpunkt von 99-100 ° C.

Beispiel 6

Wie in Beispiel 1 wird (E)-5,5-bis(Methoxyphenyl)-2,4-pentadiencarbonsäure (21,0 g) und 4-Nitrophenol (11,2 g) in Methylenchlorid (300 ml) mit DCC (14 g) behandelt. Die Mischung wird während 1 Stunde bei 0-5°C und dann über Nacht bei Raumtemperatur gerührt. Nach dem üblichen Aufarbeiten wird das Rohprodukt aus Ether/Hexan kristallisiert. Man erhält 24,7 g (E)-5,5-bis(4-Methoxyphenyl)-2,4-pentadiencarbonsäure-4-nitrophenylester mit einem Schmelzpunkt von 121-123°C. Durch Umkristallisieren dieses Materials aus 2-Propanol erhält man den reinen Ester mit einem Schmelzpunkt von 125-126°C.

Beispiel 7

Wie in Beispiel 1 wird (E)-5,5-bis(3,4-Dimethoxyphenyl)-2,4-pentadiencarbonsäure (16 g) und 4-Nitrophenol (6,6 g) in Methylenchlorid (160 ml) mit DCC (9,3 g) behandelt. Die Mischung wird während 1 Stunde bei 0-5°C und dann bei Raumtemperatur während 18 Stunden gerührt. Nach dem üblichen Aufarbeiten wird das Rohprodukt mit Ether behandelt. Man erhält 13,2 g (E)-5,5-bis(3,4-Dimethoxyphenyl)-2,4-pentadiencarbonsäure-4-nitrophenylester. Durch Umkristallisieren einer Portion aus Ether erhält man eine analytisch reine Probe mit einem Schmelzpunkt von 140-142°C.

Beispiel 8

Wie in Beispiel 1 wird (E)-5,5-bis(3-Chlorphenyl)-2,4-pentadiencarbonsäure (4,79 g) und 4-Nitrophenol (2,5 g) in Methylenchlorid (50 ml) mit DCC (3,1 g) behandelt. Die Mischung wird während 1 Stunde bei 0-5°C und dann während 1 Stunde bei Raumtemperatur gerührt. Nach dem üblichen Aufarbeiten erhält man 7,2 g (E)-5,5-bis(3-Chlorphenyl)-2,4-pentadiencarbonsäure-4-nitrophenylester als Oel.

Beispiel 9

Wie in Beispiel 1 wird (E)-5,5-bis(4-Chlorphenyl)-2,4-pentadiencarbonsäure (4,79 g) und 4-Nitrophenol (2,5 g) in Methylenchlorid (50 ml) mit DCC (3,1 g) behandelt. Die Mischung wird während 1 Stunde bei 0-5°C und dann während 1 Stunde bei Raumtemperatur gerührt. Nach dem üblichen Aufarbeiten erhält man 6,9 g (E)-5,5-bis(4-Chlorphenyl)-2,4-pentadiencarbonsäure-4-nitrophenylester als schwachgelbes Oel.

Beispiel 10

Wie in Beispiel 1 wird (E)-5,5-bis[2-(Trifluormethyl)phenyl]-2,4-pentadiencarbonsäure (1,135 g) und 4-Nitrophenol (0,450 g) in Methylenchlorid (15 ml) mit DCC (0,607 g) behandelt. Die Mischung wird während 1 Stunde bei 0-5°C und dann während 30 Minuten bei Raumtemperatur gerührt. Durch übliches Aufarbeiten erhält man 1,45 g (E)-5,5-bis-[2-(Trifluormethyl)phenyl]-2,4-pentadiencarbonsäure-4-nitrophenylester als Oel.

Beispiel 11

Wie in Beispiel 1 wird (E)-5,5-bis[3-(Trifluormethyl)phenyl]-2,4-pentadiencarbonsäure (5,8 g) und 4-Nitrophenol (2,5 g) in Methylenchlorid (50 ml) mit DCC (3,1 g) behandelt. Die Mischung wird während 1 Stunde bei 0-5°C und dann während 2 Stunden bei Raumtemperatur gerührt. Durch übliches Aufarbeiten erhält man 8 g (E)-5,5-bis[3-(Trifluormethyl) phenyl]-2,4-pentadiencarbonsäure-4-nitrophenylester als Oel.

Beispiel 12

Wie in Beispiel 1 wird (E)-5,5-bis(3-Nitrophenyl)-2,4-pentadiencarbonsäure (0,78 g) und 4-Nitrophenol (0,35 g) in Methylenchlorid (10 ml) mit DCC (0,47 g) behandelt. Die Mischung wird während 1 Stunde bei 0-5°C und dann während 1 Stunde bei Raumtemperatur gerührt. Durch übliches Aufarbeiten erhält man 1,01 g rohen (E)-5,5-bis(3-Nitrophenyl)-2,4-pentadiencarbonsäure-4-nitrophenylester.

Beispiel 13

Wie in Beispiel 1 wird (2E,4E)-5-(3-Methoxyphenyl)-5-phenyl-2,4-pentadiencarbonsäure (3,2 g) und 4-Nitrophenol (1,73 g) in Methylenchlorid (50 ml) mit DCC (2,35 g) behandelt. Die Mischung wird während 1,5

Stunden bei Raumtemperatur gerührt. Durch übliches Aufarbeiten erhält man 4,2 g Rohprodukt, das man aus 2-Propanol kristallisiert. Man erhält 3,5 g (2E,4Z)-5-(3-Methoxyphenyl)-5-phenyl-2,4-pentadien carbonsäure-4-nitrophenylester mit einem Schmelzpunkt von 101-103°C.

Beispiel 14

Wie in Beispiel 1 wird (2E,4Z)-5-(3-Methoxyphenyl)-5-phenyl-2,4-pentadiencarbonsäure (2,85 g) und 4-Nitrophenol (1,56 g) in Methylenchlorid (35 ml) mit DCC (2,1 g) behandelt und die Mischung wird während 75 Minuten bei Raumtemperatur gerührt. Durch übliches Aufarbeiten erhält man 3,5 g Rohprodukt, das man aus 2-Propanol kristallisiert. Man erhält 3,0 g (2E,4Z)-5-(3-Methoxyphenyl)-5-phenyl-2,4-pentadien carbonsäure-4-nitrophenylester mit einem Schmelzpunkt von 155-157°C.

Beispiel 15

Wie in Beispiel 1 wird (2E,4Z)-5-(3-Fluorphenyl)-5-(3-methoxyphenyl)-2,4-pentadiencarbonsäure (2,65 g) und 4-Nitrophenol (1,42 g) in Methlyenchlorid (45 ml) mit DCC (1,88 g) behandelt. Die Mischung wird während 15 Minuten bei 0-5°C und dann über Nacht bei Raumtemperatur gerührt. Durch übliches Aufarbeiten erhält man 3,65 g (2E,4Z)-5-(3-Fluorphenyl)-5-(3-methoxyphenyl)-2,4-pentadiencarbonsäure-4-nitrophenylester. Durch Kristallisieren einer Portion aus 2-Propanol erhält man eine analytisch reine Probe mit einem Schmelzpunkt von 134-135°C.

Beispiel 16

Wie in Beispiel 1 wird (2E,4E)-5-(3-Fluorphenyl)-5-(3-methoxyphenyl)-2,4-pentadiencarbonsäure (1,5 g) und 4-Nitrophenol (0,804 g) in Methylenchlorid (35 ml) mit DCC (1,067 g) behandelt. Die Mischung wird während 15 Minuten bei 0-5°C und dann über Nacht bei Raumtemperatur gerührt. Durch übliches Aufarbeiten erhält man 2,01 g (2E,4E)-5-(3-Fluorphenyl)-5-(3-methoxyphenyl)-2,4-pentadiencarbonsäure-4-nitrophenylester. Durch Kristallisieren einer Portion aus 2-Propanol erhält man eine analytisch reine Probe mit einem Schmelzpunkt von 146,5-147,5°C.

Beispiel 17

Wie in Beispiel 1 wird eine Lösung von (E)-5,5-bis-[(1,1′-Biphenyl)-4-yl]-2,4-pentadiencarbonsäure (0,673 g) und 4-Nitrophenol (0,256 g) in Methylenchlorid (7 ml) mit DCC (0,345 g) behandelt. Die Mischung wird während 1,5 Stunden bei Raumtemperatur gerührt. Durch übliches Aufarbeiten erhält man 0,88 g (E)-5,5-bis[(1,1′-Biphenyl)-4-yl]-2,4-pentadiencarbonsäure-4-nitrophenylester.

Beispiel 18

Eine Lösung von (Z)-5,5-bis(3-Methoxyphenyl)-2,4-pentadiencarbonsäure (1,85 g) und 4-Nitrophenol (0,912 g) in Methylenchlorid (25 ml) wird mit DCC (1,23 g) behandelt. Die Mischung wird während 1 Stunde bei 0-5°C und dann während 1 Stunde bei Raumtemperatur gerührt. Durch übliches Aufarbeiten erhält man 2,2 g rohen Ester. Durch Reinigung mittels HPLC (Methylenchlorid/Hexan; 3:1) erhält man 1,2 g (Z)-5,5-bis-(3-Methoxyphenyl)-2,4-pentadiencarbonsäure-4-nitrophenylester als Oel.

Beispiel 19

Wie in Beispiel 1 wird (E,E)-5-(4-Methoxyphenyl)-5-phenyl-2,4-pentadiencarbonsäure (5,2 g) und 4-Nitrophenol (3,14 g) in 30 ml Tetrachlorkohlenstoff und 10 ml Methylenchlorid mit DCC (3,8 g) behandelt. Die Mischung wird während 2 Tagen bei Raumtemperatur gerührt. Nach dem üblichen Aufarbeiten wird der Ester aus Ether/Hexan kristallisiert. Man erhält 4,14 g (E,E)-5-(4-Methoxyphenyl)-5-phenyl-2,4-pentadie ncarbonsäure-4-nitrophenylester mit einem Schmelzpunkt von 113-115°C.

Beispiel 20

Wie in Beispiel 1 wird (2E,4Z)-5-(4-Methoxyphenyl)-5-phenyl-2,4-pentadiencarbonsäure (0,85 g) und 4-Nitrophenol (0,5 g) in 5 ml Tetrachlorkohlenstoff und 5 ml Methylenchlorid mit DCC (0,62 g) behandelt. Die Mischung wird während 18 Stunden bei Raumtemperatur gerührt und dann in üblicher Weise aufgearbeitet.

Man erhält 1,0 g (2E,4Z)-5-(4-Methoxyphenyl)-5-phenyl-2,4-pentadiencarbonsäure-4-nitrophenylester als Oel.

Beispiel 21

Wie in Beispiel 1 wird (E,E)-5-(2-Methoxyphenyl)-5-(4-Methoxyphenyl)-2,4-pentadiencarbonsäure (3,4 g) und 4-Nitrophenol (1,8 g) in 25 ml Methylenchlorid mit DCC (2,27 g) behandelt. Die Mischung wird während 2 Stunden bei Raumtemperatur gerührt. Nach dem üblichen Aufarbeiten wird der Ester aus 2-Propanol kristallisiert. Man erhält 3,0 g (E,E)-5-(2-Methoxyphenyl)-5-(4-Methoxyphenyl)-2,4-pentadiencarbonsäure-4-nitrophenylester mit einem Schmelzpunkt von 149-150 ° C.

Beispiel 22

Wie in Beispiel 1 wird (2E,4Z)-5-(2-Methoxyphenyl)-5-(4-methoxyphenyl)-2,4-pentadiencarbonsäure (1 g) und 4-Nitrophenol (0,54 g) in 7 ml Methylenchlorid mit DCC (0,67 g) behandelt. Die Mischung wird während 2 Stunden bei Raumtemperatur gerührt. Nach dem üblichen Aufarbeiten wird der rohe Ester aus 2-Propanol kristallisiert. Man erhält 0,825 g (2E,4Z)-5-(2-Methoxyphenyl)-5-(4-methoxyphenyl)-2,4-pentadiencarbonsäure-4-nitrophenylester mit einem Schmelzpunkt von 133,5-135 ° C.

Beispiel 23

Wie in Beispiel 1 wird (E)-5,5-bis-(4-Methylphenyl)-2,4-pentadiencarbonsäure (7,25 g) und 4-Nitrophenol (4,4 g) in Methylenchlorid (60 ml) mit DCC (5,36 g) behandelt. Die Mischung wird während 30 Minuten bei 0-5 ° C und dann während 18 Stunden bei Raumtemperatur gerührt. Nach dem üblichen Aufarbeiten wird der Ester aus 2-Propanol kristallisiert. Man erhält 8,9 g (E)-5,5-bis(4-Methylphenyl)-2,4-pentadiencarbonsäure-4-nitrophenylester mit einem Schmelzpunkt von 153,5-154,5 ° C.

Beispiel 24

Wie in Beispiel 1 wird (E)-5,5-bis(2-Methoxyphenyl)-2,4-pentadiencarbonsäure (5 g) und 4-Nitrophenol (2,7 g) in Methylenchlorid (35 ml) mit DCC (3,34 g) behandelt. Die Mischung wird während 30 Minuten bei 0-5 ° C und dann während 18 Stunden bei Raumtemperatur gerührt. Nach dem üblichen Aufarbeiten wird das Rohprodukt aus 2-Propanol kristallisiert. Man erhält 6,1 g (E)-5,5-bis(2-Methoxyphenyl)-2,4-pentadiencarbonsäure-4nitrophenylester mit einem Schmelzpunkt von 141,5-143 ° C. Durch Kristallisieren einer Portion aus 2-Propanol erhält man eine analytisch reine Probe mit einem Schmelzpunkt von 142-143 ° C.

Beispiel 25

Wie in Beispiel 1 wird (2E,4Z)-5-(4-Methoxyphenyl)-5-(2-thienyl)-2,4-pentadiencarbonsäue (1,76 g) und 4-Nitrophenol (1 g) in 20 ml Methylenchlorid mit DCC (1,24 g) behandelt. Die Mischung wird während 18 Stunden bei Raumtemperatur gerührt und dann in üblicher Weise aufgearbeitet. Der erhaltene Ester wird aus 2-Propanol kristallisiert. Man erhält 1,45 g (2E,4Z)-5-(4-Methoxyphenyl)-5-(2-thienyl)-2,4-pentadiencarb onsäure-4-nitrophenylester mit einem Schmelzpunkt von 113-115 ° C. Durch Umkristallisieren einer Probe aus 2-Propanol erhält man eine analytisch reine Probe mit einem Schmelzpunkt von 114,5-115,5 ° C.

Beispiel 26

Wie in Beispiel 1 wird (2E,4Z)-5-Cyclopropyl-5-(4-methoxyphenyl)-2,4-pentadiencarbonsäure (1,95 g) und 4-Nitrophenol (1,33 g) in 15 ml Methylenchlorid mit DCC (1,66 g) behandelt. Die Mischung wird während 18 Stunden bei Raumtemperatur gerührt. Nach dem üblichen Aufarbeiten wird der rohe Ester aus 2-Propanol kristallisiert. Man erhält 1,9 g (2E,4Z)-5-Cyclopropyl-5-(4-methoxyphenyl)-2,4-pentadienc arbonsäure-4-nitrophenylester mit einem Schmelzpunkt von 115,5-116,5 ° C.

Beispiel 27

Wie in Beispiel 1 wird (E,E)-5-(4-Methoxyphenyl)-5-(3-pyridinyl)-2,4- pentadiencarbonsäure (2,6 g) und 4-Nitrophenol (1,42 g) in 35 ml Methylenchlorid mit DCC (1,91 g) behandelt. Die Mischung wird während 18

Stunden bei Raumtemperatur gerührt und dann in üblicher Weise aufgearbeitet. Der Ester wird aus Ether kristallisiert. Man erhält 3,65 g (E,E)-5-(4-Methoxyphenyl)-5-(3-pyridinyl)-2,4-pentadiencarbonsäure-4-nitrophenylester mit einem Schmelzpunkt von 51-56 ° C. Durch Umkristallisieren einer Portion aus Ether erhält man eine analytisch reine Probe mit einem Schmelzpunkt von 54-56 ° C.

Beispiel 28

Wie in Beispiel 1 wird (2E,4Z)-5-(4-Methoxyphenyl)-5-(3-pyridinyl)-2,4-pentadiencarbonsäure (5,7 g) und 4-Nitrophenol (3,06 g) in 80 ml Methylenchlorid mit DCC (4,2 g) behandelt. Die Mischung wird während 18 Stunden bei Raumtemperatur gerührt. Nach dem üblichen Aufarbeiten wird der Ester aus 2-Propanol kristallisiert. Man erhält 7,3 g (E,E)-5-(4-Methoxyphenyl)-5-(3-pyridinyl)-2,4-pentadiencarbonsäure-4-nitrophenlyester mit einem Schmelzpunkt von 129,5-131 ° C. Aus dem gleichen Lösungsmittel erhält man eine analytisch reine Probe mit einem Schmelzpunkt von 130-131 ° C.

Beispiel 29

Wie in Beispiel 1 wird (E,E)-5-Cyclopropyl-5-(4-methoxyphenyl)-2,4-pentadiencarbonsäure (2,7 g) und 4-Nitrophenol (1,844 g) in 20 ml Methylenchlorid mit DCC (2,3 g) behandelt. Die Mischung wird über Nacht bei Raumtemperatur gerührt und dann in üblicher Weise aufgearbeitet. Der Ester wird aus 2-Propanol kristallisiert. Man erhält 2,8 g (E,E)-5-Cyclopropyl-5-(4-methoxyphenyl)-2,4-pentadiencarbonsäure-4-nitrophenylester mit einem Schmelzpunkt von 111-112,5 ° C.

Beispiel 30

Wie in Beispiel 1 wird (E,E)-5-Cyclohexyl-5-(4-methoxyphenyl)-2,4-pentadiencarbonsäure (2,86 g) und 4-Nitrophenol (1,67 g) in 15 ml Methylenchlorid mit DCC (2,07 g) behandelt. Die Mischung wird während 18 Stunden bei Raumtemperatur gerührt und dann in üblicher Weise aufgearbeitet. Der erhaltene rohe Ester (3,5 g) wird aus 2-Propanol kristallisiert. Man erhält 3,15 g (E,E)-5-Cyclohexyl-5-(4-Methoxyphenyl-2,4-pentadiencarbonsäure-4-nitrophenylester mit einem Schmelzpunkt von 136-137 ° C.

Beispiel 31

Wie in Beispiel 1 wird (2E,4Z)-5-Cyclohexyl-5-(4-methoxyphenyl)-2,4-pentadiencarbonsäure (3,5 g) und 4-Nitrophenol (2,07 g) in 20 ml Tetrachlorkohlenstoff und 5 ml Methylenchlorid mit DCC (2,07 g) behandelt. Die Mischung wird während 3 Tagen bei Raumtemperatur gerührt. Durch übliches Aufarbeiten erhält man 4,8 g rohen Ester, den man aus 2-Propanol/Hexan kristallisiert. Man erhält 3,8 g (2E,4Z)-5-Cyclohexyl-5-(4-methoxyphenyl)-2,4-pentadiencarbonsäure-4-nitrophenylester mit einem Schmelzpunkt von 117-119 ° C.

Beispiel 32

Wie in Beispiel 1 wird (E,E)-5-(4-Methoxyphenyl)-2,4-pentadiencarbonsäure (3,47 g) und 4-Nitrophenol (2,83 g) in 50 ml Methylenchlorid mit DCC (3,53 g) behandelt. Die Mischung wird während 3 Tagen bei Raumtemperatur gerührt. Durch übliches Aufarbeiten erhält man 4,3 g rohen Ester, den man aus Tetrahydrofuran/Ether kristallisiert. Man erhält 3,7 g (E,E)-5-(4-Methoxyphenyl)-2,4-pentadiencarbonsäure-4-nitrophenylester mit einem Schmelzpunkt von 200,5-202 ° C.

Beispiel 33

Wie in Beispiel 1 wird (E,E)-5-(4-Methoxyphenyl)-2,4-hexadiencarbonsäure (3 g) und 4-Nitrophenol (2,3 g) in 20 ml Methylenchlorid mit DCC (2,85 g) behandelt. Das Gemisch wird während 3 Tagen bei Raumtemperatur gerührt und dann in üblicher Weise aufgearbeitet. Man erhält 3,1 g rohen Ester, den man aus Methylenchlorid/2-Propanol kristallisiert. Man erhält 2,95 g (E,E)-5-(4-Methoxyphenyl)-2,4-pentadiencarbonsäure-4-nitrophenylester mit einem Schmelzpunkt von 176-177 ° C.

Beispiel 34

Wie in Beispiel 1 wird (E,E)-5-(4-Methoxyphenyl)-2,4-heptadiencarbonsäure (2,8 g) und 4-Nitrophenol (2 g) in 20 ml Methylenchlorid mit DCC (2,49 g) behandelt. Die Mischung wird über Nacht bei Raumtemperatur gerührt. Durch übliches Aufarbeiten erhält man 4,3 g rohen Ester, den man aus 2-Propanol kristallisiert. Man erhält 3,1 g (E,E)-5-(4-Methoxyphenyl)-2,4-heptadiencarbonsäure-4-nitrophenylester mit einem Schmelzpunkt von 112,5-113,5 ° C.

Beispiel 35

Wie in Beispiel 1 wird (E,E)-5-(4-Methoxyphenyl)-6-methyl-2,4-heptadiencarbonsäure (2,4 g) und 4-Nitrophenol (1,63 g) in 15 ml Methylenchlorid mit DCC (2,02 g) behandelt. Die Mischung wird während 18 Stunden bei Raumtemperatur gerührt. Durch Kristallisieren des Rohproduktes aus 2-Propanol/Hexan erhält man 2,85 g (E,E)-5-(4-Methoxyphenyl)-6-methyl-2,4-heptadiencarbonsäure-4-nitrophenylester mit einem Schmelzpunkt von 95-96,5 ° C.

Beispiel 36

Wie in Beispiel 1 wird (2E,4Z)-5-(4-Methoxyphenyl)-6-methyl-2,4-heptadiencarbonsäure (3,2 g) und 4-Nitrophenol (2,2 g) in 20 ml Methylenchlorid mit DCC (2,7 g) behandelt. Das Gemisch wird während 18 Stunden bei Raumtemperatur gerührt. Nach dem üblichen Aufarbeiten wird das Rohprodukt aus 2-Propanol/Hexan kristallisiert. Man erhält 3,55 g (2E,4Z)-5-(4-Methoxyphenyl)-6-methyl-2,4-heptadiencarbonsäure-4-nitrophenylester mit einem Schmelzpunkt von 111,5-113 ° C.

Beispiel 37

Wie in Beispiel 1 wird (E,E)-5-(4-Methoxyphenyl)-2,4-octadiencarbonsäure (2,47 g) und 4-Nitrophenol (1,5 g) in 15 ml Methylenchlorid mit DCC (1,86 g) behandelt. Die Mischung wird während 3 Tagen bei Raumtemperatur gerührt. Durch Kristallisieren des Rohproduktes aus 2-Propanol erhält man 2,5 g (E,E)-5-(4-Methoxyphenyl)-2,4-octadiencarbonsäure-4-nitrophenylester mit einem Schmelzpunkt von 90-92 ° C.

Beispiel 38

Wie in Beispiel 1 wird (E,E)-5-(4-Methoxyphenyl)-2,4-nonadiencarbonsäure (3,12 g) und 4-Nitrophenol (2 g) in 20 ml Methylenchlorid mit DCC (2,49 g) behandelt. Die Mischung wird während 3 Tagen bei Raumtemperatur gerührt. Nach dem üblichen Aufarbeiten wird das Rohprodukt aus 2-Propanol kristallisiert. Man erhält 3,55 g (E,E)-5-(4-Methoxyphenyl)-2,4-nonadiencarbonsäure-4-nitrophenylester mit einem Schmelzpunkt von 78-80 ° C. Das Produkt verfestigt sich dann und schmilzt erneut bei 115 ° C.

Beispiel 39

Wie in Beispiel 1 wird (E,E)-5-(3-Methoxypenyl)-2,4-nonadiencarbonsäure (4,48 g) und 4-Nitrophenol (2,97 g) in 25 ml Methylenchlorid mit DCC (3,59 g) behandelt. Die Mischung wird während 3 Tagen bei Raumtemperatur gerührt und dann in üblicher Weise aufgearbeitet. Das Rohprodukt wird aus 2-Propanol kristallisiert. Man erhält 5,6 g (E,E)-5-(3-Methoxyphenyl)-2,4-nonadiencarbonsäure-4-nitrophenylester mit einem Schmelzpunkt von 81,5-83 ° C.

Beispiel 40

Wie in Beispiel 1 wird (2E,4Z)-5-(3-Methoxyphenyl)-2,4-nonadiencarbonsäure (7,37 g) und 4-Nitrophenol (4,89 g) in 30 ml Methylenchlorid mit DCC (5,91 g) behandelt. Die Mischung wird während 3 Tagen bei Raumtemperatur gerührt und dann in üblicher Weise aufgearbeitet. Man erhält 9,69 g (2E,4Z)-5-(3-Methoxyphenyl)-2,4-nonadiencarbonsäure-4-nitrophenylester als Oel.

Beispiel 41

Wie in Beispiel 1 wird (E,E)-5-(4-Methoxyphenyl)-2,4-decadiencarbonsäure (4,5 g) und 4-Nitrophenol (2,75 g) in 35 ml Methylenchlorid mit DCC (3,4 g) behandelt. Die Mischung wird während 4 Stunden bei Raumtemperatur gerührt. Nach dem üblichen Aufarbeiten wird der rohe Ester aus 2-Propanol kristallisiert. Man erhält 4,9 g (E,E)-5-(4-Methoxyphenyl)-2,4-decadiencarbonsäure-4-nitrophenylester mit einem Schmelzpunkt von 75-76 ° C.

Beispiel 42

Wie in Beispiel 1 wird (2E,4Z)-5-(4-Methoxyphenyl)-2,4-decadiencarbonsäure (2,8 g) und 4-Nitrophenol (1,71 g) in 20 ml Methylenchlorid mit DCC (2,1 g) behandelt. Die Mischung wird während 18 Stunden bei Raumtemperatur gerührt und dann in üblicher Weise aufgearbeitet. Man erhält 4,0 g (2E,4Z)-5-(4-Methoxyphenyl)-2,4-decadiencarbonsäure-4-nitrophenylester als Oel.

Beispiel 43

Wie in Beispiel 1 wird (E,E)-5-(3,4-Dimethoxyphenyl)-2,4-decadiencarbonsäure (3.08 g) und 4-Nitrophenol (2,75 g) in 20 ml Methylenchlorid mit DCC (2,11 g) behandelt. Die Mischung wird während 3 Tagen bei Raumtemperatur gerührt und dann in üblicher Weise aufgearbeitet. Durch Kristallisieren des rohen Esters aus 2-Propanol/Hexan erhält man 2,98 g (E,E)-5-(3,4-Dimethoxyphenyl)-2,4-decadiencarbonsäure-4-nitrophenylester mit einem Schmelzpunkt von 95-96,5 ° C.

Beispiel 44

Wie in Beispiel 1 wird (E,E)-5-(3-Fluorphenyl)-2,4-decadiencarbonsäure (3,55 g) und 4-Nitrophenol (2,33 g) in 20 ml Methylenchlorid mit DCC (2,81 g) behandelt. Die Mischung wird während 3 Tagen bei Raumtemperatur gerührt. Nach dem üblichen Aufarbeiten wird der rohe Ester aus Hexan kristallisiert. Man erhält 3,51 g (E,E)-5-(3-Fluorphenyl)-2,4-decadiencarbonsäure-4-nitrophenylester mit einem Schmelzpunkt von 46-47 ° C.

Beispiel 45

Wie in Beispiel 1 wird (2E,4Z)-5-(3-Fluorphenyl)-2,4-decadiencarbonsäure (3,3 g) und 4-Nitrophenol (2,1 g) in 20 ml Methylenchlorid mit DCC (2,67 g) behandelt. Die Mischung wird während 3 Tagen bei Raumtemperatur gerührt. Nach dem üblichen Aufarbeiten wird das Rohprodukt aus Hexan kristallisiert. Man erhält 3,1 g (2E,4Z)-5-(3-Fluorphenyl)-2,4-decadiencarbonsäure-4-nitrophenylester mit einem Schmelzpunkt von 50-51 ° C.

Beispiel 46

Wie in Beispiel 1 wird (E,E)-5-(4-Fluorphenyl)-2,4-decadiencarbonsäure (4,78 g) und 4-Nitrophenol (3,14 g) in 20 ml Methylenchlorid mit DCC (3,8 g) behandelt. Die Mischung wird während 3 Tagen bei Raumtempertur gerührt. Nach dem üblichen Aufarbeiten wird der rohe Ester aus 2-Propanol/ Hexan kristallisiert. Man erhält 4,86 g (E,E)-5-(4-Fluorphenyl)-2,4-decadiencarbonsäure-4-nitrophenylester mit einem Schmelzpunkt von 71-72,5 ° C.

Beispiel 47

Wie in Beispiel 1 wird (E,E)-5-(4-Methoxyphenyl-2,4-undecadiencarbonsäure (3,75 g) und 4-Nitrophenol (2,2 g) mit 20 ml Methylenchlorid mit DCC (2,7 g) behandelt. Die Mischung wird über Nacht bei Raumtemperatur gerührt und dann in üblicher Weise aufgearbeitet. Man erhält 4,5 g (E,E)-5-(4-Methoxyphenyl)-2,4-undecadiencarbonsäure-4-nitrophenylester als Oel.

Beispiel 48

Wie in Beispiel 1 wird (E,E)-5-(4-Methoxyphenyl)-2,4-tridecadiencarbonsäure (4,75 g) und 4-Nitrophenol (2,5 g) in 25 ml Methylenchlorid mit DCC (3,1 g) behandelt. Die Mischung wird während 18 Stunden bei Raumtemperatur gerührt. Nach dem üblichen Aufarbeiten erhält man 4,85 g (E,E)-5-(4-Methoxyphenyl)-2,4-tridecadiencarbonsäure-4-nitrophenylester als Oel.

Beispiel 49

Wie in Beispiel 1 wird (E,E)-5-(4-Methoxyphenyl)-2,4-heptadecadiencarbonsäure (7,5 g) und 4-Nitrophenol (3,77 g) in 30 ml Methylenchlorid mit DCC (4,2 g) behandelt. Die Mischung wird während 3 Tagen bei Raumtemperatur gerührt und dann in üblicher Weise aufgearbeitet. Man erhält 8,5 g rohen Ester, den man

mittels HPLC (Ether/Hexan; 1:7) reinigt. Nach Kristallisieren aus 2-Propanol erhält man 5,88 g (E,E)-5-(4-Methoxyphenyl)-2,4-heptadecadiencarbonsäure-4-nitrophenylester mit einem Schmelzpunkt von 46-47 ° C.

Beispiel 50

Wie in Beispiel 1 wird 5-Pentyl-2,4-decadiencarbonsäure (4,0 g) und 4-Nitrophenol (2,8 g) in 30 ml Methylenchlorid mit DCC (3,48 g) behandelt. Die Mischung wird während 18 Stunden bei Raumtemperatur gerührt. Nach dem üblichen Aufarbeiten wird der rohe Ester mittels HPLC (Ether/Hexan; 1:24) gereinigt. Man erhält 2,45 g 5-Pentyl-2,4-decadiencarbonsäure-4-nitrophenylester als Oel.

## Patentansprüche

## Patentansprüche für folgende Vertragsstaaten : AT, BE, CH, DE, FR, GB, IT, LI, LU, NL, SE

**1.** Verbindungen der allgemeinen Formel

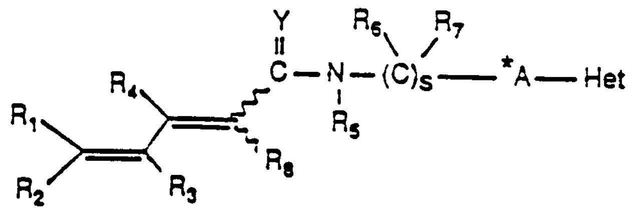

I

worin Y O oder S, *A Paraphenylen oder die Gruppe $^*$-$(CH_2)_n$-$(X)_m$-$(CH_2)_r$-, X O, S oder -CH = CH-, n und r unabhängig voneinander eine ganze Zahl von 0 bis 3, s die Zahl 0 oder 1, m die Zahl 0 oder 1, wobei n + s mindestens 2 ist, wenn m die Zahl 1 ist, $R_1$ und $R_2$ unabhängig voneinander Wasserstoff, niederes Alkyl, $C_{3-6}$-Cycloalkyl, niederes Alkenyl, Het oder Aryl, $R_3$, $R_4$ und $R_8$ unabhängig voneinander Wasserstoff, niederes Alkyl oder Aryl, $R_5$ und $R_7$ unabhängig voneinander Wasserstoff oder niederes Alkyl, $R_6$ Wasserstoff, niederes Alkyl, $C_{3-6}$-Cycloalkyl, Het-niederes Alkyl oder Aryl, Het eine monocyclische, 6-gliedrige heteroaromatische oder eine bicyclische heteroaromatische Gruppe aus der Gruppe von Pyridinyl, Chinolinyl, Isochinolinyl, Indolyl, Benzimidazolyl und Pyrimidinyl und welche gegebenenfalls durch niederes Alkyl, Halogen oder Phenyl substituiert ist, bedeuten und das Sternchen die Verknüpfungsstelle bezeichnet und die mit nieder bezeichenten Reste bis zu 7 Kohlenstoffatome besitzen, und sofern $R_6$ und $R_7$ verschieden sind, Enantiomeren und racemische Mischungen davon, sofern $R_1$ und $R_2$ verschieden sind, geometrische Isomeren davon und pharmazeutisch annehmbare Säureadditionssalze davon.

**2.** Verbindungen nach Anspruch 1 mit der allgemeinen Formel

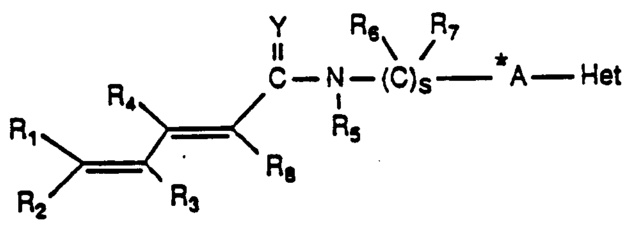

Ib

(E)- oder trans-Isomere.

3. Verbindungen nach Anspruch 1 mit der allgemeinen Formel

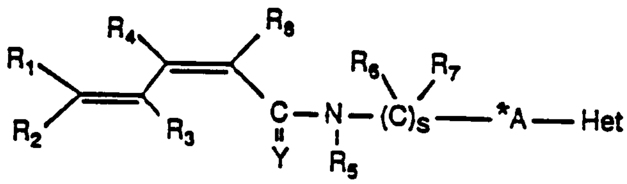

Ic

(Z)- oder cis-Isomere.

4. Verbindungen nach Anspruch 1 mit der allgemeinen Formel

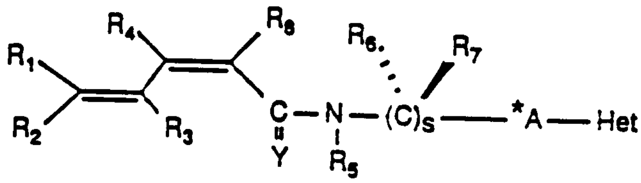

Id

[R-(Z)]-Isomere mit der (R)-Konfiguaration.

5. Verbindungen nach Anspruch 1 mit der allgemeinen Formel

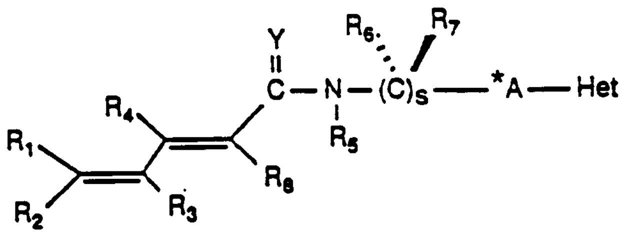

Ie

[R-(E)]-Isomere mit der (R)-Konfiguration.

6. Verbindungen nach Anspruch 1 mit der allgemeinen Formel

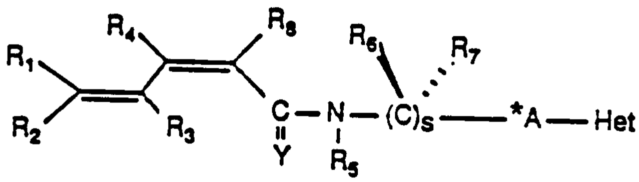

If

[S-(Z)]-Isomere mit der (S)-Konfiguration.

7. Verbindungen nach Anspruch 1 mit der allgemeinen Formel

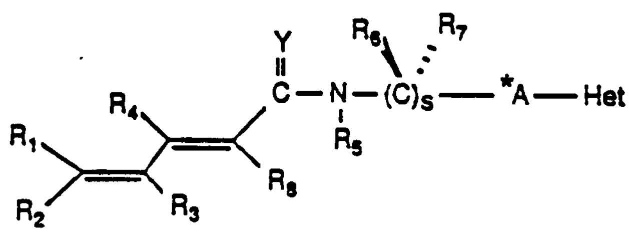

Ig

[S-(E)]-Isomere mit der (S)-Konfiguration.

8. Verbindungen nach einem der Ansprüche 1-7, worin $R_1$ und $R_2$ unabhängig voneinander niederes Alkyl oder Aryl, $R_3$, $R_4$ und $R_8$ unabhängig voneinander Wasserstoff oder niederes Alkyl, $R_5$ und $R_7$ Wasserstoff, $R_6$ Wasserstoff, niederes Alkyl oder $C_{3-6}$-Cycloalkyl, *A $*\text{-}(CH_2)_n\text{-}(X)_m\text{-}(CH_2)_r\text{-}$, n+r eine ganze Zahl von 2 bis 6, m die Zahl 0, Het eine monocyclische 6-gliedrige heteroaromatische Gruppe, welche ein oder zwei Heteroatome ausgewählt aus Stickstoff, Sauerstoff und Schwefel enthält, Y Sauerstoff oder Schwefel und s die Zahl 1 bedeuten.

9. Verbindungen nach Anspruch 8, worin $R_1$ niederes Alkyl oder Aryl, $R_2$ Aryl, $R_3$, $R_4$ und $R_8$ unabhängig von einander Wasserstoff oder niederes Alkyl, *A $*\text{-}CH_2)_n\text{-}(X)_m\text{-}(CH_2)_r\text{-}$, n+r die Zahl 3, m die Zahl 0, Het gegebenenfalls durch niederes Alkyl substituiertes Pyridinyl oder Pyrimidinyl, $R_5$ und $R_7$ je Wasserstoff, $R_6$ Wasserstoff, niederes Alkyl oder Cyclopropyl, Y Sauerstoff und s die Zahl 1 bedeuten.

10. Verbindungen nach Anspruch 9, worin $R_1$ Butyl, Pentyl oder Hexyl oder Phenyl mit bis zu 3 Substituenten ausgewählt aus Halogen und niederem Alkoxy, $R_2$ Phenyl mit bis zu 3 Substituenten ausgewählt aus Halogen und niederem Alkoxy, $R_3$, $R_4$, $R_5$, $R_7$ und $R_8$ je Wasserstoff und Het 3-Pyridinyl oder 2-Methyl-3-pyridinyl bedeuten.

11. [R-(E,E)]-5-(4-Methoxyphenyl)-N-[1-methyl-4-(3-pyridinyl)butyl]-2,4-decadienamid.

12. [R-(E,E)]-5-(4-Methoxyphenyl)-N-[1-methyl-4-(3-pyridinyl)butyl]-2,4-nonadienamid.

13. (E)-5,5-Bis(3-fluorphenyl)-N-[4-(3-pyridinyl)butyl]-2,4-pentadienamid.

14. [R-(E)]-N-[1-Ethyl]-4-(3-pyridinyl)butyl]-5,5-bis(4-methoxyphenyl)-2,4-pentadienamid.

15. [R,S-(E,E)]-5-(4-Methoxyphenyl)-N-[1-methyl-4-(2-methyl-3-pyridinyl)butyl]-2,4-decadienamid.

**16.** (E)-5,5-Bis(3-methoxyphenyl)-N-[4-(3-pyridinyl)butyl]-2,4-pentadienamid.

**17.** Verbindungen nach einem der Ansprüche 1 bis 16 zur Anwendung als therapeutisch wirksame Substanzen.

**18.** Verbindungen nach einem der Ansprüche 1 bis 16 zur Anwendung als PAF-Antagonisten.

**19.** Verfahren zur Herstellung von Verbindungen nach einem der Ansprüche 1 bis 16 und von pharmazeutisch annehmbaren Säureadditionssalzen davon, dadurch gekennzeichnet, dass man

a) ein reaktives Derivat einer Carbonsäure der allgemeinen Formel

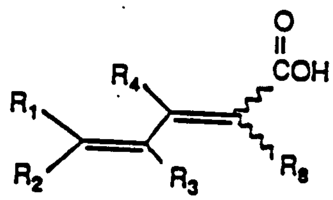

II

worin $R_1$, $R_2$, $R_3$, $R_4$ und $R_8$ die in Anspruch 1 angegebene Bedeutung besitzen, mit einer Verbindung der allgemeinen Formel

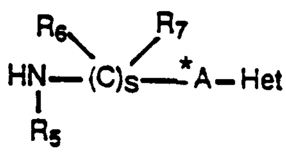

V

worin $R_5$, $R_6$, $R_7$, *A, Het und s die in Anspruch 1 angegebene Bedeutung besitzen, umsetzt, oder

b) eine Carbonsäure der obigen allgemeinen Formel II in Gegenwart eines Tri(niederen Alkyl)- oder Triarylphosphines mit einem Azid der allgemeinen Formel

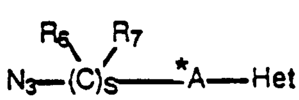

VIII

worin $R_6$, $R_7$, *A, Het und s die in Anspruch 1 angegebene Bedeutung besitzen, umsetzt, oder

c) eine Verbindung der allgemeinen Formel I, worin Y Sauerstoff bedeutet, mit Phosphorpentasulfid zu einer entsprechenden Verbindung der allgemeinen Formel I, worin Y ein Schwefelatom bedeutet, umsetzt und

d) erwünschtenfalls eine erhaltene Verbindung der allgemeinen Formel I in ein pharmazeutisch annehmbares Säureadditionssalz überführt.

**20.** Pharmazeutische Präparate enthaltend eine Verbindung nach einem der Ansprüche 1 - 16 und ein therapeutisch inertes Trägermaterial.

**21.** Pharmazeutische Präparate mit PAF-antagonistischer Wirkung enthaltend eine Verbindung nach einem der Ansprüche 1 bis 16 und ein therapeutisch inertes Trägermaterial.

**22.** Verwendung von Verbindungen nach einem der Ansprüche 1 bis 16 zur Herstellung von pharmazeutischen Präparaten, welche PAF-antagonistische Wirkung entfalten.

**Patentansprüche für folgende Vertragsstaaten : ES, GR**

**1.** Verfahren zur Herstellung von Verbindungen der allgemeinen Formel

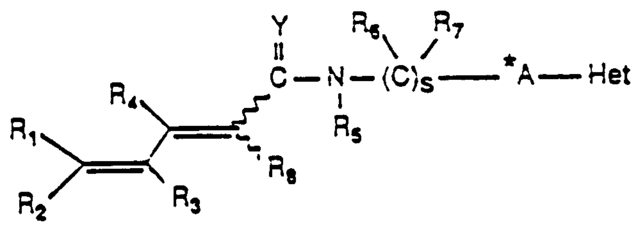

I

worin Y O oder S, *A Paraphenylen oder die Gruppe *-$(CH_2)_n$-$(X)_m$-$(CH_2)_r$-, X O, S oder -CH=CH-, n und r unabhängig voneinander eine ganze Zahl von 0 bis 3, s die Zahl 0 oder 1, m die Zahl 0 oder 1, wobei n+s mindestens 2 ist, wenn m die Zahl 1 ist, $R_1$ und $R_2$ unabhängig voneinander Wasserstoff, niederes Alkyl, $C_{3-6}$-Cycloalkyl, niederes Alkenyl, Het oder Aryl, $R_3$, $R_4$ und $R_8$ unabhängig voneinander Wasserstoff, niederes Alkyl oder Aryl, $R_5$ und $R_7$ unabhängig voneinander Wasserstoff oder niederes Alkyl, $R_6$ Wasserstoff, niederes Alkyl, $C_{3-6}$-Cycloalkyl, Het-niederes Alkyl oder Aryl, Het eine monocyclische, 6-gliedrige heteroaromatische oder eine bicyclische heteroaromatische Gruppe aus der Gruppe von Pyridinyl, Chinolinyl, Isochinolinyl, Indolyl, Benzimidazolyl und Pyrimidinyl und welche gegebenenfalls durch niederes Alkyl, Halogen oder Phenyl substituiert ist, bedeuten und das Sternchen die Verknüpfungsstelle bezeichnet und die mit nieder bezeichenten Reste bis Zu 7 Kohlenstoffatome besitzen, und sofern $R_6$ und $R_7$ verschieden sind, von Enantiomeren und racemischen Mischungen davon, sofern $R_1$ und $R_2$ verschieden sind, von geometrischen Isomeren davon und von pharmazeutisch annehmbaren Säureadditionssalzen davon, dadurch gekennzeichnet, daß man

a) einen reaktives Derivat einer Carbonsäure der allgemeinen Formel

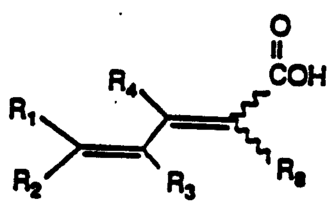

II

worin $R_1$, $R_2$, $R_3$, $R_4$ und $R_8$ obige Bedeutung besitzen, mit einer Verbindung der allgemeinen Formel

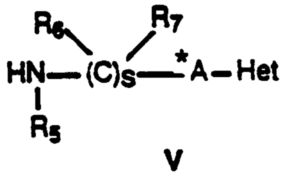

V

worin $R_5$, $R_6$, $R_7$, *A, Het und s obige Bedeutung besitzen,

umsetzt, oder

b) eine Carbonsäure der obigen allgemeinen Formel II in Gegenwart eines Tri(niederen Alkyl)- oder Triarylphosphines mit einem Azid der allgemeinen Formel

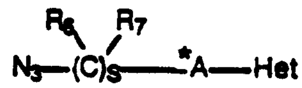

VIII

worin $R_6$, $R_7$, *A, Het und s obige Bedeutung besitzen,

umsetzt, oder

c) eine Verbindung der allgemeine Formel I, worin Y ein Sauerstoffatom bedeutet, mit Phosphorpentasulfid zu einer entsprechenden Verbindung der allgemeinen Formel I umsetzt, worin Y ein Schwefelatom bedeutet, und

d) erwünschtenfalls eine erhaltene Verbindung der Formel I in ein pharmazeutisch annehmbares Säureadditionssalz umsetzt.

**2.** Ein Verfahren nach Anspruch 1, dadurch gekennzeichnet, dass man eine Verbindung der allgemeinen Formel

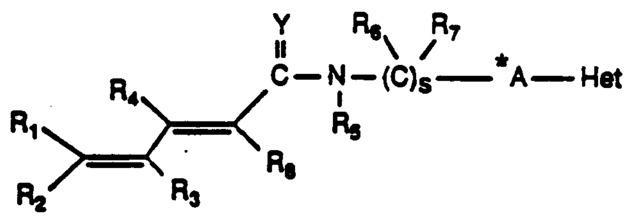

Ib

herstellt, d.h. das (E)- oder trans-Isomere.

**3.** Verfahren nach Anspruch 1, dadurch gekennzeichnet, das man eine Verbindung der allgemeinen Formel

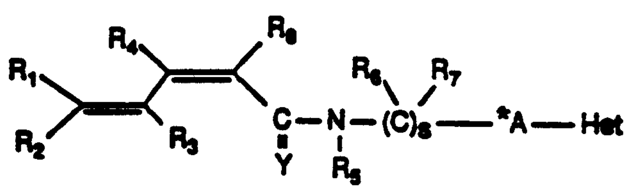

Ic

herstellt, d.h. das (Z)- oder cis-Isomere.

**4.** Verfahren nach Anspruch 1, dadurch gekennzeichnet, dass man eine verbindung der allgemeinen Formel

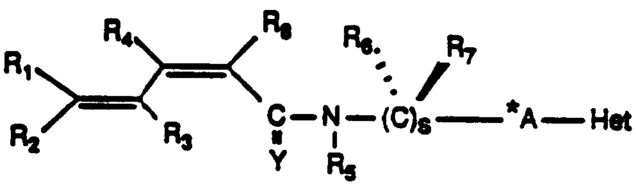

Id

herstellt, d.h. das [R-(Z)]-Isomere mit der (R)-Konfiguration.

**5.** Verfahren nach Anspruch 1, dadurch gekennzeichnet, dass man eine Verbindung der allgemeinen Formel

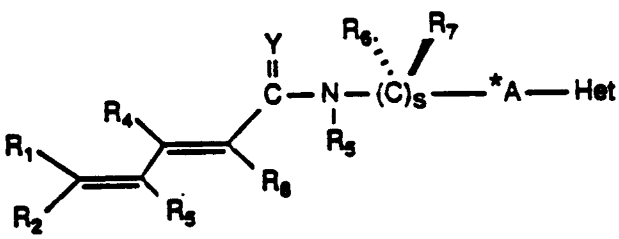

Ie

herstellt, d.h. das [R-(E)]-Isomere mit der (R)-Konfiguration.

**6.** Verfahren nach Anspruch 1, dadurch gekennzeichnet, dass man eine Verbindung der allgemeinen Formel

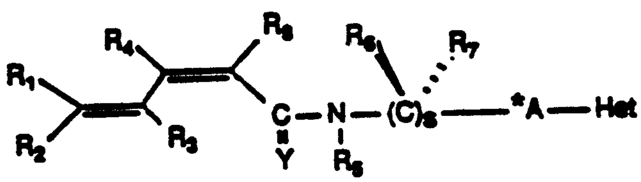

If

herstellt, d.h., das [S-(Z)]-Isomere mit der (S)-Konfiguration.

**7.** Verfahren nach Anspruch 1, dadurch gekennzeichnet, dass man eine Verbindung der allgemeinen Formel

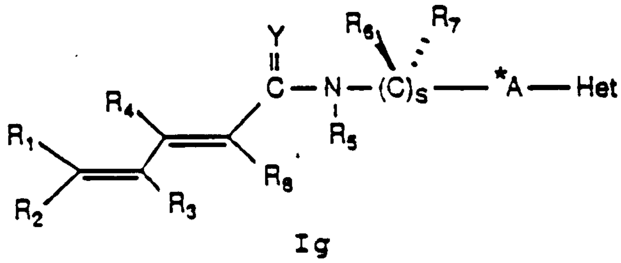

Ig

herstellt, d.h. das [S-(E)]-Isomere mit der (S)-Konfiguration.

**8.** Verfahren nach einem der Ansprüche 1-7, worin $R_1$ und $R_2$ unabhängig voneinander niederes Alkyl oder Aryl, $R_3$, $R_4$ und $R_8$ unabhängig voneinander Wasserstoff oder niederes Alkyl, $R_5$ und $R_7$ Wasserstoff, $R_6$ Wasserstoff, niederes Alkyl oder $C_{3-6}$ Cycloalkyl, *A *-$(CH_2)_n$-$(X)_m$-$(CH_2)_r$-, n + r eine ganze Zahl von 2 bis 6, m die Zahl 0, Het eine monocyclische 5- oder 6-gliedrige heteroaromatische Gruppe, welche ein oder zwei Heteroatome ausgewählt aus Stickstoff, Sauerstoff und Schwefel enthält, Y Sauerstoff oder Schwefel und s die Zahl 1 bedeuten.

**9.** Verfahren nach Anspruch 8, worin $R_1$ niederes Alkyl oder Aryl, $R_2$ Aryl, $R_3$, $R_4$ und $R_8$ unabhängig voneinander Wasserstoff oder niederes Alkyl, *A *-$(CH_2)_n$-$(X)_m$-$(CH_2)_r$-, n + r die Zahl 3, m die Zahl 0, Het gegebenenfalls durch niederes Alkyl substituiertes Pyridinyl oder Pyrimidinyl, $R_5$ und $R_7$ je Wasserstoff, $R_6$ Wasserstoff, niederes Alkyl oder Cyclopropyl, Y Sauerstoff und s die Zahl 1 bedeuten.

**10.** Verfahren nach Anspruch 9, worin $R_1$ Butyl, Pentyl oder Hexyl oder Phenyl mit bis zu 3 Substituenten ausgewählt aus Halogen und niederem Alkoxy, $R_2$ Phenyl mit bis zu 3 Substituenten ausgewählt aus Halogen und niederem Alkoxy, $R_3$, $R_4$, $R_5$, $R_7$ und $R_8$ je Wasserstoff und Het 3-Pyridinyl oder 2-Methyl-3-pyridinyl bedeuten.

**11.** Verfahren nach Anspruch 1, dadurch gekennzeichnet, dass man [R-(E,E)]-5-(4-Methoxyphenyl)-N-[1-methyl-4-(3-pyridinyl)butyl]-2,4-decadienamid herstellt.

**12.** Verfahren nach Anspruch 1, dadurch gekennzeichnet, dass man [R-(E,E)]-5-(4-Methoxyphenyl)-N-[1-methyl-4-(3-pyridinyl)butyl]-2,4-nonadienamid herstellt.

**13.** Verfahren nach Anspruch 1, dadurch gekennzeichnet, dass man (E)-5,5-Bis(3-fluorphenyl)-N-[4-(3-pyridinyl)-butyl]-2,4-pentadienamid herstellt.

**14.** Verfahren nach Anspruch 1, dadurch gekennzeichnet, dass man [R-(E)]-N-[1-Ethyl-4-(3-pyridinyl)butyl]-5,5-bis(4-methoxyphenyl)-2,4-pentadienamid herstellt.

**15.** Verfahren nach Anspruch 1, dadurch gekennzeichnet, dass man [R,S-(E,E)]-5-(4-Methoxyphenyl)-N-[1-methyl-4-(2-methyl-3-pyridinyl)butyl]-2,4-decadienamid herstellt.

**16.** Verfahren nach Anspruch 1, dadurch gekennzeichnet, dass man (E)-5,5-Bis(3-methoxyphenyl)-N-[4-(3-pyridinyl)butyl]-2,4-pentadienamid herstellt.

**17.** Verfahren zur Herstellung von pharmazeutischen Präparaten, insbesondere von Präparaten zur Verhütung oder Behandlung von Krankheiten, welche durch erhöhtes PAF gekennzeichnet sind, oder von Kreislauferkrankungen, Lungenkrankheiten, immunologischen Störungen, Entzündungen, dermatologischen Störungen, Schocks oder Transplantatabstossungen, dadurch gekennzeichnet, dass man eine der in Anspruch 1 definierten Verbindungen der Formel I oder ein pharmazeutisch annehmbares Säureadditionssalz davon und erwünschtenfalls eine oder mehrere andere therapeutisch wirksame Substanzen zusammen mit einem therapeutisch inerten Trägermaterial in eine galenische Darreichungsform bringt.

**18.** Verwendung von Verbindungen der in Anspruch I definierten Formel I oder von pharmazeutisch annehmbaren Säureadditionssalzen davon zur Herstellung von pharmazeutischen Präparaten, welche PAF-antagonistische Wirkungen entfalten.

**Claims**

**Claims for the following Contracting States : AT, BE, CH, DE, FR, GB, IT, LI, LU, NL, SE**

1. Compounds of the general formula

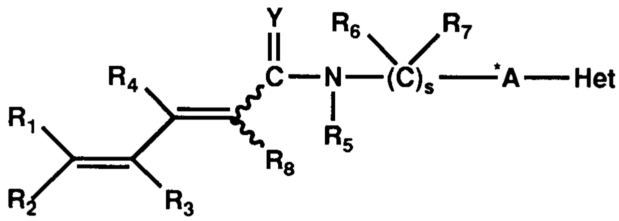

I

wherein Y signifies O or S, *A signifies paraphenylene or the group *-$(CH_2)_n$-$(X)_m$-$(CH_2)_r$-, X signifies O, S or -CH=CH-, n and r each independently signify a whole number of 0 to 3, s signifies the number 0 or 1, m signifies the number 0 or 1, provided that n+s is at least 2 when m is the number 1, $R_1$ and $R_2$ each independently signify hydrogen, lower alkyl, $C_{3-6}$-cycloalkyl, lower alkenyl, Het or aryl, $R_3$, $R_4$ and $R_8$ each independently signify hydrogen, lower alkyl or aryl, $R_5$ and $R_7$ each independently signify hydrogen or lower alkyl, $R_6$ signifies hydrogen, lower alkyl, $C_{3-6}$-cycloalkyl, Het-lower alkyl or aryl, Het signifies a monocyclic 6-membered heteroaromatic or a bicyclic heteroaromatic group from the group of pyridinyl, quinolinyl, isoquinolinyl, indolyl, benzimidazolyl and pyrimidinyl which is optionally substituted by lower alkyl, halogen or phenyl, and the asterisk denotes the point of attachment and the residues denoted by lower have up to 7 carbon atoms,
and enantiomers and racemic mixtures thereof when $R_6$ and $R_7$ are different, geometric isomers thereof when $R_1$ and $R_2$ are different and pharmaceutically acceptable acid addition salts thereof.

2. Compounds in accordance with claim 1 having the general formula

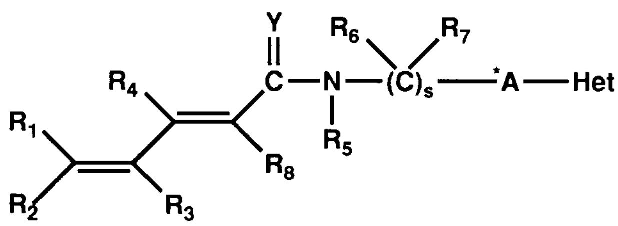

Ib

(E) or trans isomer.

3. Compounds in accordance with claim 1 having the general formula

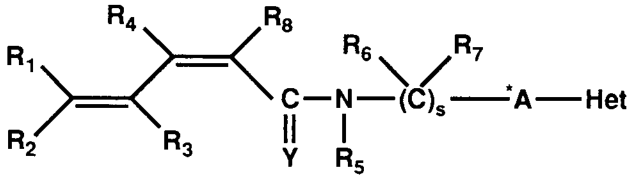

Ic

(Z) or cis isomer.

4. Compounds in accordance with claim 1 having the general formula

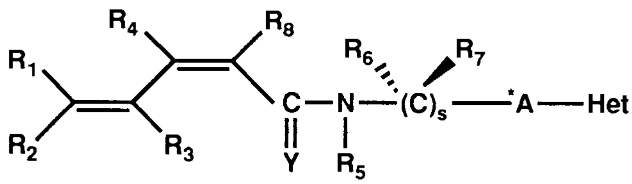

Id

[R-(Z)] isomer with the (R) configuration.

5. Compounds in accordance with claim 1 having the general formula

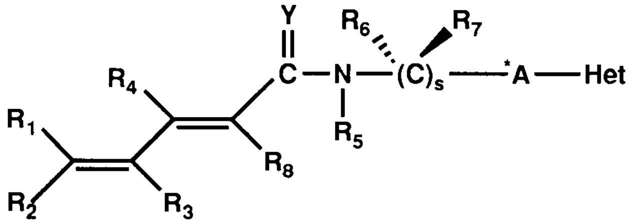

Ie

[R-(E)] isomer with the (R) configuration.

6. Compounds in accordance with claim 1 having the general formula

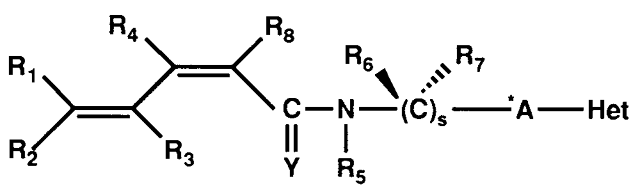

If

[S-(Z)] isomer with the (S) configuration.

7. Compounds in accordance with claim 1 having the general formula

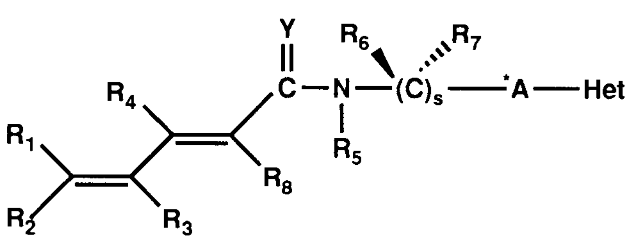

Ig

[S-(E)] isomer with the (S) configuration.

**8.** Compounds in accordance with any one of claims 1-7, wherein $R_1$ and $R_2$ each independently signify lower alkyl or aryl, $R_3$, $R_4$ and $R_8$ each independently signify hydrogen or lower alkyl, $R_5$ and $R_7$ signify hydrogen, $R_6$ signifies hydrogen, lower alkyl or $C_{3-6}$-cycloalkyl, *A signifies *-$(CH_2)_n$-$(X)_m$-$(CH_2)_r$-, n+r signify a whole number of 2 to 6, m signifies the number 0, Het signifies a monocyclic 6-membered heteroaromatic group containing one or two hetero atoms selected from nitrogen, oxygen and sulphur, Y signifies oxygen or sulphur and s signifies the number 1.

**9.** Compounds in accordance with claim 8, wherein $R_1$ signifies lower alkyl or aryl, $R_2$ signifies aryl, $R_3$, $R_4$ and $R_8$ each independently signify hydrogen or lower alkyl, *A signifies *-$(CH_2)_n$-$(X)_m$-$(CH_2)_r$-, n+r signify the number 3, m signifies the number 0, Het signifies pyridinyl or pyrimidinyl optionally substituted by lower alkyl, $R_5$ and $R_7$ each signify hydrogen, $R_6$ signifies hydrogen, lower alkyl or cyclopropyl, Y signifies oxygen and s signifies the number 1.

**10.** Compounds in accordance with claim 9, wherein $R_1$ signifies butyl, pentyl or hexyl or phenyl with up to 3 substituents selected from halogen and lower alkoxy, $R_2$ signifies phenyl with up to 3 substituents selected from halogen and lower alkoxy, $R_3$, $R_4$, $R_5$, $R_7$ and $R_8$ each signify hydrogen and Het signifies 3-pyridinyl or 2-methyl-3-pyridinyl.

**11.** [R-(E,E)]-5-(4-Methoxyphenyl)-N-[1-methyl-4-(3-pyridinyl)butyl]-2,4-decadienamide.

**12.** [R-(E,E)]-5-(4-Methoxyphenyl)-N-[1-methyl-4-(3-pyridinyl)butyl]-2,4-nonadienamide.

**13.** (E)-5,5-Bis(3-fluorophenyl)-N-[4-(3-pyridinyl)butyl]-2,4-pentadienamide.

**14.** [R-(E)]-N-[1-Ethyl-4-(3-pyridinyl)butyl]-5,5-bis(4-methoxyphenyl)-2,4-pentadienamide.

**15.** [R,S-(E,E)]-5-(4-Methoxyphenyl)-N-[1-methyl-4-(2-methyl-3-pyridinyl)butyl]-2,4-decadienamide.

**16.** (E)-5,5-Bis(3-methoxyphenyl)-N-[4-(3-pyridinyl)-butyl]-2,4-pentadienamide.

**17.** Compounds in accordance with any one of claims 1 to 16 for use as therapeutically active substances.

**18.** Compounds in accordance with any one of claims 1 to 16 for use as PAF antagonists.

**19.** A process for the manufacture of compounds in accordance with any one of claims 1 to 16 and of pharmaceutically acceptable acid addition salts thereof, characterized by

a) reacting a reactive derivative of a carboxylic acid of the general formula

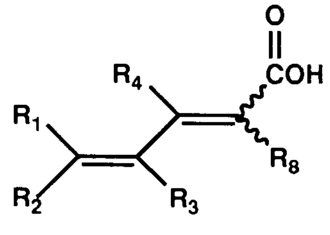

II

wherein $R_1$, $R_2$, $R_3$, $R_4$ and $R_8$ have the significance given in claim 1, with a compound of the general formula

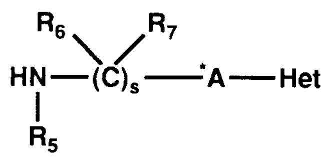

V

wherein $R_5$, $R_6$, $R_7$, *A, Het and s have the significance given in claim 1,

or

b) reacting a carboxylic acid of general formula II above in the presence of a tri(lower alkyl)- or triarylphosphine with an azide of the general formula

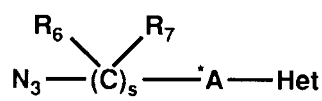

VIII

wherein $R_6$, $R_7$, *A, Het and s have the significance given in claim 1,

or

c) reacting a compound of general formula I in which Y signifies oxygen with phosphorus pentasulphide to give a corresponding compound of general formula I in which Y signifies a sulphur atom, and

d) if desired, converting a compound of the general formula I obtained into a pharmaceutically acceptable acid addition salt.

**20.** A pharmaceutical preparation containing a compound in accordance with any one of claims 1-16 and a therapeutically inert carrier.

**21.** A pharmaceutical preparation having PAF-antagonist activity, containing a compound in accordance with any one of claims 1 to 16 and a therapeutically inert carrier.

**22.** The use of a compound in accordance with any one of claims 1 to 16 for the manufacture of pharmaceutical preparations which exhibit PAF-antagonistic activity.

**Claims for the following Contracting States : ES, GR**

**1.** A process for the manufacture of compounds of the general formula

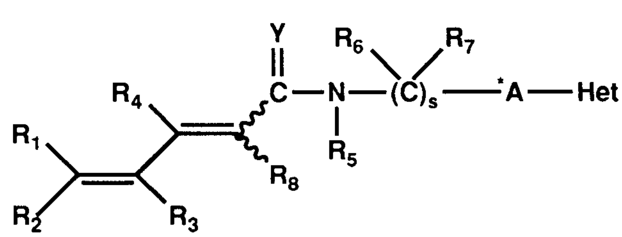

I

wherein Y signifies O or S, *A signifies paraphenylene or *$-(CH_2)_n-(X)_m-(CH_2)_r-$, X signifies the group O, S or -CH=CH-, n and r each independently signify a whole number of 0 to 3, s signifies the number 0 or 1, m signifies the number 0 or 1, provided that n+s is at least 2 when m is the number 1, $R_1$ and $R_2$ each independently signify hydrogen, lower alkyl, $C_{3-6}$-cycloalkyl, lower alkenyl, Het or aryl, $R_3$, $R_4$ and $R_8$ each independently signify hydrogen, lower alkyl or aryl, $R_5$ and $R_7$ each independently signify hydrogen or lower alkyl, $R_6$ signifies hydrogen, lower alkyl, $C_{3-6}$-cycloalkyl, Het-lower alkyl or aryl, Het signifies a monocyclic 6-membered heteroaromatic or a bicyclic heteroaromatic group from the group of pyridinyl, quinolinyl, isoquinolinyl, indolyl, benzimidazolyl and pyrimidinyl which is optionally substituted by lower alkyl, halogen or phenyl, and the asterisk denotes the point of attachment and the

residues denoted by lower have up to 7 carbon atoms,
and enantiomers and racemic mixtures thereof when $R_6$ and $R_7$ are different, geometric isomers thereof when $R_1$ and $R_2$ are different and pharmaceutically acceptable acid addition salts thereof,
characterized in that

a) reacting a reactive derivative of a carboxylic acid of the general formula

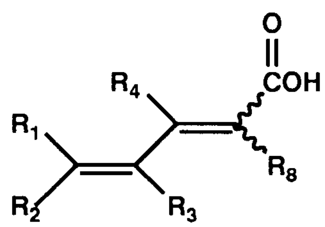

II

wherein $R_1$, $R_2$, $R_3$, $R_4$ and $R_8$ have the above significance, with a compound of the general formula

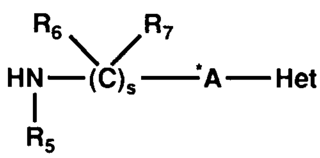

V

wherein $R_5$, $R_6$, $R_7$, *A, Het and s have above significance

or

b) reacting a carboxylic acid of general formula II above in the presence of a tri(lower alkyl)- or triarylphosphine with an azide of the general formula

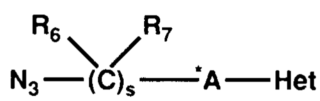

VIII

wherein $R_6$, $R_7$, *A, Het and s have the above significance,

or

c) reacting a compound of general formula I in which Y signifies oxygen with phosphorus pentasulphide to give a corresponding compound of general formula I in which Y signifies a sulphur atom, and

d) if desired, converting a compound of the general formula I obtained into a pharmaceutically acceptable acid addition salt.

**2.** A process in accordance with claim 1, characterized in that a compound of the general formula

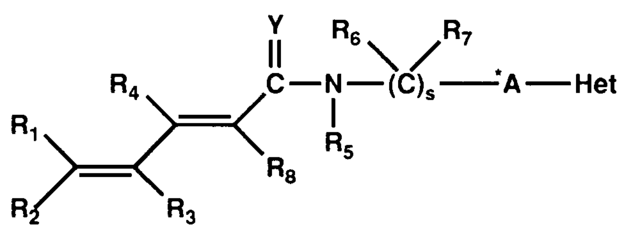

Ib

is manufactured, i.e. the (E) or trans isomer.

**3.** A process in accordance with claim 1, characterized in that a compound of the general formula

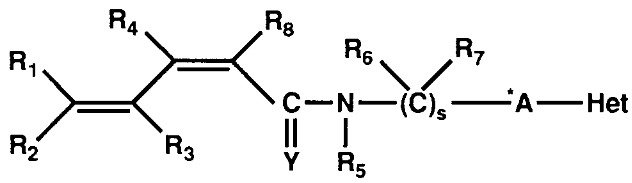

Ic

is manufactured, i.e. the (Z) or cis isomer.

**4.** A process in accordance with claim 1, characterized in that a compound of the general formula

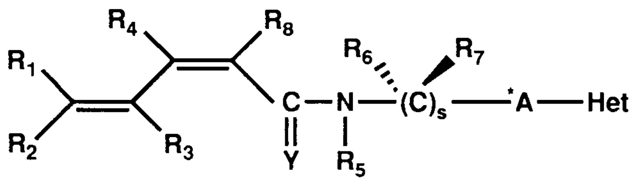

Id

is manufactured, i.e. the (R-(Z)] isomer with the (R) configuration.

**5.** A process in accordance with claim 1, characterized in that a compound of the general formula

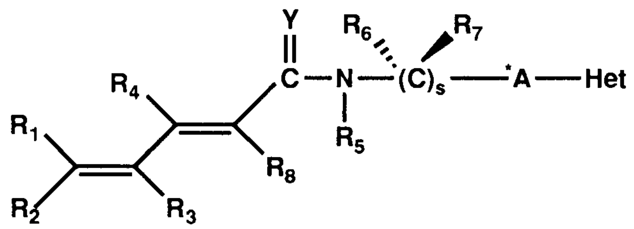

Ie

is manufactured, i.e. the [R-(E)] isomer with the (R) configuration.

**6.** A process in accordance with claim 1, characterized in that a compound of the general formula

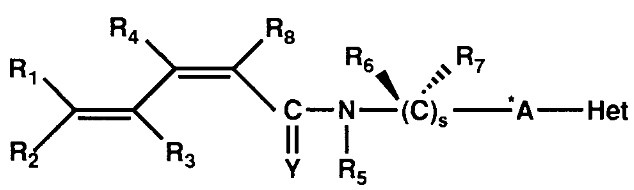

If

is manufactured, i.e. the [S-(Z)] isomer with the (S) configuration.

7. A process in accordance with claim 1, characterized in that a compound of the general formula

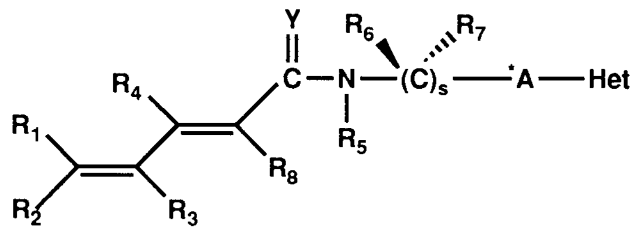

Ig

is manufactured, i.e. the [S-(E)] isomer with the (S) configuration.

8. A process in accordance with any one of claims 1-7 wherein $R_1$ and $R_2$ each independently signify lower alkyl or aryl, $R_3$, $R_4$ and $R_8$ each independently signify hydrogen or lower alkyl, $R_5$ and $R_7$ signify hydrogen, $R_6$ signifies hydrogen, lower alkyl or $C_{3-6}$-cycloalkyl, *A signifies *-$(CH_2)_n$-$(X)_m$-$(CH_2)_r$-, n+r signify a whole number of 2 to 6, m signifies the number 0, Het signifies a monocyclic 6-membered heteroaromatic group containing one or two hetero atoms selected from nitrogen, oxygen and sulphur, Y signifies oxygen or sulphur and s signifies the number 1.

9. A process in accordance with claim 8, wherein $R_1$ signifies lower alkyl or aryl, $R_2$ signifies aryl, $R_3$, $R_4$ and $R_8$ each independently signify hydrogen or lower alkyl, *A signifies *-$(CH_2)_n$-$(X)_m$-$(CH_2)_r$-, n+r signify the number 3, m signifies the number 0, Het signifies pyridinyl or pyrimidinyl optionally substituted by lower alkyl, $R_5$ and $R_7$ each signify hydrogen, $R_6$ signifies hydrogen, lower alkyl or cyclopropyl, Y signifies oxygen and s signifies the number 1.

10. A process in accordance with claim 9, wherein $R_1$ signifies butyl, pentyl or hexyl or phenyl with up to 3 substituents selected from halogen and lower alkoxy, $R_2$ signifies phenyl with up to 3 substituents selected from halogen and lower alkoxy, $R_3$, $R_4$, $R_5$, $R_7$ and $R_8$ each signify hydrogen and Het signifies 3-pyridinyl or 2-methyl-3-pyridinyl.

11. A process in accordance with claim 1, characterized in that [R-(E,E)]-5-(4-methoxyphenyl)-N-[1-methyl-4-(3-pyridinyl)butyl]-2,4-decadienamide is manufactured.

12. A process in accordance with claim 1, characterized in that [R-(E,E)]-5-(4-methoxyphenyl)-N-[1-methyl-4-(3-pyridinyl)butyl]-2,4-decadienamide is manufactured.

13. A process in accordance with claim 1, characterized in that (E)-5,5-bis(3-fluorophenyl)-N-[4-(3-pyridinyl)-butyl]-2,4-pentadienamide is manufactured.

14. A process in accordance with claim 1, characterized in that [R-(E)]-N-[1-ethyl-4-(3-pyridinyl)butyl]-5,5-bis(4-methoxyphenyl)-2,4-pentadienamide is manufactured.

15. A process in accordance with claim 1, characterized in that [R,S-(E,E)]-5-(4-methoxyphenyl)-N-[1-methyl-4-(2-methyl-3-pyridinyl)butyl]-2,4-decadienamide is manufactured.

16. A process in accordance with claim 1, characterized in that (E)-5,5-bis(3-methoxyphenyl)-N-[4-(3-pyridinyl)butyl]-2,4-pentadienamide is manufactured.

17. A process for the manufacture of pharmaceutical preparations, particularly of preparations for the prevention or treatment of diseases which are characterized by excess PAF or of cardiovascular diseases, pulmonary diseases, immunological disorders, inflammatory diseases, dermatological disorders, shock or transplant rejections. characterized by bringing a compound of formula I defined in claim 1 or a pharmaceutically acceptable acid addition salt thereof and, if desired, one or more other therapeutically active substances together with a therapeutically inert carrier into a galenical administration form.

**18.** The use of compounds of formula I defined in claim 1 or of pharmaceutically acceptable acid addition salts thereof for the manufacture of pharmaceutical preparations which exhibit PAF-antagonist activity.

## Revendications

**Revendications pour les Etats contractants suivants : AT, BE, CH, DE, FR, GB, IT, LI, LU, NL, SE**

**1.** Composés de formule générale

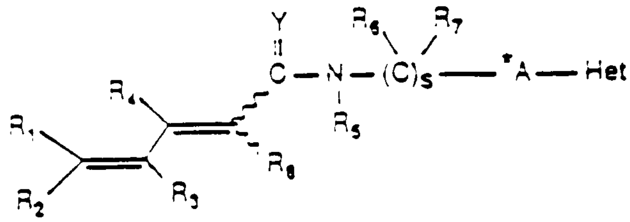

I

dans laquelle Y représente O ou S, *A un paraphénylène ou le groupe *-$(CH_2)_n$-$(X)_m$-$(CH_2)_r$-, X représente O, S ou -CH=CH-, n et r représentent indépendamment l'un de l'autre un nombre entier allant de 0 à 3, s le nombre 0 ou 1, m le nombre 0 ou 1, où n + s vaut au moins 2 lorsque m est le nombre 1, $R_1$ et $R_2$ représentent indépendamment l'un de l'autre un hydrogène, alcoyle inférieur, cycloalcoyle en $C_{3-6}$, alcényle inférieur, Het ou aryle, $R_3$, $R_4$ et $R_8$ représentent indépendamment l'un de l'autre un hydrogène, alcoyle inférieur ou aryle, $R_5$ et $R_7$ représentent indépendamment l'un de l'autre un hydrogène ou un alcoyle inférieur, $R_6$ représente un hydrogène, alcoyle inférieur, cycloalcoyle en $C_{3-6}$, Het-alcoyle inférieur ou aryle, Het représente un groupe hétéroaromatique monocyclique à six chaînons ou hétéroaromatique bicyclique du groupe de pyridinyle, quinolinyle, isoquinolinyle, indolyle, benzimidazolyle, et pyrimidinyle et qui est éventuellement substitué par un alcoyle inférieur, halogène ou un phényle, et l'astérisque représente le point de jonction et les radicaux décrits comme inférieurs possèdent jusqu'à sept atomes de carbone, et, dans la mesure où $R_6$ et $R_7$ sont différents, leurs énantiomères et mélanges racémiques, dans la mesure où $R_1$ et $R_2$ sont différents, leurs isomères géométriques et leurs sels d'addition d'acides pharmaceutiquement acceptables.

**2.** Composés selon la revendication 1, de formule générale

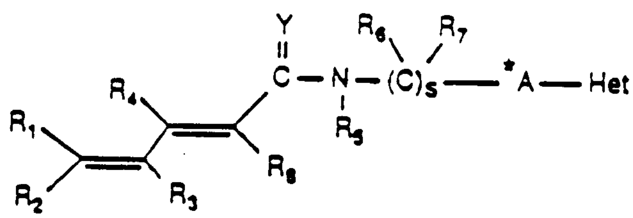

Ib

Isomères (E) ou trans

**3.** Composés selon la revendication 1, de formule générale

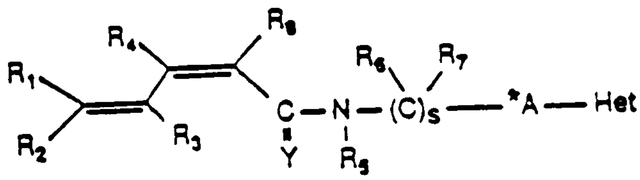

Ic

Isomères (Z) ou cis

**4.** Composés selon la revendication 1, de formule générale

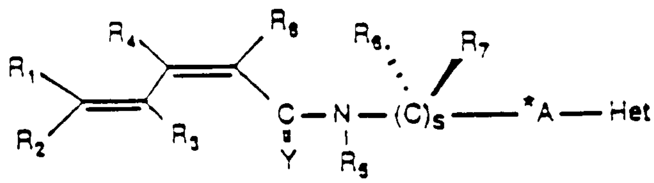

Id

Isomères [R-(Z)] à configuration (R).

**5.** Composés selon la revendication 1, de formule générale

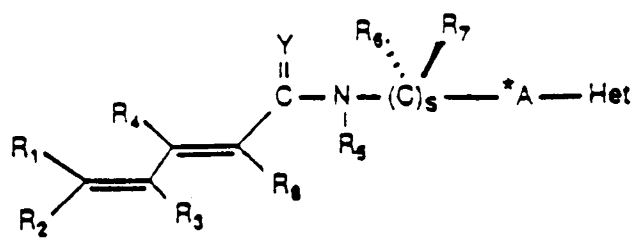

Ie

Isomères [R-(E)] à configuration (R)

**6.** Composés selon la revendication 1, de formule générale

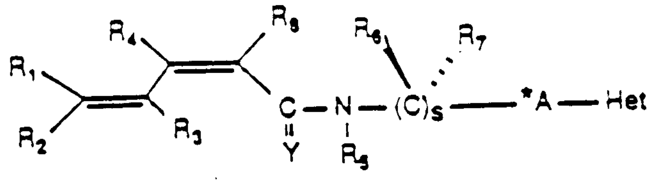

Iℓ

Isomères [S-(Z)] à configuration (S).

**7.** Composés selon la revendication 1, de formule générale

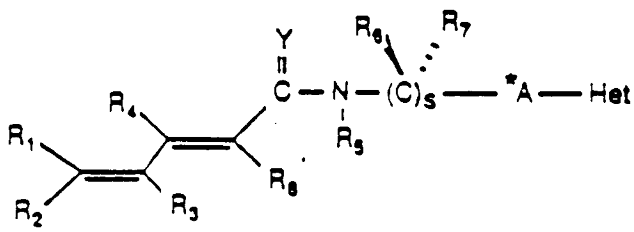

Ig

Isomères [S-(E)] à configuration (S)

**8.** Composés selon l'une des revendications 1-7, où $R_1$ et $R_2$ représentent indépendamment l'un de l'autre un alcoyle inférieur ou un aryle, $R_3$, $R_4$ et $R_8$ représentent indépendamment l'un de l'autre un hydrogène ou un alcoyle inférieur, $R_5$ et $R_7$ représentent un hydrogène, $R_6$ représente un hydrogène, un alcoyle inférieur ou un cycloalcoyle en $C_{3-6}$, *A représente *-$(CH_2)_n$-$(X)_m$-$(CH_2)_r$- n + r représente un nombre entier allant de 2 à 6, m le nombre 0, Het un groupe hétéroaromatique à 6 chaînons monocyclique qui contient un ou deux hétéroatomes choisis parmi l'azote, l'oxygène et le soufre, Y représente un oxygène ou un soufre, et s représente le nombre 1.

**9.** Composés selon la revendication 8, où $R_1$ représente un alcoyle inférieur ou un aryle, $R_2$ un aryle, $R_3$, $R_4$ et $R_8$ indépendamment l'un de l'autre un hydrogène ou un alcoyle inférieur, *A représente *-$(CH_2)_n$-$(X)_m$-$(CH_2)_r$-, n + r représente le nombre 3, m le nombre 0, Het un pyridinyle ou pyrimidinyle éventuellement substitué par un alcoyle inférieur, $R_5$ et $R_7$ chacun un hydrogène, $R_6$ un hydrogène, un alcoyle inférieur ou un cyclopropyle, Y un oxygène et s le nombre 1.

**10.** Composés selon la revendication 9, où $R_1$ représente un butyle, pentyle ou hexyle ou un phényle ayant jusqu'à trois substituants choisis parmi halogène et alcoxy inférieur, $R_2$ un phényle ayant jusqu'à 3 substituants choisis parmi halogène et alcoxy inférieur, $R_3$, $R_4$, $R_5$, $R_7$ et $R_8$ chacun un hydrogène et Het un 3-pyridinyle ou un 2-méthyl-3-pyridinyle.

**11.** [R-(E,E)]-5-(4-méthoxyphényl)-N-[1-méthyl-4-(3-pyridinyl)butyl]-2,4-décadiénamide.

**12.** [R-(E,E)]-5-(4-méthoxyphényl)-N-[1-méthyl-4-(3-pyridinyl)butyl]-2,4-nonadiénamide.

**13.** (E)-5,5-bis-(3-fluorophényl)-N-[4-3-pyridinyl)butyl]-2,4-pentadiénamide.

**14.** [R-(E)]-N-[1-éthyl]-4-(3-pyridinyl)butyl]-5,5-bis(4-méthoxyphényl)-2,4-pentadiénamide.

**15.** [R,S-(E,E)]-5-(4-méthoxyphényl)-N-[1-méthyl-4-(2-méthyl-3-pyridinyl)butyl]-2,4-décadiénamide.

**16.** (E)-5,5-bis-(3-méthoxyphényl)-N-[4-(3-pyridinyl)butyl]2,4-pentadiénamide.

**17.** Composés selon l'une des revendications 1 à 16, aux fins d'application aux substances thérapeutiquement actives.

**18.** Composés selon l'une quelconque des revendications 1 à 16, aux fins d'application comme antagonistes de PAF.

**19.** Procédé de préparation de composés selon l'une des revendications 1 à 16 et de leurs sels d'addition d'acides pharmaceutiquement acceptables, caractérisé en ce que

a) On fait réagir un dérivé réactif d'un acide carboxylique de formule générale

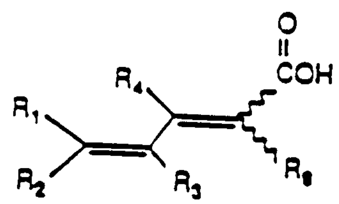

II

dans laquelle $R_1$, $R_2$, $R_3$, $R_4$ et $R_8$ ont la signification indiquée dans la revendication 1, avec un composé de formule générale

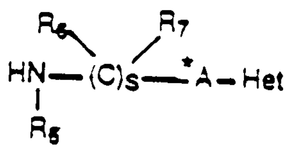

V

dans laquelle $R_5$, $R_6$, $R_7$, *A, Het et s ont la signification indiquée dans la revendication 1, ou

b) On fait réagir un acide carboxylique de formule générale II ci-dessus en présence d'une tri-(alcoyle inférieur)- ou triarylphosphine, avec un azide de formule générale

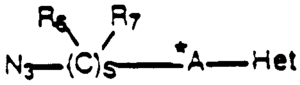

VIII

dans laquelle $R_6$, $R_7$, *A, Het, et s ont la signification indiquée dans la revendication 1, ou

c) On fait réagir un composé de formule générale I, dans laquelle Y représente un oxygène, avec du pentasulfure de phosphore pour donner un composé correspondant de formule générale I dans

laquelle Y représente un atome de soufre, et

d) si on le désire, on transforme un composé de formule générale I obtenu en un sel d'addition d'acides pharmaceutiquement acceptable.

**20.** Préparations pharmaceutiques contenant un composé selon l'une des revendications 1-16 et un support thérapeutiquement inerte.

**21.** Préparations pharmaceutiques à action antagoniste de PAF contenant un composé selon l'une des revendications 1 à 16 et un support thérapeutiquement inerte.

**22.** Application de composés selon l'une des revendications 1 à 16 à la préparation de préparations pharmaceutiques qui déploient une activité antagoniste de PAF.

**Revendications pour les Etats contractants suivants : ES, GR**

**1.** Procédé de préparation de composés de formule générale

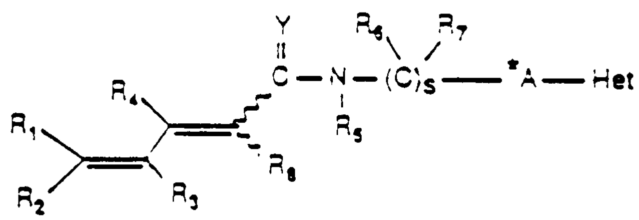

I

dans laquelle Y représente O ou S, *A un paraphénylène ou le groupe $*-(CH_2)_n-(X)_m-(CH_2)_r-$, X représente O, S ou -CH=CH-, n et r représentent indépendamment l'un de l'autre un nombre entier allant de 0 à 3, s le nombre 0 ou 1, m le nombre 0 ou 1, où n + s vaut au moins 2 lorsque m est le nombre 1, $R_1$ et $R_2$ représentent indépendamment l'un de l'autre un hydrogène, alcoyle inférieur, cycloalcoyle en $C_{3-6}$, alcényle inférieur, Het ou aryle, $R_3$, $R_4$ et $R_8$ représentent indépendamment l'un de l'autre un hydrogène, alcoyle inférieur ou aryle, $R_5$ et $R_7$ représentent indépendamment l'un de l'autre un hydrogène ou un alcoyle inférieur, $R_6$ représente un hydrogène, alcoyle inférieur, cycloalcoyle en $C_{3-6}$, Het-alcoyle inférieur ou aryle, Het représente un groupe hétéroaromatique monocyclique à six chaînons ou hétéroaromatique bicyclique du groupe de pyridinyle, quinolinyle, isoquinolinyle, indolyle, benzimidazolyle, et pyrimidinyle et qui est éventuellement substitué par un alcoyle inférieur, halogène ou un phényle, et l'astérisque représente le point de jonction et les radicaux décrits comme inférieurs possèdent jusqu'à sept atomes de carbone, et, dans la mesure où $R_6$ et $R_7$ sont différents, leurs énantiomères et mélanges racémiques, dans la mesure où $R_1$ et $R_2$ sont différents, leurs isomères géométriques et leurs sels d'addition d'acides pharmaceutiquement acceptables, caractérisé en ce que

a) On fait réagir un dérivé réactif d'un acide carboxylique de formule générale

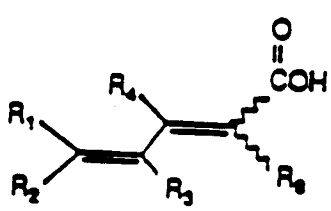

II

dans laquelle $R_1$, $R_2$, $R_3$, $R_4$ et $R_8$ ont la signification indiquée dans la revendication 1, avec un composé de formule générale

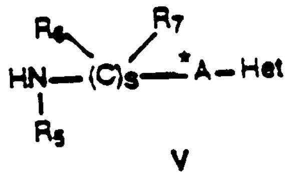

V

dans laquelle $R_5$, $R_6$, $R_7$, *A, Het et s ont la signification indiquée dans la revendication 1,
ou
b) On fait réagir un acide carboxylique de formule générale II ci-dessus en présence d'une tri-(alcoyle inférieur)- ou triarylphosphine, avec un azide de formule générale

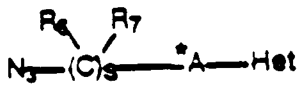

VIII

dans laquelle $R_6$, $R_7$, *A, Het, et s ont la signification indiquée dans la revendication 1, ou
c) On fait réagir un composé de formule générale I, dans laquelle Y représente un oxygène, avec du pentasulfure de phosphore pour donner un composé correspondant de formule générale I dans laquelle Y représente un atome de soufre, et
d) si on le désire, on transforme un composé de formule générale I obtenu en un sel d'addition d'acides pharmaceutiquement acceptable.

**2.** Procédé selon la revendication 1, caractérisé en ce qu'on prépare un composé de formule générale

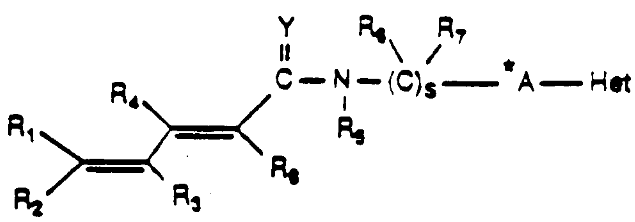

Ib

c'est-à-dire l'isomère (E) ou trans.

**3.** Procédé selon la revendication 1, caractérisé en ce qu'on prépare un composé de formule générale

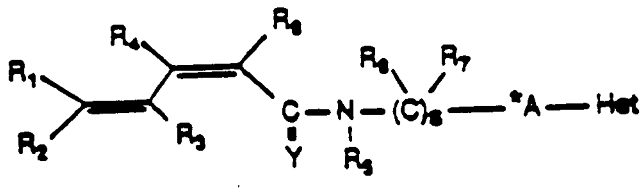

Ic

c'est-à-dire l'isomère (Z) ou cis.

**4.** Procédé selon la revendication 1, caractérisé en ce qu'on prépare un composé de formule générale

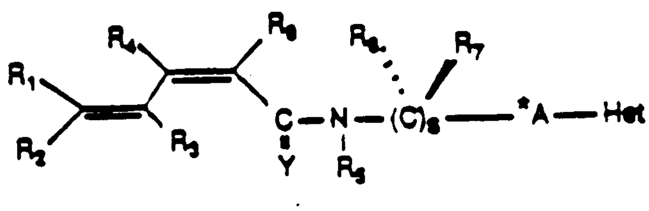

Id

c'est-à-dire l'isomère [R-(Z)] à configuration (R).

**5.** Procédé selon la revendication 1, caractérisé en ce qu'on prépare un composé de formule générale

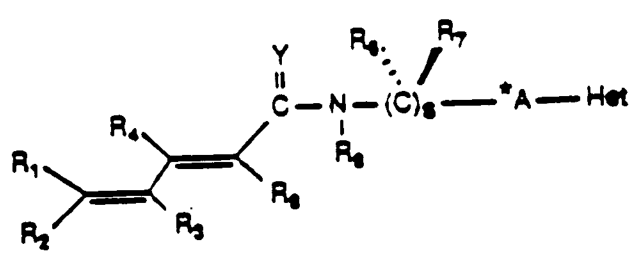

Ie

c'est-à-dire l'isomère [R-(E)] à configuration (R).

**6.** Procédé selon la revendication 1, caractérisé en ce qu'on prépare un composé de formule générale

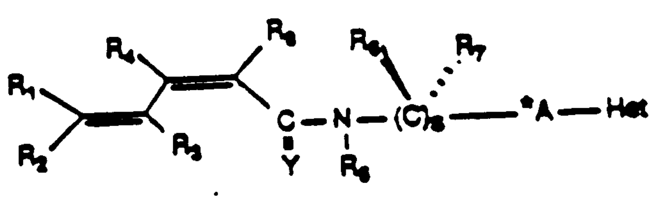

If

c'est-à-dire l'isomère [S-(Z)] à configuration (S).

**7.** Procédé selon la revendication 1, caractérisé en ce qu'on prépare un composé de formule générale

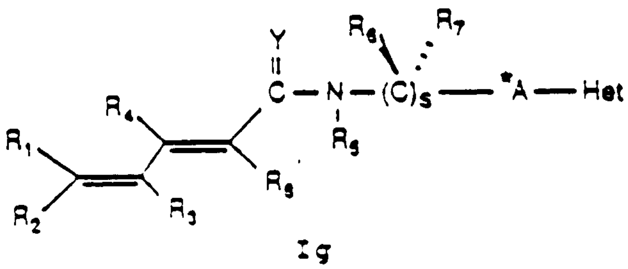

Ig

c'est-à-dire l'isomère [S-(E)] à configuration (S).

**8.** Procédé selon l'une des revendications 1-7, où $R_1$ et $R_2$ représentent indépendamment l'un de l'autre un alcoyle inférieur ou un aryle, $R_3$, $R_4$ et $R_8$ représentent indépendamment l'un de l'autre un hydrogène ou un alcoyle inférieur, $R_5$ et $R_7$ représentent un hydrogène, $R_6$ représente un hydrogène, un alcoyle inférieur ou un cycloalcoyle en $C_{3-6}$, *A représente $^*-(CH_2)_n-(X)_m-(CH_2)_r-$ n + r représente un nombre entier allant de 2 à 6, m le nombre 0, Het un groupe hétéroaromatique à 6 chaînons monocyclique qui contient un ou deux hétéroatomes choisis parmi l'azote, l'oxygène et le soufre, Y représente un oxygène ou un soufre, et s représente le nombre 1.

**9.** Procédé selon la revendication 8, où $R_1$ représente un alcoyle inférieur ou un aryle, $R_2$ un aryle, $R_3$, $R_4$ et $R_8$ indépendamment l'un de l'autre un hydrogène ou un alcoyle inférieur, *A représente $^*-(CH_2)_n-(X)_m-(CH_2)_r-$, n + r représente le nombre 3, m le nombre 0, Het un pyridinyle ou pyrimidinyle éventuellement substitué par un alcoyle inférieur, $R_5$ et $R_7$ chacun un hydrogène, $R_6$ un hydrogène, un alcoyle inférieur ou un cyclopropyle, Y un oxygène et s le nombre 1.

**10.** Procédé selon la revendication 9, où $R_1$ représente un butyle, pentyle ou hexyle ou un phényle ayant jusqu'à trois substituants choisis parmi halogène et alcoxy inférieur, $R_2$ un phényle ayant jusqu'à 3 substituants choisis parmi halogène et alcoxy inférieur, $R_3$, $R_4$, $R_5$, $R_7$ et $R_8$ chacun un hydrogène et Het un 3-pyridinyle ou un 2-méthyl-3-pyridinyle.

**11.** Procédé selon la revendication 1, caractérisé en ce qu'on prépare le [R-(E,E)]-5-(4-méthoxyphényl)-N-[1-méthyl-4-(3-pyridinyl)butyl]-2,4-décadiénamide.

**12.** Procédé selon la revendication 1, caractérisé en ce qu'on prépare le [R-(E,E)]-5-(4-méthoxyphényl)-N-[1-méthyl-4-(3-pyridinyl)butyl]-2,4-nonadiénamide.

**13.** Procédé selon la revendication 1, caractérisé en ce qu'on prépare le (E)-5,5-bis(3-fluorophényl)-N-[4-(3-pyridinyl)-butyl]-2,4-pentadiénamide.

**14.** Procédé selon la revendication 1, caractérisé en ce qu'on prépare le [R-(E)]N-51-éthyl-4-(3-pyridinyl)-butyl]-5,5èbis(4-méthoxyphényl)-2,4-pentadiénamide.

**15.** Procédé selon la revendication 1, caractérisé en ce qu'on prépare le [R,S-(E,E)]-5-(4-méthoxyphényl)-N-[1-méthyl-4-(2-méthyl-3-pyridinyl)butyl]-2,4-décadiénamide.

**16.** Procédé selon la revendication 1, caractérisé en ce qu'on prépare le (E)-5,5-bis(3-méthoxyphényl)-N-54-(3-pyridinyl)-butyl]-2,4-pentadiénamide.

**17.** Procédé de préparation de préparations pharmaceutiques, en particulier de préparations pour prévenir ou traiter les maladies qui se caractérisent par un accroissement du PAF, de maladies de la circulation, de maladies des poumons et de désordres immunologiques, d'inflammations, de désordres dermatologiques, de chocs ou de rejets de greffes, caractérisé en ce qu on met sous une forme d'administration

galénique un des composés de formule I définis dans la revendication 1 ou un de ses sels d'addition d'acides pharmaceutiquement acceptables et si on le désire une ou plusieurs autres substances thérapeutiquement actives avec un support thérapeutiquement inerte.

**18.** Application de composés de formule I définis dans la revendication 1 ou de leurs sels d'addition d'acides pharmaceutiquement acceptables à la préparation de préparations pharmaceutiques qui déploient des effets antagonistes de PAF.